(19) Europäisches Patentamt
European Patent Office
Office européen des brevets

(11) EP 1 447 401 A1

(12) EUROPEAN PATENT APPLICATION
published in accordance with Art. 158(3) EPC

(43) Date of publication:
**18.08.2004 Bulletin 2004/34**

(21) Application number: **02783591.7**

(22) Date of filing: **21.11.2002**

(51) Int Cl.$^7$: **C07D 211/76**, C07D 401/12, C07D 211/86, C07D 211/88, C07D 211/96, C07D 417/04, C07D 409/06, C07D 401/10, C07D 413/10, C07D 405/12, C07D 239/10, C07D 241/08, C07D 265/10, C07D 265/32, A61K 31/451, A61K 31/454, A61K 31/4545, A61K 31/496, A61K 31/5377, A61K 31/4535, A61K 31/4433

(86) International application number:
**PCT/JP2002/012174**

(87) International publication number:
**WO 2003/043988 (30.05.2003 Gazette 2003/22)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **22.11.2001 JP 2001357348**

(71) Applicant: **ONO PHARMACEUTICAL CO., LTD.
Osaka-shi, Osaka 541-8526 (JP)**

(72) Inventors:
- **TAKAHASHI, K., Minase Res. Ins. Ono Pharm. Co. Ltd Mishima-gun, Osaka 618-8585 (JP)**
- **YAMAMOTO, S., Minase Res. Ins. Ono Pharm. Co. Ltd Mishima-gun, Osaka 618-8585 (JP)**
- **NAKA, M., Minase Res. Ins. Ono Pharm. Co. Ltd Mishima-gun, Osaka 618-8585 (JP)**

(74) Representative: **Teipel, Stephan, Dr. et al
Lederer & Keller
Patentanwälte
Prinzregentenstrasse 16
80538 München (DE)**

(54) **PIPERIDIN-2-ONE DERIVATIVE COMPOUNDS AND DRUGS CONTAINING THESE COMPOUNDS AS THE ACTIVE INGREDIENT**

(57) A piperidin-2-one derivative compound represented by formula (I):



Printed by Jouve, 75001 PARIS (FR)

(Cont. next page)

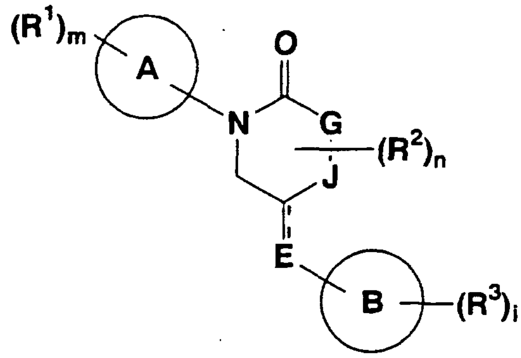

(I)

wherein all symbols are described in the specification, or a non-toxic salt thereof.

The compound represented by formula (I) inhibits activation of p38MAP kinase, and is useful for prevention and/or treatment of various inflammatory diseases, rheumatoid arthritis, osteoarthritis, arthritis, osteoporosis, autoimmune diseases, infectious diseases, sepsis, cachexia, cerebral infarction, Alzheimer's disease, asthma, chronic pulmonary inflammatory diseases, reperfusion injury, thrombosis, glomerulonephritis, diabetes, graft versus host rejection, inflammatory bowel disease, Crohn's disease, ulcerative colitis, multiple sclerosis, tumor growth and metastasis, multiple myeloma, plasma cell leukemia, Castleman's disease, atrial myxoma, psoriasis, dermatitis, gout, adult respiratory distress syndrome (ARDS), arteriosclerosis, post-percutaneous transluminal coronary angioplasty (PTCA) restenosis or pancreatitis.

FIGURE 1

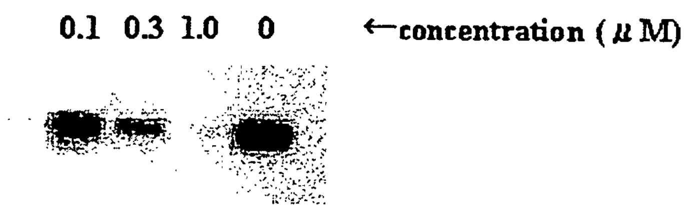

## Description

### TECHNICAL FIELD

The present invention relates to piperidin-2-one derivatives.

Particularly, the present invention relates to

(1) a piperidin-2-one derivatives represented by formula (I)

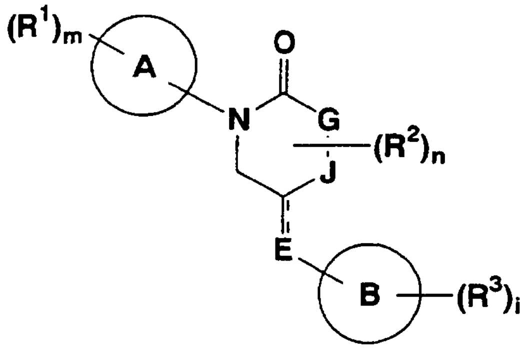

(I)

(wherein all symbols have tha same meanings as described below)
or a non-toxic salt thereof,
(2) a method for preparation of the same, and
(3) a pharmaceutical composition comprising the same as an active ingredient.

### BACKGROUND ART

p38 mitogen-activated protein (MAP) kinase (p38α / Mpk2 / RK / SAPK2a / CSBP) (hereinafter referred to as "p38MAP kinase") was cloned as an enzyme which induces tyrosine phosphorylation in monocyte after stimulation with lipopolysaccharide (LPS) (Nature, 372, 739 (1994)), and is activated by various extracellular stimuli (physical stimuli: osmotic shock, heat shock, UV irradiation, chemical stimuli: endotoxin, hydrogen peroxide, arsenic trioxide, an inflammatory cytokine and a growth factor). Also, since p38MAP kinase relates to the production of an inflammatory cytokine and chemokine such as tumor necrosis factor-α (TNF-α), interleukin 1 (IL-1), IL-6 or IL-8, an association between the activation of this enzyme and diseases is strongly suggested. Therefore, an improvement effect on various disease symptoms represented by inflammatory diseases is expected by the suppression of p38MAP kinase activation.

Accordingly, a p38MAP kinase inhibitor is expected to be useful in the prevention and/or the treatment of diseases whose cause or deterioration is thought to be related to the abnormal production of an inflammatory cytokine or chemokine or over response to them, such as various inflammatory diseases, rheumatoid arthritis, osteoarthritis, arthritis, osteoporosis, autoimmune diseases, infectious diseases, sepsis, cachexia, cerebral infarction, Alzheimer's disease, asthma, chronic pulmonary inflammatory diseases, reperfusion injury, thrombosis, glomerulonephritis, diabetes, graft versus host rejection, inflammatory bowel disease, Crohn's disease, ulcerative colitis, multiple sclerosis, tumor growth and metastasis, multiple myeloma, plasma cell leukemia, Castleman's disease, atrial myxoma, psoriasis, dermatitis, gout, adult respiratory distress syndrome (ARDS), arteriosclerosis, post-percutaneous transluminal coronary angioplasty (PTCA) restenosis and pancreatitis.

As p38MAP kinase inhibitors, for example, WO99/64400 discloses that compounds shown by formulae (A) to (G) are useful for p38MAP kinase inhibitors:

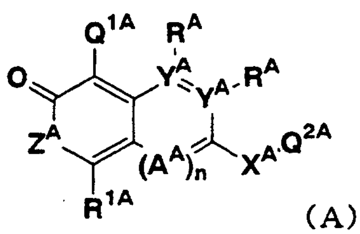
(A)
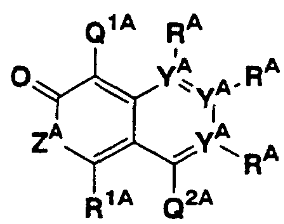
(B)
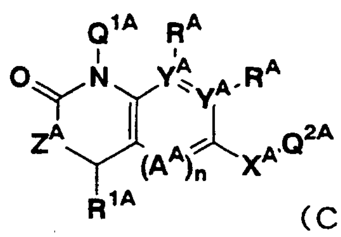
(C)

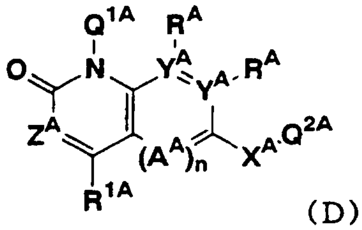
(D)
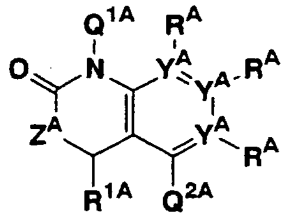
(E)
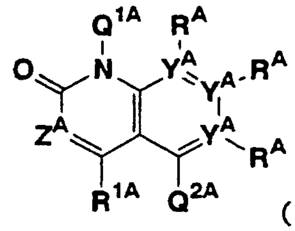
(F)

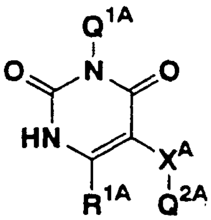
(G)

wherein $Q^{1A}$ and $Q^{2A}$ each independently is selected from a 5- to 6-membered aromatic carbocyclic ring, a heterocyclic ring and the like;

$Q^{1A}$ each independently is substituted with 1 to 4 substituent(s) selected from a halogen atom, $C_1$-$C_3$ alkyl optionally substituted with $NR'_2$, OR', $CO_2R'$ or $CONR'_2$, and the like;

$Q^{2A}$ each independently is substituted with at most 4 substituent(s) selected from halogen or $C_1$-$C_3$ straight-chain or branched alkyl optionally substituted with $NR'_2$, OR', $CO_2R'$, $S(O_2)N(R')_2$, $N{=}C{-}N(R')_2$, $R^3$ or $CONR'_2$;

$X^A$, when present, is selected from -S-, -O-, $-N(R^2)-$, and the like;

$R^{1A}$ is selected from a hydrogen atom, ($C_1$-$C_3$) alkyl, OH or O-($C_1$-$C_3$) alkyl;

wherein necessary ones of the symbols in the groups are extracted.

Also, DE10002509 discloses that compounds represented by formula by formula (H) are useful as IL-12 inhibitors:

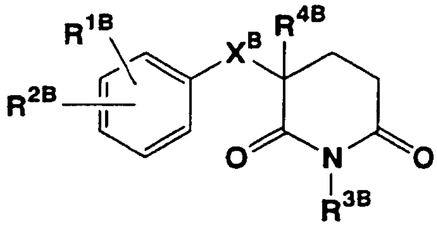
(H)

wherein $R^{1B}$ and $R^{2B}$ each represents a hydrogen atom, a chlorine atom, a fluorine atom, OH, or the like;

$R^{3B}$ represents a hydrogen atom, OH, or $CH_2NR^{6B}R^{7B}$;

$R^{6B}$ and $R^{7B}$, taken together, form pyrrolidine, piperidine, morpholine, or the like;

$R^{4B}$ represents a hydrogen atom, C1-3 alkyl, a fluorine atom, trifluoromethyl, or difluoromethyl.

$X^B$ represents $(CH_2)_n$-$NR^{8B}$, $(CH_2)_n$-S, $(CH_2)_q$, or the like,

wherein necessary ones of the symbols in the groups are extracted.

## DISCLOSURE OF THE INVENTION

As a result of the present inventors intensively studied to find compounds which are useful as agents for treatment of diseases by suppressing the activity of p38MAP as the subject, they found that the object was accomplished by novel piperidin-2-one derivatives represented by the following formula (I), and thus the present invention has been completed.

That is, the present invention relates to

[1] a piperidin-2-one derivative compound represented by formula (I):

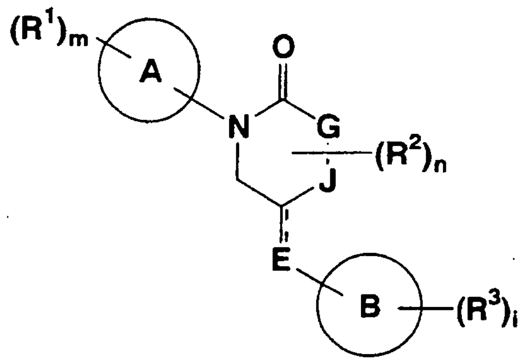

(I)

wherein ring A represents a C5-10 mono- or bi- carbocyclic ring, or a 5-to 10-membered mono- or bi- heterocyclic ring having 1 to 5 nitrogen atom(s), 1 to 2 oxygen atom(s) and/or a sulfur atom;

$R^1$ represents

(1) C1-8 alkyl,
(2) C2-8 alkenyl,
(3) C2-8 alkynyl,
(4) a halogen atom,
(5) $-OR^4$,
(6) $-NR^5R^6$,
(7) $-NR^7COR^8$,
(8) $-CONR^9R^{10}$,
(9) $-COOR^{11}$,
(10) $-SO_2NR^{12}R^{13}$,
(11) $-NR^{14}SO_2R^{15}$,
(12) $-SR^{16}$,
(13) $-S(O)R^{17}$,
(14) $-SO_2R^{18}$,
(15) $-NR_{22}COOR^{23}$,
(16) $-NR^{24}CONR^{25}R^{26}$,
(17) $-COR^{27}$,
(18) $-NO_2$,
(19) -CN,
(20) $-CF_3$,
(21) $-OCF_3$,
(22) Cyc1, or
(23) C1-8 alkyl, C2-8 alkenyl or C2-8 alkynyl substituted with 1 to 5 substituent(s) selected from the group consisting of (a) a halogen atom, (b) $-OR^4$, (c) $-NR^5R^6$, (d) $-NR^7COR^8$, (e) $-CONR^9R^{10}$, (f) $-COOR^{11}$, (g) $-SO_2NR^{12}R^{13}$, (h) $-NR^{14}SO_2R^{15}$, (i) $-SR^{16}$, (j) $-S(O)R^{17}$, (k) $-SO_2R^{18}$, (l) $-NR^{22}COOR^{23}$, (m) $-NR^{24}CONR^{25}R^{26}$, (n) $-COR^{27}$, (o) $-NO_2$, (p) -CN, (q) $-CF_3$, (r) $-OCF_3$, and (s) Cyc1;

$R^4$-$R^{18}$ or $R^{22}$-$R^{27}$ each independently represents

(1) a hydrogen atom,
(2) C1-8 alkyl,
(3) Cyc1, or
(4) C1-8 alkyl substituted with 1 to 5 substituent(s) selected from the group consisting of (a) $-OR^{28}$, (b) $-NR^{29}R^{30}$, (c) $-NR^{31}COR^{32}$, (d) $-CONR^{33}R^{34}$, (e) $-COOR^{35}$, (f) $-SO_2NR^{36}R^{37}$, (g) $-NR^{38}SO_2R^{39}$, (h) $-CONR^{40}NR^{41}R^{42}$, (i) $-CONR^{43}OR^{44}$, and (j) Cyc1,

$R^{28}$-$R^{44}$ each independently represents

(1) a hydrogen atom,
(2) C1-8 alkyl,
(3) Cyc1, or
(4) C1-8 alkyl substituted with 1 to 5 substituent(s) selected from the group consisting of $-OR^{45}$, $-NR^{46}R^{47}$, and Cyc1;

$R^{45}$-$R^{47}$ each independently represents a hydrogen atom, C1-8 alkyl, Cyc1, or C1-8 alkyl substituted with Cyc1;

Cyc1 represents a C5-10 mono- or bi- carbocyclic ring or a 5- to 10-membered mono- or bi- heterocyclic ring having 1 to 5 nitrogen atom(s), 1 to 2 oxygen atom(s) and/or a sulfur atom, in which the carbocyclic ring or the heterocyclic ring may be substituted with 1 to 5 of $R^{48}$;

$R^{48}$ represents

(1) C1-8 alkyl,
(2) a halogen atom,
(3) $-NO_2$,
(4) -CN,
(5) $-OR^{49}$,
(6) $-NR^{50}R^{51}$
(7) $-COOR^{52}$,
(8) $-COR^{53}$,
(9) $-CONR^{54}R^{55}$,
(10) $-NR^{56}COR^{57}$,
(11) $-SO_2NR^{58}R^{59}$,
(12) $-NR^{60}SO_2R^{61}$,
(13) $-SR^{62}$,
(14) $-SO_2R^{63}$,
(15) oxo,
(16) $-CF_3$,
(17) $-OCF_3$, or
(18) C1-8 alkyl substituted with 1 to 5 substituent(s) selected from the group consisting of (a) a halogen atom, (b) $-NO_2$, (c) -CN, (d) $-OR^{49}$, (e) $-NR^{50}R^{51}$, (f) $-COOR^{52}$, (g) $-COR^{53}$, (h) $-CONR^{54}R^{55}$, (i) $-NR^{56}COR^{57}$, (j) $-SO_2NR^{58}R^{59}$, (k) $-NR^{60}SO_2R^{61}$, (l) $-SR^{62}$, (m) $-SO_2R^{63}$, (n) oxo, (o) $-CF_3$, and (p) $-OCF_3$;

$R^{49}$-$R^{63}$ each independently represents, a hydrogen atom, C1-8 alkyl, phenyl, or C1-8 alkyl substituted with phenyl;

$R^2$ represents

(1) C1-8 alkyl,
(2) $-OR^{20}$,
(3) $-NR^{64}R^{65}$,
(4) $-COOR^{66}$,
(5) $-CONR^{67}R^{68}$,
(6) $-NR^{69}COR^{70}$,
(7) $-SO_2R^{71}$,
(8) $-SO_2NR^{72}R^{73}$,
(9) $-NR^{74}SO_2R^{75}$,
(10) $-NR^{76}COOR^{77}$,
(11) Cyc2, or
(12) C1-8 alkyl, C2-8 alkenyl or C2-8 alkynyl substituted with 1 to 5 substituent(s) selected from the group consisting of (a) $-OR^{20}$, (b) $-NR^{64}R^{65}$, (c) $-COOR^{66}$, (d) $-CONR^{67}R^{68}$, (e) $-NR^{69}COR^{70}$, (f) $-SO_2R^{71}$, (g) $-SO_2NR^{72}R^{73}$, (h) $-NR^{74}SO_2R^{75}$, (i) $-NR^{76}COOR^{77}$, and (j) Cyc2;

$R^{20}$ and $R^{64}$-$R^{77}$ each independently represents (1) a hydrogen atom, (2) C1-8 alkyl, (3) Cyc2 or (4) C1-8 alkyl substituted with Cyc2;

Cyc2 represents a C5-6 monocarbocyclic ring, or a 5- to 6-membered monoheterocyclic ring having 1 to 4 nitrogen atom(s), 1 to 2 oxygen atom(s) and/or a sulfur atom, in which the carbocyclic ring or the heterocyclic ring may be substituted with 1 to 5 substituent(s) selected from the group consisting of C1-8 alkyl, C1-8 alkoxy, a

halogen atom, $-CF_3$ and $OCF_3$;

G or J each independently represents, a carbon atom, a nitrogen atom, an oxygen atom, or a sulfur atom;

E represents C1-4 alkylene, C2-4 alkenylene; -O-, -S-, $-NR^{21}-$,

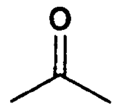

,

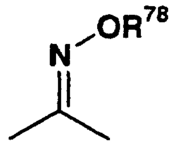

,

$-NR^{79}SO_2-$ or $-SO_2NR^{80}-$, in which the C1-4 alkylene may be substituted with 1 to 5 of C1-8 alkyl, C1-8 alkoxy, a halogen atom, or hydroxyl;

$R^{21}$ and $R^{78}$-$R^{80}$ each independently represents (1) a hydrogen atom, (2) C1-8 alkyl, (3) Cyc3, or (4) C1-8 alkyl substituted with 1 to 5 of Cyc3 or hydroxyl;

Cyc3 represents a C5-6 monocarbocyclic ring, or a 5- to 6-membered monoheterocyclic ring having 1 to 4 nitrogen atom(s), 1 to 2 oxygen atom(s) and/or a sulfur atom, in which the carbocyclic ring or the heterocyclic ring may be substituted with 1 to 5 of C1-8 alkyl, C1-8 alkoxy, a halogen atom, $-CF_3$ or $OCF_3$;

Ring B represents a C5-10 mono- or bi- carbocyclic ring, or a 5-to 10-membered mono- or bi- heterocyclic ring having 1 to 4 nitrogen atom(s), 1 to 2 oxygen atom(s) and/or a sulfur atom;

$R^3$ represents

(1) C1-8 alkyl,
(2) C2-8 alkenyl,
(3) C2-8 alkynyl,
(4) a halogen atom,
(5) $-OR^{81}$,
(6) $-NR^{82}R^{83}$,
(7) $-NR^{84}COR^{85}$,
(8) $-CONR^{86}R^{87}$,
(9) $-COOR^{88}$,
(10) $-SO_2NR^{89}R^{90}$,
(11) $-NR^{91}SO_2R^{92}$,
(12) $-SR^{93}$,
(13) $-S(O)_R{}^{94}$,
(14) $-SO_2R^{95}$,
(15) $-NR^{96}COOR^{97}$,
(16) $-NR^{98}CONR^{99}R^{100}$,
(17) $-OCONR^{101}R^{102}$,
(18) $-NO_2$,
(19) -CN,
(20) $-CF_3$,
(21) $-OCF_3$,
(22) Cyc4, or
(23) C1-8 alkyl, C2-8 alkenyl or C2-8 alkynyl substituted with 1 to 5 substituent(s) selected from the group consisting of (a) a halogen atom, (b) $-OR^{81}$, (c) $-NR^{82}R^{83}$, (d) $-NR^{84}COR^{85}$, (e) $-CONR^{86}R^{87}$, (f) $-COOR^{88}$, (g) $-SO_2NR^{89}R^{90}$, (h) $-NR^{91}SO_2R^{92}$, (i) $-SR^{93}$, (j) $-S(O)R^{94}$, (k) $-SO_2R^{95}$, (l) $-NR^{96}COOR^{97}$, (m) $-NR^{98}CONR^{99}R^{100}$, (n) $-OCONR^{101}R^{102}$, (o) $-NO_2$, (p) -CN, (q) $-CF_3$, (r) $-OCF_3$, and (s) Cyc4;

$R^{81}$-$R^{102}$ each independently represents (1) a hydrogen atom, (2) C1-8 alkyl, (3) Cyc4 or (4) C1-8 alkyl substituted with 1 to 5 substituent(s) selected from the group consisting of Cyc4, $-OR^{103}$, $-CONR^{104}R^{105}$, and $-COOR^{106}$;

$R^{103}$-$R^{106}$ each independently represents, (1) a hydrogen atom, (2) C1-8 alkyl, (3) Cyc4 or (4) C1-8 alkyl substituted with 1 to 5 substituent(s) selected from Cyc4 and $-OR^{107}$;

$R^{107}$ represents (1) a hydrogen atom, (2) C1-8 alkyl, (3) Cyc4 or (4) C1-8 alkyl substituted with Cyc4;

Cyc4 is a C5-10 mono- or bi- carbocyclic ring, or a 5- to 10-membered mono- or bi- heterocyclic ring having 1 to 5 nitrogen atom(s), 1 to 2 oxygen atom(s) and/or a sulfur atom, in which the carbocyclic ring or the heterocyclic ring may be substituted with 1 to 5 of C1-8 alkyl, C1-8 alkoxy, a halogen atom, $-CF_3$ or $-OCF_3$;

m represents 0 or an integer of 1 to 5;

n represents 0 or an integer of 1 to 7;

i represents 0 or an integer of 1 to 12,

wherein

(1) when m is 2 or more, $R^1$ is the same or different,
(2) when n is 2 or more, $R^2$ is the same or different,
(3) when i is 2 or more, $R^3$ is the same or different,
(4) when E is C1-4 alkylene, $\overline{----}$ is a single bond or a double bond,
(5) when E is other than C1-4 alkylene, $\overline{----}$ is a single bond,

or a non-toxic salt thereof,

[2] a process for producing the same, and

[3] a pharmaceutical composition comprising the same as an active ingredient.

In the present invention, C1-8 alkyl means methyl, ethyl, propyl, butyl, pentyl, hexyl, heptyl, octyl or isomeric groups thereof.

C2-8 alkenyl means ethenyl, propenyl, butenyl, butadienyl, pentenyl, pentadienyl, hexenyl, hexadienyl, heptenyl, heptadienyl, octenyl, octadienyl or isomeric groups thereof.

C2-8 alkynyl means ethynyl, propynyl, butynyl, butadiynyl, pentynyl, pentadiynyl, hexynyl, hexadiynyl, heptynyl, heptadiynyl, octynyl, octadiynyl or isomeric groups thereof.

C1-8 alkoxy means methoxy, ethoxy, propoxy, butoxy, pentyloxy, hexyloxy, heptyloxy, octyloxy or isomeric groups thereof.

C1-4 alkylene means methylene, ethylene, trimethylene, tetramethylene or isomeric groups thereof.

C2-4 alkenylene means ethenylene, propenylene, butenylene or isomeric groups thereof.

Halogen atom means chlorine, bromine, fluorine and iodine atom.

C5-10 mono- or bi- carbocyclic ring means, for example, cyclopentane, cyclohexane, cycloheptane, cyclooctane, cyclononane, cyclodecane, cyclopentene, cyclohexene, cycloheptene, cyclooctene, cyclopentadiene, cyclohexadiene, cycloheptadiene, cyclooctadiene, benzene, pentalene, perhydropentalene, azulene, perhydroazulene, indene, perhydroindene, indan, naphthalene, dihydronaphthalene, tetrahydronaphthalene, perhydronaphthalene, *etc.*

C5-6 monocarbocyclic ring means, for example, cyclopentane, cyclohexane, cyclopentene, cyclohexene, cyclopentadiene, cyclohexadiene, benzene, *etc.*

5- to 10-membered monoheterocyclic ring containing 1-5 nitrogen atom(s), 1-2 oxygen atom(s) and/or 1 sulfur atom means 5- to 10-membered monocyclic heteroaryl containing 1-5 nitrogen atom(s), 1-2 oxygen atom(s) and/or 1 sulfur atom, and partially or fully saturated one. It includes, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, *etc.*

5- to 10-membered biheterocyclic ring containing 1-5 nitrogen atom(s), 1-2 oxygen atom(s) and/or 1 sulfur atom means 5- to 10-membered bicyclic heteroaryl containing 1-5 nitrogen atom(s), 1-2 oxygen atom(s) and/or 1 sulfur atom, and partially or fully saturated one. It includes, for example, indole, isoindole, indolizine, benzofuran, isobenzofuran, benzothiophene, isobenzothiophene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzofurazan, benzothiadiazole, benzotriazole, benzofurazan, benzothiadiazole, benzotriazole, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dioxaindan, benzodioxane, chroman, *etc*.

5- to 6-membered monoheterocyclic ring containing 1-4 nitrogen atom(s), 1-2 oxygen atom(s) and/or 1 sulfur atom means 5- to 6-membered monocyclic heteroaryl containing 1-4 nitrogen atom(s), 1-2 oxygen atom(s) and/or 1 sulfur atom, and partially or fully saturated one. It includes, for example, pyrrole, imidazole, triazole, tetrazole, pyrazole, pyridine, pyrazine, pyrimidine, pyridazine, furan, pyran, thiophene, thiopyran, oxazole, isoxazole, thiazole, isothiazole, furazan, oxadiazole, oxazine, oxadiazine, thiadiazole, thiazine, thiadiazine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine, dihydrothiadiazine, tetrahydrothiadiazine, morpholine, thiomorpholine, oxathiane, dioxolane, dioxane, *etc*.

In the present invention, all isomers are included unless otherwise specified. For example, alkyl, alkoxy and alkylene group includes straight or branched ones. In addition, isomers on double bond, ring, fused ring (E-, Z-, cis-, trans-isomer), isomers generated from asymmetric carbon atom(s) (R-, S-, α-, β-isomer, enantiomer, diastereomer), optically active isomers (D-, L-, d-, I-isomer), polar compounds generated by chromatographic separation (more polar compound, less polar compound), equilibrium compounds, mixtures thereof at voluntary ratios and racemic mixtures are also included in the present invention.

In the present invention, unless otherwise indicated and as is apparent for those skilled in the art, symbol 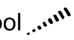 indicates that it is bound to the opposite side of the sheet (namely α-configuration), symbol 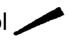 indicates that it is bound to the front side of the sheet (namely β-configuration), symbol 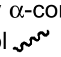 indicates that it is α-, β- or a mixture thereof, and symbol 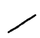 indicates that it is a mixture of α-configuration and β-configuration.

[SALT]

In the present invention, non-toxic salts include all pharmaceutically acceptable salts. It includes, for example, general salts, acid addition salts, *etc*.

The compounds represented by formula (I) of the present invention may be converted into the corresponding salts by conventional means. Non-toxic and water-soluble salts are preferred. Suitable salts, for example, include; salts of alkali metals (e.g. potassium, sodium, *etc.*), salts of alkaline earth metals (e.g. calcium, magnesium, *etc.*), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris (hydroxymethyl)amine, lysine, arginine, N-methyl-D-glucamine, *etc.*),*etc.*

The compounds represented by formula (I) of the present invention may be converted into the corresponding acid addition salts by conventional means. Non-toxic and water-soluble salts are preferred. Suitable acid addition salts, for example, include ; salts of inorganic acids e.g. hydrochloride, hydrobromide, sulfate, phosphate, nitrate, *etc.;* salts of organic acids e.g. acetate, trifluoroacetate, lactate, tartrate, oxalate, fumarate, maleate, citrate, benzoate, methanesulfonate, ethanesulfonate, benzenesulfonate, toluenesulfonate, isethionate, glucuronate, gluconate, *etc.*

The compounds represented by formula (I) of the present invention or the salts thereof may be converted into the corresponding solvates by conventional means.

Non-toxic and water-soluble solvates are preferred. Suitable solvates include, for example, hydrates, solvates

of the alcohols (e.g. ethanol *etc.), etc.*

In the present invention, all groups represented by $R^1$, $R^2$ and $R^3$ are preferred.

In formula (1), $R^1$ is preferably C1-8 alkyl, a halogen atom, $-NR^5R^6$, $-NR^7COR^8$, $-COOR^{11}$, $-SO_2NR^{12}R^{13}$, $-NR^{14}SO_2R^{15}$ or C1-4 alkyl substituted with $OR^4$, more preferably C1-4 alkyl, a halogen atom, $-NR^5R^6$ or $-NR^7COR^8$, and most preferably methyl, ethyl, a fluorine atom or a chlorine atom.

In formula (I), $R^2$ is preferably C1-8 alkyl, $-OR^{20}$, $-COOR^{66}$ or C1-4 alkyl substituted with Cyc2, more preferably C1-4 alkyl, $-OR^{20}$ or $-COOR^{66}$, and most preferably methyl, ethyl, hydroxyl, methoxy, -COOH or $-COOCH_3$.

In formula (I), $R^3$ is preferably C1-8 alkyl, a halogen atom, $OR^{81}$, $-NR^{98}CONR^{99}R^{100}$, $-OCONR^{101}R^{102}$, C1-8 alkyl substituted with $-NR^{98}CONR^{99}R^{100}$, or C1-8 alkyl substituted with $-OCONR^{101}R^{102}$, more preferably C1-4 alkyl, a halogen atom, C1-4 alkyl substituted with $-NR^{98}$ $CONR^{99}R^{100}$, or C1-4 alkyl substituted with $-OCONR^{101}R^{102}$, and most preferably methyl, a fluorine atom, a chorine atom, $-CH_2-NR^{98}CONR^{99}R^{100}$ or $-CH_2-OCONR^{101}R^{102}$.

In formula (I), the ring A is preferably a C5-10 mono- or bi- carbocyclic ring or a mono- or bi- heterocyclic ring having 1 to 2 nitrogen atom(s), 1 to 2 oxygen atom(s) and/or a sulfur atom, more preferably a C5-6 monocarbocyclic ring, or a 5- to 6-membered monocyclic heteroaryl having 1 to 2 nitrogen atom(s), an oxygen atom and/or a sulfur atom, and most preferably a benzene ring, a pyridine ring, a thiophene ring, or a cyclohexane ring.

In formula (I), the ring B is preferably a C5-10 mono- or bi- carbocyclic ring, and most preferably a benzene ring or a naphthalene ring.

In formula (I), G is preferably a carbon atom, a nitrogen atom, an oxygen atom, or a sulfur atom.

In formula (I), J is preferably a carbon atom, an oxygen atom, or a sulfur atom.

In formula (I), E is preferably C1-4 alkylene, C1-4 alkylene substituted with hydroxyl, C1-4 alkylene substituted with C1-4 alkoxy, -S- or

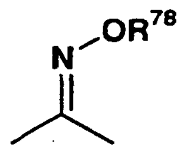

,

and most preferably methylene, hydroxymethylene, methoxymethylene, or hydroxyiminomethylene.

In formula (I), m is preferably 0 or an integer of 1 to 3.

In formula (I), n is preferably 0 or an integer of 1 to 3.

In formula (I), i is preferably 0 or an integer of 1 to 3.

In the present invention, when m is 2 or more, n is 2 or more, and i is 2 or more, each of $R^1$, each of $R^2$ and each of $R^3$, respectively, is the same or different.

In formula (1), Cyc1 is preferably a C5-6 monocarbocyclic ring, or a 5- to 6-membered monoheterocyclic ring having 1 to 5 nitrogen atom(s), 1 to 2 oxygen atom(s) and/or a sulfur atom; more preferably C5-6 monocarbocyclic ring, and most preferably a benzene ring.

In formula (I), Cyc2 is preferably a C5-6 monocarbocyclic ring, or a 5-6 membered monocyclic hetero ring having 1 to 4 nitrogen atom(s), 1 to 2 oxygen atom(s) and/or a sulfur atom, more preferably a C5-6 monocarbocyclic ring, and most preferably a benzene ring.

In formula (I), Cyc3 is preferably a C5-6 monocarbocyclic ring, or a 5- to 6-membered monoheterocyclic ring having 1 to 4 nitrogen atom(s), 1 to 2 oxygen atom(s) and/or a sulfur atom, and most preferably a benzene ring.

Among the compounds represented by formula (I), preferred compounds includes:

a compound represented by formula (I-A-1):

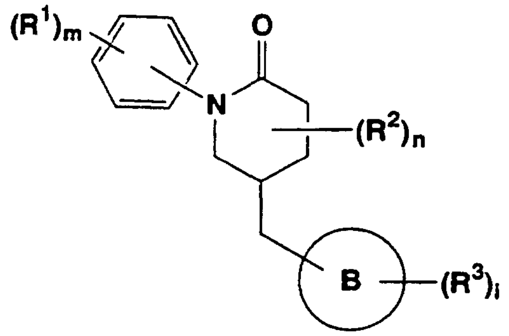

(I-A-1)

(wherein all symbols in the formula have the same meanings as defined above), a compound represented by formula (I-A-2):

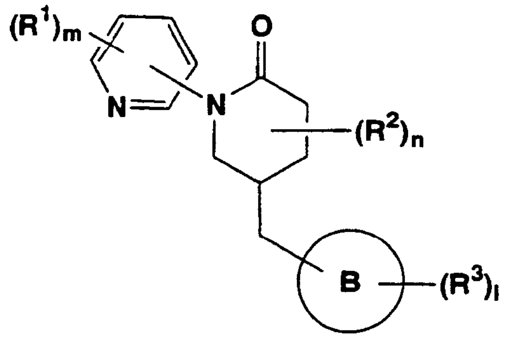

(I-A-2)

(wherein all symbols in the formula have the same meanings as defined above), a compound represented by formula (I-A-3):

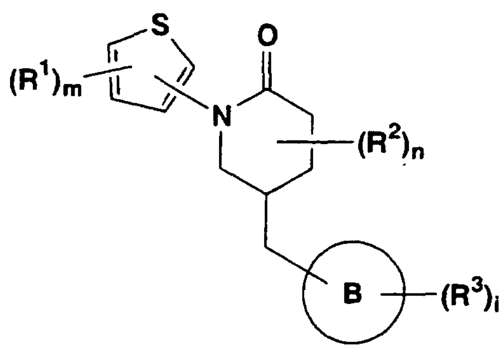

(I-A-3)

(wherein all symbols in the formula have the same meanings as defined above), a compound represented by formula (I-A-4):

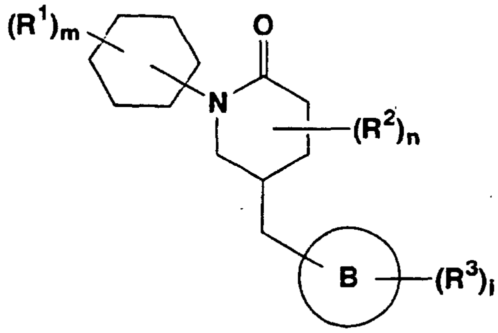

(I -A-4)

(wherein all symbols in the formula have the same meanings as defined above), a compound represented by formula (I-B-1):

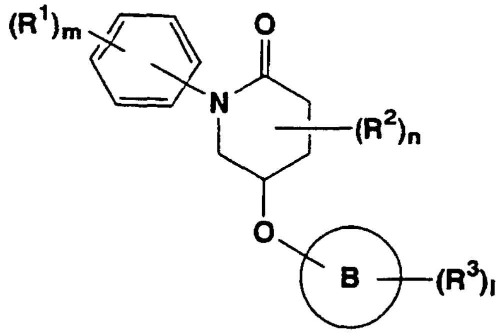

(I -B-1)

(wherein all symbols in the formula have the same meanings as defined above), a compound represented by formula (I-B-2):

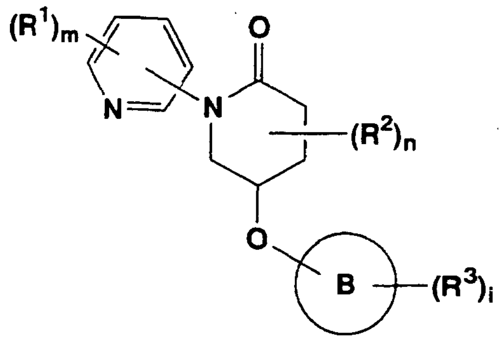

(I -B-2)

(wherein all symbols in the formula have the same meanings as defined above), a compound represented by formula (I-B-3):

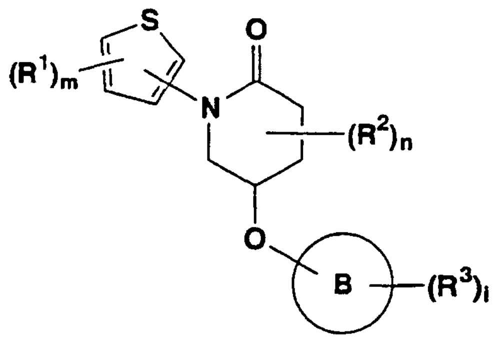

( I -B-3)

(wherein all symbols in the formula have the same meanings as defined above), a compound represented by formula (I-B-4):

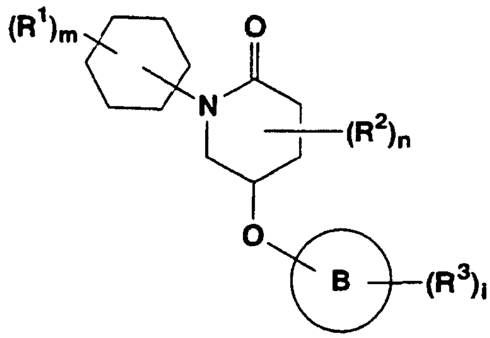

( I -B-4)

(wherein all symbols in the formula have the same meanings as defined above), a compound represented by formula (I-C-1):

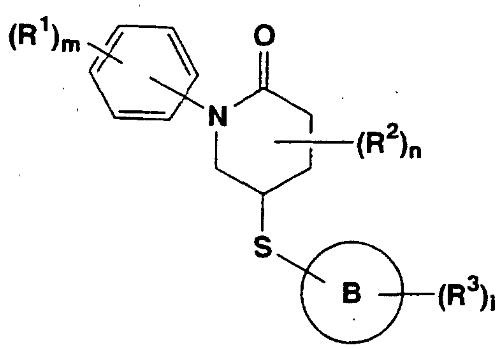

( I -C-1)

(wherein all symbols in the formula have the same meanings as defined above), a compound represented by formula (I-C-2):

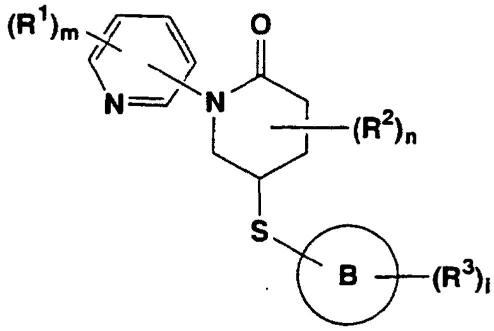

( I -C-2)

(wherein all symbols in the formula have the same meanings as defined above), a compound represented by formula (I-C-3):

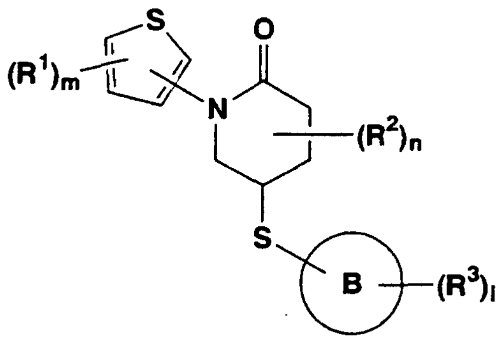

( I -C-3)

(wherein all symbols in the formula have the same meanings as defined above), a compound represented by formula (I-C-4):

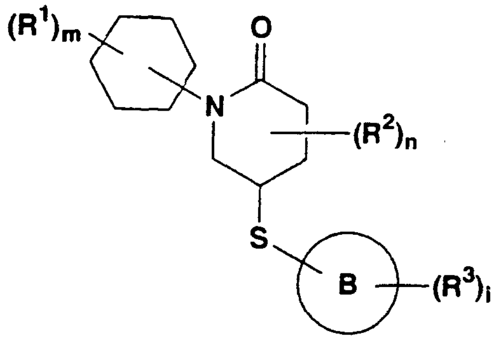

( I -C-4)

(wherein all symbols in the formula have the same meanings as defined above), a compound represented by formula (I-D-1):

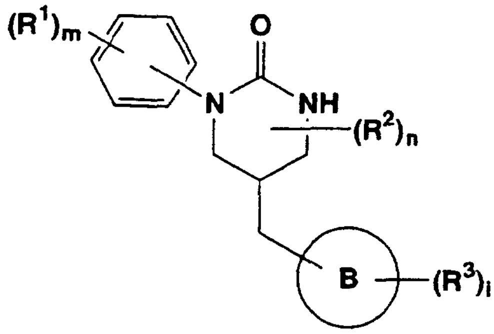

(I-D-1)

(wherein all symbols in the formula have the same meanings as defined above), a compound represented by formula (I-D-2):

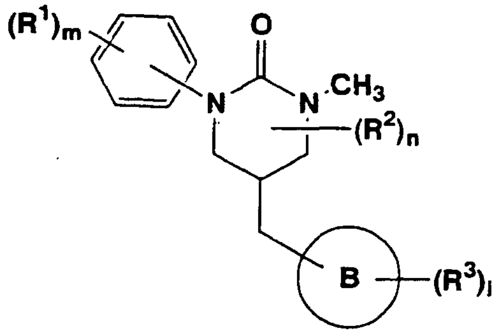

(I-D-2)

(wherein all symbols in the formula have the same meanings as defined above), a compound represented by formula (I-D-3):

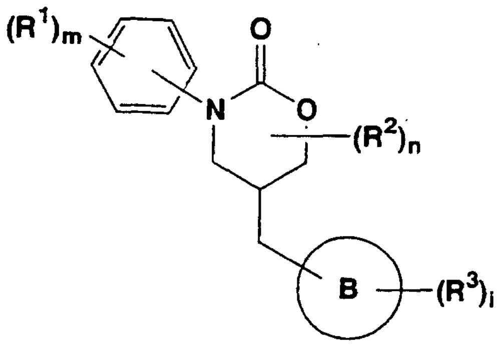

(I-D-3)

(wherein all symbols in the formula have the same meanings as defined above), a compound represented by formula (I-D-4):

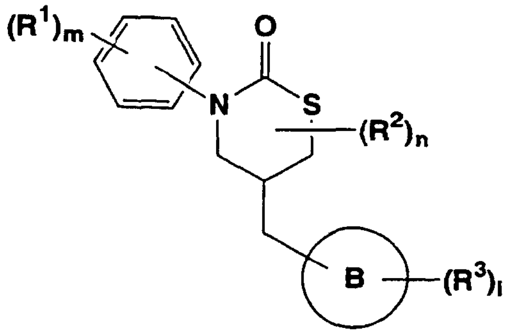

(I-D-4)

(wherein all symbols in the formula have the same meanings as defined above), a compound represented by formula (I-E-1):

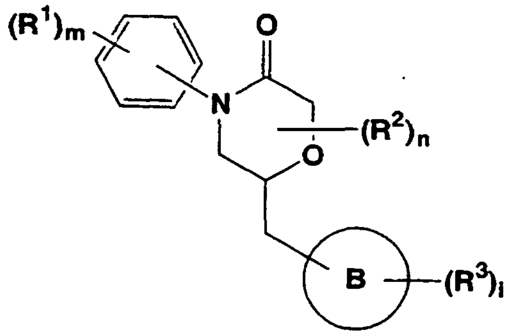

(I-E-1)

(wherein all symbols in the formula have the same meanings as defined above), and a compound represented by formula (I-E-2):

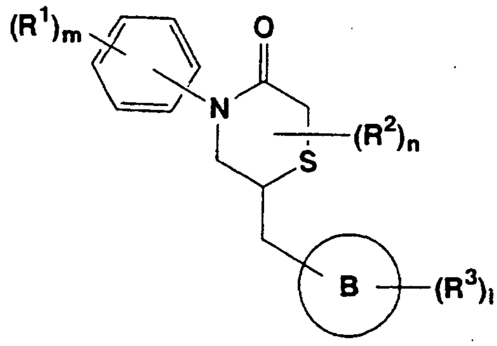

(I-E-2)

(wherein all symbols in the formula have the same meanings as defined above).

Specific preferred compounds of the present invention includes compounds shown in Tables 1 to 42, compounds described in Examples, and non-toxic salts thereof.

In Tables, Ph represents phenyl, and other symbols have the same meanings as defined above.

Table 1

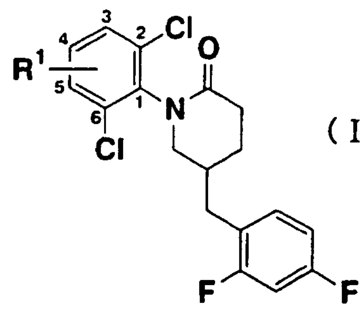

(I-A-1-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 2

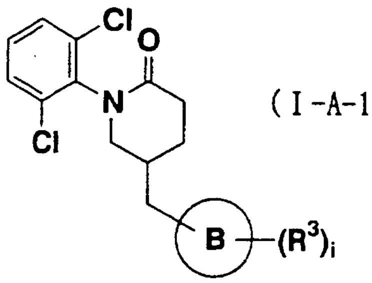

(I-A-1-2).

| No. | 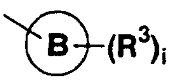 | No. | 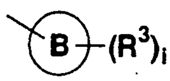 | No. | 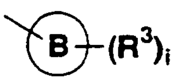 |
|---|---|---|---|---|---|
| 1 | 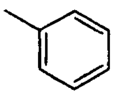 | 8 | 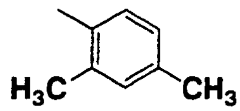 | 15 | 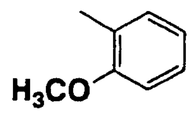 |
| 2 | 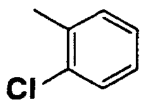 | 9 | 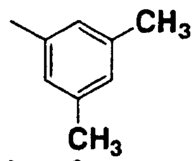 | 16 | 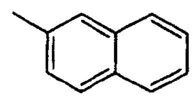 |
| 3 | 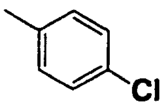 | 10 | 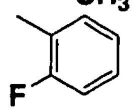 | 17 | 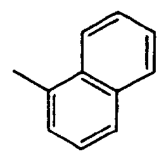 |
| 4 | 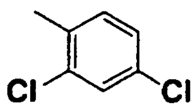 | 11 | 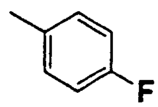 | | |
| 5 | 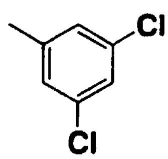 | 12 | 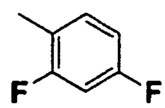 | | |
| 6 | 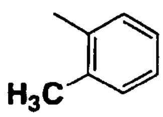 | 13 | 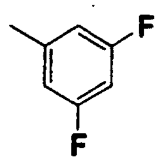 | | |
| 7 | 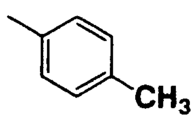 | 14 | 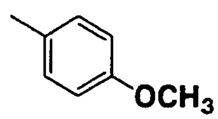 | | |

Table 3

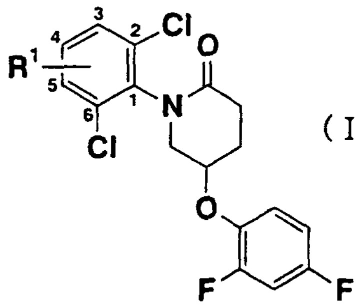

(I-B-1-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 4

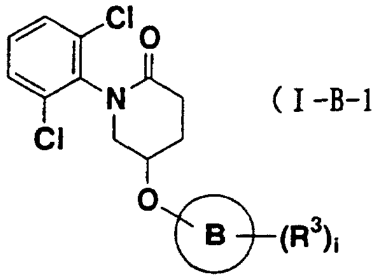

(I-B-1-2)

| No. | 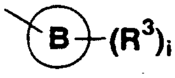 | No. | 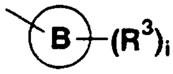 | No. | 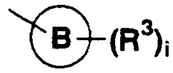 |
|---|---|---|---|---|---|
| 1 | 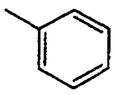 | 8 | 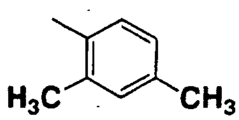 | 15 | 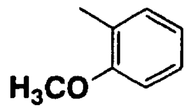 |
| 2 | 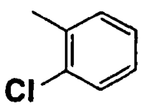 | 9 | 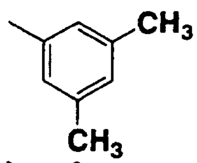 | 16 | 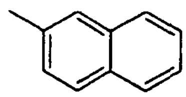 |
| 3 | 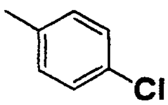 | 10 | 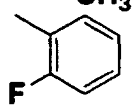 | 17 | 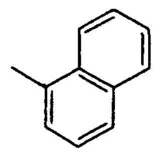 |
| 4 | 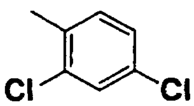 | 11 | 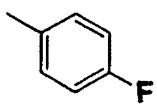 | | |
| 5 | 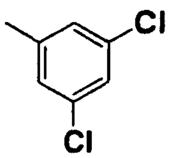 | 12 | 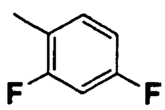 | | |
| 6 | 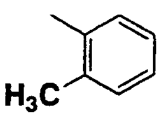 | 13 | 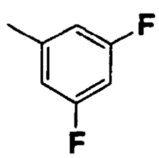 | | |
| 7 | 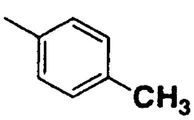 | 14 | 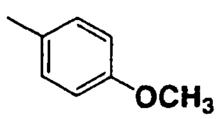 | | |

Table 5

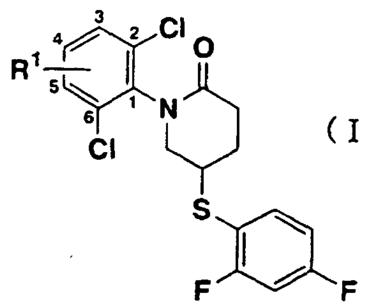

(I-C-1-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 6

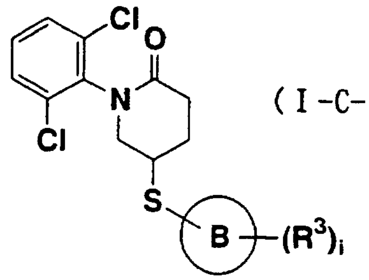

(I-C-1-2)

| No. | 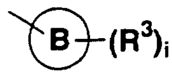 | No. | 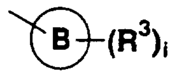 | No. | 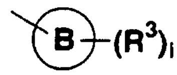 |
|---|---|---|---|---|---|
| 1 | 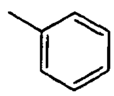 | 8 | 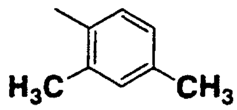 | 15 | 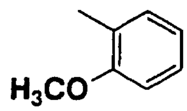 |
| 2 | 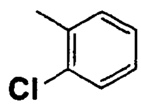 | 9 | 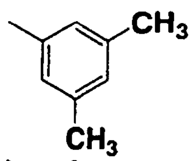 | 16 | 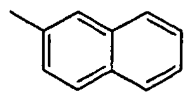 |
| 3 | 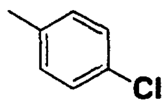 | 10 | 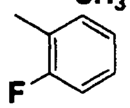 | 17 | 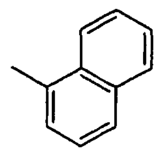 |
| 4 | 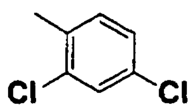 | 11 | 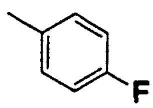 | | |
| 5 | 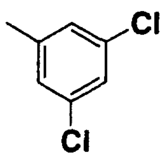 | 12 | 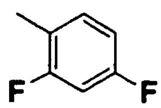 | | |
| 6 | 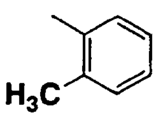 | 13 | 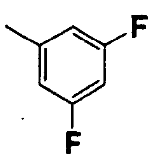 | | |
| 7 | 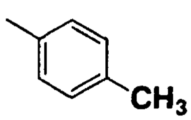 | 14 | 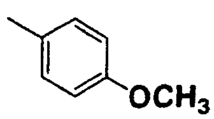 | | |

Table 7

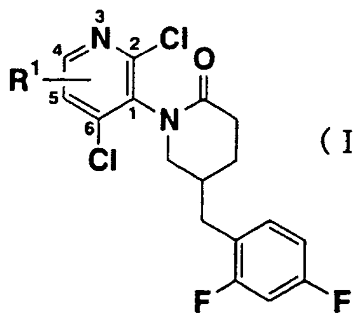

( I -A-2-1)

| No. | $R^1$ | No. | $R^1$ |
|---|---|---|---|
| 1 | H | 8 | 5-OH |
| 2 | 4-OH | 9 | 5-$CH_2OH$ |
| 3 | 4-$CH_2OH$ | 10 | 5-$NH_2$ |
| 4 | 4-$NH_2$ | 11 | 5-$NHCH_3$ |
| 5 | 4-$NHCH_3$ | 12 | 5-$NHCOCH_3$ |
| 6 | 4-$NHCOCH_3$ | 13 | 5-$NHCOCH_2Ph$ |
| 7 | 4-$NHCOCH_2Ph$ | | |

Table 8

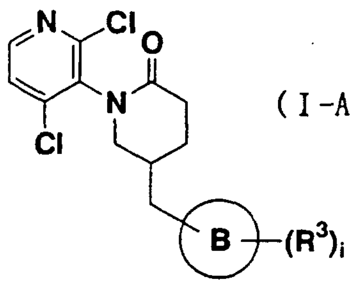

(I-A-2-2)

| No. | 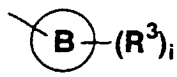 | No. | 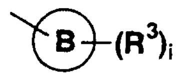 | No. | 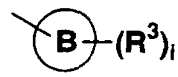 |
|---|---|---|---|---|---|
| 1 | 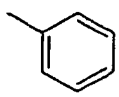 | 8 | 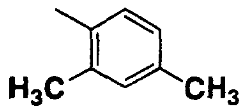 | 15 | 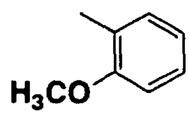 |
| 2 | 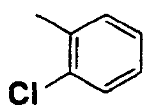 | 9 | 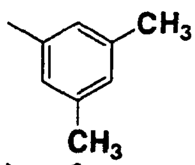 | 16 | 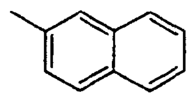 |
| 3 | 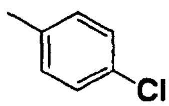 | 10 | 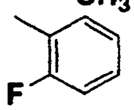 | 17 | 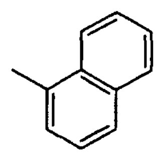 |
| 4 | 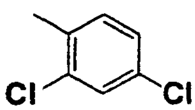 | 11 | 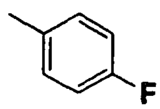 | | |
| 5 | 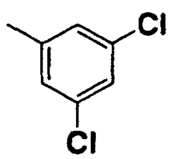 | 12 | 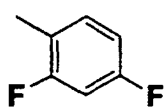 | | |
| 6 | 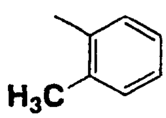 | 13 | 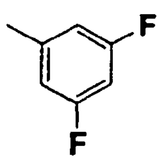 | | |
| 7 | 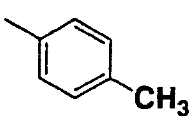 | 14 | 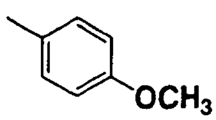 | | |

Table 9

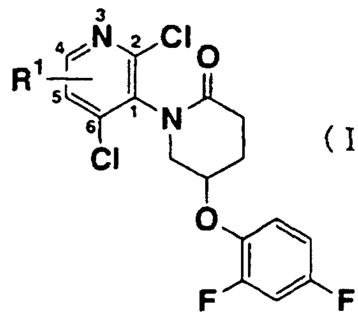

(I -B-2-1)

| No. | $R^1$ | No. | $R^1$ |
|---|---|---|---|
| 1 | H | 8 | 5-OH |
| 2 | 4-OH | 9 | 5-$CH_2OH$ |
| 3 | 4-$CH_2OH$ | 10 | 5-$NH_2$ |
| 4 | 4-$NH_2$ | 11 | 5-$NHCH_3$ |
| 5 | 4-$NHCH_3$ | 12 | 5-$NHCOCH_3$ |
| 6 | 4-$NHCOCH_3$ | 13 | 5-$NHCOCH_2Ph$ |
| 7 | 4-$NHCOCH_2Ph$ | | |

Table 10

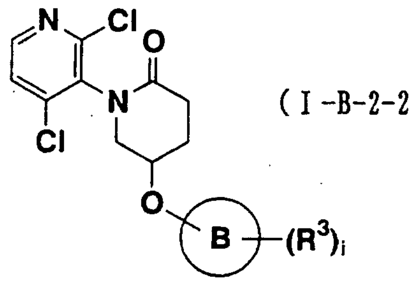

(I-B-2-2)

| No. | 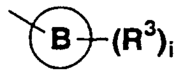 | No. | 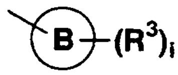 | No. | 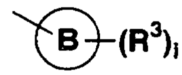 |
|---|---|---|---|---|---|
| 1 | 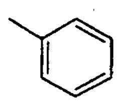 | 8 | 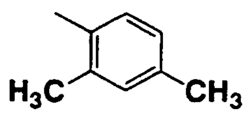 | 15 | 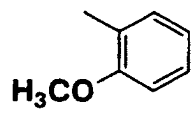 |
| 2 | 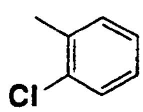 | 9 | 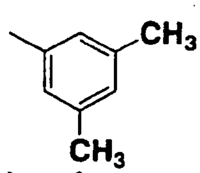 | 16 | 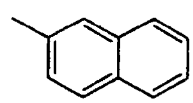 |
| 3 | 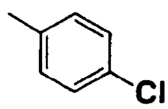 | 10 | 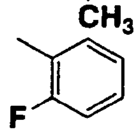 | 17 | 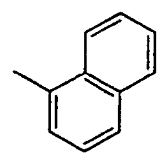 |
| 4 | 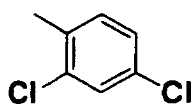 | 11 | 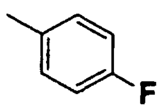 | | |
| 5 | 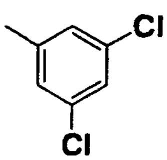 | 12 | 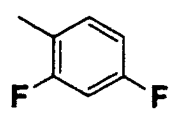 | | |
| 6 | 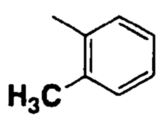 | 13 | 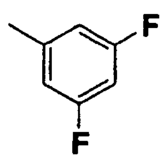 | | |
| 7 | 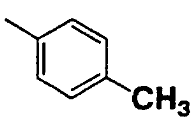 | 14 | 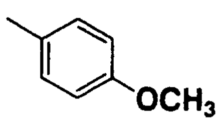 | | |

Table 11

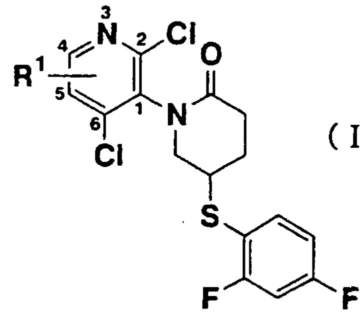

(I-C-2-1)

| No. | $R^1$ | No. | $R^1$ |
|---|---|---|---|
| 1 | H | 8 | 5-OH |
| 2 | 4-OH | 9 | 5-$CH_2OH$ |
| 3 | 4-$CH_2OH$ | 10 | 5-$NH_2$ |
| 4 | 4-$NH_2$ | 11 | 5-$NHCH_3$ |
| 5 | 4-$NHCH_3$ | 12 | 5-$NHCOCH_3$ |
| 6 | 4-$NHCOCH_3$ | 13 | 5-$NHCOCH_2Ph$ |
| 7 | 4-$NHCOCH_2Ph$ | | |

Table 12

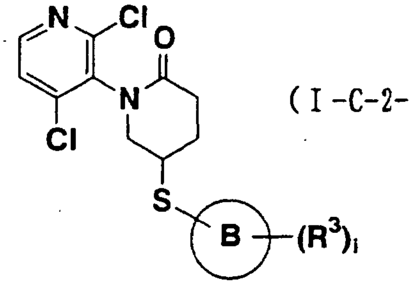

(I-C-2-2)

| No. | 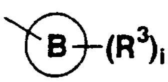 | No. | 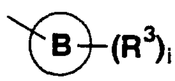 | No. | 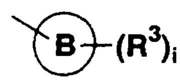 |
|---|---|---|---|---|---|
| 1 | 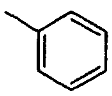 | 8 | 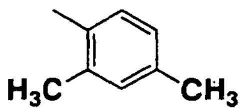 | 15 | 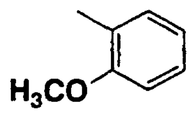 |
| 2 | 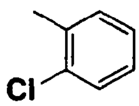 | 9 | 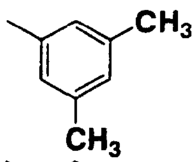 | 16 | 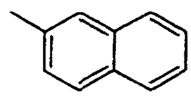 |
| 3 | 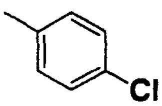 | 10 | 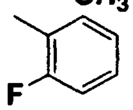 | 17 | 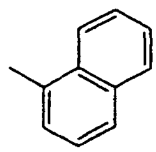 |
| 4 | 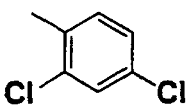 | 11 | 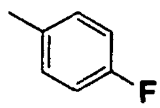 | | |
| 5 | 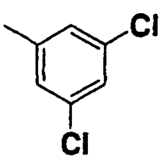 | 12 | 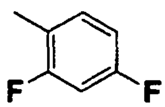 | | |
| 6 | 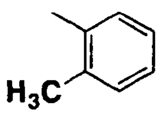 | 13 | 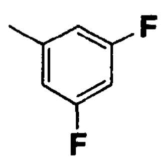 | | |
| 7 | 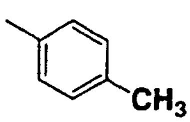 | 14 | 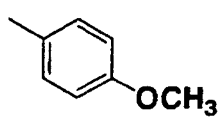 | | |

Table 13

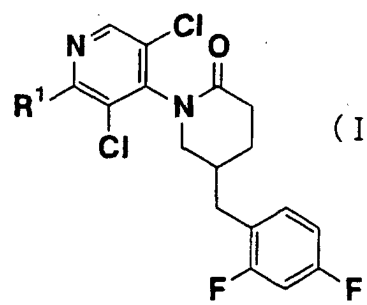

(I-A-2-3)

| No. | $R^1$ |
|---|---|
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 14

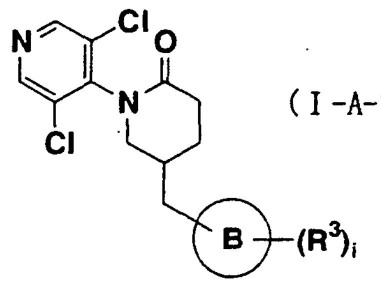

( I -A-2-4)

| No. | 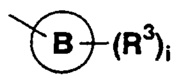 | No. | 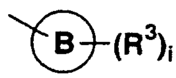 | No. | 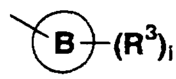 |
|---|---|---|---|---|---|
| 1 | 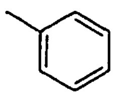 | 8 | 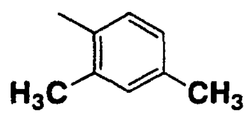 | 15 | 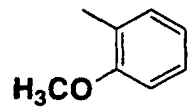 |
| 2 | 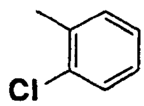 | 9 | 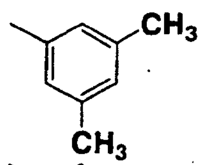 | 16 | 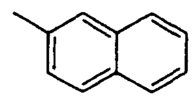 |
| 3 | 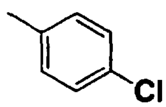 | 10 | 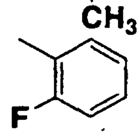 | 17 | 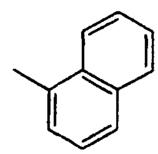 |
| 4 | 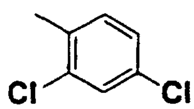 | 11 | 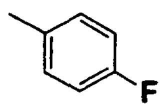 | | |
| 5 | 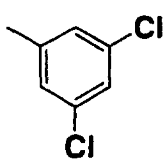 | 12 | 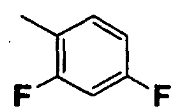 | | |
| 6 | 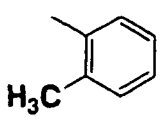 | 13 | 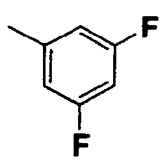 | | |
| 7 | 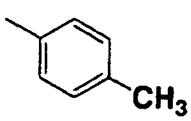 | 14 | 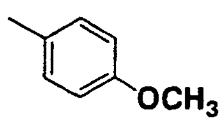 | | |

Table 15

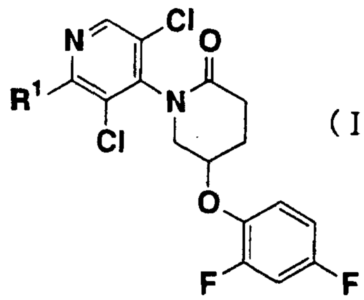

(I-B-2-3)

| No. | $R^1$ |
|---|---|
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 16

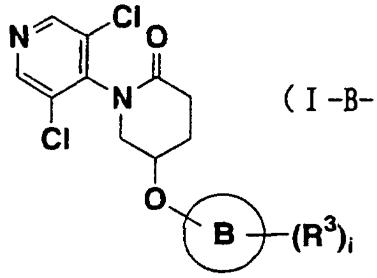

(I-B-2-4)

| No. | 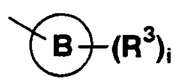 | No. | 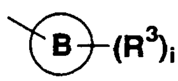 | No. | 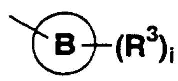 |
|---|---|---|---|---|---|
| 1 | 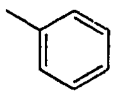 | 8 | 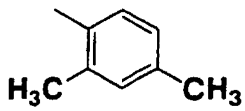 | 15 | 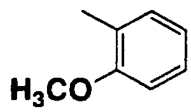 |
| 2 | 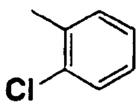 | 9 | 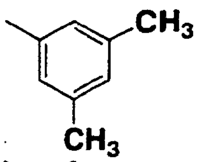 | 16 | 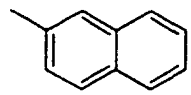 |
| 3 | 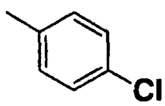 | 10 | 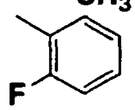 | 17 | 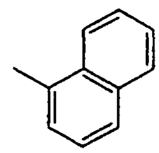 |
| 4 | 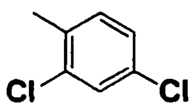 | 11 | 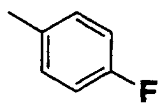 | | |
| 5 | 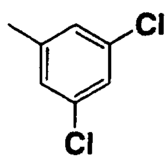 | 12 | 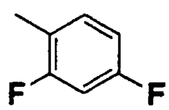 | | |
| 6 | 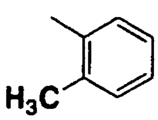 | 13 | 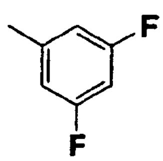 | | |
| 7 | 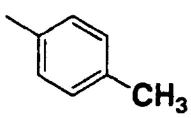 | 14 | 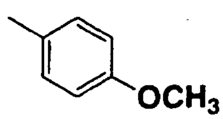 | | |

Table 17

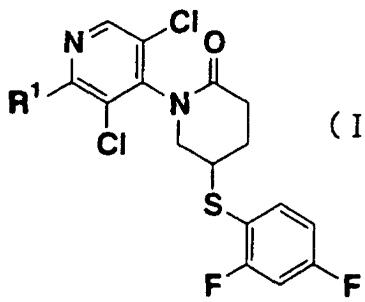

(I-C-2-3)

| No. | $R^1$ |
| --- | --- |
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 18

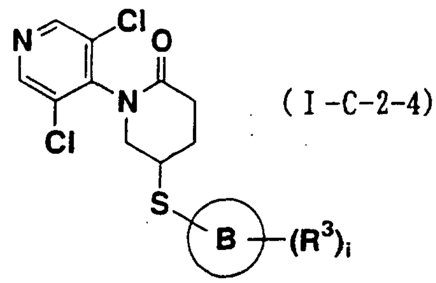

(I-C-2-4)

| No. | 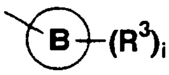 | No. | 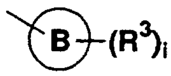 | No. | 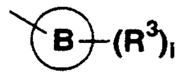 |
|---|---|---|---|---|---|
| 1 | 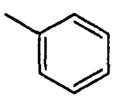 | 8 | 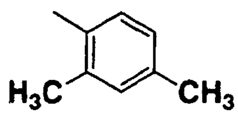 | 15 | 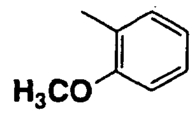 |
| 2 | 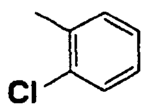 | 9 | 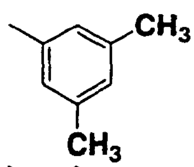 | 16 | 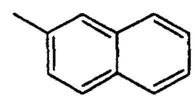 |
| 3 | 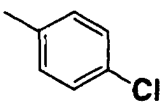 | 10 | 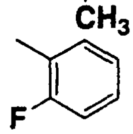 | 17 | 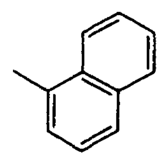 |
| 4 | 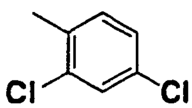 | 11 | 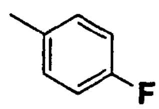 | | |
| 5 | 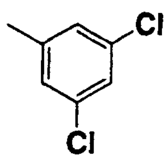 | 12 | 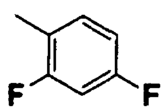 | | |
| 6 | 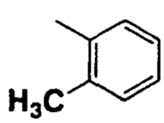 | 13 | 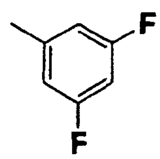 | | |
| 7 | 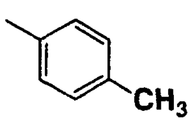 | 14 | 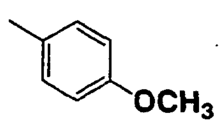 | | |

Table 19

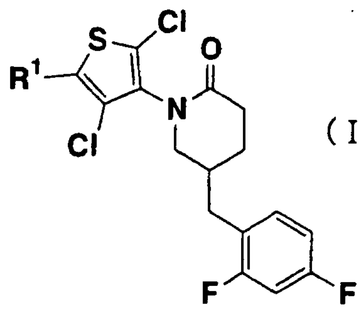

(I-A-3-1)

| No. | $R^1$ |
|---|---|
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 20

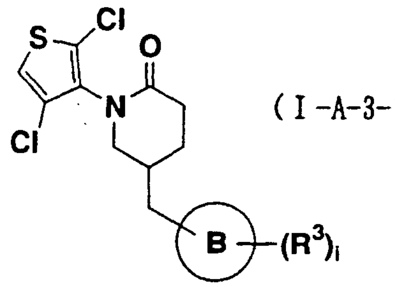

(I-A-3-2)

| No. | 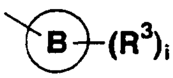 | No. | 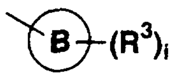 | No. | 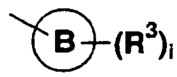 |
|---|---|---|---|---|---|
| 1 | 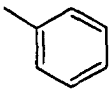 | 8 | 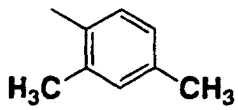 | 15 | 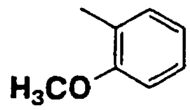 |
| 2 | 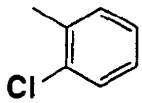 | 9 | 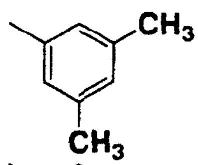 | 16 | 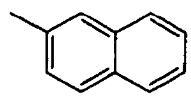 |
| 3 | 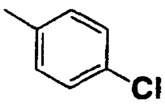 | 10 | 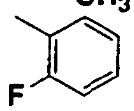 | 17 | 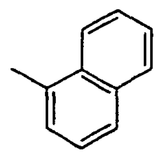 |
| 4 | 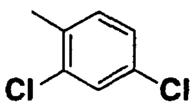 | 11 | 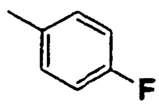 | | |
| 5 | 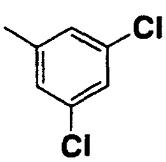 | 12 | 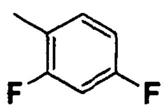 | | |
| 6 | 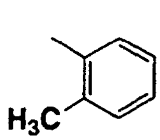 | 13 | 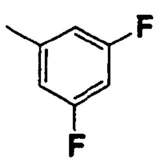 | | |
| 7 | 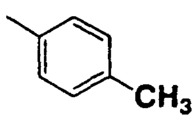 | 14 | 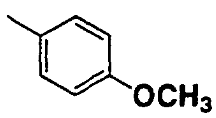 | | |

Table 21

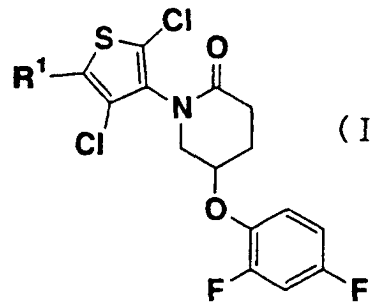

(I-B-3-1)

| No. | $R^1$ |
|---|---|
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 22

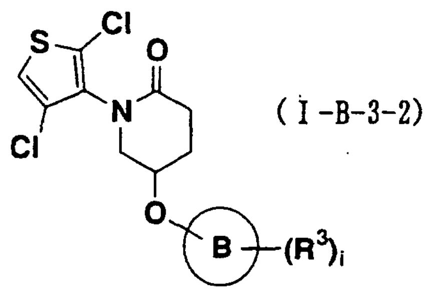

( I -B-3-2)

| No. | 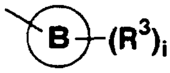 | No. | 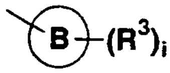 | No. | 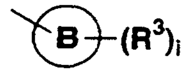 |
|---|---|---|---|---|---|
| 1 | 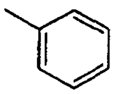 | 8 | 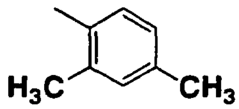 | 15 | 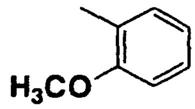 |
| 2 | 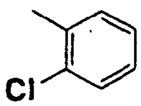 | 9 | 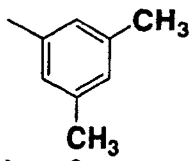 | 16 | 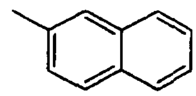 |
| 3 | 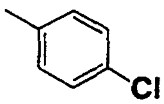 | 10 | 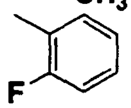 | 17 | 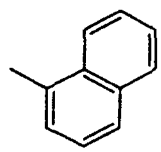 |
| 4 | 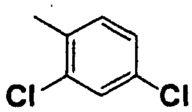 | 11 | 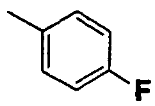 | | |
| 5 | 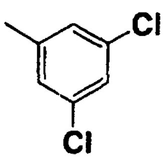 | 12 | 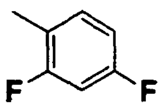 | | |
| 6 | 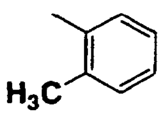 | 13 | 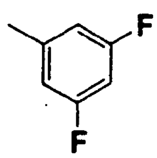 | | |
| 7 | 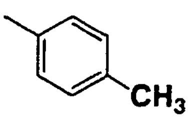 | 14 | 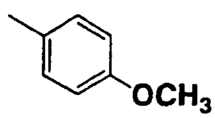 | | |

Table 23

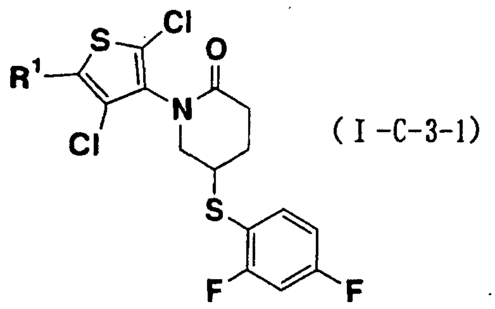

(I -C-3-1)

| No. | $R^1$ |
|---|---|
| 1 | H |
| 2 | OH |
| 3 | $CH_2OH$ |
| 4 | $NH_2$ |
| 5 | $NHCH_3$ |
| 6 | $NHCOCH_3$ |
| 7 | $NHCOCH_2Ph$ |

Table 24

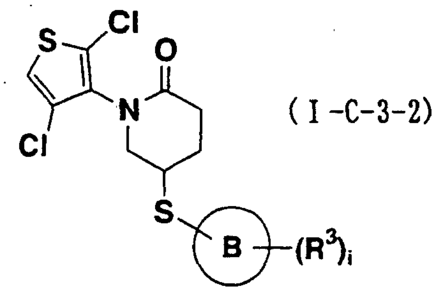

(I-C-3-2)

| No. | 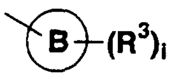 | No. | 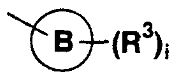 | No. | 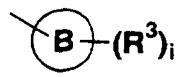 |
|---|---|---|---|---|---|
| 1 | 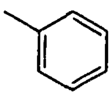 | 8 | 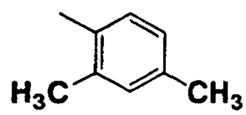 | 15 | 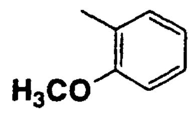 |
| 2 | 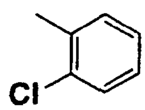 | 9 | 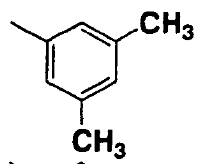 | 16 | 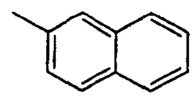 |
| 3 | 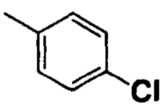 | 10 | 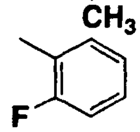 | 17 | 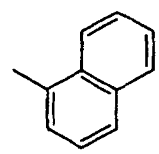 |
| 4 | 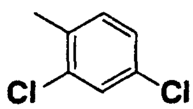 | 11 | 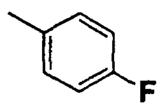 | | |
| 5 | 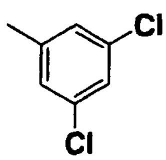 | 12 | 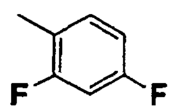 | | |
| 6 | 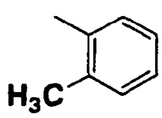 | 13 | 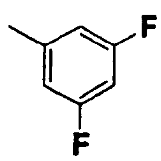 | | |
| 7 | 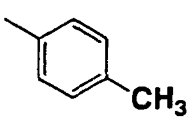 | 14 | 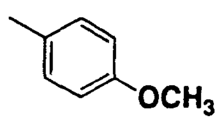 | | |

Table 25

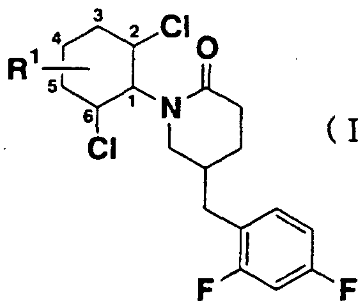

(I-A-4-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 26

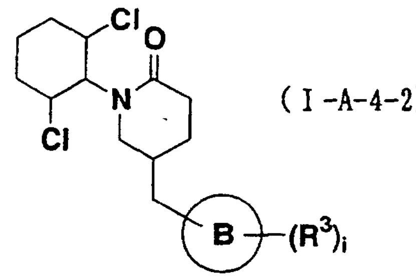

( I -A-4-2)

| No. | 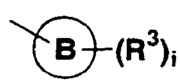 | No. | 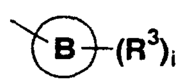 | No. | 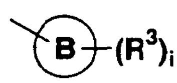 |
|---|---|---|---|---|---|
| 1 | 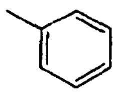 | 8 | 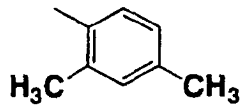 | 15 | 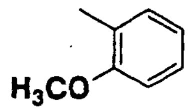 |
| 2 | 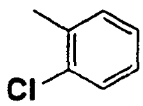 | 9 | 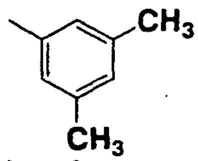 | 16 | 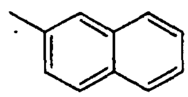 |
| 3 | 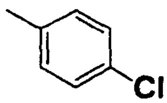 | 10 | 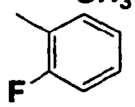 | 17 | 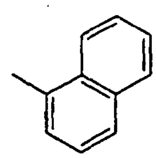 |
| 4 | 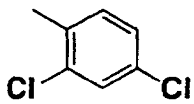 | 11 | 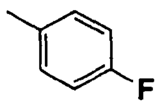 | | |
| 5 | 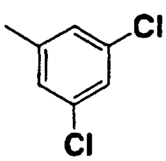 | 12 | 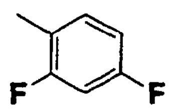 | | |
| 6 | 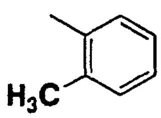 | 13 | 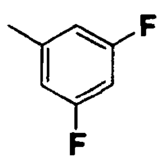 | | |
| 7 | 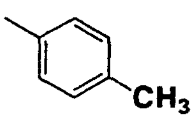 | 14 | 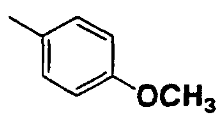 | | |

Table 27

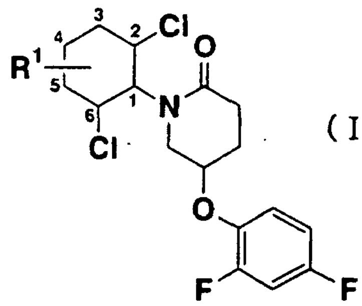

(I -B-4-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 28

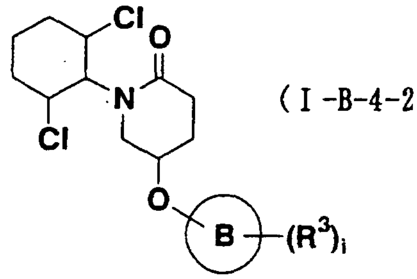

( I -B-4-2)

| No. | 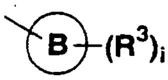 | No. | 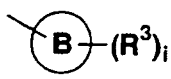 | No. | 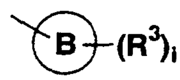 |
|---|---|---|---|---|---|
| 1 | 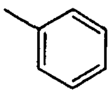 | 8 | 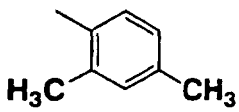 | 15 | 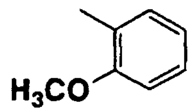 |
| 2 | 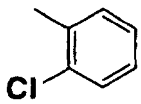 | 9 | 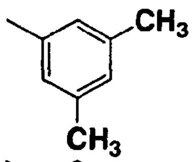 | 16 | 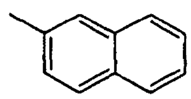 |
| 3 | 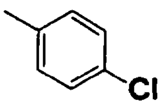 | 10 | 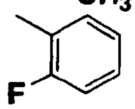 | 17 | 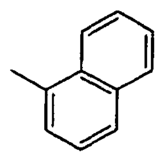 |
| 4 | 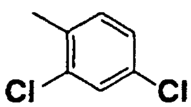 | 11 | 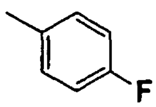 | | |
| 5 | 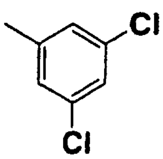 | 12 | 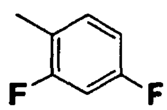 | | |
| 6 | 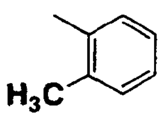 | 13 | 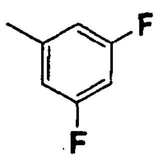 | | |
| 7 | 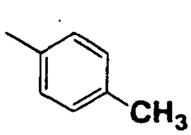 | 14 | 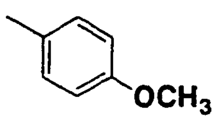 | | |

Table 29

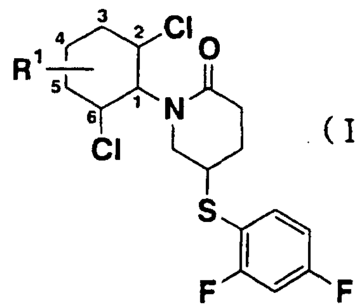

(I -C-4-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 30

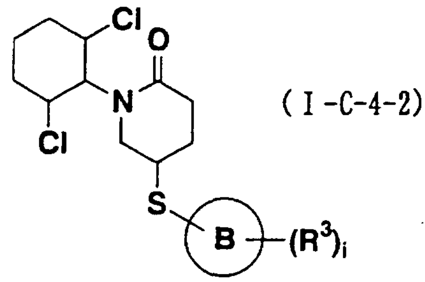

(I-C-4-2)

| No. | 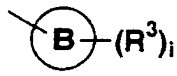 | No. | 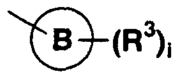 | No. | 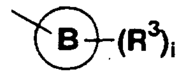 |
|---|---|---|---|---|---|
| 1 | 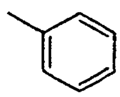 | 8 | 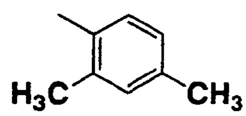 | 15 | 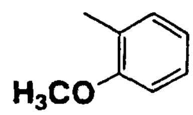 |
| 2 | 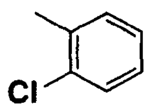 | 9 | 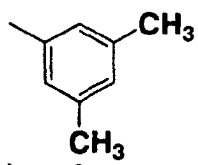 | 16 | 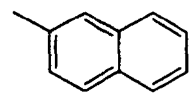 |
| 3 | 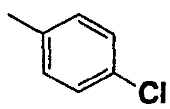 | 10 | 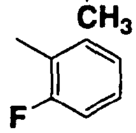 | 17 | 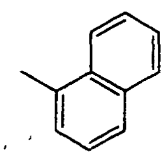 |
| 4 | 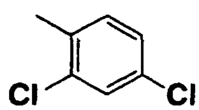 | 11 | 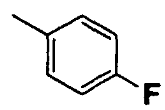 | | |
| 5 | 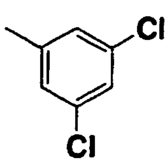 | 12 | 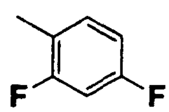 | | |
| 6 | 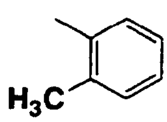 | 13 | 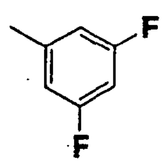 | | |
| 7 | 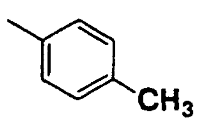 | 14 | 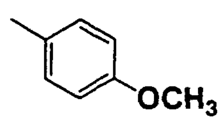 | | |

Table 31

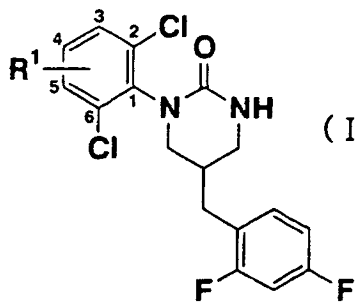

(I-D-1-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 32

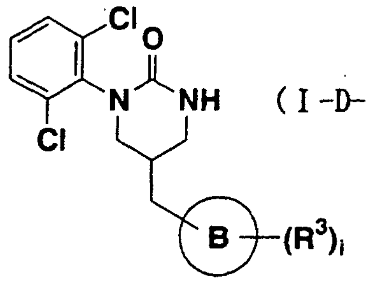

(I-D-1-2)

| No. | 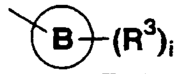 | No. | 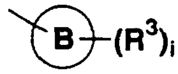 | No. | 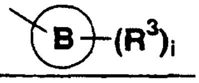 |
|---|---|---|---|---|---|
| 1 | 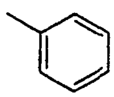 | 8 | 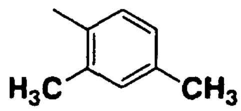 | 15 | 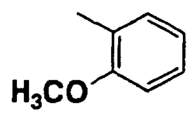 |
| 2 | 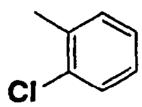 | 9 | 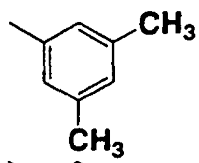 | 16 | 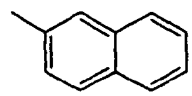 |
| 3 | 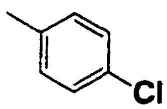 | 10 | 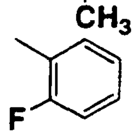 | 17 | 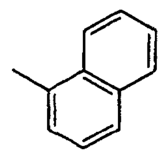 |
| 4 | 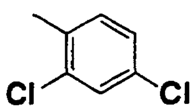 | 11 | 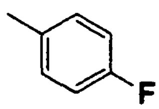 | | |
| 5 | 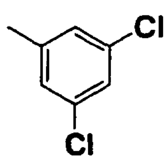 | 12 | 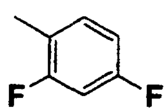 | | |
| 6 | 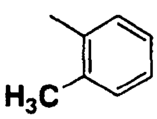 | 13 | 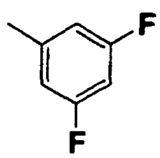 | | |
| 7 | 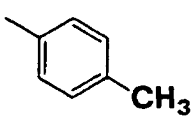 | 14 | 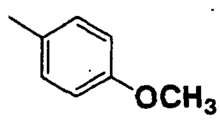 | | |

Table 33

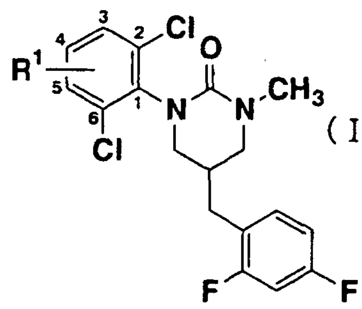

(I-D-2-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 34

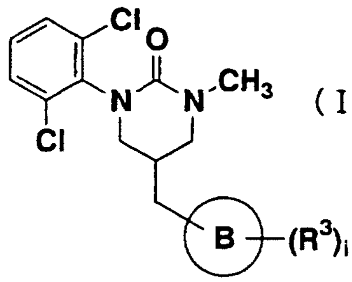 (I -D-2-2)

| No. | 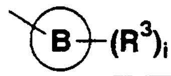 | No. | 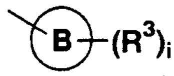 | No. | 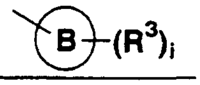 |
|---|---|---|---|---|---|
| 1 | 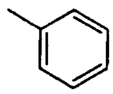 | 8 | 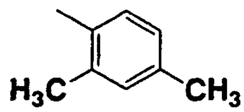 | 15 | 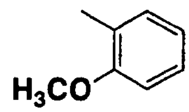 |
| 2 | 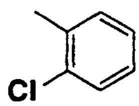 | 9 | 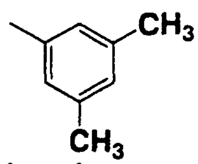 | 16 | 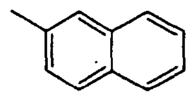 |
| 3 | 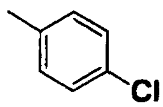 | 10 | 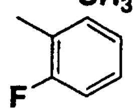 | 17 | 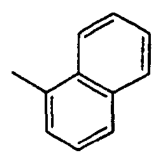 |
| 4 | 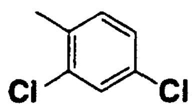 | 11 | 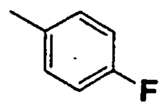 | | |
| 5 | 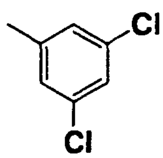 | 12 | 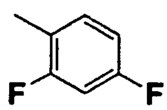 | | |
| 6 | 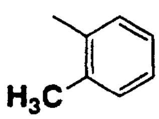 | 13 | 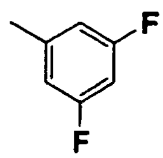 | | |
| 7 | 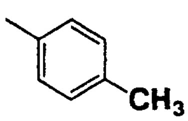 | 14 | 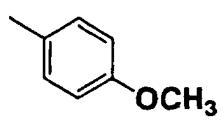 | | |

Table 35

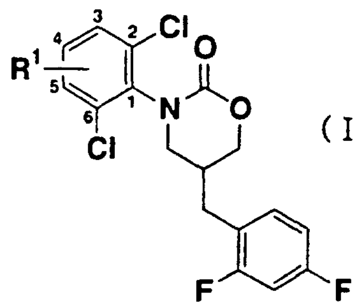

(I-D-3-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

## Table 36

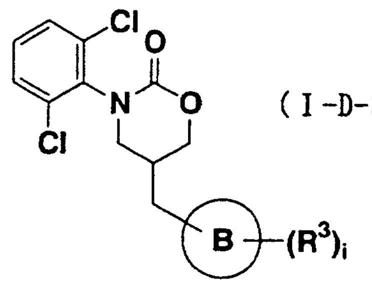

(I-D-3-2)

| No. | 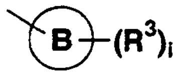 | No. | 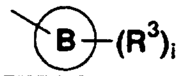 | No. | 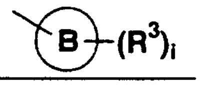 |
|---|---|---|---|---|---|
| 1 | 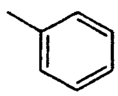 | 8 | 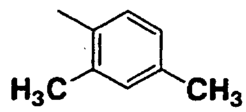 | 15 | 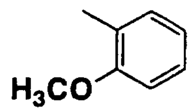 |
| 2 | 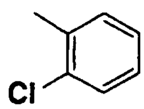 | 9 | 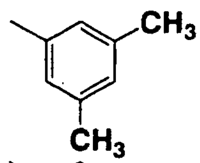 | 16 | 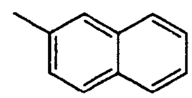 |
| 3 | 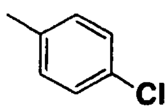 | 10 | 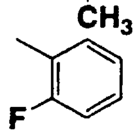 | 17 | 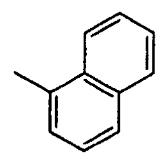 |
| 4 | 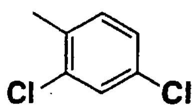 | 11 | 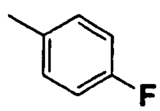 | | |
| 5 | 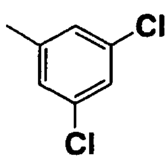 | 12 | 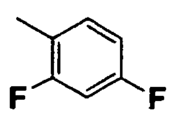 | | |
| 6 | 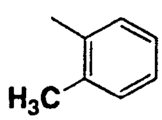 | 13 | 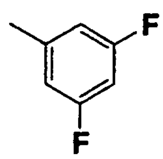 | | |
| 7 | 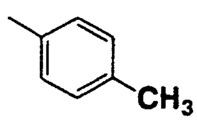 | 14 | 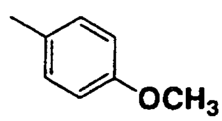 | | |

Table 37

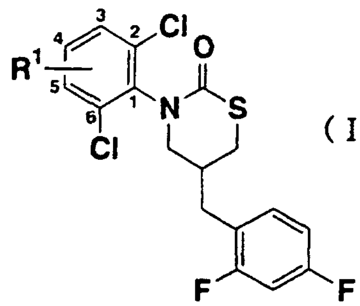

(I-D-4-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 38

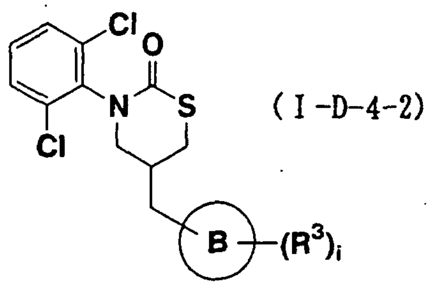

(I-D-4-2)

| No. | 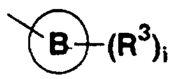 | No. | 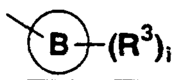 | No. | 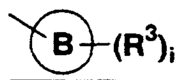 |
|---|---|---|---|---|---|
| 1 | 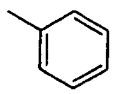 | 8 | 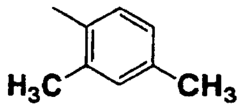 | 15 | 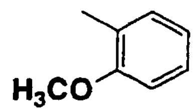 |
| 2 | 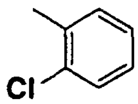 | 9 | 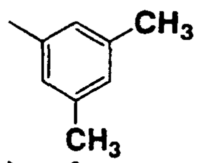 | 16 | 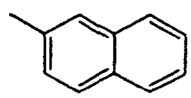 |
| 3 | 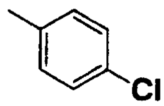 | 10 | 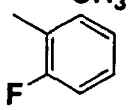 | 17 | 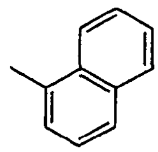 |
| 4 | 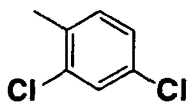 | 11 | 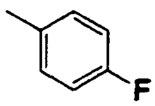 | | |
| 5 | 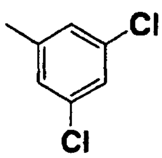 | 12 | 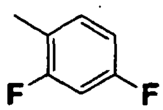 | | |
| 6 | 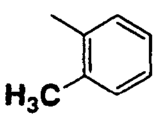 | 13 | 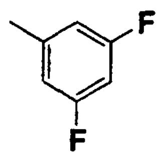 | | |
| 7 | 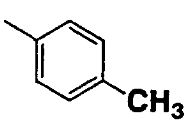 | 14 | 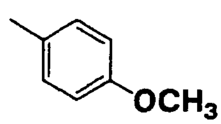 | | |

Table 39

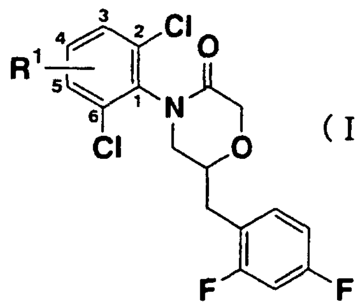

(I-E-1-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 40

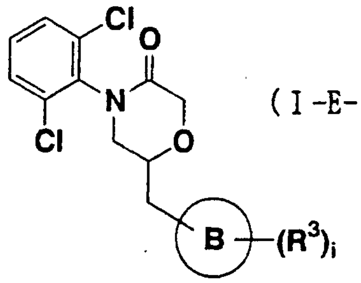

(I-E-1-2)

| No. | 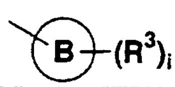 | No. | 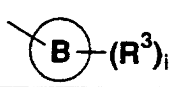 | No. | 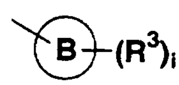 |
|---|---|---|---|---|---|
| 1 | 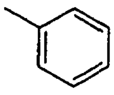 | 8 | 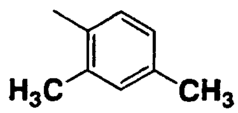 | 15 | 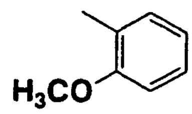 |
| 2 | 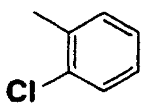 | 9 | 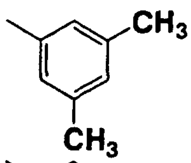 | 16 | 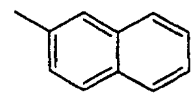 |
| 3 | 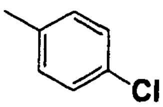 | 10 | 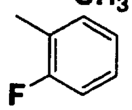 | 17 | 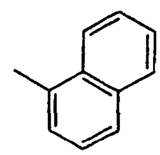 |
| 4 | 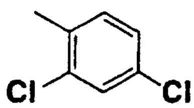 | 11 | 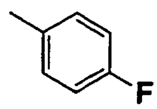 | | |
| 5 | 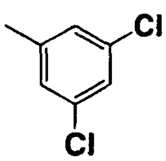 | 12 | 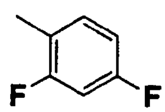 | | |
| 6 | 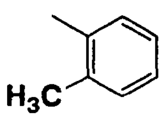 | 13 | 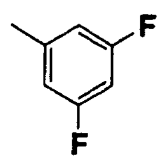 | | |
| 7 | 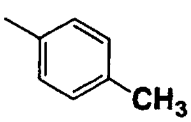 | 14 | 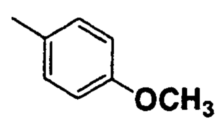 | | |

Table 41

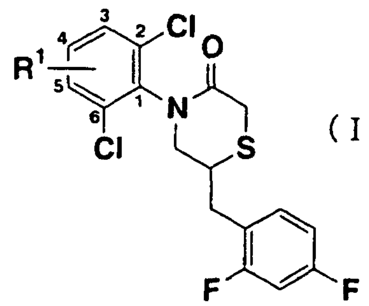

( I -E-2-1)

| No. | $R^1$ | No. | $R^1$ |
|---|---|---|---|
| 1 | H | 8 | 4-OH |
| 2 | 3-OH | 9 | 4-$CH_2OH$ |
| 3 | 3-$CH_2OH$ | 10 | 4-$NH_2$ |
| 4 | 3-$NH_2$ | 11 | 4-$NHCH_3$ |
| 5 | 3-$NHCH_3$ | 12 | 4-$NHCOCH_3$ |
| 6 | 3-$NHCOCH_3$ | 13 | 4-$NHCOCH_2Ph$ |
| 7 | 3-$NHCOCH_2Ph$ | | |

Table 42

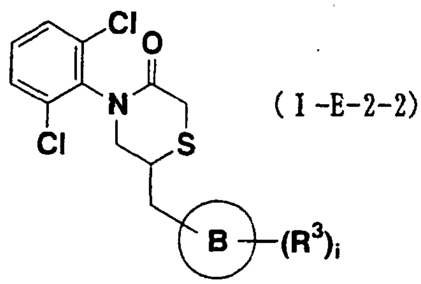

(I-E-2-2)

| No. | 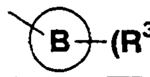 | No. | 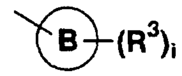 | No. | 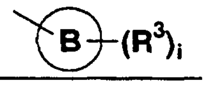 |
|---|---|---|---|---|---|
| 1 | 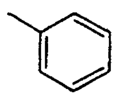 | 8 | 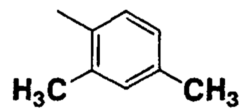 | 15 | 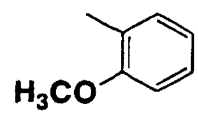 |
| 2 | 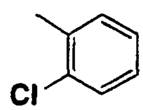 | 9 | 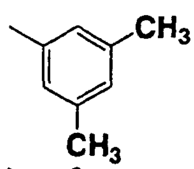 | 16 | 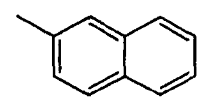 |
| 3 | 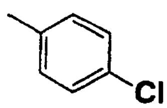 | 10 | 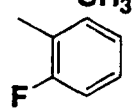 | 17 | 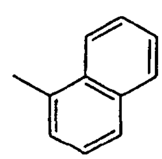 |
| 4 | 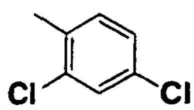 | 11 | 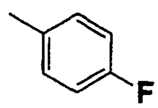 | | |
| 5 | 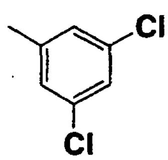 | 12 | 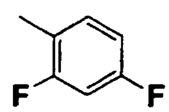 | | |
| 6 | 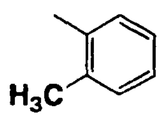 | 13 | 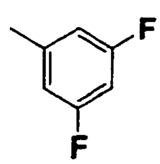 | | |
| 7 | 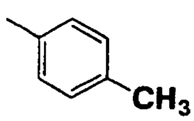 | 14 | 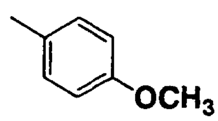 | | |

[Method for preparing compounds of the present invention]

The compound represented by formula (I) of the present invention can be produced by the following method or the method described in Examples.

[A] Among the compounds represented by formula (I) of the present invention, a compound wherein 

represents a single bond, i.e., a compound represented by formula (I-A):

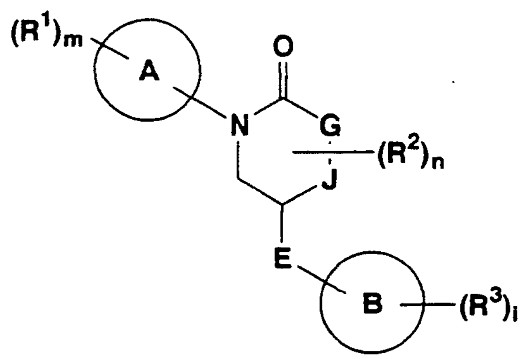

(I-A)

(wherein all symbols have the same meanings as defined above) can be produced by the following method.

[1] The compound represented by formula (I-A) can be produced by subjecting a compound represented by formula (II):

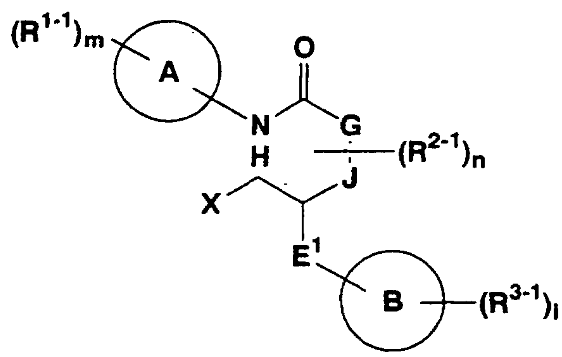

(II)

(wherein X represents a leaving group (e.g., chlorine atom, bromine atom, iodine atom, tosyl, mesyl) and $R^{1-1}$, $R^{2-1}$, $R^{3-1}$ and $E^1$ have the same meanings as $R^1$, $R^2$, $R^3$, and E, respectively, wherein hydroxyl, amino, thiol or carboxyl contained in the group represented by $R^{1-1}$, $R^{2-1}$, $R^{3-1}$ or $E^1$ is optionally protected, if necessary, and other symbols have the same meanings as defined above) to a cyclization reaction to thereby give a compound represented by formula (I-1):

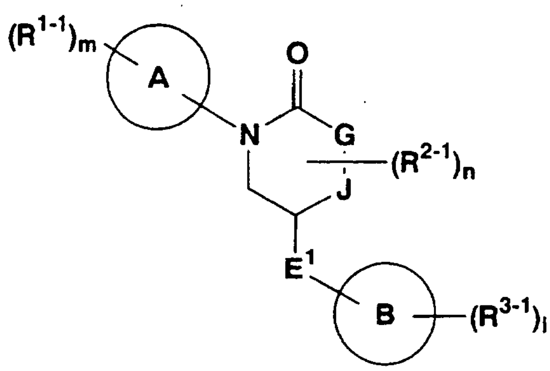

(I − 1)

(wherein all symbols have the same meanings as defined above), followed by a deprotection reaction of a protective group, if necessary.

The cyclization reaction of the compound represented by formula (II) is known, and for example, can be carried out by reacting the compound represented by formula (II) in an organic solvent (diethyl ether, tetrahydrofuran, *etc.*) in the presence of a base (*tert*-butoxy potassium, sodium methoxide, sodium ethoxide, sodium hydride, potassium hydride, *etc.*) at a temperature of -20 to 40°C.

The deprotection reaction of a protective group can be carried out by the following method.

The deprotection reaction of a protective group for hydroxyl, amino, thiol or carboxyl is known and it includes

(1) alkaline hydrolysis,
(2) deprotection reaction under acidic conditions,
(3) deprotection reaction by hydrogenolysis,
(4) deprotection reaction of a silyl group,

and the like.

These methods are described specifically as follows.

(1) The deprotection reaction by alkaline hydrolysis is, for example, carried out in an organic solvent (methanol, tetrahydrofuran, dioxane, or a mixture thereof, *etc.*) using a hydroxide of an alkali metal (sodium hydroxide, potassium hydroxide, lithium hydroxide, *etc.*), a hydroxide of an alkali earth metal (barium hydroxide or calcium hydroxide, *etc.*), a carbonate (sodium carbonate or potassium carbonate, *etc.*), an aqueous solution thereof, or a mixture thereof at a temperature of 0 to 40°C.
(2) The deprotection reaction under acidic conditions is carried out, for example, in an organic solvent (methylene chloride, chloroform, dioxane, ethyl acetate, anisole, *etc.*) or in the absence of an organic solvent, in an organic acid (acetic acid, trifluoroacetic acid, methanesulfonic acid, *etc.*), an inorganic acid (hydrochloric acid, sulfuric acid, *etc.*) or a mixture thereof (hydrogen bromide/acetic acid, *etc.*) at a temperature of 0 to 100°C.
(3) The deprotection reaction by hydrogenolysis is carried out, for example, in a solvent (ethers (tetrahydrofuran, dioxane, dimethoxyethane, diethyl ether, *etc.*), alcohols (methanol, ethanol, *etc.*), benzenes (benzene, toluene, *etc.*), ketones (acetone, methylethylketone, *etc.*), nitriles (acetonitrile, *etc.*), amides (dimethylformamide, *etc.*), water, ethyl acetate, acetic acid or a mixed solvent of at least two of these) in the presence of a catalyst (palladium-carbon, palladium black, palladium hydroxide, platinum oxide, Raney nickel, *etc.*) under the hydrogen atmosphere at normal pressure or under pressurization, or in the presence of ammonium formate at a temperature of 0 to 200°C.
(4) The deprotection reaction of a silyl group is carried out, for example, in a water-miscible organic solvent (tetrahydrofuran, acetonitrile, *etc.*) using tetrabutylammonium fluoride at a temperature of 0 to 40°C.

The protective group for hydroxyl includes methoxymethyl, 2-tetrahydropyranyl, t-butyldimethylsilyl, t-butyldiphenylsilyl, acetyl, benzyl and triphenylmethyl.

The protective group for amino includes benzyloxylcarbonyl, t-butoxycarbonyl, trifluoroacetyl and 9-fluorenylmethoxycarbonyl.

The protective group for thiol includes benzyl, methoxybenzyl, acetoamidemethyl, triphenylmethyl and acetyl.

The protective group for carboxyl includes methyl, ethyl, t-butyl and benzyl.

The protective group for hydroxyl, amino, thiol or carboxyl is not particularly limited to the above-described groups, so long as it can be easily and selectively left. For example, those described in T. W. Greene, Protective Groups in Organic Synthesis 3rd edition, Wiley, New York, 1999 can be used.

As is easily understood by those skilled in the art, an object compound of the present invention can be produced easily by using a different deprotection reaction depending on usage.

[2] The compound represented by formula (I-A) can also be produced by subjecting a compound represented by formula (III):

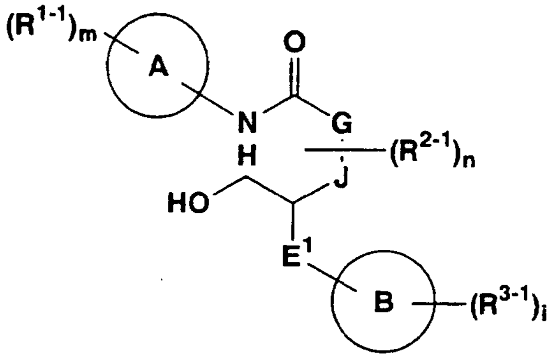

(III)

(wherein all symbols have the same meanings as defined above) to a cyclization reaction to thereby give a compound

represented by formula (I-1), followed by deprotection reaction of a protective group, if necessary.

The above-described cyclization reaction is known and is carried out, for example, in an organic solvent (dichloromethane, diethyl ether, tetrahydrofuran, acetonitrile, benzene, toluene, *etc.*) in the presence of an azo compound (diethyl azodicarboxylate, diisopropyl azodicarboxylate, 1,1'-(azodicarbonyl)dipiperidine, 1,1'-azobis(N,N-dimethylfonmamide), *etc.* ) and a phosphine compound (triphenylphosphine, tributylphosphine, trimethylphosphine, *etc.*) at a temperature of 0 to 60°C.

The deprotection reaction of a protective group can be carried out in the same manner as described above.

[3] Among the compounds represented by formula (I-A) of the present invention, a compound wherein $E^2$ represents an oxygen atom or a sulfur atom, i.e., a compound represented by formula (1-2):

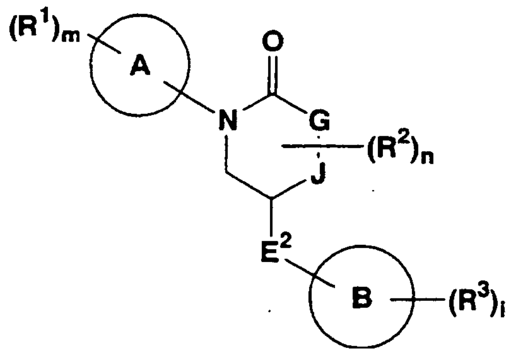

(I − 2)

(wherein $E^2$ represents an oxygen atom or a sulfur atom and other symbols have the same meanings as defined above) can also be produced by reacting a compound represented by formula (IV):

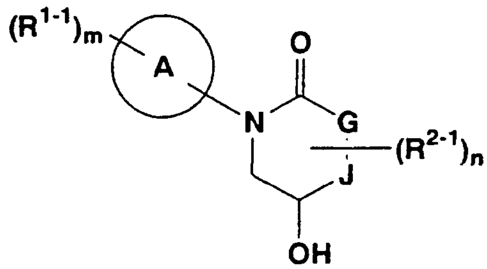

(IV)

(wherein all symbols have the same meanings as defined above) with a compound represented by formula (V):

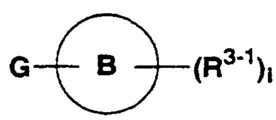

(V)

(wherein G represents hydroxyl or a thiol and other symbols have the same meanings as defined above) to thereby give a compound represented by formula (I-2'):

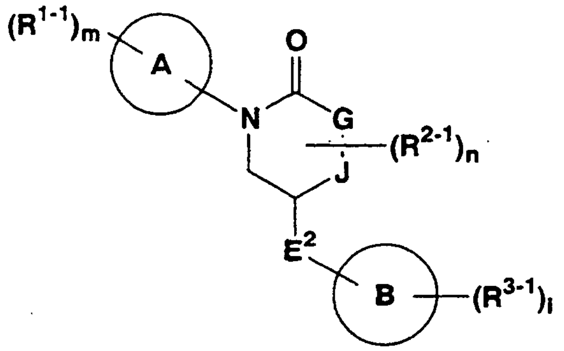

(I − 2')

(wherein all symbols have the same meanings as defined above), followed by deprotection reaction of a protective group, if necessary.

The reaction of the compound represented by formula (IV) with the compound represented by formula (V) is known and carried out, for example, in an organic solvent (dichloromethane, diethyl ether, tetrahydrofuran, acetonitrile, benzene, toluene, *etc.*) in the presence of an azo compound (diethyl azodicarboxylate, diisopropyl azodicarboxylate, 1,1'-(azodicarbonyl) dipiperidine, 1,1'-azobis(N,N-dimethylformamide), *etc.*) and a phosphine compound (triphenylphosphine, tributylphosphine, trimethylphosphinem, *etc.*) at a temperature of 0 to 60°C.

The deprotection reaction of a protective group can be carried out in the same manner as described above.

[4] Among the compounds represented by formula (I-A) of the present invention, a compound wherein G represents a carbon atom and only one $R^2$ is bound at the 3-position of piperidin-2-one, i.e., a compound represented by formula (I-3):

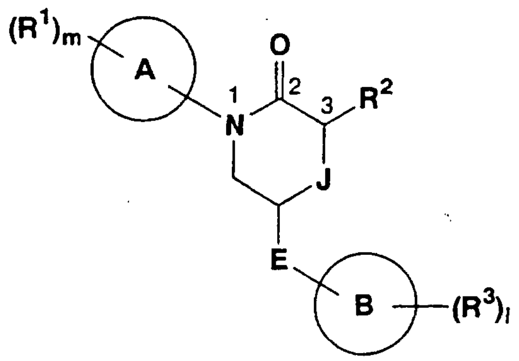

(I − 3)

(wherein all symbols have the same meanings as defined above) can be also produced by reacting a compound among the compounds (I-1) produced by the above-described method, in which n represents O, i.e., a compound represented by formula (I-1-4):

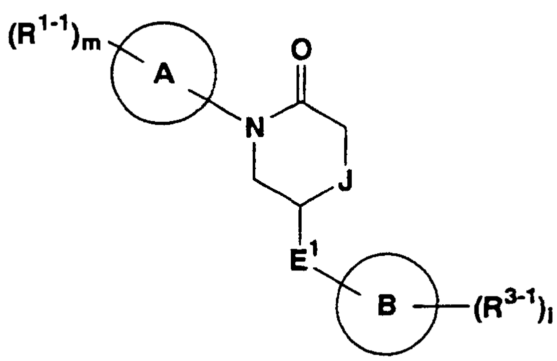

(I − 1 − 4)

(wherein all symbols have the same meanings as defined above) with a compound represented by formula (VI):

$$R^{2-1}\text{-}X \qquad \text{(VI)}$$

(wherein all symbols have the same meanings as defined above) to thereby give a compound represented by formula (I-4):

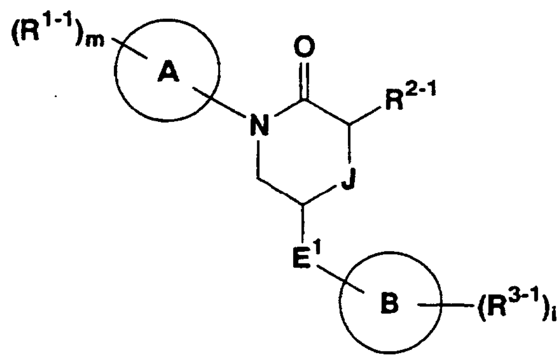

(I－4)

(wherein all symbols have the same meanings as defined above), followed by deprotection reaction of a protective group, if necessary.

The reaction of the compound represented by formula (I-1-4) with the compound represented by formula (VI) is known and can be carried out, for example, in an organic solvent (diethyl ether, tetrahydrofuran, *etc.*) in the presence of a base (lithium bis(trimethylsilyl)amide, potassium bis(trimethylsilyl)amide, lithium diisopropylamide, butyl lithium, *tert*-butoxy potassium, sodium methoxide, sodium ethoxide, sodium hydride, potassium hydride, *etc.*) at a temperature of -80 to 20°C.

The deprotection reaction of a protective group can be carried out in the same manner as described above.

[5] Among the compounds represented by formula (I-A) of the present invention, a compound wherein at least one of $R^1$ represents amino, i.e., a compound represented by formula (I-5):

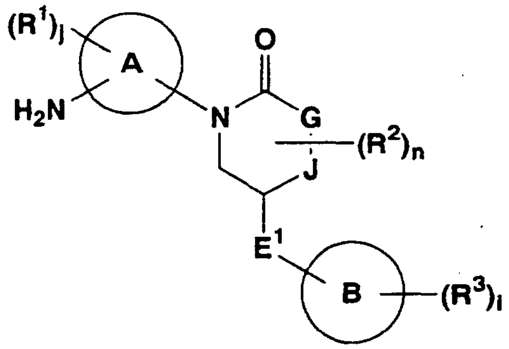

(I－5)

(wherein j represents 0 or an integer of 1, 2, 3, or 4 and other symbols have the same meanings as defined above) can also be produced by reducing a compound among the compounds (I-1) produced by the above-described method, in which at least one of $R^1$ represents nitro, i.e., a compound represented by formula (I-1-5):

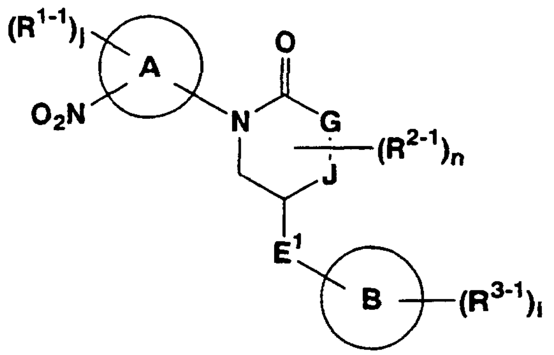

(I-1-5)

(wherein all symbols have the same meanings as defined above) to thereby give a compound represented by formula (1-6):

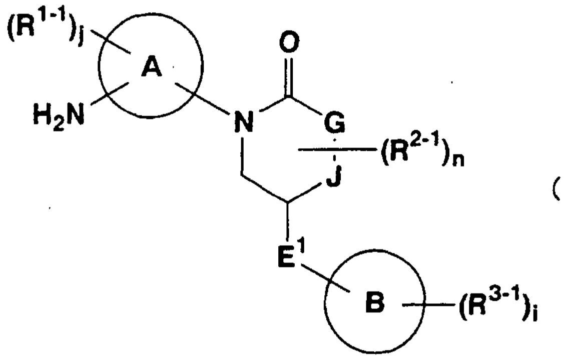

(I-6)

(wherein all symbols have the same meanings as defined above), followed by deprotection reaction of a protective group, if necessary.

The reduction reaction of nitro is known and carried out, for example, by a hydrogenation reaction and a reduction reaction using a metal.

The hydrogenation reaction is known and a deprotection reaction by hydrogenation is carried out, for example, in an inactive solvent [ethers (e.g., tetrahydrofuran, dioxane, dimethoxyethane, diethyl ether, *etc.*), alcohols (e.g., methanol, ethanol, *etc.*), benzenes (e.g., benzene, toluene, *etc.*), ketones (e.g., acetone, methylethylketone, *etc.*), nitriles (e.g., acetonitrile, *etc.*), amides (e.g., dimethylformamide, *etc.*), water, ethyl acetate, acetic acid or a mixture of at least two of them] in the presence of a hydrogenation catalyst (e.g., palladium-carbon, palladium black, palladium, palladium hydroxide, platinum dioxide, nickel, Raney nickel, ruthenium chloride, *etc.*) in the presence or in the absence of an inorganic acid (e.g., hydrochloric acid, sulfuric acid, hypochlorous acid, boric acid, tetrafluoroboric acid, *etc.*) or an organic acid (e.g., acetic acid, p-toluenesulfonic acid, oxalic acid, trifluoroacetic acid, formic acid, *etc.*) under the hydrogen atmosphere at normal pressure or under pressurization, or in the presence of ammonium formate at a temperature of 0 to 200°C. When an acid is used, a salt thereof may be used.

The reduction reaction using a metal is known and is carried out, for example, in a water-miscible solvent (ethanol, methanol, acetic acid, *etc.*) in the presence or in the absence of an aqueous hydrochloric acid solution using a metal (zinc, iron, tin, tin chloride, iron chloride, *etc.*) at a temperature of 50 to 150°C.

Among the compounds represented by formula (I-A) of the present invention, a compound wherein at least one of $R^3$ represents amino can be produced in the same manner.

[6] Among the compounds represented by formula (I-A) of the present invention, a compound wherein at least one of $R^1$ represents $-NR^5R^6$, $R^5$ represents C1-4 alkyl or C1-4 alkyl substituted with 1 to 3 of Cyc1 (wherein a carbon atom adjacent to a nitrogen atom is substituted with one Cycl) and $R^6$ represents a hydrogen atom, i.e., a compound represented by formula (I-7):

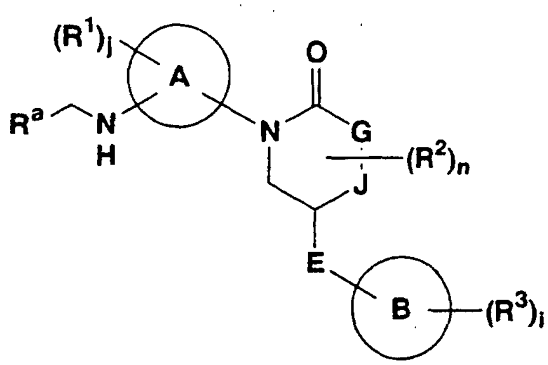

(I − 7)

(wherein $R^a$ represents a hydrogen atom, C1-3 alkyl, Cyc1 or C1-3 alkyl substituted with 1 to 3 of Cyc1 and other symbols have the same meanings as defined above) can also be produced by subjecting the compound represented by formula (I-6) produced in the above-described method and a compound represented by formula (VII):

$$R^{a-1}\text{-CHO} \qquad \text{(VII)}$$

(wherein $R^{a-1}$ has the same meaning as $R^a$, however, hydroxyl, amino, thiol or carboxyl contained in Cyc1 is protected when a protection is necessary, and other symbols have the same meanings as defined above) to a reductive amination reaction to thereby give a compound represented by formula (1-8):

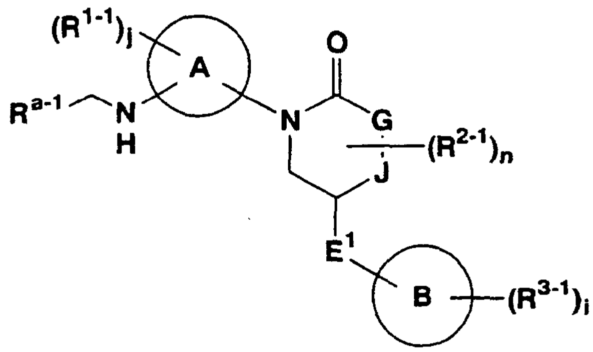

(I − 8)

(wherein all symbols have the same meanings as defined above), followed by deprotection reaction of a protective group, if necessary.

The reductive amination reaction may be carried out after isolating an imine generated from the compound represented by formula (I-6) and the compound represented by formula (VII), or an imine is generated in a reaction system and a reduction may be carried out (in one pot) without isolation.

The above-described imine generation reaction is known and is carried out, for example, in an organic solvent (e.g., methanol, ethanol, methylene chloride, chloroform, dichloroethane, benzene, toluene, *etc.*) in the presence or in the absence of a dehydrating agent (e.g., anhydrous magnesium sulfate, Molecular Sieve (proprietary name), *etc.*), in the presence or in the absence of an acid (e.g., hydrochloric acid, acetic acid, *etc.*) at a temperature of 20°C to the reflux temperature.

The above-described reduction reaction of imine is known and is carried out, for example, in an organic solvent (e.g., tetrahydrofuran, diethyl ether, dichloroethane, dichloromethane, dimethylformamide, acetic acid, methanol, ethanol, a mixture thereof, *etc.*) in the presence of a reducer (sodium triacetoxyborohydride, sodium cyanoborohydride, sodium borohydride, zinc borohydride, diisobutyl aluminum hydride, etc.) at a temperature of 0 to 40°C, or in a solvent (ethers (tetrahydrofuran, dioxane, dimethoxyethane, diethyl ether, *etc.*), alcohols (methanol, ethanol, *etc.*), benzenes (benzene, toluene, *etc.*), ketones (acetone, methylethylketone, *etc.*), nitriles (acetonitrile, *etc.*), amides (dimethylformamide, *etc.*), water, ethyl acetate, acetic acid, a mixture of at least two of them, *etc.*) in the presence of a catalyst (palladium-carbon, palladium black, palladium hydroxide, platinum dioxide, Raney nickel, *etc.*) under the hydrogen atmosphere at normal pressure or under pressurization at a temperature of 0 to 200°C.

The above-described reductive amination reaction is known and is carried out, for example, in an organic solvent (e.g., dichloroethane, dichloromethane, dimethylformamide, acetic acid or a mixture thereof) in the presence of a reducer (sodium triacetoxyborohydride, sodium cyanoborohydride or sodium borohydride) at a temperature of 0 to 40°C.

The deprotection reaction of a protective group can be carried out in the same manner as described above.

[7] Among the compounds represented by formula (I-A) of the present invention, a compound wherein at least one of $R^1$ represents $-NR^7COR^8$ and $R^7$ represents hydrogen, i.e., a compound represented by formula (I-9):

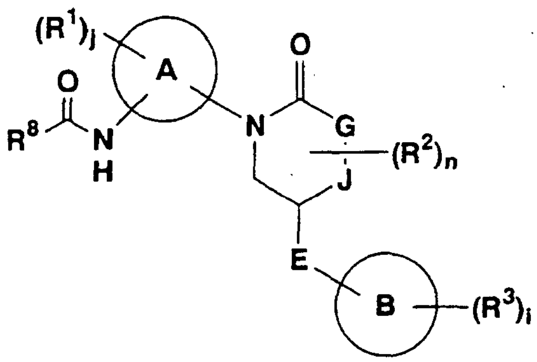

(I − 9)

(wherein all symbols have the same meanings as defined above) can also be produced by subjecting the compound represented by formula (I-6) produced by the above-described method and a compound represented by formula (VIII):

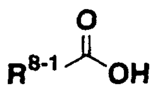

(VIII)

(wherein $R^{8-1}$ has the same meaning as $R^8$, however, hydroxyl, amino, thiol or a carboxyl contained in the group represented by $R^{8-1}$ is protected when a protection is necessary) to an amidation reaction to thereby give a compound represented by formula (1-10):

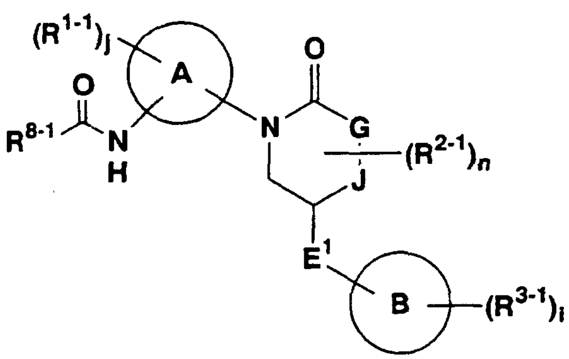

(I − 1 0)

(wherein all symbols have the same meanings as defined above), followed by deprotection reaction of a protective group, if necessary.

The above-described amidation reaction is known and it includes, for example,

(1) a method using an acid halide,
(2) a method using a mixed acid anhydride
(3) a method using a condensing agent

and the like.

These methods are described specifically as follows.

(1) The method using an acid halide is carried out, for example, by reacting a carboxylic acid in an organic solvent (chloroform, methylene chloride, diethyl ether, tetrahydrofuran, etc.) or without a solvent, with an acid halogenation agent (oxalyl chloride, thionyl chloride, *etc.*) at a temperature of -20°C to the reflux temperature, and by reacting the obtained acid halide in the presence of a tertiary amine (pyridine, triethylamine, dimethylaniline, dimethylaminopyridine, *etc.*) in an amine and an inactive organic solvent (chloroform, methylene chloride, diethyl ether, tetrahydrofuran, *etc.*) at a temperature of 0 to 40°C. Further, this method can also be carried out in an organic solvent (dioxane, tetrahydrofuran, *etc.*) using an aqueous alkaline solution (aqueous sodium bicarbonate solution, sodium hydroxide solution, *etc.*) with an acid halide at a temperature of 0 to 40°C.

(2) The method using a mixed acid anhydride is carried out, for example, by reacting a carboxylic acid in an organic solvent (chloroform, methylene chloride, diethyl ether, tetrahydrofuran, *etc.*) or without a solvent in the presence of a tertiary amine (pyridine, triethylamine, dimethylaniline, dimetylaminopyridine, *etc.*) with an acid halide (pivaloyl chloride, tosyl chloride, mesyl chloride, *etc.*) or an acid derivative (ethyl chloroformate, isobutyl chloroformate, *etc.*) at a temperature of 0 to 40°C, and reacting the obtained mixed acid anhydride in an organic solvent (chloroform, methylene chloride, diethyl ether, tetrahydrofuran, *etc.*) with an amine at a temperature of 0 to 40°C.

(3) The method using a condensing agent is carried out, for example, by reacting a carboxylic acid and an amine in an organic solvent (chloroform, methylene chloride, dimethylformamide, diethyl ether, tetrahydrofuran, *etc.*) or without a solvent in the presence or in the absence of a tertiary amine (pyridine, triethylamine, dimethylaniline, dimetylaminopyridine, *etc.*) using a condensing agent (1,3-dicyclohexylcarbodiimide (DCC), 1-ethyl-3-[3-(demethylamino)propyl]carbodiimide (EDC), 1-1'-carbonyldiimidazole (CDI), 2-chloro-1-methylpyridinium iodine, 1-propylphosphonic acid cyclic anhydride (PPA), *etc.*) with or without 1-hydroxylbenztriazole (HOBt) at a temperature of 0 to 40°C.

These (1), (2) and (3) reactions are preferably carried out under the inactive gas (argon, nitrogen, *etc.*) atmosphere and anhydrous conditions.

The deprotection reaction of a protective group can be carried out in the same manner as described above.

[8] Among the compounds represented by formula (I-A) of the present invention, a compound wherein at least one of $R^1$ represents $-NR^7COR^8$ and $R^7$ does not represent a hydrogen atom, i.e., a compound represented by formula (I-11):

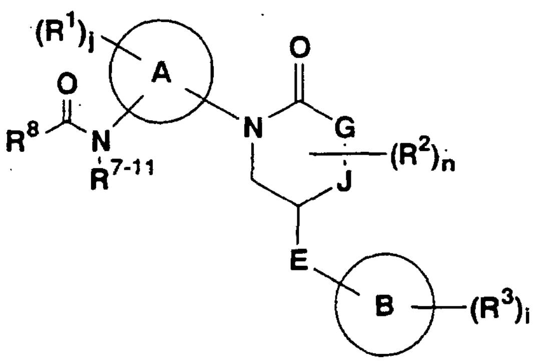

(I-11)

(wherein $R^{7-11}$ has the same meaning as $R^7$, however, $R^{7-11}$ does not represent a hydrogen atom and other symbols have the same meanings as defined above) can also be produced by reacting the compound represented by formula (I-10) and a compound represented by formula (IX):

$$R^{7-11-1}\text{-}X \qquad \text{(IX)}$$

(wherein $R^{7-11-1}$ has the same meaning as $R^{7-11}$, however, hydroxyl, an amino, a thiol or a carboxyl contained in a group represented by $R^{7-11-1}$ is protected when a protection is necessary) to thereby give a compound represented by formula (I-12):

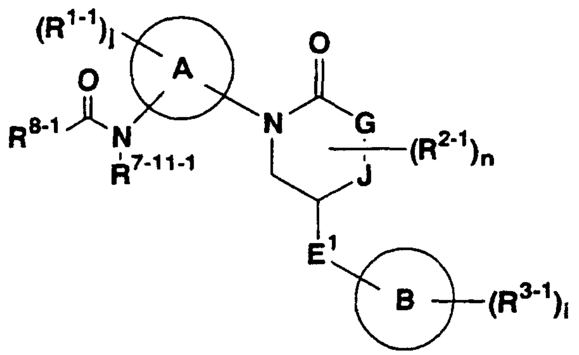

(I-12)

(wherein all symbols have the same meanings as defined above), followed by deprotection reaction of a protective group, if necessary.

The reaction of the compound represented by formula (I-11) with the compound represented by formula (IX) is known and can be carried out, for example, in an organic solvent (diethyl ether, tetrahydrofuran, N,N-dimethylformamide, *etc.*) in the presence of a base (sodium hydride, potassium hydride, *etc.*) at a temperature of -20 to 60°C.

The deprotection reaction of a protective group can be carried out in the same manner as described above.

[9] Among the compounds represented by formula (I-A) of the present invention, a compound wherein $E^2$ represents $-NR^{21}$, i.e., a compound represented by formula (I-13):

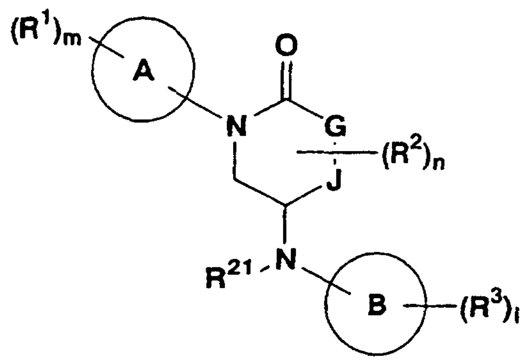

(I-13)

(wherein all symbols have the same meanings as defined above) can also be produced by subjecting a compound represented by formula (II-13):

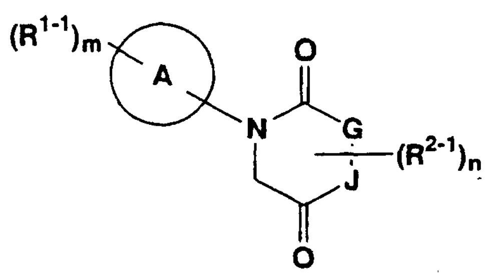

(II-13)

(wherein all symbols have the same meanings as defined above), and a compound represented by formula (III-13):

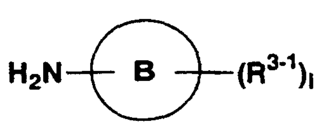

(III-13)

(wherein all symbols have the same meanings as defined above) to a reductive amination reaction to thereby give a

compound represented by formula (1-13'):

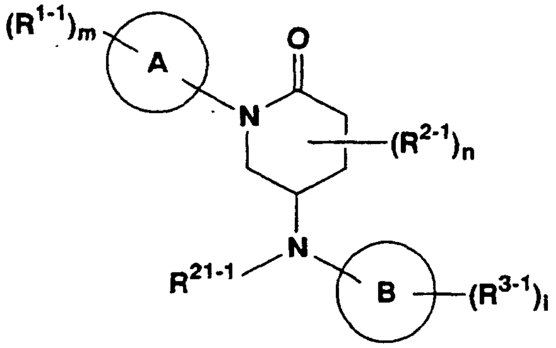

(I-13')

(wherein $R^{21-1}$ has the same meaning as $R^{21}$, however, hydroxyl, amino, thiol or carboxyl contained in the group represented by $R^{21}$ is protected when a protection is necessary, and other symbols have the same meanings as defined above), followed by deprotection reaction of a protective group, if necessary.

The reductive amination reaction and the deprotection reaction of a protective group can be carried out in the same manner as described above.

[10] Among the compounds represented by formula (I-A) of the present invention, a compound wherein $E^2$ represents $-NR^{79}SO_2-$ i.e., a compound represented by formula (I-14):

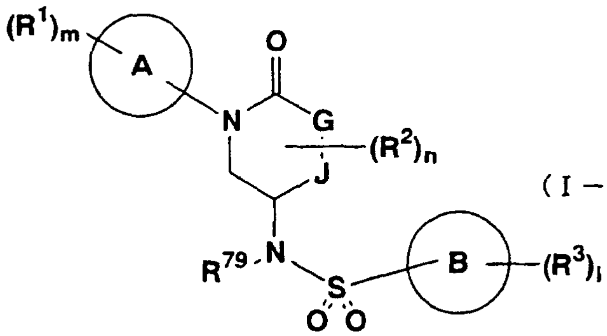

(I-14)

(wherein all symbols have the same meanings as defined above) can also be produced by subjecting a compound represented by formula (II-13):

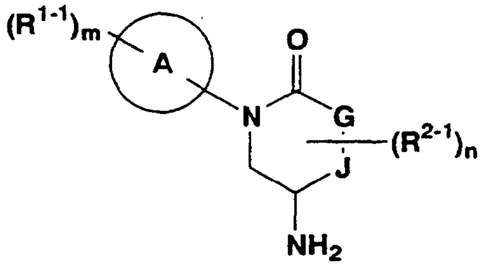

(II-14)

(wherein all symbols have the same meanings as defined above), and a compound represented by formula (III-13):

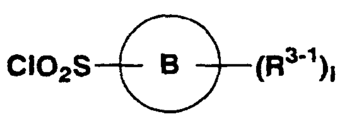

(III-14)

(wherein all symbols have the same meanings as defined above) to a sulfonamidation reaction to thereby give a compound represented by formula (I-14'):

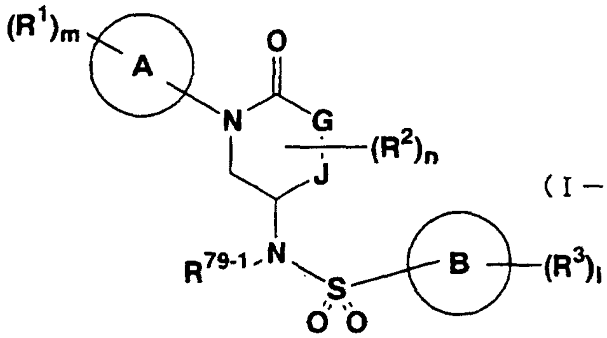

(I-14')

(wherein $R^{79-1}$ has the same meaning as $R^{21}$, however, hydroxyl, amino, thiol or carboxyl contained in the group represented by $R^{79}$ is protected when a protection is necessary, and other symbols have the same meanings as defined above), followed by deprotection reaction of a protective group, if necessary.

The sulfonamidation reaction is known and is carried out, for example, by reacting a sulfonyl halide with an amine in the presence of a base (diisopropylethylamine, pyridine, triethylamine, dimethylaniline, dimethylaminopyridine, *etc.*) in an organic solvent (chloroform, dichloromethane, dichloroethane, diethyl ether, tetrahydrofuran, *etc.*) at a temperature of 0 to 40°C.

The deprotection reaction of a protective group can be carried out in the same manner as described above.

[11] The compound represented by formula (I-A) of the present invention can also be produced by reacting a compound represented by formula (II-15-1):

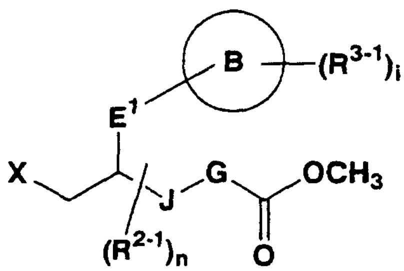

(II-15-1)

(wherein X represents a leaving group (e.g., chlorine atom, bromine atom, iodine atom, tosyl, mesyl) and other symbols have the same meanings as defined above) or a compound represented by formula (II-15-2):

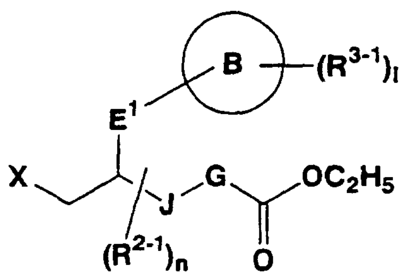

(II-15-2)

(wherein X represents a leaving group (e.g., chlorine atom, bromine atom, iodine atom, tosyl, mesyl) and other symbols have the same meanings as defined above) with a compound represented by formula (III-15):

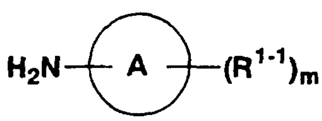

(III-15)

(wherein all symbols have the same meanings as defined above), followed by cyclization to thereby give a compound represented by formula (1-15'):

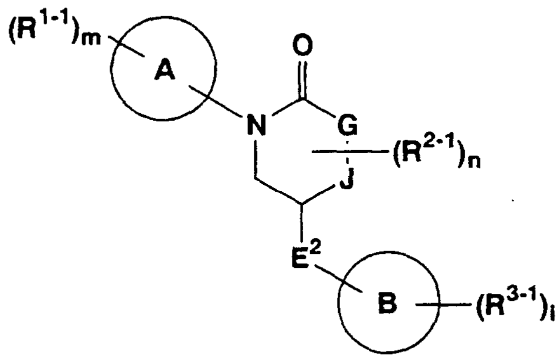

(I −15')

(wherein all symbols have the same meanings as defined above), followed by deprotection reaction of a protective group, if necessary.

This reaction is known and carried out in an organic solvent (benzene, toluene, xylene, *etc.*) with an amine at a temperature of 20 to 150°C.

The deprotection reaction of a protective group can be carried out in the same manner as described above.

[B] Among the compounds represented by formula (I) of the present invention a compound wherein ---- represents a double bond, i.e., a compound represented by formula (I-B):

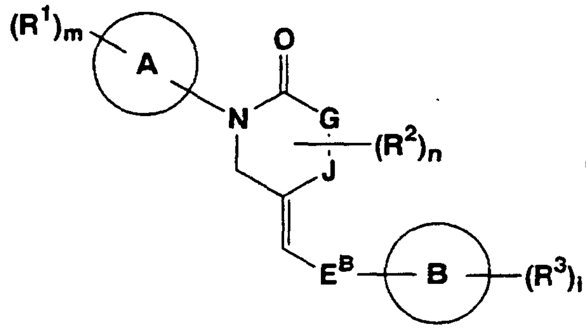

(I-B)

(wherein $E^B$ represents C1-3 alkylene and other symbols have the same meanings as defined above) can be produced by subjecting a compound produced by the above-described method, i.e., a compound represented by formula (I-16):

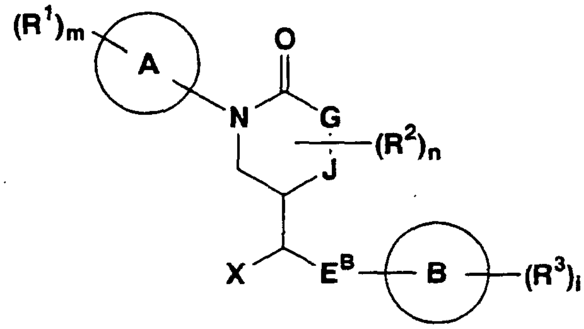

(I-16)

(wherein X represents a leaving group (e.g., chlorine atom, bromine atom, iodine atom, tosyl, mesyl) and other symbols have the same meanings as defined above) to an elimination reaction to thereby give a compound represented by formula (I-B'):

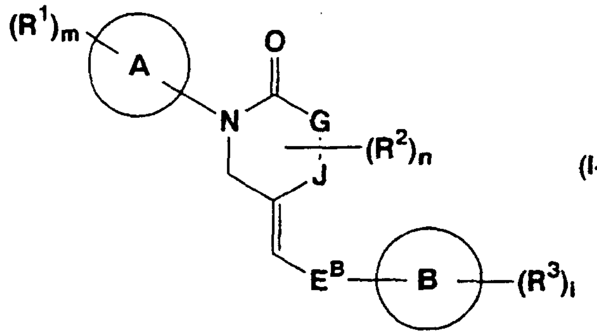

(I-B')

(wherein all symbols have the same meanings as defined above), followed by deprotection reaction of a protective group, if necessary.

The elimination reaction is known and carried out by a reaction in an organic solvent (methanol, ethanol, *etc.*) with a base (sodium hydroxide, potassium hydroxide or an aqueous solution thereof, *etc.*) at a temperature of 0 to 40°C.

The deprotection reaction of a protective group can be carried out in the same manner as described above.

The compounds represented by formulae (II) to (IX) are either known per se or can be produced easily by a known method.

For example, the compounds represented by formulae (II) and (III) can be produced by the method described in the following reaction process 1.

In the reaction process, Et represents ethyl and other symbols have the same meanings as defined above.

Reaction Process 1

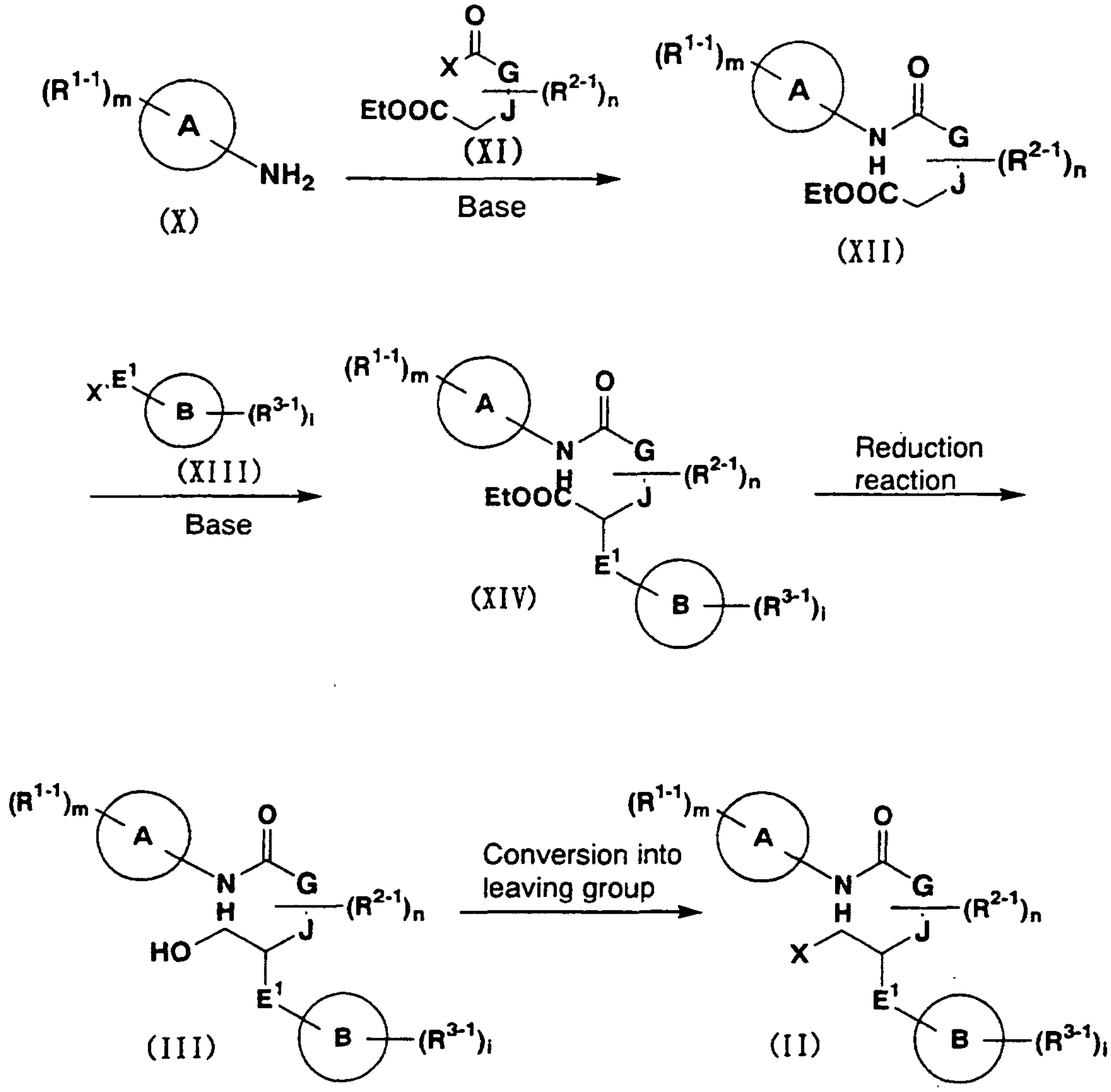


Each reaction in the above reaction process is carried out by a known method. In the reaction process, the compounds represented by formulae (X), (XI) and (XIII) used as the starting materials are either known or can be produced easily by a known method.

In each reaction in this description, a reaction product can be purified by a general purification method, for example, distillation under normal or reduced pressure, high speed liquid chromatography using silica gel or magnesium silicate, thin layer chromatography or column chromatography, washing, re-crystallization or the like. Purification may be carried out at each reaction or after completion of several reactions.

Other staring materials and each reagent of the present invention are either known *per se* or can be produced easily by a known method.

[Brief Description of the Drawing]

Fig. 1 is a photograph showing the suppression of ATF-2 phosphorylation by p38αMAP kinase by the compound of the present invention produced in Example 1 (1).

[Pharmacological activity of compound of the present invention]

It was demonstrated that the compound of the present invention has p38αMAP kinase inhibition activity by the following experiments.

(1) Study of p38αMAP kinase inhibitory activity (1)

Using activation transcription factor 2 (activating transcription factor 2; ATF-2, Cell Signaling Inc., #9224L) which is a substrate of p38αMAP kinase, the inhibitory effect of the compound of the present invention on the ATF-2 phosphorylation by recombinant human p38αMAP kinase (Upstate Biotechnology Inc., #14-251) was studied by the Western-blotting method using the anti-phosphorylated ATF-2 antibody (Cell Signaling Inc., #9221L). In other words, 10 μL of a solution of the compound of the present invention at a known concentration was added to 10 μL of the kinase buffer (Cell Signaling Inc., #9802) containing recombinant human p38αMAP kinase (100 ng/tube) and pre-incubated for 10 minutes at 30°C. Then, 20 μL of adenosine triphosphate (ATP)/ATF-2 mixture was added and after the incubation of 30 minutes at 30°C, 20 μL of SDS buffer (187.5 mM Tris / 6% SDS / 30% glycerol / 150 mM DTT / 0.03% bromophenol blue) was added to stop the enzyme reaction. After heating at 100°C for 5 minutes, mixing and centrifugation were conducted. After re-mixing, 20 μL of the sample was subjected to an electrophoresis on SDS-PAGE gel (10-20%, Daiichi Pure Chemicals Co., Ltd.). After the electrophoresis, blotting was conducted on PVDF membrane (Sequi-Blot (proprietary name), 0.2 μm, BIO-RAD) by a conventional method. After that, the PVDF membrane was treated with Block Ace (Snow Brand Milk Product Co., Ltd.) (at room temperature, for 1 hour). After reacted with the anti-phosphorylated ATF-2 antibody for 1.5 hours, it was washed with TBS-T solution (0.02 M Tris / 0.137 M NaCl / 0.05% Tween 20, pH 7.6). Furthermore, the reaction with a secondary antibody (anti-rabbit IgG, horseradish peroxide linked whole antibody, Amersham LIFE SCIENCE) was conducted for 1 hour. After washing with TBS-T solution, phosphorylated ATF-2 was detected using Western blotting detection reagent (Amersham Pharmacia Biotech).
Fig. 1 shows the result using the compound of the present invention produced in Example 1 (1).
As is shown in Fig. 1, the compound of the present invention produced in Example 1 (1) inhibited the ATF-2 phosphorylation by p38αMAP kinase at the concentration of 0.3 μM or more. Further, other compounds of the present invention inhibited the ATF-2 phosphorylation by p38αMAP kinase at the concentration of 1 μM or more.

(2) Study of p38αMAP kinase inhibitory activity (2)

Using activation transcription factor 2 (hereinafter abbreviated as ATF-2) which is a substrate of p38αMAP kinase, the inhibition effect of the compound of the present invention on the ATF-2 phosphorylation by recombinant human p38αMAP kinase was examined.

Experimental method:

A kinase buffer (25 mM Tris-HCl (pH 7.5), 5 mM β-glycerophosphate, 2 mM dithiothreitol, 0.1 mM $Na_3VO_4$, 10 mM $MgCl_2$) containing recombinant human p38αMAP kinase (Upstate Biotechnology #14-251) (5 μL) was added to a 384-well plate for fluorescence measurement (6.25 μg protein/well). Furthermore, a kinase solution containing the compound of the present invention (5 μL) was added and incubated at room temperature for 20 minutes. Separately, 5 μL of a substrate mixture prepared with the kinase buffer (biotinylated ATF-2 (5μg/mL) (Upstate Biotechnology, #14-432), adenosine triphosphate (90 μmol/L) (Sigma #FL-AAS) and anti-phosphorylated ATF-2 antibody (20-fold dilution) (Cell Signaling Technology, #9221L) were added and an enzyme reaction was carried out at 30°C for 30 minutes. After completion of the reaction, the enzyme reaction was terminated by adding 5 μL of Hepes buffer containing 0.25% BSA and 100 mM EDTA. The amount of complex of phosphorylated ATF2 and anti-phosphorylated ATF2 generated by this reaction was measured using Alpha Screen™ Rabbit Detection kit (Packard #6760607).
The enzyme inhibitory activity against p38MAP kinase, which is the effect of the compound of the present invention, was calculated as an inhibition rate (%) by the following formula.

$$\text{Inhibition rate (\%)} = \{(A_C - A_X)/(Ac - A_B)\} \times 100$$

$A_B$: Measured value without enzyme addition
$A_C$: Measured value with enzyme addition in the absence of the compound
$A_X$: Measured value with enzyme addition in the presence of the compound

As a result, the compound of the present invention showed the $IC_{50}$ of 10 μM or less. For example, the $IC_{50}$ of the compound described in Example 1 (1) was 42.9 nM.

(3) Effect on mouse cytokine production

The compound of the present invention suspended in 0.5% methyl cellulose (MC) was orally administered to

a male Balb/c mouse (Charles River Japan, Inc.), and after 0.5 hour, lipopolysaccharide (LPS, 055:B5, Difco) was intraperitoneally administered at the dose of 1 mg/kg (5 animals /group). MC (0.5%) was orally administered to a control group (5 animals). Ninety minutes after the LPS treatment, heparinized blood collection was performed via the abdominal main vain under anesthesia with ether and blood plasma was obtained by centrifugation (12,000 rpm, 3 minutes, 4°C). The obtained blood plasma sample was stored at -80°C until it was used. TNF-α and IL-6 in the blood plasma were measured using ELISA kits from R&D Inc. (#MTA00) and Endogen Inc. (#EM2IL6), respectively.

From the result of the above measurement, the compound of the present invention was found to significantly suppress cytokine production.

(4) Inhibition activity against TNF-α production using human cell line

Using THP-1, which is a human monocyte cell line, the inhibition effect of the compound of the present invention on the TNF-α production induced by lipopolyliposaccharide (LPS) stimulation was studied.

Experimental method:

Each 50 µL of lipopolyliposaccharide (LPS; Difco #3120-25-0) prepared at the concentration of 40 ng/mL using RPMI-1640 medium containing 10% fetal calf serum (hereinafter abbreviated as RPMI-1640) and RPMI-1640 containing the compound of the present invention was added to a 96-well plate for tissue culture. A portion of 100 µL of the cell suspension solution of THP-1 (Dainippon Pharmaceutical Co., Ltd, #06-202) prepared at the cell density of $2x10^6$ cells/mL using RPMI-1640 was added and cultured for 90 minutes at 37°C in an incubator (5% $CO_2$, 95% Air). After completion of the reaction, the culture medium supernatant was recovered and the amount of produced TNF-α was measured using an ELISA kit (Invitrogen, #850090192).

The inhibition activity against TNF-α production, which is the effect of the compound of the present invention, was calculated as an inhibition rate (%) by the following formula.

$$\text{Inhibition rate (\%)} = \{(A_C - A_X)/(A_C - A_B)\} \times 100$$

$A_B$: Measured value without LPS induction
$A_C$: Measured value with LPS induction in the absence of the compound
$A_X$: Measured value with LPS induction in the presence of the compound

As a result, the compound of the present invention showed the $IC_{50}$ of 10 µM or less. For example, the $IC_{50}$ of the compound described in Example 1 (1) was 14.5 nM.

[Toxicity]

Toxicity of the compound represented by formula (I) of the present invention is sufficiently low, and it was confirmed to be safe enough for use as a pharmaceutical agent.

[Application for pharmaceuticals]

The compound represented by formula (I) of the present invention suppresses p38MAP kinase activation, therefore it is expected to be useful in the prevention and/or the treatment of various inflammatory diseases, rheumatoid arthritis, osteoarthritis, arthritis, osteoporosis, autoimmune diseases, infectious diseases, sepsis, cachexia, cerebral infarction, Alzheimer's disease, asthma, chronic pulmonary inflammatory diseases, reperfusion injury, thrombosis, glomerulonephritis, diabetes, graft versus host rejection, inflammatory bowel disease, Crohn's disease, ulcerative colitis, multiple sclerosis, tumor growth and metastasis, multiple myeloma, plasma cell leukemia, Castleman's disease, atrial myxoma, psoriasis, dermatitis, gout, adult respiratory distress syndrome (ARDS), arteriosclerosis, post-percutaneous transluminal coronary angioplasty restenosis and pancreatitis.

The compounds represented by formula (I) or the non-toxic salts thereof may be administered in combination with other drugs for the purpose of 1) complement and/or enhancement of preventing and/or treating effect, 2) improvement of dynamics and absorption of the compound, and lowering of dose, and/or 3) alleviation of side effect of the compound.

The compounds represented by formula (I) may be administered in combination with other drugs as a composition in one drug product comprising these components, or may be administered separately. When they are administered independently, they may be administered simultaneously or with time lag. Administering with time lag includes

the method of administering the compounds represented by formula (I) before other drugs and vice versa; they may be administered in the same route or not.

The above combination drugs takes effect on whichever disease preventing and/or treatment effect of the compound of formula (I) is complemented and/or enhanced.

The weight proportion of the compounds represented by formula (I) and the other drugs is not specifically limited.

Arbitrary two or more of the other drugs may be administered in combination.

Examples of the other drugs for compensating for and/or enhancing the preventive and/or treatment effect of the compounds represented by formula (I) include not only those which have so far been found but also those which will be found on the basis of the aforementioned mechanism.

Other agents to complement and/or enhance a prevention and/or a treatment effect of the compound represented by formula (I) on rheumatoid arthritis, osteoarthritis, arthritis or the like include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, an anti-TNF-α agent, an immunosuppressing agent, a disease modifying anti-rheumatic agent, a non-steroidal anti-inflammatory agent and the like.

Other agents to complement and/or enhance prevention and/or treatment effect of the compound represented by formula (I) on inflammatory bowel disease, Crohn's disease or ulcerative colitis include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, an anti-TNF-α agent, an immunosuppressing agent, a leukotoriene receptor antagonist, an anti-choline agent, a 5-lipoxygenase inhibitor, a nitric monooxide synthase inhibitor, an interleukin 8 antagonist, a poly(ADP)-ribose polymerase inhibitor, a mitochondrial benzodiazepine receptor agonist, an anti-oxidation agent, a topical anesthetic, an agent for digestive tract ulcer, a defense factor enhancing agent, mesalazine, salazosulfapyridine and the like.

Other agents to complement and/or enhance prevention and/or treatment effect of the compound represented by formula (I) on asthma, chronic pulmonary inflammatory diseases or adult respiratory distress syndrome (ARDS) include a steroidal agent, an elastase inhibitor, a cannabinoid-2 receptor stimulating agent, a prostaglandin, a prostaglandin synthase inhibitor, a phosphodiesterase inhibitor, a metalloproteinase inhibitor, an adhesion molecule inhibitor, a leukotoriene receptor antagonist, an anti-choline agent, a thromboxane A2 receptor antagonist, a thromboxane synthase inhibitor, a β2 adrenaline receptor stimulating agent, a xanthine derivative, an expectorant agent, an antibiotic, an anti-histamine agent, a cytokine inhibitor, a forskolin agent, a mediator release inhibitor and the like.

The steroidal agent includes clobetasol propionate, diflorasone diacetate, fluocinonide, mometasone furancarboxylate, betamethasone dipropionate, betamethasone butyrate propionate, betamethasone valerate, difluprednate, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclometasone dipropionate, triamcinolone acetonide, flumetasone pivalate, alclometasone dipropionate, clobetasone butyrate, prednisolone, fludroxycortide, cortisone acetate, hydrocortisone, hydrocortisone sodium phosphate, hydrocortisone sodium succinate, fludrocortisone acetate, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, dexamethasone sodium phosphate, dexamethasone palmitate, paramethasone acetate, betamethasone, fluticasone propionate, budesonide, flunisolide, ST-126P, ciclesonide, dexamethasone palmitate, mometasone furancarbonate, prasterone sulfonate, deflazacort, methylprednisolone suleputanate, methylprednisolone sodium succinate and the like.

The elastase inhibitor includes ONO-5046, ONO-6818, MR-889, PBI-1101, EPI-HNE-4, R-665, ZD-0892, ZD-8321, GW-311616, DMP-777, L-659286, L-658758, L-680833, L-683845, AE-3763 and the like.

The prostaglandin (hereinafter referred to as "PG") includes a PG receptor agonist, a PG receptor antagonist and the like.

The PG receptor includes a PGE receptor (EP1, EP2, EP3, EP4), a PGD receptor (DP, CRTH2), a PGF receptor (FP) a PGI receptor (IP), a TX receptor (TP) and the like.

The prostaglandin synthase inhibitor includes salazosulfapyridine, mesalazine, osalazine, 4-amino salicylic acid, JTE-522, auranofin, carprofen, difenpiramide, flunoxaprofen, flurbiprofen, indometacin, ketoprofen, lomoxicam, loxoprofen, meloxicam, oxaprozin, parsalmide, piproxen, piroxicam, piroxicam betadex, piroxicam cinnamate, tropine indometacinate, zaltoprofen, pranoprofen and the like.

The phosphodiesterase inhibitor includes a PDE 4 inhibitor such as rolipram, cilomilast (Proprietary name: Ariflo), Bay19-8004, NIK-616, roflumilast (BY-217), cipamfylline (BRL-61063), atizoram (CP-80633), SCH-351591, YM-976, V-11294A, PD-168787, D-4396 or IC-485, and a PDE 5 inhibitor such as sildenafil.

The adhesion molecule inhibitor includes an antagonist such as α4 integrin, and the like.

The anti-TNF-α agent includes an antibody against TNF-α, a soluble TNF-α receptor, an antibody against a

TNF-α receptor and the like, and the anti-TNF-α agent includes infliximab, etanercept and the like.

The immunosuppressing agent includes methotrexate, cyclosporin, ascomycin, leflunomide, bucillamine, salazosulfapyridine, azathioprine, tacrolimus, cyclophosphamide and the like.

The disease modifying anti-rheumatic agent includes aurothioglucose, sodium aurothiomalate, auranofin, actarit, D-penicillamine preparation, lobenzarit disodium, bucillamine, hydroxychloroquine, salazosulfapyridine and the like.

The non-steroidal anti-inflammatory agent includes sasapyrine, sodium salicylic acid, aspirin, aspirin dialuminate combinations, diflunisal, indomethacin, suprofen, ufenamate, dimethyl-isopropyl-azulene, bufexamac, felbinac, diclofenac, tolmetin sodium, clinoril, fenbufen, napumetone, proglumetacin, indomethacin farnesil, acemetacin, proglumetacin maleate, amfenac sodium, mofezolac, etodolac, ibuprofen, ibuprofen piconol, naproxen, flurbiprofen, flurbiprofen axetil, ketoprofen, fenoprofen calcium, tiaprofen, oxaprozin, pranoprofen, loxoprofen sodium, alminoprofen, zaltoprofen, mefenamic acid, aluminum mefenamaic acid, tolfenamic acid, floctafenine, ketophenylbutazone, oxyphenbutazone, pyroxicam, tenoxicam, ampiroxicam, Napageln ointment, epirizole, tiaramide hydrochloride, tinoridine hydrochloride, emorfazone, sulpyrine, migrenin, Saridon, Sedes G, Amipylo N, sorbone, pilin derivatives for cough and cold preparations, acetaminophen, phenacetin, dimetotiazine mesilate, simetride combinations, non-pilin derivatives for cough and cold preparations and the like.

The leukotoriene receptor antagonist includes pranlukast hydrate, montelukast, zafirlukast, seratrodast, MCC-847, KCA-757, CS-615, YM-158, L-740515, CP-195494, LM-1484, RS-635, A-93178, S-36496, BIIL-284, ONO-4057 and the like.

The anti-choline agent includes ipratropium bromide, oxitropium bromide, flutropium bromide, cimetropium bromide, temiverine, thiotropium bromide, revatropate (UK-112166) and the like.

The topical anesthetic includes cocaine hydrochloride, procaine hydrochloride, lidocaine, dibucaine hydrochloride, tetracaine hydrochloride and the like.

The defense factor enhancing agent includes sucralfate, aldioxa, teprenone, cetraxate hydrochloride, omoprostil and the like.

The thromboxane A2 receptor antagonist includes seratrodast, ramatroban, domitroban calcium hydrate, KT-2-962 and the like.

The thromboxane synthase inhibitor includes ozagrel hydrochloride, imitrodast sodium and the like.

The β2 adrenaline receptor stimulating agent includes fenoterol hydrobromide, salbutamol sulfate, terbutaline sulfate, formoterol fumarate, salmeterol xinafoate, isoproterenol sulfate, orciprenaline sulfate, chlorprenaline sulfate, epinephrine, trimetoquinol hydrochloride, hexoprenalinemesyl sulfate, procaterol hydrochloride, tulobuterol hydrochloride, tulobuterol, pirbuterol hydrochloride, clenbuterol hydrochloride, mabuterol hydrochloride, ritodrine hydrochloride, bambuterol, dopexamine hydrochloride, meruadrine tartrate, AR-C68397, levosalbutamol, R,R-formoterol, KUR-1246, KUL-7211, AR-C89855, S-1319 and the like.

The xanthine derivative includes aminophylline, theophylline, doxofylline, sipamphylline, diprophylline and the like.

The expectorant agent includes foeniculated ammonia spirit, sodium hydrogen carbonate, bromhexine hydrochloride, carbocysteine, ambroxol hydrochloride, ambroxol hydrochloride sustained preparation, methylcysteine hydrochloride, acetylcysteine, ethyl L-cysteine hydrochloride, tyloxapol and the like.

The antibiotic includes sodium cefuroxime, meropenem trihydrate, netilmicin sulfate, sisomicin sulfate, ceftibuten, PA-1806, IB-367, tobramycin, PA-1420, doxorubicin, astromicin sulfate, cefetamet pivoxil hydrochloride and the like. As the antibiotic for an inhalant, for example, PA-1806, IB-367, tobramycin, PA-1420, doxorubicin, astromicin sulfate, cefetamet pivoxil hydrochloride and the like.

The anti-histamine agent includes ketotifen fumarate, mequitazine, azelastine hydrochloride, oxatomide, terfenadine, emedastine difumarate, epinastine hydrochloride, astemizole, ebastine, cetirizine hydrochloride, bepotastine, fexofenadine, loratadine, desloratadine, olopatadine hydrochloride, TAK-427, ZCR-2060, NIP-530, mometasone furoate, mizolastine, BP-294, andolast, auranofin, acrivastine and the like.

The cytokine inhibitor includes suplatast tosylate (proprietary name: IPD) and the like.

The mediator release inhibitor includes tranilast, sodium cromoglicate, amlexanox, repirinast, ibudilast, dazanolast, pemirolast potassium and the like.

In order to use the compounds of the present invention represented by formula (I) or the non-toxic salts thereof, these compounds are normally administered to the entire or local part of human body orally or parenterally.

The dose of these compounds depends on the age, weight and symptom of the patient, the remedial value, the administration method, the treatment time, *etc.* In practice, however, these compounds are administered orally once or several times per day each in an amount of from 1 μg to 100 mg per adult, parenterally once or several times per day each in an amount of from 0.1 μg to 10 mg per adult or continuously administered into vein for 1 hour to 24 hours per day.

It goes without saying that the dose of these compounds may be less than the aforementioned value or may

need to exceed the aforementioned range because the dose varies under various conditions as mentioned above.

The compound of the present invention may be administered in the composition of, for example, solid compositions, liquid compositions or other compositions each for oral administration, or injections, liniments or suppositories, each for parenteral administration.

Solid compositions for oral administration include compressed tablets, pills, capsules, powders and granules.

Capsules include hard capsules and soft capsules.

In such solid compositions, one or more of the active substance(s) may be used in combination with at least one diluting agent such as lactose, mannitol, mannnit, glucose, hydroxypropylcellulose, microcrystallite cellulose, starch, polyvinylpyrrolidone, magnesium aluminometasilicate, *etc.*

Solid compositions may comprise other additives by the law of the art, for example, lubricants (e.g. magnesium stearate *etc.*), disintegrants (e.g. cellulose calcium glycolate *etc.*), solubilizing agent (e.g. glutamic acid, aspartic acid, *etc.*), *etc.* in addition of diluting agent. If necessary, compressed tablets or pills may be coated with a gastric or enteric film (e.g. sucrose, gelatin, hydroxypropyl cellulose, hydroxypropyl cellulose phthalate, *etc.*), or with two or more layers. Furthermore, capsules made of a substance which can be absorbed in the body, for example, gelatin, are included.

Liquid compositions for oral administration include pharmaceutically acceptable emulsions, solutions, syrups, elixirs, *etc.* In such liquid compositions, one or more of the active substance(s) may be solved, suspended or emulsified in generally used inert diluent(s) (e.g. purified water, ethanol, *etc.*). The compositions may comprise, in addition to the inert diluent, humectants, suspending agents, emulsifying agent, sweetening agents, flavoring agents, aromatic agents and preservatives.

The other compositions for oral administration include sprays which comprise one or more of the active substance(s) and may be prepared by methods known per se. Sprays may comprise in addition to a generally used diluent, a stabilizer such as sodium bisulfite and an isotonization buffer such as sodium chloride, sodium citrate or citric acid. The preparation process of sprays is described in detail in, for example, U.S. Patent No. 2,868,691 and 3,095,355.

Injections for parenteral administration include sterile aqueous or nonaqueous solutions, suspensions and emulsions. Solvent(s) for aqueous solutions or suspentions may include, for example, distilled water for injection, physiological salt solution. Solvent(s) for nonaqueous solutions or suspentions may include, for example, propylene glycol, polyethylene glycol, vegetable oil (e.g. olive oil *etc.*), alcohol (e.g. ethanol *etc.*), POLYSORBATE80 (registered trade mark), *etc.*

Moreover, these injections may comprise some additives, such as presertives, humectants, emulsifying agents, dispersing agents, stabilizing agents, solution adjuvants (e.g. glutamic acid, aspartic acid, *etc.*). They may be sterilized by filtering through bacteria removal filter, blending of bactericidal substance, or irradiation. They may also be manufactured in the form of sterile solid forms which may be dissolved in sterile water or some other sterile diluent (s) for injection immediately before use.

The other compositions for parenteral administration include liquid compositions for external use, ointments, embrocations, suppositories for rectal administration and pessaries for vaginal administration, *etc.* which comprise one or more of the active substance(s) and may be prepared by methods known per se.

## BEST MODE FOR CARRYING OUT THE INVENTION

The present invention is explained below in detail based on Reference Examples and Examples, however, the present invention is not limited thereto.

The solvents in the parentheses show the developing or eluting solvents and the ratios of the solvents used are by volume in chromatographic separations or TLC. The solvents in the parentheses in NMR show the solvents for measurement.

Reference Example 1

ethyl 4-[N-(2,6-dichlorophenyl)carbamoyl]butanoate

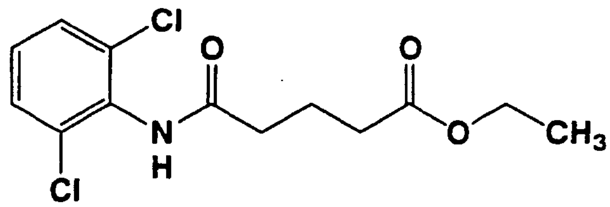

Under an atmosphere of argon, to a solution of 2,6-dichloroaniline (5.96 g) in N,N-dimethylformamide (50 ml) was added ethyl glutaryl chloride (9.87 g) in ice bath and the mixture was stirred at room temperature for 5 hours. The reaction mixture was poured in water and extracted with ethyl acetate. The organic layer was washed with a saturated aqueous sodium hydrogen carbonate solution, water and brine sequentially, and dried over anhydrous magnesium sulfate. The solvent was evaporated and the obtained residue was washed with isopropyl ether to give the title compound (7.67 g) having the following physical data.
TLC:Rf 0.41 (ethyl acetate:hexane=2:3);
NMR ($CDCl_3$):d 7.37 (d, J = 7.8 Hz, 2H), 7.23-7.05 (m, 2H), 4.15 (q, J = 7.2 Hz, 2H), 2.60-2.42 (m, 4H), 2.18-2.00 (m, 2H), 1.27 (t, J = 7.2 Hz, 3H).

Reference Example 2

ethyl 4-[N-(2,6-dichlorophenyl)carbamoyl]-2-benzylbutanoate

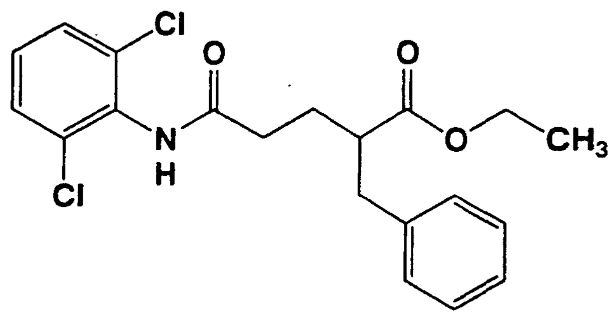

Under an atmosphere of argon, a solution of the compound prepared in Reference Example 1 (909mg) in anhydrous tetrahydrofuran (20ml) was cooled to -78°C, and thereto was dropped lithium diisopropylamide (1.5M solution in cyclohexane, 4.4ml). The mixture was stirred for 30 minutes and thereto was added benzyl bromide (0.39 ml). The mixture was stirred at -60°C for 1 hour. A saturated aqueous ammonium chloride solution was added to the reaction mixture, which was extracted with ethyl acetate. The organic layer was washed with water and brine and dried over anhydrous magnesium sulfate. The solvent was evaporated and the obtained residue was purified by column chromatography on silica gel (ethyl acetate:hexane=1:4→3:7) to give the title compound (188 mg) having the following physical data.
TLC:Rf 0.53 (ethyl acetate:hexane=2:3);
NMR ($CDCl_3$):d 7.37 (d, J = 8.1 Hz, 2H), 7.30-7.13 (m, 6H), 7.07 (s, 1H), 4.10 (q, J = 7.2 Hz, 2H), 3.04-2.77 (m, 3H), 2.60-2.30 (m, 2H), 2.11-1.97 (m, 2H), 1.17 (t, J = 7.2 Hz, 3H).

Reference Example 3

4-[N-(2,6-dichlorophenyl)carbamoyl]-2-benzylbutanol

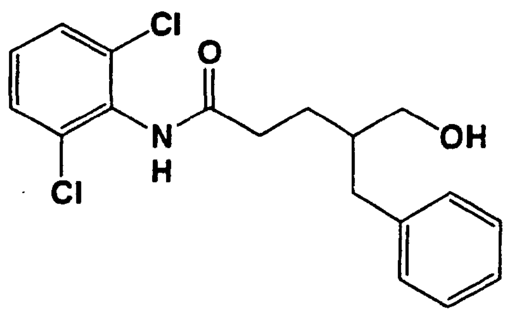

Under an atmosphere of argon, to a solution of the compound prepared in Reference Example 2 (180 mg) in anhydrous tetrahydrofuran (5 ml) was added lithium borohydride (170 mg) in ice bath and the mixture was stirred at room temperature for 48 hours. The reaction mixture was poured in iced water and thereto was added 1N hydrochloric acid. The reaction mixture was extracted with ethyl acetate, washed with a saturated aqueous sodium hydrogen carbonate solution, water and brine sequentially, and dried over anhydrous magnesium sulfate. The solvent was evaporated and the obtained residue was purified by column chromatography on silica gel (ethyl acetate:hexane=3:2) to give the title compound (117 mg) having the following physical data.
TLC:Rf 0.17 (ethyl acetate:hexane=2:3);
NMR ($CDCl_3$):d 7.37 (d, J = 8.1 Hz, 2H), 7.34-7.15 (m, 6H), 7.10 (s, 1H), 3.64 (m, 1H), 3.57 (m, 1H), 2.78-2.40 (m, 4H), 2.17 (br, 1H), 2.01-1.75 (m, 3H).

Reference Example 4

4-[N-(2,6-dichlorophenyl)carbamoyl]-2-benzylbuthylbromide

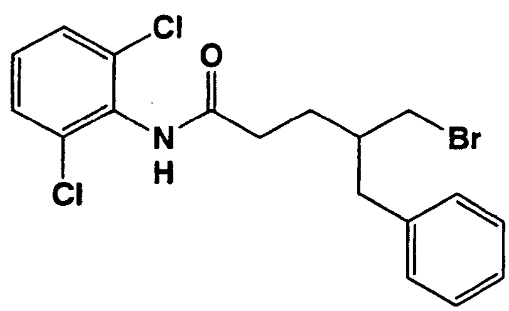

Under an atmosphere of argon, to a solution of the compound prepared in Reference Example 3 (115 mg) in methylene chloride (5 ml) were added triphenylphosphine (155 mg) and carbon tetrabromide (247 mg) and the mixture was stirred at room temperature for 1.5 hours. Ethyl acetate was added to the reaction mixture, which was washed with a saturated aqueous sodium hydrogen carbonate solution, water and brine, and dried over anhydrous magnesium sulfate. The solvent was evaporated and the obtained residue was purified by column chromatography on silica gel (ethyl acetate:hexane=1:4) to give the title compound (112 mg) having the following physical data.
TLC:Rf 0.61 (ethyl acetate:hexane=2:3);
NMR ($CDCl_3$):d 7.37 (d, J = 7.8 Hz, 2H), 7.37-7.14 (m, 6H), 6.91 (s, 1H), 3.48 (m, 1H), 3.38 (m, 1H), 2.81-2.62 (m, 2H), 2.60-2.40 (m, 2H), 2.12 (m, 1H), 2.00-1.87 (m, 2H).

Example 1

1-(2,6-dichlorophenyl)-5-benzylpiperidin-2-one

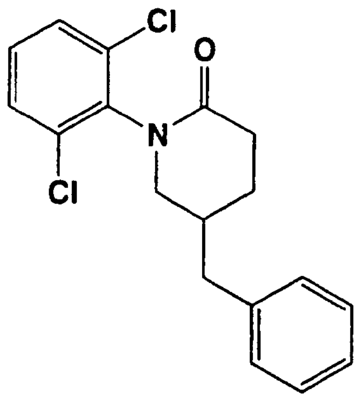

Under an atmosphere of argon, to a solution of the compound prepared in Reference Example 4 (110 mg) in anhydrous tetrahydrofuran (5 ml) was added potassium tert-butoxide (33 mg) in ice bath and the mixture was stirred at 0°C for 15 minutes. 1N hydrochloric acid was added to the reaction mixture, which was extracted with ethyl acetate. The organic layer was washed with a saturated aqueous sodium hydrogen carbonate solution, water and brine sequentially, and dried over anhydrous magnesium sulfate. The solvent was evaporated and the obtained residue was purified by column chromatography on silica gel (ethyl acetate:hexane=3:7) to give the compound of the present invention (87 mg) having the following physical data.
TLC:Rf 0.47 (ethyl acetate:hexane=1:1);
NMR ($CDCl_3$):d 7.40-7.16 (m, 8H), 3.39-3.29 (m, 2H), 2.77-2.62 (m, 3H), 2.58-2.30 (m, 2H), 2.01 (m, 1H), 1.72 (m, 1H).

Examples 1(1)~1(12)

By the same procedure as described in Reference Example 2→Reference Example 3→Reference Example 4→Example 1 using the corresponding halide instead of benzybromide, the following compounds of the present invention were obtained.

Example 1(1)

1-(2,6-dichlorophenyl)-5-(2,4-difluorobenzyl)piperidin-2-one

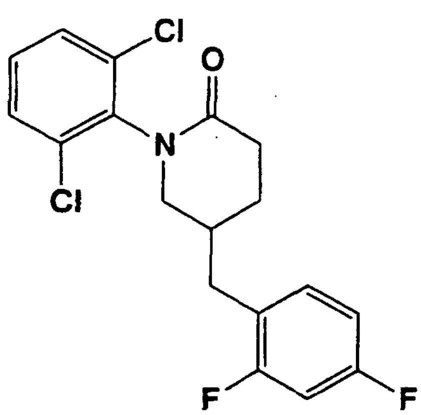

TLC:Rf 0.34 (ethyl acetate:hexane=2:3);
NMR ($CDCl_3$):d 7.42-7.34 (m, 2H), 7.24-7.08 (m, 2H), 6.87-6.77 (m, 2H), 3.36 (d, J = 8.1 Hz, 2H), 2.77-2.62 (m, 3H), 2.60-2.29 (m, 2H), 1.99 (m, 1H), 1.73 (m, 1H).

Example 1(2)

1-(2,6-dichlorophenyl)-5-(2-naphthylmethyl)piperidin-2-one

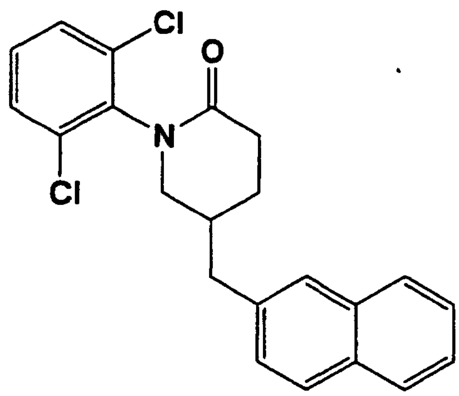

TLC:Rf 0.39 (ethyl acetate:hexane=2:3);
NMR ($CDCl_3$):d 7.83-7.75 (m, 3H), 7.63 (s, 1H), 7.50-7.40 (m, 2H), 7.39-7.30 (m, 3H), 7.18 (t, J = 8.1 Hz, 1H), 3.42-3.30 (m, 2H), 2.97-2.82 (m, 2H), 2.70 (ddd, J = 18.0, 6.0, 3.6 Hz, 1H), 2.61-2.42 (m, 2H), 2.02 (m, 1H), 1.78 (m, 1H).

Example 1(3)

1-(2,6-dichlorophenyl)-5-(2,4-dimethylbenzyl)piperidin-2-one

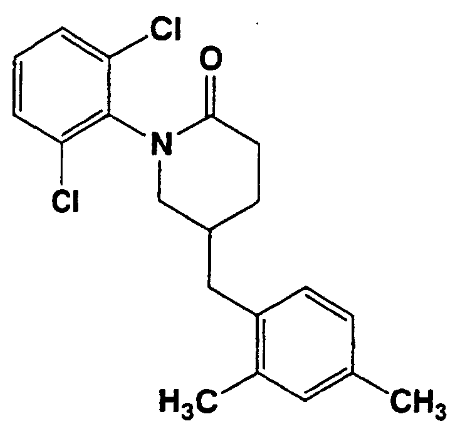

TLC:Rf 0.42 (ethyl acetate:hexane=2:3);
NMR ($CDCl_3$):d 7.41-7.34 (m, 2H), 7.20 (t, J = 8.1 Hz, 1H), 7.03-6.91 (m, 3H), 3.43-3.30 (m, 2H), 2.74-2.58 (m, 3H), 2.49 (m, 1H), 2.40-2.23 (m, 7H), 2.01 (m, 1H), 1.72 (m, 1H).

Example 1(4)

1-(2,6-dichlorophenyl)-5-(4-trifluoromethoxybenzyl)piperidin-2-one

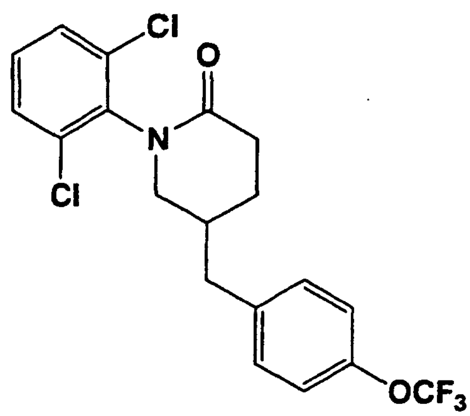

TLC:Rf 0.20 (ethyl acetate:hexane=3:7);
NMR ($CDCl_3$):d 7.42-7.36 (m, 2H), 7.25-7.12 (m, 5H), 3.41-3.29 (m, 2H), 2.80-2.63 (m, 3H), 2.52 (ddd, J = 17.4, 10.8, 6.3 Hz, 1H), 2.38 (m, 1H), 2.01 (m, 1H), 1.72 (m, 1H).

Example 1(5)

1-(2,6-dichlorophenyl)-5-(4-trifluoromethylbenzyl)piperidin-2-one

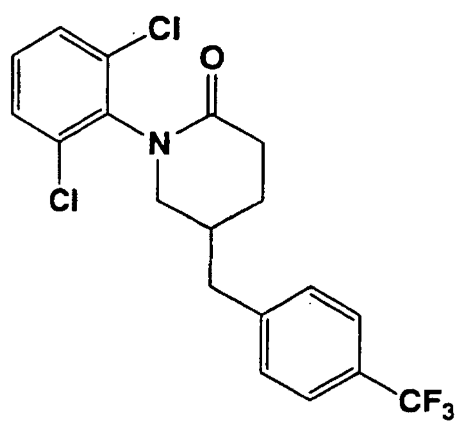

TLC:Rf 0.27 (ethyl acetate:hexane=2:3);
NMR ($CDCl_3$):d 7.57 (d, J = 8.1 Hz, 2H), 7.41-7.36 (m, 2H), 7.32 (d, J = 8.1 Hz, 2H), 7.21 (m, 1H), 3.36 (d, J = 7.8 Hz, 2H), 2.79 (d, J = 7.2 Hz, 2H), 2.69 (ddd, J = 17.7, 5.7, 3.6 Hz, 1H), 2.60-2.32 (m, 2H), 2.00 (m, 1H), 1.72 (m, 1H).

Example 1(6)

1-(2,6-dichlorophenyl)-5-(3,5-difluorobenzyl)piperidin-2-one

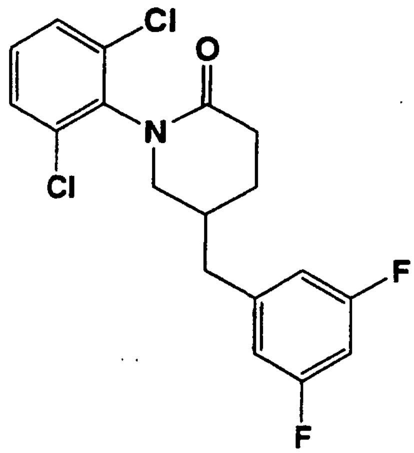

TLC:Rf 0.36 (ethyl acetate:hexane=2:3);
NMR ($CDCl_3$):d 7.42-7.34 (m, 2H), 7.21 (m, 1H), 6.80-6.63 (m, 3H), 3.41-3.28 (m, 2H), 2.75-2.62 (m, 3H), 2.54 (ddd, J = 17.7, 11.1, 6.3 Hz, 1H), 2.38 (m, 1H), 2.00 (m, 1H), 1.72 (m, 1H).

Example 1(7)

1-(2,6-dichlorophenyl)-5-(2-chlorobenzyl)piperidin-2-one

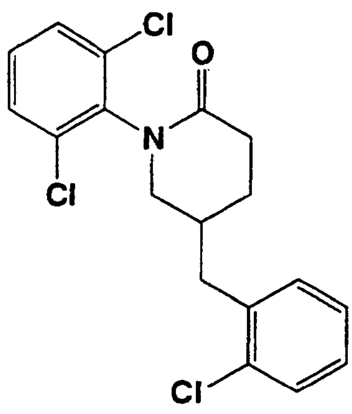

TLC:Rf 0.38 (ethyl acetate:hexane=2:3);
NMR ($CDCl_3$):d 7.42-7.34 (m, 3H), 7.25-7.14 (m, 4H), 3.46-3.34 (m, 2H), 2.94-2.77 (m, 2H), 2.69 (ddd, J = 17.7, 5.7, 3.3 Hz, 1H), 2.59-2.40 (m, 2H), 2.00 (m, 1H), 1.77 (m, 1H).

Example 1(8)

1-(2,6-dichlorophenyl)-5-(4-fluorobenzyl)piperidin-2-one

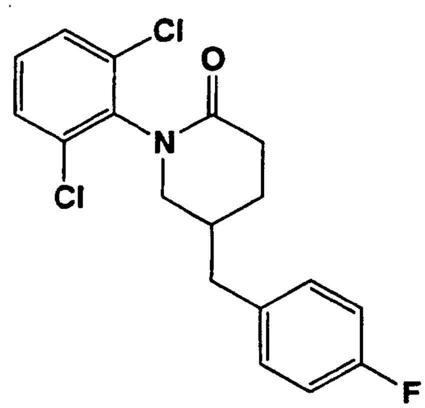

TLC:Rf 0.29 (ethyl acetate:hexane=2:3);
NMR ($CDCl_3$):d 7.41-7.34 (m, 2H), 7.23-7.09 (m, 3H), 7.04-6.95 (m, 2H), 3.37-3.27 (m, 2H), 2.74-2.61 (m, 3H), 2.52 ( ddd, J = 17.7, 11.4, 6.3 Hz, 1H), 2.37 (m, 1H), 2.00 (m, 1H), 1.69 (m, 1H).

Example 1(9)

1-(2,6-dichlorophenyl)-5-(2-fluorobenzyl)piperidin-2-one

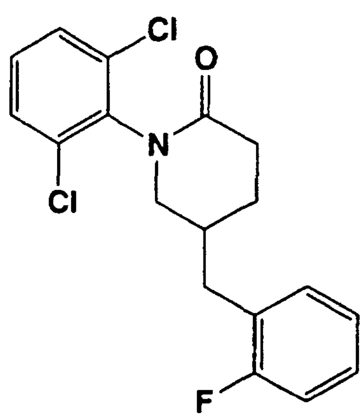

TLC:Rf 0.36 (ethyl acetate:hexane=2:3);
NMR ($CDCl_3$):d 7.41-7.34 (m, 2H), 7.25-7.15 (m, 3H), 7.14-6.99 (m, 2H), 3.38 (d, J = 8.1 Hz, 2H), 2.76 (d, J = 6.9 Hz, 2H), 2.69 (ddd, J = 18.3, 6.0, 3.6 Hz, 1H), 2.60-2.34 (m, 2H), 2.00 (m, 1H), 1.74 (m, 1H).

Example 1(10)

2-(2,6-dichlorophenyl)-5-(1-naphthylmethyl)piperidin-2-one

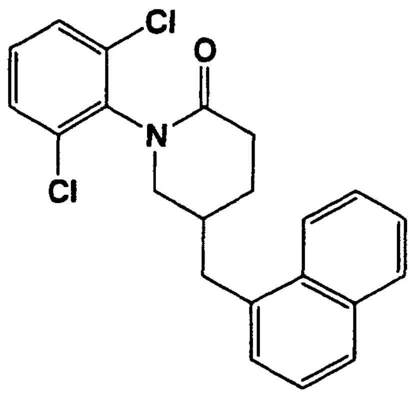

TLC:Rf 0.48 (hexane:ethyl acetate=1:1);
NMR ($CDCl_3$):d 8.01 (d, J=8.1Hz, 1H), 7.90-7.87 (m, 1H), 7.76 (d, J=8.1Hz, 1H), 7.58-7.48 (m, 2H), 7.43-7.32 (m, 4H), 7.20 (t, J=8.1Hz, 1H), 3.53-3.40 (m, 2H), 3.22 (dd, J=6.6Hz, 12.8Hz, 1H), 3.11 (dd, J=7.8Hz, 12.8Hz, 1H), 2.73-2.42 (m, 3H), 2.08-1.97 (m, 1H), 1.88-1.74 (m, 1H).

Example 1(11)

1-(2,6-dichlorophenyl)-5-(2-methoxybenzyl)piperidin-2-one

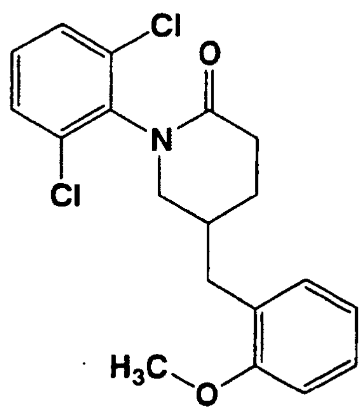

TLC:Rf 0.46 (ethyl acetate:hexane=2:3);
NMR ($CDCl_3$):d 7.41-7.34 (m, 2H), 7.25-7.08 (m, 3H), 6.92-6.84 (m, 2H), 3.83 (s, 3H), 3.35 (d, J = 8.4 Hz, 2H), 2.75-2.62 (m, 3H), 2.59-2.37 (m, 2H), 1.98 (m, 1H), 1.70 (m, 1H).

Example 1(12)

1-(2,6-dichlorophenyl)-5-(4-ethylbenzyl)piperidin-2-one

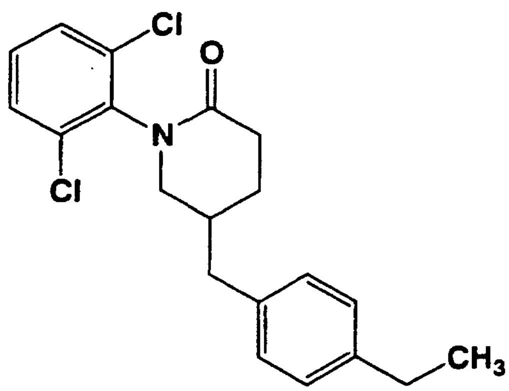

TLC:Rf 0.28 (ethyl acetate:hexane=3:7);
NMR ($CDCl_3$):d 7.40-7.35 (m, 2H), 7.19 (m, 1H), 7.13 (d, J = 8.7 Hz, 2H), 7.10 (d, J = 8.7 Hz, 2H), 3.42-3.28 (m, 2H), 2.73-2.60 (m, 5H), 2.50 (m, 1H), 2.38 (m, 1H), 2.00 (m, 1H), 1.70 (m, 1H), 1.23 (t, J = 7.8 Hz, 3H).

Example 2

1-(2,6-dichlorophenyl)-5-benzylpiperidin-2-one

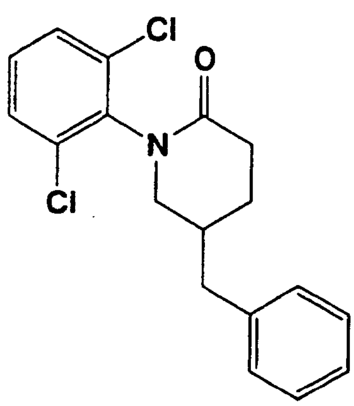

Under an atmosphere of argon, a solution of the compound prepared in Reference Example 3 (3.65 g) in tetrahydrofuran (50 ml) was cooled in ice bath and thereto were added triphenylphosphine (4.92 g) and diethyl azodicarboxylate (40% solution in toluene, 7.41 ml). The reaction mixture was stirred at 0°C for 15 minutes. The reaction mixture was concentrated. The obtained residue was purified by column chromatography on silica gel (ethyl acetate: hexane=3:7) and washed with t-butyl methyl ether to give the compound of the present invention (1.89 g) having the following physical data.
TLC:Rf 0.47 (ethyl acetate:hexane=1:1);
NMR ($CDCl_3$):d 7.40-7.16 (m, 8H), 3.39-3.29 (m, 2H), 2.77-2.62 (m, 3H), 2.58-2.30 (m, 2H), 2.01 (m, 1H), 1.72 (m, 1H).

Example 3

1-(2-chlorophenyl)-5-benzylpiperidin-2-one

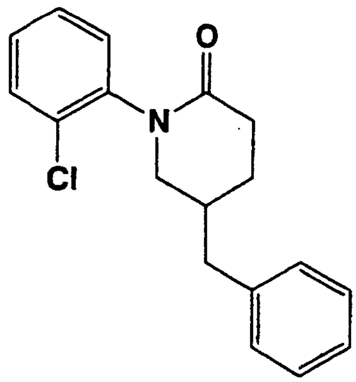

By the same procedure as described in Reference Example 1→Reference Example 2→Reference Example 3→Reference Example 4→Example 1 using 2-chloroaniline instead of 2,6-dichloroaniline, the compound of the present invention having the following physical data was obtained.
TLC:Rf 0.30 (hexane:ethyl acetate=1:1);
NMR ($CDCl_3$):d 7.48-7.43, 7.33-7.16 (m, 9H), 3.49-3.39, 3.29-3.22 (m, 2H), 2.73-2.27 (m, 5H), 2.07-1.95 (m, 1H), 1.79-1.65 (m, 1H).

Example 4

1-(2,6-difluorophenyl)-5-benzylpiperidin-2-one

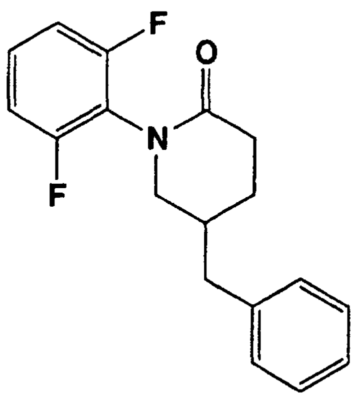

By the same procedure as described in Reference Example 1→Reference Example 2→Reference Example 3→Reference Example 4→Example 1 using 2,6-difluoroaniline instead of 2,6-dichloroaniline, the compound of the present invention having the following physical data was obtained.
TLC:Rf 0.53 (hexane:ethyl acetate=1:1);
NMR ($CDCl_3$):d 7.33-7.17 (m, 6H), 6.99-6.91 (m, 2H), 3.46 (ddd, J=1.3Hz, 5.1Hz, 11.4Hz, 1H), 3.37 (dd, J=9.3Hz, 11.4Hz, 1H), 2.71 (d, J=7.5Hz, 2H), 2.68-2.62 (m, 1H), 2.58-2.46 (m, 1H), 2.44-2.30 (m, 1H), 2.08-1.97 (m, 1H), 1.79-1.65 (m, 1H).

Example 5

1-(2,6-dimethylphenyl)-5-(2,4-difluorobenzyl)piperidin-2-one

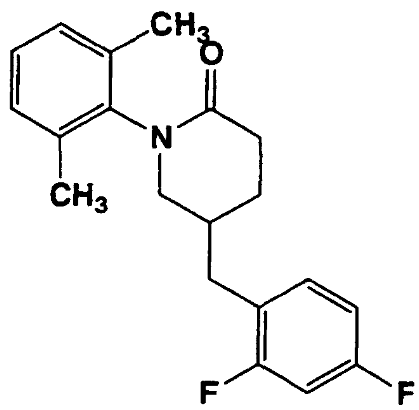

By the same procedure as described in Reference Example 1→Reference Example 2→Reference Example 3→Reference Example 4→Example 1 using 2,6-dimethylaniline instead of 2,6-dichloroaniline and 2,4-difluorobenzylbromide instead of benzylbromide, the compound of the present invention having the following physical data was obtained.
TLC:Rf 0.31 (ethyl acetate:hexane=3:2);
NMR ($CDCl_3$):d 7.17-7.05 (m, 4H), 6.87-6.75 (m, 2H), 3.29-3.18 (m, 2H), 2.75-2.62 (m, 3H), 2.52 (ddd, J = 18.3, 11.7, 6.6 Hz, 1H), 2.30 (m, 1H), 2.21 (s, 3H), 2.12 (s, 3H), 2.00 (m, 1H), 1.66 (m, 1H).

Reference Example 5

2,6-dichloro-4-hydroxymethylaniline

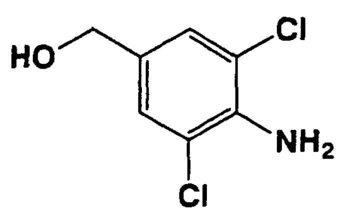

Under an atmosphere of argon, to a solution of 4-amino-3,5-dichlorobenzoic acid (2.5g) in anhydrous tetrahydrofuran (100ml) was added Borane-tetrahydrofuran complex (1.04 M solution in tetrahydrofuran, 35ml) in ice bath the mixture was stirred at room temperature for 1 hours. Methanol was added to the reaction mixture, which was concentrated. The residue was dissolved in ethyl acetate, washed with 1N hydrochloric acid, water, a saturated aqueous sodium hydrogen carbonate solution, water and brine sequentially, and dried over anhydrous magnesium sulfate. The solvent was evaporated to give the title compound having the following physical data. The obtained compound was used in next reaction without purification.
TLC:Rf 0.32 (ethyl acetate:hexane=3:7);
NMR ($CDCl_3$):d 7.21 (s, 2H), 4.54 (d, J = 4.5 Hz, 2H), 4.43 (s, 2H), 1.59 (t, J = 4.5 Hz, 1H).

Reference Example 6

2,6-dichloro-4-(1,1,1-triphenylmethoxymethyl)aniline

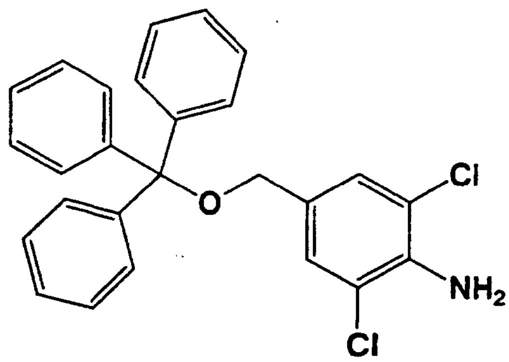

Under an atmosphere of argon, to a solution of the compound prepared in Reference Example 5 in N,N-dimethylformamide (20 ml) were added triethylamine (3.68 ml), trityl chloride (3.71 g) and N,N-dimethylaminopyridine (65 mg) in ice bath and the mixture was stirred at room temperature for 12 hours. Ethyl acetate was added to the reaction mixture, which was washed with 1N hydrochloric acid, water, a saturated aqueous sodium hydrogen carbonate solution, water and brine sequentially, and dried over anhydrous magnesium sulfate. The solvent was evaporated and the obtained residue was washed with isopropyl ether to give the title compound (3.0g) having the following physical data.
TLC:Rf 0.49 (ethyl acetate:hexane=1:9);
NMR ($CDCl_3$):d 7.50-7.43 (m, 6H), 7.37-7.20 (m, 9H), 7.16 (s, 2H), 4.40 (s, 2H), 4.01 (s, 2H).

Example 6

1-[2,6-dichloro-4-(1,1,1-triphenylmethoxymethyl)phenyl]-5-(2,4-difluorobenzyl)pipe ridin-2-one

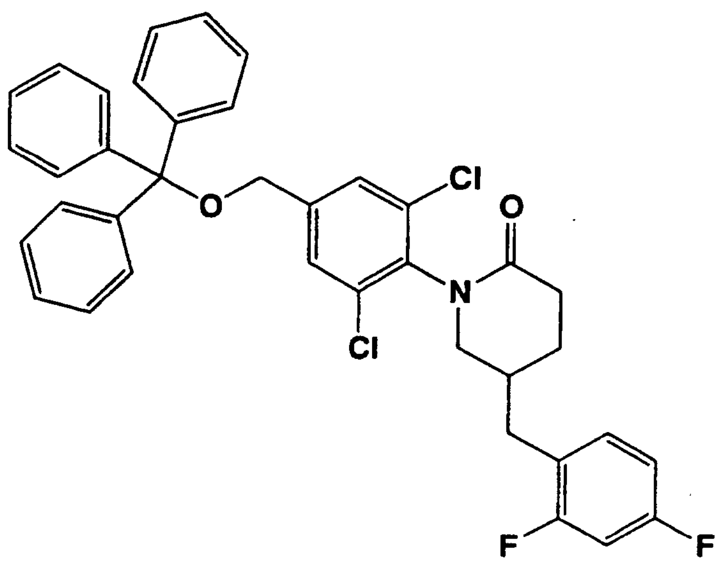

By the same procedure as described in Reference Example 1→Reference Example 2→Reference Example 3→Reference Example 4→Example 1 using the compound prepared in Reference Example 6 instead of 2,6-dichloroaniline and 2,4-difluorobenzylbromide instead of benzylbromide, the compound of the present invention having the following physical data was obtained.
TLC:Rf 0.32 (ethyl acetate:hexane=2:3);
NMR ($CDCl_3$):d 7.50-7.43 (m, 6H), 7.39-7.20 (m, 11H), 7.17 (m, 1H), 6.81 (m, 2H), 4.14 (s, 2H), 3.34 (d, J = 7.8 Hz, 2H), 2.76-2.62 (m, 3H), 2.52 (ddd, J = 17.7, 11.1, 6.3 Hz, 1H), 2.38 (m, 1H), 1.99 (m, 1H), 1.72 (m, 1H).

Example 7

1-(2,6-dichloro-4-hydroxymethylphenyl)-5-(2,4-difluorobenzyl)piperidin-2-one

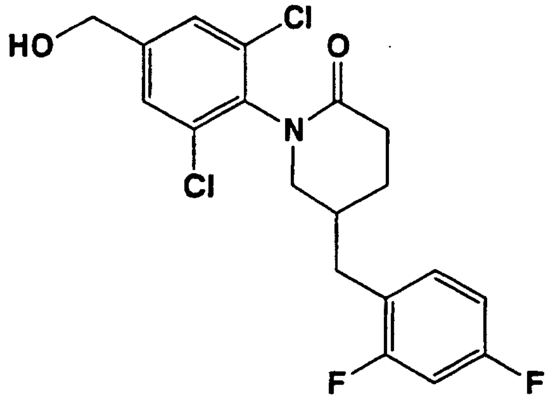

The compound prepared in Example 6 (380 mg) was dissolved in a mixed solvent of methylene chloride (1 ml) and water (0.1 ml). Trifluoroacetic acid (1 ml) was added thereto in ice bath and the mixture was stirred at 0°C for 1 hour. The reaction mixture was poured in an iced saturated aqueous sodium hydrogen carbonate solution and extracted with ethyl acetate. The organic layer was washed with water and brine and dried over anhydrous magnesium sulfate. The solvent was evaporated. The obtained residue was purified by column chromatography on silica gel (ethyl acetate:hexane=3:2) and washed with isopropyl ether to give the title compound (162 mg) having the following physical data.
TLC:Rf 0.40 (ethyl acetate:hexane=3:2);
NMR ($CDCl_3$):d 7.34 (s, 2H), 7.14 (m, 1H), 6.87-6.75 (m, 2H), 4.62-4.53 (m, 2H), 3.34 (d, J = 7.8 Hz, 2H), 2.77-2.61 (m, 3H), 2.59-2.28 (m, 3H), 1.99 (m, 1H), 1.72 (m, 1H).

Reference Example 7

Ethyl 4-[N-(2,6-dichlorophenyl)carbamoyl]-2-hydroxybutanoate

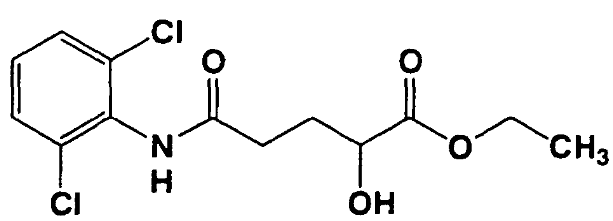

Under an atmosphere of argon, a solution of the compound prepared in Reference Example 1 (2.09 g) in anhydrous tetrahydrofuran (15 ml) was cooled to -78°C. Lithium bis(trimethylsilyl)amide (1.0M solution in tetrahydrofuran, 15.2 ml) was added thereto and the mixture was stirred for 30 minutes. A solution of 2-(phenylsulfonyl)-3-phenyloxaziridine (2.16 g) in anhydrous tetrahydrofuran (10 ml) was added to the reaction mixture, which was stirred at -70~-60°C for 4 hours. A saturated aqueous ammonium chloride solution was added to the reaction mixture, which

was extracted with ethyl acetate. The organic layer was washed with water and brine and dried over anhydrous magnesium sulfate. The solvent was evaporated and the obtained residue was purified by column chromatography on silica gel (ethyl acetate:hexane =3:7→2:3) to give the title compound (1.30 g) having the following physical data.
TLC:Rf 0.30 (ethyl acetate:hexane=3:2);
NMR ($CDCl_3$):d 7.37 (d, J = 8.1 Hz, 2H), 7.31 (s, 1H), 7.19 (t, J = 8.1 Hz, 1H), 4.38-4.19 (m, 3H), 3.18 (br, 1H), 2.77-2.51 (m, 2H), 2.37 (m, 1H), 2.03 (m, 1H), 1.31 (t, J = 7.8 Hz, 3H).

Reference Example 8

Ethyl 4-[N-(2,6-dichlorophenyl)carbamoyl]-2-methoxymethoxybutanoate

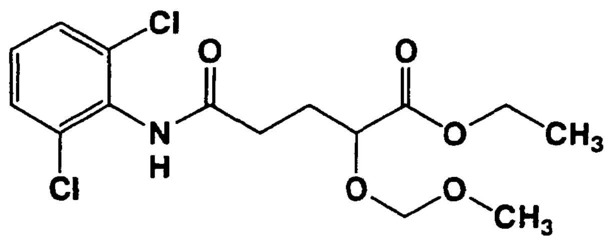

Under an atmosphere of argon, to a solution of the compound prepared in Reference Example 7 (650 mg) in methylene chloride (5 ml) were added diisopropylethylamine (2.1 ml) and chloromethyl methyl ether (0.70 ml) in ice bath and the mixture was stirred at room temperature for 12 hours. Ethyl acetate was added to the reaction mixture, which was washed with 1N hydrochloric acid, water, a saturated aqueous sodium hydrogen carbonate solution, water and brine sequentially, and dried over anhydrous magnesium sulfate. The solvent was evaporated and the obtained residue was purified by column chromatography on silica gel (ethyl acetate:hexane=3:7→2:3) to give the title compound (330 mg) having the following physical data.
TLC:Rf 0.43 (ethyl acetate:hexane=1:1);
NMR ($CDCl_3$):d 7.37 (d, J = 8.1 Hz, 2H), 7.31 (s, 1H), 7.19 (t, J = 8.1 Hz, 1H), 4.79-4.68 (m, 2H), 4.49 (m, 1H), 4.21 (q, J = 7.8 Hz, 2H), 3.43 (s, 3H), 2.72-2.48 (m, 2H), 2.40-2.08 (m, 2H), 1.32 (t, J = 7.8 Hz, 3H).

Reference Example 9

4-[N-(2,6-dichlorophenyl)carbamoyl]-2-methoxymethoxybutanol

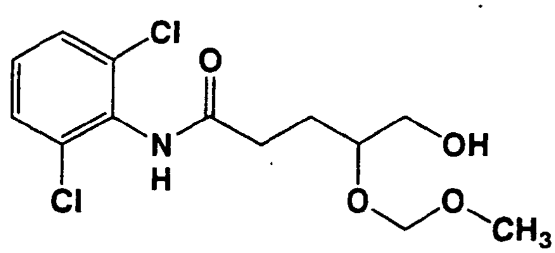

By the same procedure as described in Reference Example 3 using the compound prepared in Reference Example 8 instead of the compound prepared in Reference Example 2, the title compound having the following physical data was obtained.
TLC:Rf 0.22 (ethyl acetate:hexane=4:1);
NMR ($CDCl_3$):d 7.37 (d, J = 8.1 Hz, 2H), 7.31 (s, 1H), 7.19 (t, J = 8.1 Hz, 1H), 4.91-4.68 (m, 2H), 3.78 (m, 1H), 3.78-3.50 (m, 2H), 3.46 (s, 3H), 3.01 (br, 1H), 2.65-2.50 (m, 2H), 2.05-1.86 (m, 2H).

Reference Example 10

4-[N-(2,6-dichlorophenyl)carbamoyl]-2-methoxymethoxybutyl 4-methylbenzensulfonate

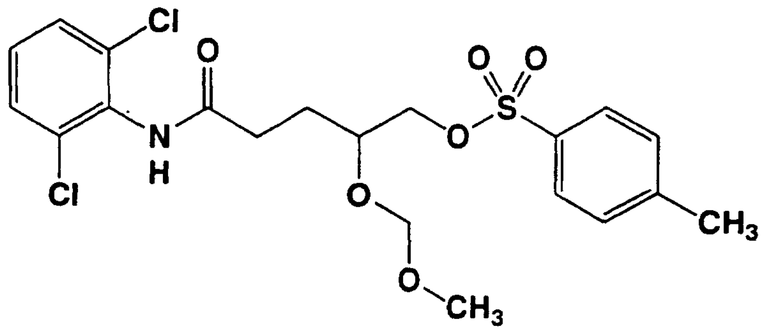

Under an atmosphere of argon, a solution of the compound prepared in Reference Example 9 (190 mg) in pyridine (5 ml) was cooled to 0°C. p-toluenesulfonyl chloride (180mg) was added thereto and the mixture was stirred at room temperature for 12 hours. The reaction mixture was diluted with ethyl acetate, washed with 1N hydrochloric acid, water, a saturated aqueous sodium hydrogen carbonate solution, water and brine sequentially, and dried over anhydrous magnesium sulfate. The solvent was evaporated to give the title compound having the following physical data.
TLC:Rf 0.44 (ethyl acetate:hexane=1:1);
NMR ($CDCl_3$):d 7.80 (d, J = 8.1 Hz, 2H), 7.42-7.32 (m, 5H), 7.19 (m, 1H), 4.75-4.60 (m, 2H), 4.08-4.02 (m, 2H), 3.97 (m, 1H), 3.38 (s, 3H), 2.61-2.47 (m, 2H), 2.45(s, 3H), 2.02-1.80 (m, 2H).

Reference Example 11

1-(2,6-dichlorophenyl)-5-methoxymethoxypiperidin-2-one

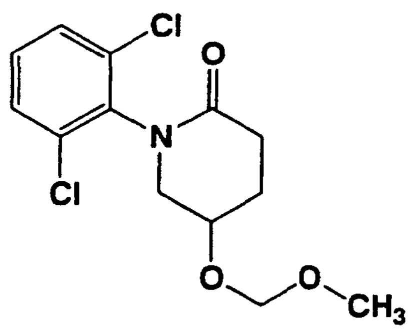

By the same procedure as described in Example 1 using the compound prepared in Reference Example 10 instead of the compound prepared in Reference Example 4, the title compound having the following physical data was obtained.
TLC:Rf 0.39 (ethyl acetate:hexane=1:1);
NMR ($CDCl_3$):d 7.41-7.38 (m, 2H), 7.22 (m, 1H), 4.79-4.74 (m, 2H), 4.19 (m, 1H), 3.67 (dd, J = 12.0, 4.5 Hz, 1H), 3.54 (dd, J = 12.0, 5.4 Hz, 1H), 3.41 (s, 3H), 2.87-2.74 (m, 1H), 2.56 (dt, J = 17.7, 6.3 Hz, 1H), 2.20-2.10 (m, 2H).

Reference Example 12

1-(2,6-dichlorophenyl)-5-hydroxypiperidin-2-one

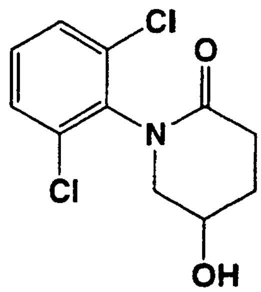

The compound prepared in Reference Example 11 (178 mg) dissolved in a mixed solvent of methanol (5 ml) and water (1 ml). Concentrated hydrochloric acid (0.12ml) was added thereto and the mixture was stirred at 70°C for 6 hours. The reaction mixture was neutralized with a saturated aqueous sodium hydrogen carbonate solution and concentrated. The residue was dissolved in ethyl acetate, washed with water and brine and dried over anhydrous magnesium sulfate. The solvent was evaporated and washed with isopropyl ether to give the title compound (102 mg) having the following physical data.
TLC:Rf 0.21 (ethyl acetate:hexane=3:2);
NMR ($CDCl_3$):d 7.42-7.38 (m, 2H), 7.22 (m, 1H), 4.38 (m, 1H), 3.74 (dd, J = 12.0, 4.2, 1H), 3.45 (dd, J = 12.0, 4.8 Hz, 1H), 2.83 (ddd, J = 18.0, 8.4, 6.6 Hz, 1H), 2.59 (dt, J = 18.0, 6.3 Hz, 1H), 2.20-2.07 (m, 2H), 1.91 (d, J = 4.8 Hz, 1H).

Example 8

1-(2,6-dichlorophenyl)-5-(2,4-difluorophenoxy)piperidin-2-one

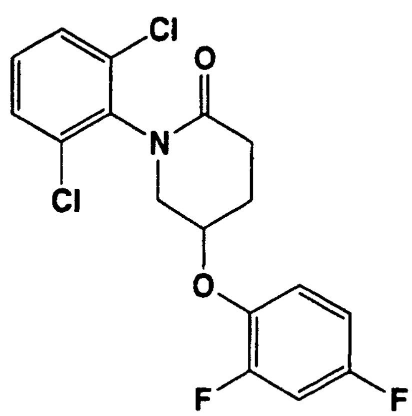

Under an atmosphere of argon, the compound prepared in Reference Example 12 (100 mg) was dissolved in tetrahydrofuran (5 ml). 2,4-difluorophenol (64 mg) and triphenylphosphine (232 mg) were added thereto and the reaction mixture was iced. Diethyl azodicarboxylate (40% solution in toluene, 0.35 ml) was added to the reaction mixture, which was stirred at room temperature for 12 hours. The reaction mixture was concentrated and the obtained residue was purified by column chromatography on silica gel (ethyl acetate:hexane=3:7) to give the compound of the present invention (51 mg) having the following physical data.
TLC:Rf 0.41 (ethyl acetate:hexane=2:3);
NMR ($CDCl_3$):d 7.43-7.38 (m, 2H), 7.23 (m, 1H), 7.04 (m, 1H), 6.93-6.76 (m, 2H), 4.77 (m, 1H), 3.83-3.68 (m, 2H),

2.92 (ddd, J = 17.4, 9.6, 6.0 Hz, 1H), 2.61 (dt, J = 17.4, 6.0 Hz, 1H), 2.41-2.18 (m, 2H).

Example 8(1)

1-(2,6-dichlorophenyl)-5-(2,4-difluorothiophenoxy)piperidin-2-one

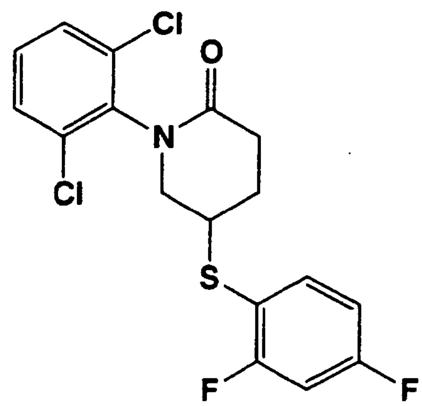

By the same procedure as described in Example 8 using 2,4-difluorothiophenol instead of 2,4-difluorophenol, the compound of the present invention having the following physical data was obtained.
TLC:Rf 0.47 (ethyl acetate:hexane=2:3);
NMR ($CDCl_3$):d 7.60 (m, 1H), 7.40-7.35 (m, 2H), 7.21 (m, 1H), 6.94-6.83 (m, 2H), 3.65 (m, 1H), 3.62-3.53 (m, 2H), 2.81 (ddd, J = 17.7, 6.0, 4.8 Hz, 1H), 2.61 (ddd, J = 17.7, 9.6, 6.3 Hz, 1H), 2.28 (m, 1H), 1.98 (m, 1H).

Example 9

1-(2,6-dichloro-4-nitrophenyl)-5-benzylpiperidin-2-one

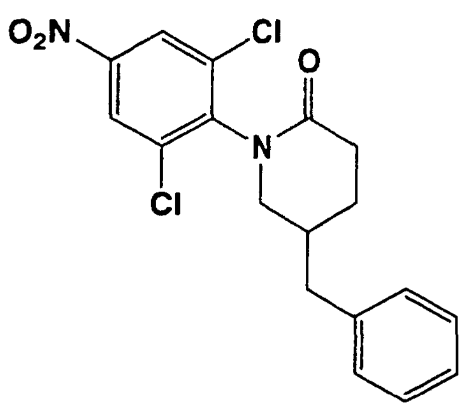

By the same procedure as described in Reference Example 1→Reference Example 2→Reference Example 3→Example 2 using 2,6-dichloro-4-nitroaniline instead of 2,6-dichloroaniline, the compound of the present invention having the following physical data was obtained.
TLC:Rf 0.63 (hexane:ethyl acetate=1:1);
NMR ($CDCl_3$):d 8.26-8.23 (m, 2H), 7.34-7.17 (m, 5H), 3.38-3.32 (m, 2H), 2.80-2.65 (m, 3H), 2.60-2.36 (m, 2H), 2.08-2.01 (m, 1H), 1.80-1.67 (m, 1H).

Example 9(1)

1-(2,6-dichloro-4-nitrophenyl)-5-(2,4-difluorobenzyl)piperidin-2-one

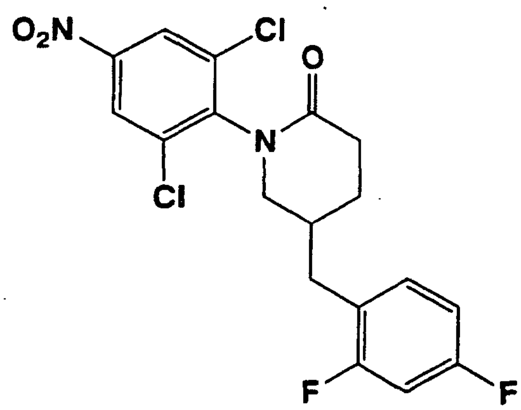

By the same procedure as described in Reference Example 1→Reference Example 2→Reference Example 3→Example 2 using 2,6-dichloro-4-nitroaniline instead of 2,6-dichloroaniline and 2,4-difluorobenzylbromide instead of benzylbromide, the compound of the present invention having the following physical data was obtained.
TLC:Rf 0.57 (hexane:ethyl acetate=1:1);
NMR ($CDCl_3$):d 8.26 (dd, J=2.3Hz, 4.1Hz, 2H), 7.18-7.10 (m, 1H), 6.87-6.78 (m, 2H), 3.40-3.29 (m, 2H), 2.74 (d, J=6.9Hz, 2H), 2.68 (dd, J=3.3Hz, 5.7Hz, 1H), 2.60-2.48 (m, 1H), 2.45-2.32 (m, 1H), 2.08-1.97 (m, 1H), 1.82-1.68 (m, 1H).

Example 10

1-(2,6-dichloro-4-aminophenyl)-5-benzylpiperidin-2-one

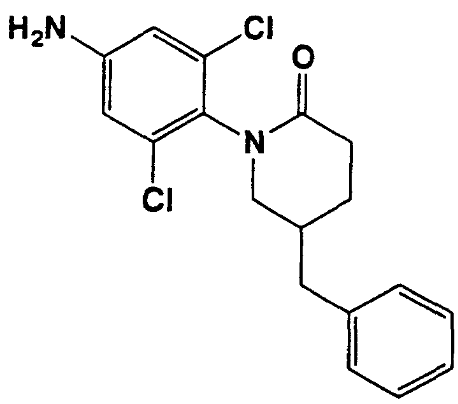

The compound prepared in Example 9 (421mg) was dissolved in a mixed solvent of acetic acid (11 ml) and water (2 ml). Iron powder (470mg) was added thereto and the mixture was stirred at room temperature for 10 minutes. The reaction mixture was diluted with ethyl acetate and filtrated through Celite (proprietary name). The filtrate was concentrated and filtrated with Floridil (proprietary name). The filtrate was concentrated and the obtained residue was washed with isopropyl ether to give the compound of the present invention (373mg) having the following physical data.
TLC:Rf 0.14 (hexane:ethyl acetate=1:1);
NMR ($CDCl_3$):d 7.33-7.17 (m, 5H), 6.62-6.59 (m, 2H), 3.87 (s, 2H), 3.35-3.26 (m, 2H), 2.74-2.60 (m, 3H), 2.54-2.42 (m, 1H), 2.38-2.30 (m, 1H), 2.01-1.92 (m, 1H), 1.73-1.62 (m, 1H).

Example 10(1)

1-(2,6-dichloro-4-aminophenyl)-5-(2,4-difluorobenzyl)piperidin-2-one

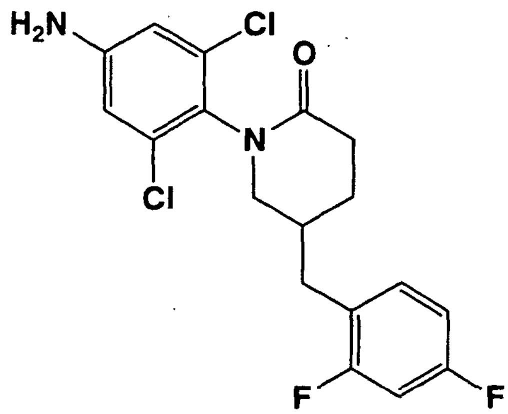

By the same procedure as described in Example 10 using the compound prepared in Example 9(1), the compound of the present invention having the following physical data was obtained.
TLC:Rf 0.21 (hexane:ethyl acetate=1:1);
NMR ($CDCl_3$):d 7.18-7.10 (m, 1H), 6.85-6.76 (m, 2H), 6.60-6.58 (m, 2H), 3.92 (s, 2H), 3.31 (d, J=7.8Hz, 2H), 2.72-2.62 (m, 3H), 2.55-2.43 (m, 1H), 2.38-2.29 (m, 1H), 1.99-1.92 (m, 1H), 1.75-1.65 (m, 1H).

Example 11

1-[2,6-dichloro-4-(N-(4-methylimidazol-5-ylmethyl)amino)phenyl]-5-benzylpiperidin-2-one

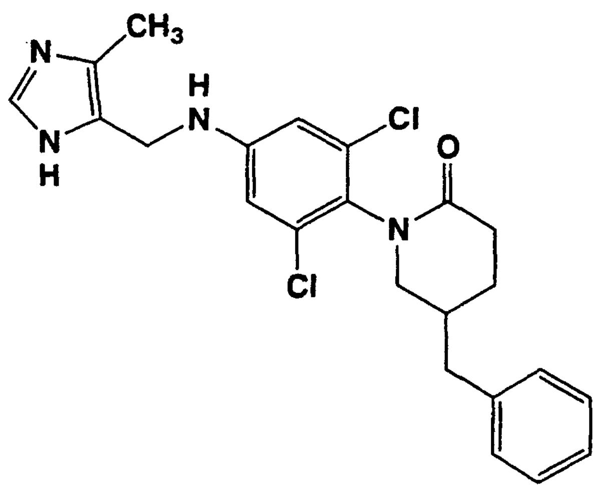

The compound prepared in Example 10 (100 mg), 4-methyl-5-formylimidazole (158 mg) and anhydrous magnesium sulfate (35 mg) were suspended in toluene (3 ml) and the mixture was refluxed for 1.5 hours. The reaction mixture was concentrated and the residue was suspended in methanol (3 ml). Sodium borohydride (55mg) was added to the suspension, which was stirred for 30 minutes. The reactioin mixture was diluted with ethyl acetate and water and extracted with ethyl acetate. The organic layer was washed with water and brine and dried over anhydrous sodium sulfate. The solvent was evaporated and the obtained residue was washed with ethyl acetate to give the compound

of the present invention (93mg) having the following physical data.
TLC:Rf 0.28 (chloroform:methanol=9:1);
NMR (DMSO-$d_6$):d 11.70 (s, 1H), 7.42 (s, 1H), 7.30-7.15 (m, 5H), 6.71 (s, 2H), 6.43 (t, J=5.1Hz, 1H), 4.00 (s, 2H), 3.22-3.12 (m, 2H), 2.66 (d, J=7.5Hz, 2H), 2.41-2.23 (m, 3H), 2.15 (s, 3H), 1.90-1.80 (m, 1H), 1.6 2-1.48 (m, 1H).

Example 11(1)

1-[2,6-dichloro-4-(N-methylamino)phenyl]-5-(2,4-difluorobenzyl)piperidin-2-one

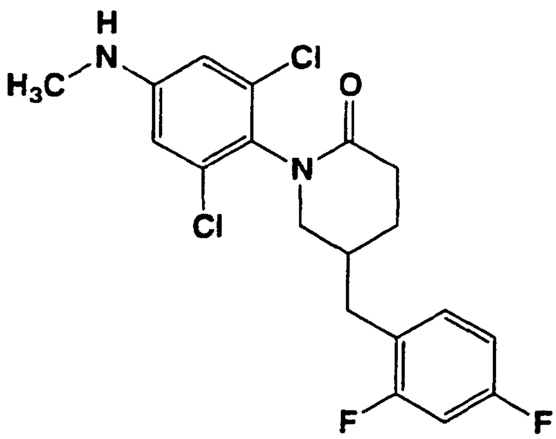

By the same procedure as described in Example 11 using paraformaldehyde instead of 4-methyl-5-formylimidazole, the compound of the present invention having the following physical data was obtained.
TLC:Rf 0.44 (hexane:ethyl acetate=1:2);
NMR ($CDCl_3$):d 7.18-7.10 (m, 1H), 6.85-6.76 (m, 2H), 6.52-6.50 (m, 2H), 4.17-4.08 (m, 1H), 3.32 (d, J=8.1Hz, 2H), 2.76 (d, J=4.8Hz, 3H), 2.72-2.61 (m, 3H), 2.55-2.43 (m, 1H), 2.37-2.28 (m, 1H), 2.00-1.90 (m, 1H), 1.75-1.62 (m, 1H).

Example 12

1-[2,6-dichloro-4-(2-phenylacetylamino)phenyl]-5-(2,4-difluorobenzyl)piperidin-2-one

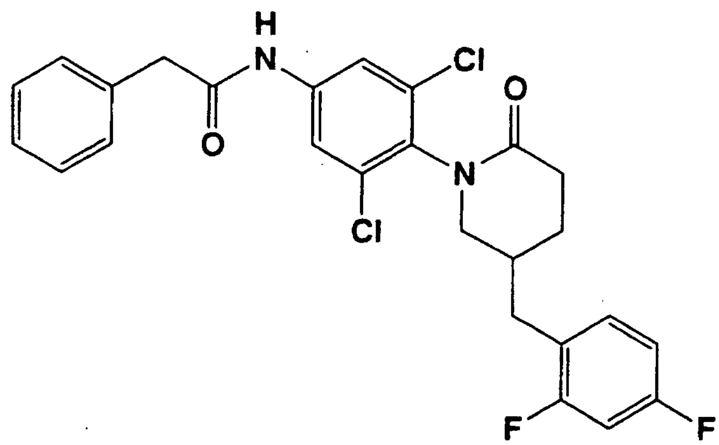

A solution of the compound prepared in Example 10(1) (115mg) and triethylamine (50 μl) in methylene chloride (3 ml) was iced. Phenylacetyl chloride (48 μl) was added thereto and the mixture was stirred at room temperature

overnight. Water was added to the reaction mixture, which was extracted with methylene chloride. The organic layer was washed with 1N hydrochloric acid, 1N aqueous sodium hydroxide solution, water and brine sequentially, and dried over anhydrous magnesium sulfate. The solvent was evaporated and the obtained residue washed with isopropyl ether to give the compound of the present invention (104 mg) having the following physical data.
TLC:Rf 0.36 (hexane:ethyl acetate=1:1);
NMR ($CDCl_3$):d 9.03 (s, 1H), 7.40 (d, J=2.4Hz, 1H), 7.36 (d, J=2.4Hz, 1H), 7.32-7.28 (m, 5H), 7.16-7.08 (m, 1H), 6.85-6.76 (m, 2H), 3.58 (s, 2H), 3.32-3.29 (m, 2H), 2.78-2.70 (m, 3H), 2.63-2.51 (m, 1H), 2.44-2.30 (m, 1H), 2.05-1.94 (m, 1H), 1.80-1.70 (m, 1H).

Example 12(1)

1-(2,6-dichloro-4-acetylaminophenyl)-5-(2,4-difluorobenzyl)piperidin-2-one

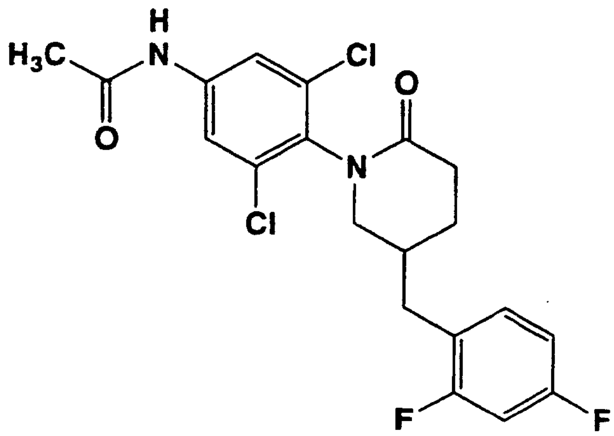

By the same procedure as described in Example 12 using acetyl chloride instead of phenylacetyl chloride, the compound of the present invention having the following physical data was obtained.
TLC:Rf 0.23 (hexane:ethyl acetate=1:2);
NMR ($CDCl_3$):d 9.12 (s, 1H), 7.35 (d, J=2.3Hz, 1H), 7.29 (d, J=2.3Hz, 1H), 7.17-7.09 (m, 1H), 6.86-6.77 (m, 2H), 3.31 (d, J=8.4Hz, 2H), 2.75-2.67 (m, 3H), 2.57(m, 1H), 2.37(m, 1H), 2.05-1.94(m, 4H), 1.73(m, 1H).

Example 13

1-[2,6-dichloro-4-(N-methyl-2-phenylacetylamino)phenyl]-5-(2,4-difluorobenzyl)piperidin-2-one

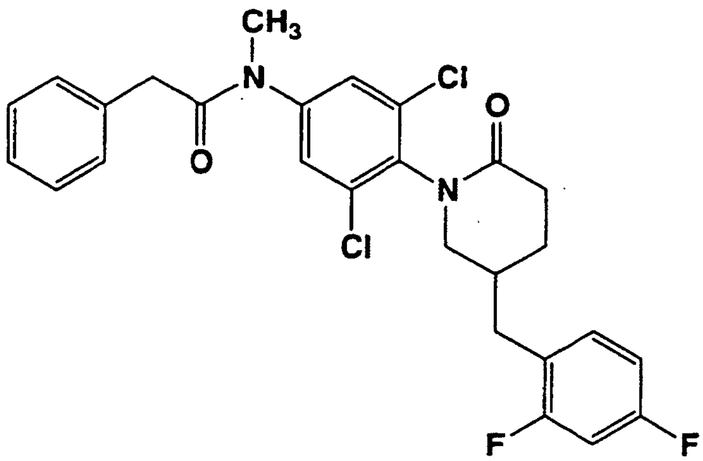

To solution of the compound prepared in Example 12. (65 mg) in N,N-dimethylformamide (1.3 ml) were added sodium hydride (5 mg) and methyl iodide (9 µl) and the mixture was stirred at room temperature for 1 hour. Water was added to the reaction mixture, which was extracted with ethyl acetate. The organic layer was washed with a saturated aqueous sodium thiosulfate solution, 1N hydrochloric acid and brine and dried over anhydrous sodium sulfate. The solvent was evaporated and the obtained residue was purified by column chromatography on silica gel (ethyl acetate: hexane=1:1) to give the compound of the present invention (34 mg) having the following physical data.
TLC:Rf 0.30 (hexane:ethyl acetate=1:1);
NMR ($CDCl_3$):d 7.26-7.01 (m, 8H), 6.87-6.79 (m, 2H), 3.57 (s, 2H), 3.36 (d, J=9.0Hz, 2H), 3.25 (s, 3H), 2.76-2.65 (m, 3H), 2.59-2.47 (m, 1H), 2.46-2.32 (m, 1H), 2.05-1.96 (m, 1H), 1.81-1.71 (m, 1H).

Example 14

(3RS,5RS)-1-(2,6-dichlorophenyl)-5-(2,4-difluorobenzyl)-3-methylpiperidin-2-one

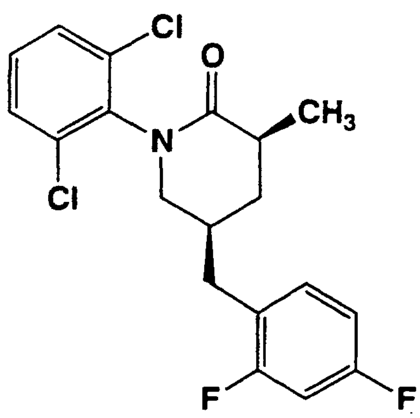

Under an atmosphere of argon, a solution of the compound prepared in Example 1(1) (130mg) in anhydrous tetrahydrofuran (10ml) was cooled to -78°C. Lithium bis(trimethylsilyl)amide (1.0M solution in tetrahydrofuran, 0.39 ml) was added thereto and the mixture was stirred for 30 minutes. Methyl iodide (24 µl) was added to the reaction mixture, which was stirred at -70~-60°C for 30 minutes. A saturated aqueous ammonium chloride solution was added thereto

and the mixture was extracted with ethyl acetate. The organic layer was washed with water and brine and dried over anhydrous magnesium sulfate. The solvent was evaporated and the obtained residue was purified by column chromatography on silica gel (ethyl acetate:hexane=1:4) and washed with isopropyl ether-hexane to give the compound of the present invention (29 mg) having the following physical data and the compound of the present invention (20 mg) represented in Example 14a.
TLC:Rf 0.58 (ethyl acetate:hexane=2:3);
NMR ($CDCl_3$):d 7.36 (d, J = 7.8 Hz, 2H), 7.22-7.08 (m, 2H), 6.88- 6.77 (m, 2H), 3.42-3.24 (m, 2H), 2.67 (d, J = 7.2 Hz, 2H), 2.61-2.35 (m, 2H), 2.02 (m, 1H), 1.51 (m, 1H), 1.30 (d, J = 7.2 Hz, 3H).

Example 14a

(3RS,5SR)-1-(2,6-dichlorophenyl)-5-(2,4-difluorobenzyl)-3-methylpiperidin-2-one

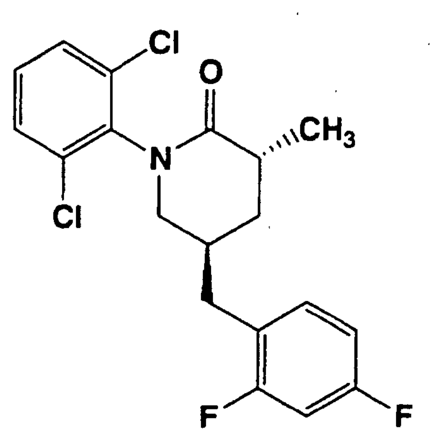

TLC:Rf 0.50 (ethyl acetate:hexane=2:3);
NMR ($CDCl_3$):d 7.41-7.34 (m, 2H), 7.23-7.12 (m, 2H), 6.88-6.77 (m, 2H), 3.37 (d, J = 7.5 Hz, 2H), 2.83-2.63 (m, 3H), 2.54 (m, 1H), 1.91 (ddd, J = 13.8, 9.3, 6.3 Hz, 1H), 1.77 (dt, J = 13.8, 4.5 Hz, 1H), 1.33 (d, J = 7.5 Hz, 3H).

Example 14(1)

(3RS,5RS)-1-(2,6-dichlorophenyl)-5-(2,4-difluorobenzyl)-3-ethylpiperidin-2-one

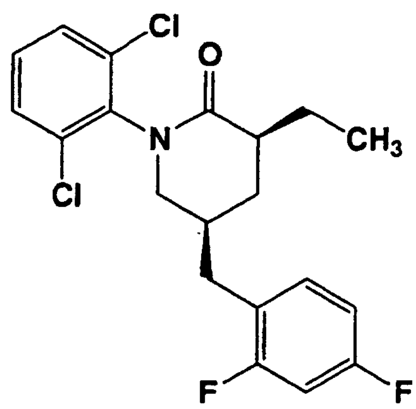

By the same procedure as described in Example 14 using ethyl iodide instead of methyl iodide, the compound of the present invention having the following physical data and the compound of the present invention represented in

Example 14(1)a were obtained.
TLC:Rf 0.60 (ethyl acetate:hexane=2:3);
NMR ($CDCl_3$):d 7.36 (d, J= 8.4 Hz, 2H), 7.21-7.08 (m, 2H), 6.87 (m, 2H), 3.36 (t, J = 11.4 Hz, 1H), 3.23 (ddd, J = 11.4, 4.8, 2.1 Hz, 1H), 2.78-2.61 (m, 2H), 2.51- 2.33 (m, 2H), 2.00 (m, 1H), 1.89 (m, 1H), 1.76 (m, m, 1H), 1.57 (m, 1H), 0.97 (t, J = 7.5 Hz, 3H).

Example 14(1)a

(3RS,5SR)-1-(2,6-dichlorophenyl)-5-(2,4-difluorobenzyl)-3-ethylpiperidin-2-one

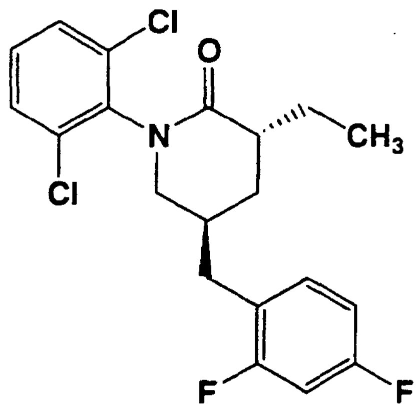

TLC:Rf 0.56 (ethyl acetate:hexane=2:3);
NMR ($CDCl_3$):d 7.41-7.36 (m, 2H), 7.23-7.12 (m, 2H), 6.87-6.77 (m, 2H), 3.41 (dd, J = 11.7, 5.7 Hz, 1H), 3.32 (dd, J = 11.7, 8.4 Hz, 1H), 2.84-2.70 (m, 2H), 2.60-2.42 (m, 2H), 2.02-1.76 (m, 3H), 1.62 (m, 1H), 0.96 (t, J = 7.8 Hz, 3H).

Example 14(2)

1-(2,6-dichlorophenyl)-5-(2,4-difluorobenzyl)-3-methoxymethylpiperidin-2-one

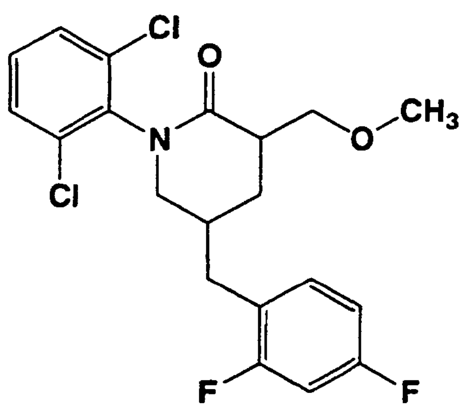

By the same procedure as described in Example 14 using bromomethyl methyl ether instead of methyl iodide, the compound of the present invention having the following physical data was obtained.
TLC:Rf 0.51 (ethyl acetate:hexane=2:3);
NMR ($CDCl_3$):d 7.40-7.34 (m, 2H), 7.22-7.08 (m, 2H), 6.87-6.76 (m, 2H), 3.80-3.68 (m, 2H), 3.45-3.21 (m, 5H), 2.98-2.34 (m, 4H), 2.12 (m, 1H), 1.90-1.65 (m, 1H).

Example 15

1-(2,6-dichlorophenyl)-5,5-bis(2,4-difluorobenzyl)piperidin-2-one

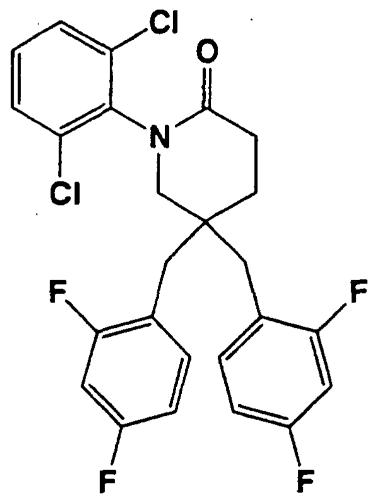

By the same procedure as described in Reference Example 3-Example 2 using ethyl 4-(N-(2,6-dichlorophenyl) carbamoyl]-2,2-bis(2,4-difluorobenzyl)butanoate instead of the compound prepared in Reference Example 2, the compound of the present invention having the following physical data was obtained.
TLC:Rf 0.30 (hexane:ethyl acetate=2:1);
NMR (DMSO-$d_6$):d 7.60-7.50 (m, 2H), 7.42-7.30 (m, 3H), 7.25-7.15 (m, 2H), 7.08-6.98 (m, 2H), 3.34 (s, 2H), 3.00 (d, J = 14.1 Hz, 2H), 2.71 (d, J = 14.1 Hz, 2H), 2.60 (t, J = 6.9 Hz, 2H), 1.58 (t, J = 6.9 Hz, 2H).

Examples 16(1)~16(13)

By the same procedure as described in Reference Example 1→Reference Example 2→Reference Example 3→Example 2 using the corresponding amine derivatives and the corresponding halobenzyl derivatives, the following compounds of the present invention were obtained.

Example 16(1)

1-(4-bromo-2,6-dimethylphenyl)-5-(2,4-difluorobenzyl)piperidin-2-one

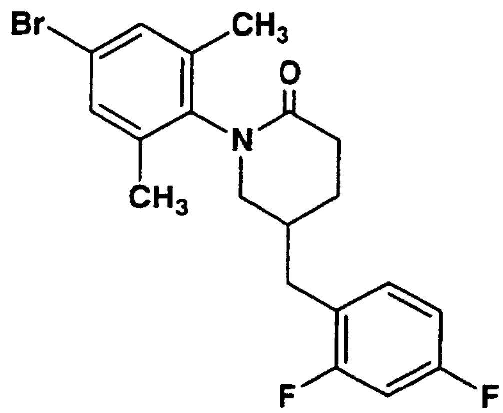

TLC:Rf 0.40 (ethyl acetate:hexane=3:1);
NMR ($CDCl_3$):d 7.24 (s, 2H), 7.15-7.07 (m, 1H), 6.87-6.73 (m, 2H), 3.27-3.10 (m, 2H), 2.73-2.60 (m, 3H), 2.55-2.42 (m, 1H), 2.35-1.92 (m, 8H), 1.74-1.60 (m, 1H).

Example 16(2)

1-(2-methylnaphthalene-1-yl)-5-(2,4-difluorobenzyl)piperidin-2-one

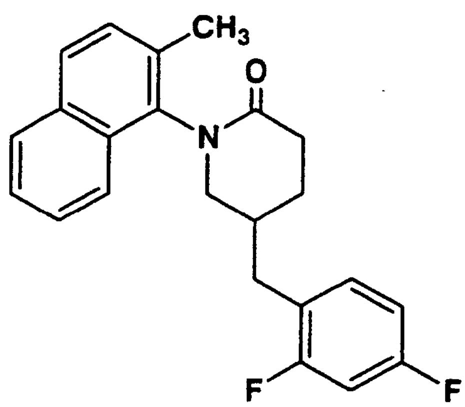

TLC:Rf 0.27 (ethyl acetate:hexane=2:1);
NMR ($CDCl_3$):d 7.88-7.80 (m, 1H), 7.78-7.60 (m, 2H), 7.53-7.33 (m, 3H), 7.19-7.07 (m, 1H), 6.88-6.72 (m, 2H), 3.53-3.25 (m, 2H), 2.88-2.25 (m, 8H), 2.14-2.04 (m, 1H), 1.93-1.68 (m, 1H).

Example 16(3)

1-(2,6-dimethylphenyl)-5-(2-methoxyethoxymethoxybenzyl)piperidin-2-one

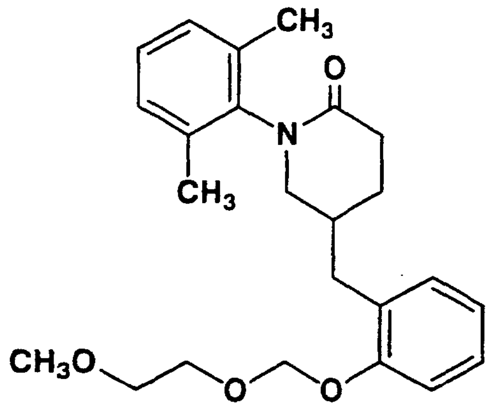

TLC:Rf 0.28 (ethyl acetate:hexane=7:3);
NMR ($CDCl_3$):d 7.22-7.04 (m, 6H), 6.93 (m, 1H), 5.29 (s, 2H), 3.78-3.72 (m, 2H), 3.54-3.49 (m, 2H), 3.37 (s, 3H), 3.31-3.17 (m, 2H), 2.70 (d, J = 6.9 Hz, 2H), 2.67 (ddd, J = 18.3, 5.7, 3.0 Hz, 1H), 2.49 (ddd, J = 18.3, 12.0, 6.6 Hz, 1H), 2.35 (m, 1H), 2.22 (s, 3H), 2.10 (s, 3H), 2.00 (m, 1H), 1.66 (m, 1H).

Example 16(4)

1-(2,6-dichlorophenyl)-5-(4-nitrobenzyl)piperidin-2-one

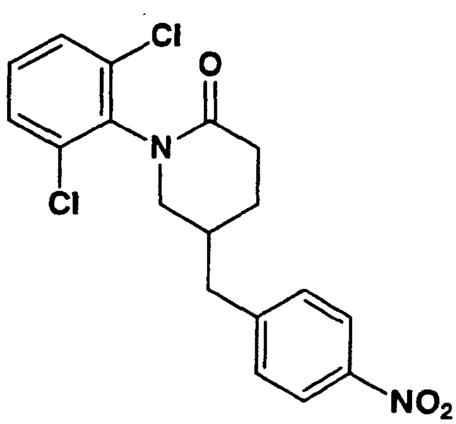

TLC:Rf 0.40 (ethyl acetate:hexane=2:1);
NMR ($CDCl_3$):d 8.19 (d, J = 8.7 Hz, 2H), 7.45-7.32 (m, 4H), 7.26-7.18 (m, 1H), 3.40-3.30 (m, 2H), 2.90-2.86 (m, 2H), 2.75-2.65 (m, 1H), 2.61-2.35 (m, 2H), 2.08-1.93 (m, 1H), 1.86-1.68 (m, 1H).

Example 16(5)

1-(2,6-dichlorophenyl)-5-(2-cyanobenzyl)piperidin-2-one

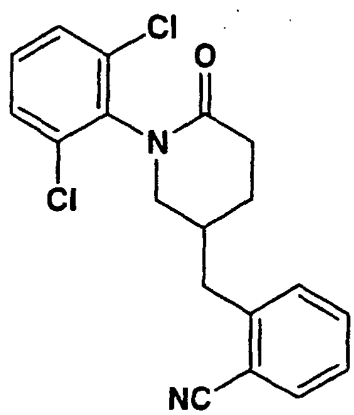

TLC:Rf 0.39 (ethyl acetate:hexane=2:1);
NMR ($CDCl_3$):d 1.82 (m, 1 H) 2.00 (m, 1 H) 2.52 (m, 2 H) 2.70 (m, 1 H) 2.96 (m, 2 H) 3.40 (m, 2 H) 7.21 (m, 1 H) 7.37 (m, 4 H) 7.56 (m, 1 H) 7.67 (m, 1 H).

Example 16(6)

1-(2,6-dimethylphenyl)-5-(2-bromobenzyl)piperidin-2-one

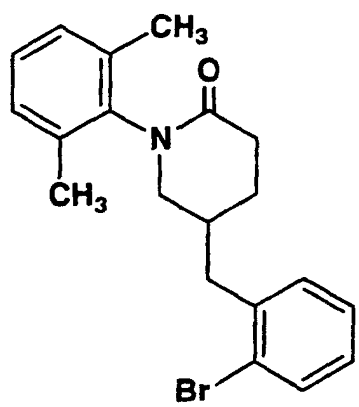

TLC:Rf 0.35 (ethyl acetate);
NMR ($CDCl_3$):d 1.75 (m, 1 H) 2.04 (m, 1 H) 2.12 (s, 3 H) 2.23 (s, 3 H) 2.50 (m, 2 H) 2.69 (m, 1 H) 2.81 (m, 2 H) 3.27 (m, 2 H) 7.10 (m, 4 H) 7.21 (m, 2 H) 7.56 (dd, J=7.97, 1. 10 Hz, 1 H).

Example 16(7)

1-(thiazol-2-yl)-5-(2,4-difluorobenzyl)piperidin-2-one

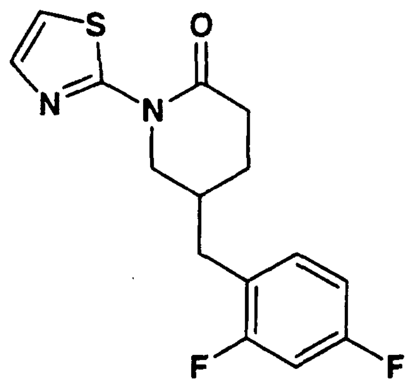

TLC:Rf 0.73 (hexane:ethyl acetate=1:2);
NMR ($CDCl_3$):d 1.62 (m, 1 H) 1.95 (m, 1 H) 2.26 (m, 1 H) 2.73 (m, 4 H) 3.66 (dd, J=13.00,10.44 Hz, 1 H) 4.51 (m, 1 H) 6.85 (m, 2 H) 7.01 (d, J=3.48 Hz, 1 H) 7.16 (m, 1 H) 7. 51 (d, J=3.66 Hz, 1 H).

Example 16(8)

1-(2,6-dichlorophenyl)-5-(2-methoxymethoxymethylbenzyl)piperidin-2-one

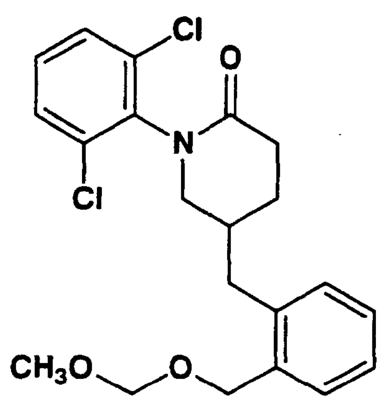

TLC:Rf 0.61 (hexane:ethyl acetate=2:3);
NMR ($CDCl_3$):d 1.75 (m, 1H), 2.01 (m, 1H), 2.60-2.36 (m, 2H), 2.86-2.63 (m, 3H), 3.45-3.32 (m, 5H), 4.60 (d, J = 9.6 Hz, 1H), 4.64 (d, J = 9.6 Hz, 1H), 4.69 (s, 2H), 7.32-7.17 (m, 4H), 7.41-7.36 (m, 3H).

Example 16(9)

1-(4-t-butoxycarbonylamino-2-chlorophenyl)-5-(2,4-difluorobenzyl)piperidin-2-one

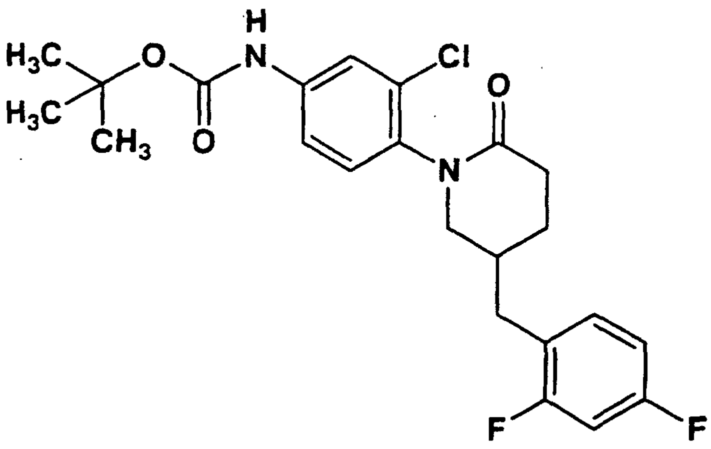

TLC:Rf 0.33 (hexane:ethyl acetate=1:2);
NMR ($CDCl_3$):d 7.58 (s, 1H), 7.20-7.02 (m, 3H), 6.90-6.70 (m, 3H), 3.45-3.30 (m, 2H), 2.78-2.42 (m, 4H), 2.40-2.20 (m, 1H), 2.02-1.90 (m, 1H), 1.78-1.60 (m, 1H), 1.51 (s, 9H).

Example 16(10)

1-(4-t-butoxycarbonylamino-2,6-dichlorophenyl)-5-(2,4-difluorobenzyl)piperidin-2-one

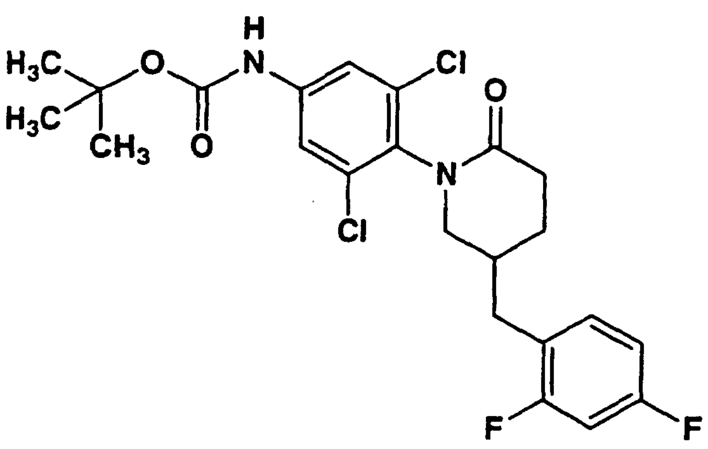

TLC:Rf 0.48 (hexane:acetone=2:3);
NMR ($CDCl_3$):d 7.66-7.53 (m, 2H), 7.20-7.08 (m, 1H), 6.93-6.74 (m, 2H), 6.45-6.32 (m, 1H), 3.13-3.28 (m, 2H), 2.73-2.69 (m, 2H), 2.67-2.61 (m, 1H), 2.55-2.45 (m, 1H), 2.40-2.28 (m, 1H), 2.03-1.90 (m, 1H), 1.75-1.65 (m, 1H), 1.50 (s, 9H).

Example 16(11)

1-(3-t-butoxycarbonylamino-6-chlorophenyl)-5-(2,4-difluorobenzyl)piperidin-2-one

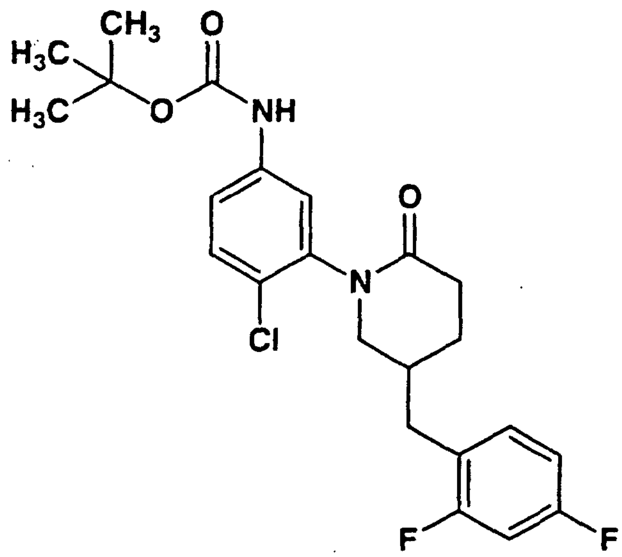

TLC:Rf 0.29 (hexane:ethyl acetate=1:1);
NMR ($CDCl_3$):d 7.53-7.47 (m, 1H), 7.33-7.28 (m, 1H), 7.18-7.08 (m, 1H), 7.06-7.01 (m, 1H), 6.84-6.72 (m, 3H), 3.43-3.25 (m, 2H), 2.71-2.20 (m, 5H), 2.04-1.91 (m, 1H), 1.76-1.64 (m, 1H), 1.50-1.49 (m, 9H).

Example 16(12)

1-(4-t-butoxycarbonylamino-2,6-dichlorophenyl)-5-(2-fluorobenzyl)piperidin-2-one

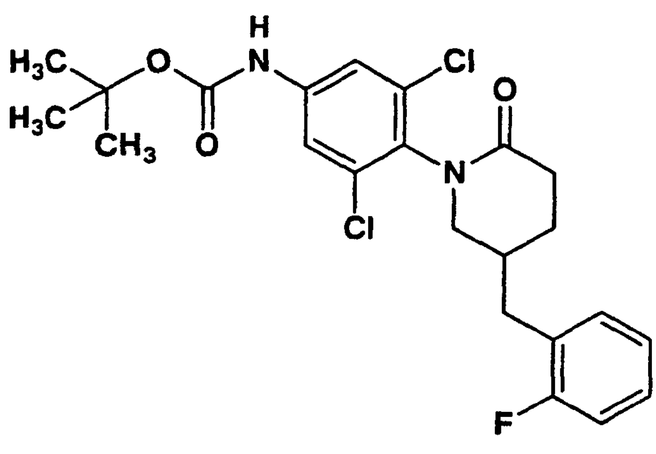

TLC:Rf 0.55 (hexane:ethyl acetate=1:2);
NMR ($CDCl_3$):d 1.49 (s, 9 H) 1.72 (m, 1 H) 1.98 (m, 1 H) 2.38 (m, 1 H) 2.52 (m, 1 H) 2.71 (m, 3 H) 3.33 (d, J=8.00 Hz, 2 H) 7.06 (m, 2 H) 7.19 (m, 2 H) 7.35 (m, 3 H).

Example 16(13)

1-(4-bromo-2,6-dichlorophenyl)-5-(2,4-difluorobenzyl)piperidin-2-one

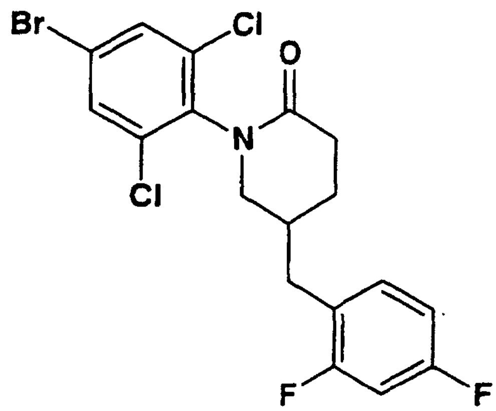

TLC:Rf 0.56 (hexane:ethyl acetate=1:2);
NMR ($CDCl_3$):d 1.71 (m, 1 H) 1.98 (m, 1 H) 2.35 (m, 1 H) 2.51 (m, 1 H) 2.68 (m, 3 H) 3.32 (m, 2 H) 6.83 (m, 2 H) 7.14 (m, 1 H) 7.54 (m, 2 H).

Example 17

1-(2,6-dichlorophenyl)-5-(2,4-difluorobenzyl)-5-hydroxymethylpiperidin-2-one

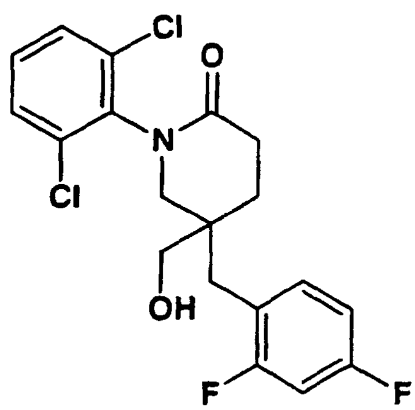

By the same procedure as described in Reference Example 1→Reference Example 2→ Example 2 using 4,4-bis(ethoxycarbonyl)-5-phenylpentanoic acid instead of ethyl glutaryl chloride, the compound of the present invention having the following physical data was obtained.
TLC:Rf 0.37 (ethyl acetate:hexane=4:1);
NMR ($CDCl_3$):d 7.43-7.37 (m, 2H), 7.30-7.20 (m, 2H), 6.90-6.78 (m, 2H), 3.70 (d, J = 5.0 Hz, 2H), 3.55 (d, J = 12.0 Hz, 1H), 3.31 (dd, J = 12.0, 1.5 Hz, 1H), 2.92 (dd, J = 13.8, 1.8 Hz, 1H), 2.83 (dd, J = 13.8, 1.8 Hz, 1H), 2.69 (ddd, J = 18.9, 6.9, 5.1 Hz, 1H), 2.56 (ddd, J = 18.9, 9.9, 6.9 Hz, 1H), 1.93-1.75 (m, 3H).

Example 18

1-(2,6-dimethylphenyl)-5-(3-phenylprop-1-enyl)-5-hydroxymethylpiperidin-2-one

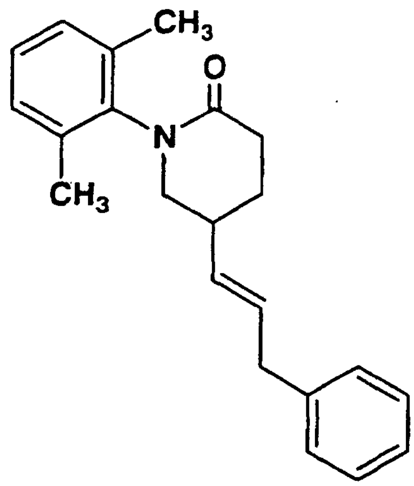

By the same procedure as described in Reference Example 1→Reference Example 2→Reference Example 3→Example 2 using 2,6-dimethylaniline and ((1E)-3-bromoprop-1-enyl)benzene, the compound of the present invention having the following physical data was obtained.
TLC:Rf 0.33 (hexane:ethyl acetate=3:2);
NMR ($CDCl_3$):d 7.38-7.18 (m, 5H), 7.16-7.04 (m, 3H), 6.42 (d, J = 15.9, 1H), 6.18 (dt, J = 15.9, 7.5 Hz, 1H), 3.34 (m, 1H), 3.21 (dd, J = 11.9, 9.3 Hz, 1H), 2.68 (m, 1H), 2.57 (m, 1H), 2.43-2.25 (m, 2H), 2.23-2.02 (m, 8H), 1.69 (m, 1H).

Reference Example 13

3-(2,6-dichlorophenylaminocarbonylamino)-2-(2,4-difluorophenylmethyl)propanoic acid ethyl ester

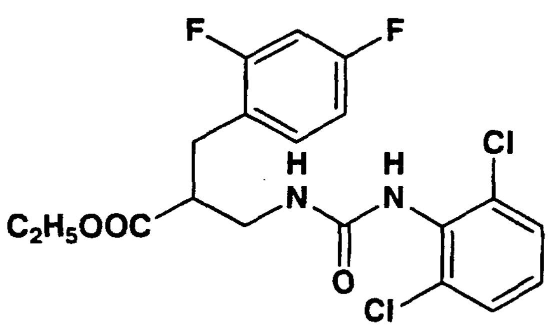

Under an atmosphere of argon, to a solution of 3-amino-2-(2,4-difluorophenylmethyl)propanoic acid ethyl ester (837 mg) and 2,6-dichlorophenyl isocyanate (617 mg) in tetrahydrofuran (20 ml) was added triethylamine (415 µl) at 0°C. The reaction mixture was stirred at room temperature for 2 hours. To the reaction mixture were water and ethyl acetate. The organic layer was washed with brine, dried over anhydrous magnesium sulfate and concentrated. The residue was powdered with isopropyl ether and methanol to give the title compound (803 mg) having the following physical data.
TLC:Rf 0.37 (hexane:ethyl acetate=3:2);
NMR ($CDCl_3$):d 7.38 (d, J = 8.1 Hz, 2H), 7.21-7.11 (m, 2H), 6.82-6.70 (m, 2H), 6.15 (br, 1H), 5.06 (br, 1H), 4.06 (q, J

= 7.2 Hz, 2H), 3.53 (m, 1H), 3.39 (m, 1H), 3.02-2.79 (m, 3H), 1.14 (t, J = 7.2 Hz, 3H).

Example 19

1-(2,6-dichlorophenyl)-5-(2,4-difluorobenzyl)tetrahydropyrimidin-2(1H)-one

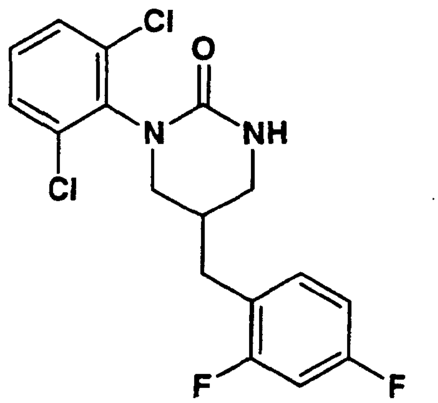

By the same procedure as described in Reference Example 3→Example 2 using the compound prepared in Reference Example 19, the compound of the present invention having the following physical data was obtained.
TLC:Rf 0.32 (ethyl acetate:hexane=4:1);
NMR ($CDCl_3$):d 7.41-7.35 (m, 2H), 7.22-7.13 (m, 2H), 6.88-6.77 (m, 2H), 4.98 (br, 1H), 3.45-3.35 (m, 3H), 3.19 (m, 1H), 2.88-2.72 (m, 2H), 2.59 (m, 1H).

Example 20

1-(4-bromo-2,6-dichlorophenyl)-5-(2,4-difluorobenzyl)tetrahydropyrimidin-2(1H)-one

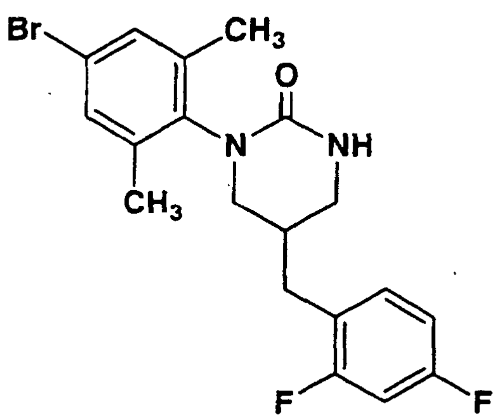

By the same procedure as described in Reference Example 13→Example 19 using 4-bromo-2,6-dichlorophenyl isocyanate instead of 2,6-dichlorophenyl isocyanate, the compound of the present invention having the following physical data was obtained.
TLC:Rf 0.47 (ethyl acetate:toluene= 2:1);
NMR ($CDCl_3$):d 2.15 (s, 3 H) 2.25 (s, 3 H) 2.51 (m, 1 H) 2.74 (m, J=7.83, 7.83 Hz, 2 H) 3.27 (m, 4 H) 4.88 (m, J=3.02 Hz, 1 H) 6.83 (m, 2 H) 7.13 (m, 1 H) 7.22 (s, 2 H).

Reference Example 14

3-(2,6-dichlorophenylaminocarbonyloxy)-2-(2,4-difluorophenylmethyl)propanoic acid ethyl ester

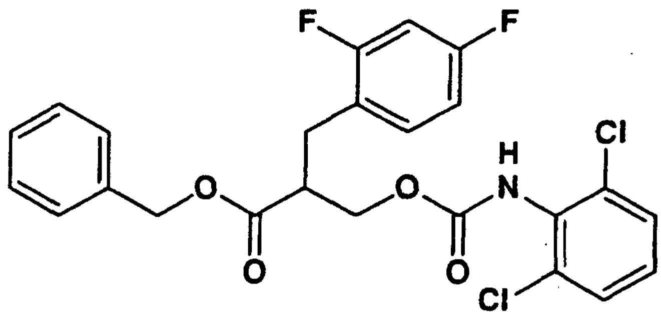

By the same procedure as described in Reference Example 13 using 3-hydroxy-2-(2,4-difluorophenylmethyl) propanoic acid benzyl ester instead of 3-amino-2-(2,4-difluorophenylmethyl)propanoic acid ethyl ester, the title compound having the following physical data was obtained.
TLC:Rf 0.43 (hexane:ethyl acetate=7:3);
NMR ($CDCl_3$):d 2.96 (m, 3 H) 4.37 (m, 2 H) 5.09 (s, 2 H) 6.20 (m, 1 H) 6.74 (m, 2 H) 7.06 (m, 1 H) 7.23 (m, 3 H) 7.35 (m, 5 H).

Reference Example 15

3-(2,6-dichlorophenylaminocarbonyloxy)-2-(2,4-difluorophenylmethyl)propanoic acid

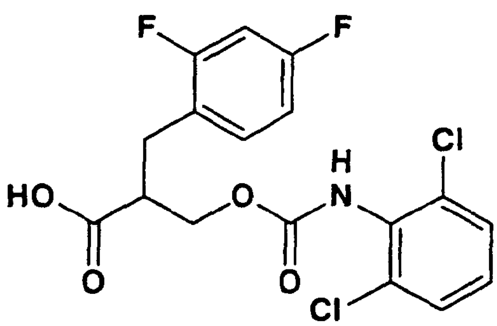

To a solution of the compound prepared in Reference Example 14 (256 mg) in methanol (5 ml) was added 5% palladium-carbon (50 mg). The mixture was stirred at room temperature for 30 minutes under an atmosphere of hydrogen. The reaction mixture was filtrated through Celite (proprietary name) and concentrated to give the title compound having the following physical data.
TLC:Rf 0.15 (hexane:ethyl acetate=2:3);
NMR ($CDCl_3$):d 3.03 (m, 3 H) 4.36 (m, 2 H) 6.44 (m, 1 H) 6.81 (m, 2 H) 7.17 (m, 2 H) 7.35 (m, 2 H).

Reference Example 16

3-(2,6-dichlorophenylaminocarbonyloxy)-2-(2,4-difluorophenylmethyl)propanoic acid

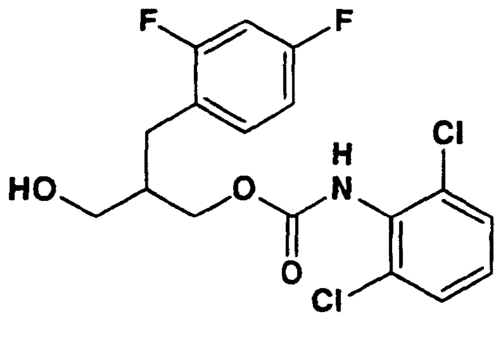

By the same procedure as described in Reference Example 5 using the compound prepared in Reference Example 15 instead of 4-amino-3,5-dichlorobenzoic acid, the title compound having the following physical data was obtained.
TLC:Rf 0.35 (hexane:ethyl acetate=3:2);
NMR ($CDCl_3$):d 2.12 (m, 1 H) 2.44 (t, J=6.59 Hz, 1 H) 2.65 (m, 2 H) 3.60 (m, 2 H) 4.17 (m, 1 H) 4.32 (m, 1 H) 6.33 (m, 1 H) 6.80 (m, 2 H) 7.15 (m, 2 H) 7.40 (m, 2 H).

Example 21

3-(2,6-dichlorophenyl)-5-(2,4-difluorobenzyl)-1,3-tetrahydrooxazin-2-one

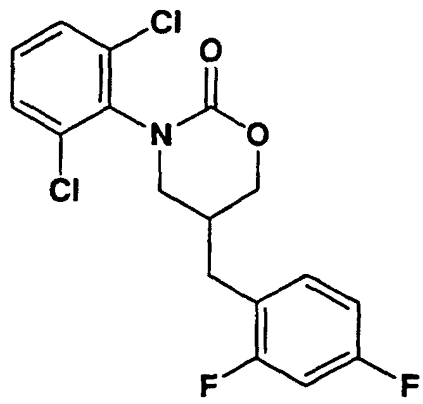

By the same procedure as described in Example 2 using the compound prepared in Reference Example 16, the compound of the present invention having the following physical data was obtained.
TLC:Rf 0.39 (ethyl acetate:toluene= 1:4);
NMR ($CDCl_3$):d 2.64 (m, 1 H) 2.86 (m, 2 H) 3.40 (m, J=10.99, 7.42 Hz, 1 H) 3.56 (m, 1 H) 4.19 (dd, J=10.99, 7.69 Hz, 1 H) 4.39 (m, 1 H) 6.85 (m, 2 H) 7.23 (m, 2 H) 7.41 (m, 2 H).

Reference Example 17

2-((2,6-dichlorophenyl)aminocarbonylmethylamino)-3-(2,4-difluorophenyl)propanoic acid methyl ester

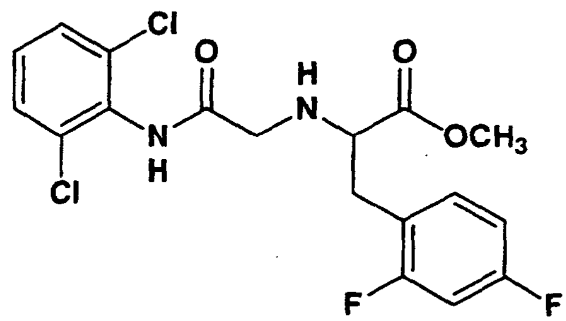

To a solution of 2-amino-3-(2,4-difluorophenyl)propanoic acid methyl ester (1 g) in dimethylforrmamide (10 ml) were added triethylamine (1.1 ml) and N-(2,6-dichlorophenyl)-2-bromoacetamide (1.69 g) at room temperature. The reaction mixture was stirred at 90°C for 3 hours. To the reaction mixture were added water and ethyl acetate. The organic layer was washed with brine, dried over anhydrous magnesium sulfate and concentrated. The obtained residue was purified by column chromatography on silica gel (hexane:ethyl acetate=7:3→3:2) to give the title compound (0.92 g) having the following physical data.
TLC:Rf 0.50 (hexane:ethyl acetate=1:1);
NMR ($CDCl_3$):d 2.31 (m, 1 H) 2.86 (m, 1 H) 3.09 (m, 1 H) 3.20 (d, J=17.86 Hz, 1 H) 3.57 (m, 2 H) 3.77 (s, 3 H) 6.69 (m, 2 H) 7.16 (m, 2 H) 7.33 (d, J=8.24 Hz, 2 H) 8.25 (s, 1 H).

Reference Example 18

2-(N-((2,6-dichlorophenyl)aminocarbonyl)methyl-N-acetylamino)-3-(2,4-difluorophenyl)propanoic acid methyl ester

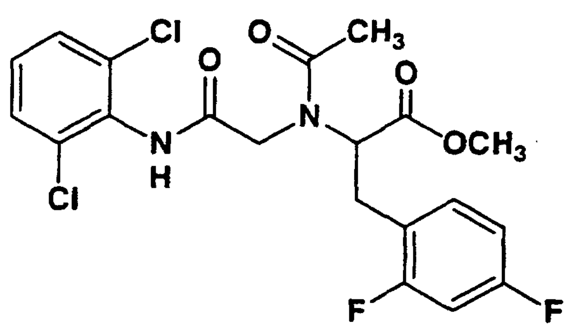

To a solution of the compound prepared in Reference Example 17 (130mg) in dichloromethane were added acetyl chloride (24 µl) and triethylamine (42 µl) at 0°C. The reaction mixture was stirred for 15 minutes. To the reaction mixture were added water and ethyl acetate. The organic layer was washed with brine, dried over anhydrous magnesium sulfate and concentrated to give the title compound (93 mg) having the following physical data.
TLC:Rf 0.52 (hexane:ethyl acetate=1:1);
NMR ($CDCl_3$):d 2.18 (s, 3 H) 3.52 (m, 3 H) 3.87 (s, 3 H) 4.02 (m, 2 H) 6.87 (m, 2 H) 7.16 (m, 2 H) 7.39 (m, 2 H) 10.06 (s, 1 H).

Example 22

1-(2,6-dichlorophenyl)-5-(2,4-difluorobenzyl)-4-acetylpiperazin-2-one

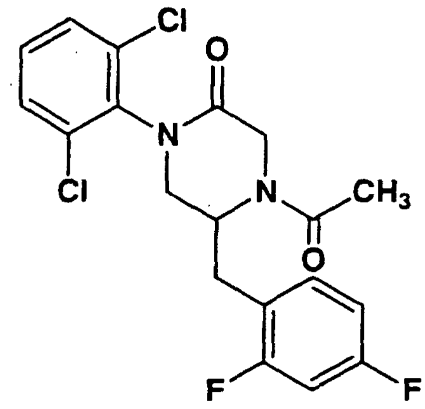

TLC:Rf 0.40 (toluene:ethyl acetate=3:7);
NMR ($CDCl_3$):d 2.05 and 1.80 (s and s, 3H), 3.45-3.05 (m, 3H), 4.12 and 3.97 (m, 1H), 4.27 and 3.96 (d and d, J = 18.1 Hz and J = 19.5 Hz, 1H), 5.08 and 4.43 (d and d, J = 19.5 Hz, and J = 18.1 Hz, 1H), 5.35 and 4.39 (m, 1H), 6.91-6.77 (m, 2H), 7.34-7.13 (m, 2H), 7.46-7.40 (m, 2H).

Reference Example 19

2-((2,6-dichlorophenyl)aminocarbonyl)methyloxy)-3-(2,4-difluorophenyl)propanoic acid ethyl ester

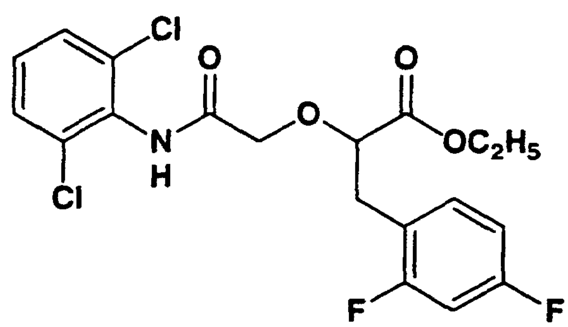

Under an atmosphere of argon, to a solution of 1-((2,6-dichlorophenyl)aminocarbonyl)methyloxyacetic acid methyl ester (290 mg) and 2,4-difluorobenzyl bromide (134 μl) in tetrahydrofuran (10 ml) was added lithium bis(trimethylsilyl)amide (1.0M solution in tetrahydrofuran, 2.09 ml) at -78°C. The reaction mixture was stirred at below -60°C for 15 minutes. A saturated aqueous ammonium chloride solution was added to the reaction mixture, which was extracted ethyl acetate. The extract was washed with water and brine, dried over anhydrous magnesium sulfate and concentrated. The obtained residue was purified by column chromatography on silica gel (hexane:ethyl acetate=4:1→7:3) to give the title compound (53 mg) having the following physical data.
TLC:Rf 0.56 (toluene:ethyl acetate=1:1);
NMR ($CDCl_3$):d 7.96 (s, 1H), 7.39 (d, J = 7.8 Hz, 2H), 7.32-7.15 (m, 2H), 6.85-6.72 (m, 2H), 4.34-4.04 (m, 5H), 3.27 (m, 1H), 3.08 (m, 1H), 1.28 (t, J = 7.8 Hz, 3H).

Example 23

4-(2,6-dichlorophenyl)-6-(2,4-difluorobenzyl)morpholin-3-one

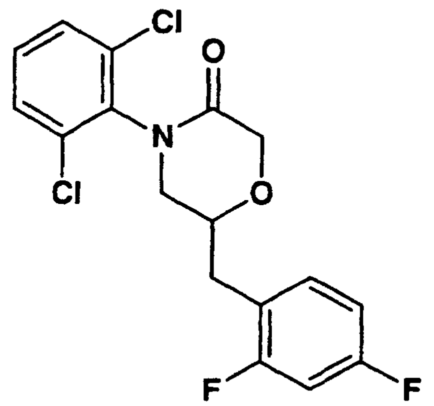

By the same procedure as described in Reference Example 3→Example 2 using the compound prepared in Reference Example 19, the compound of the present invention having the following physical data was obtained.
TLC:Rf0.49 (ethyl acetate:hexane=2:3);
NMR ($CDCl_3$):d 2.92 (m, 2 H) 3.29 (dd, J=11.40, 2.88 Hz, 1 H) 3.70 (t, J=11.40 Hz, 1 H) 4.19 (m, 1 H) 4.30 (d, J=17.03 Hz, 1 H) 4.48 (d, J=17.03 Hz, 1 H) 6.82 (m, 2 H) 7.24 (m, 2 H) 7.40 (m, 2 H).

Reference Example 20

4-((2,6-dichlorophenyl)aminocarbonyl)-2-((2,4-difluorophenyl)hydroxymethyl)butanoic acid ethyl ester

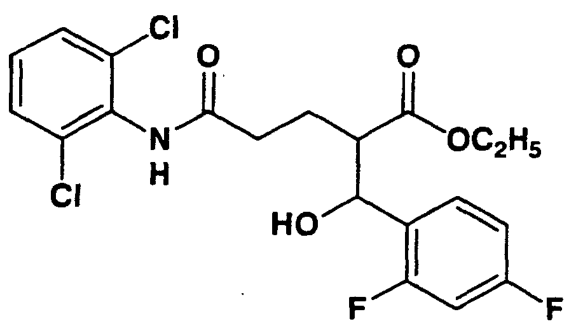

Under an atmosphere of argon, to a solution of the compound prepared in Reference Example 1 (1.52 g) in tetrahydrofuran (15 ml) was added lithium bis(trimethylsilyl)amide (1.0M solution in tetrahydrofuran, 11.0 ml) at -78°C. The mixture was stirred for 30 minutes and 2,4-difluorobenzaldehyde (602 µl) was added thereto. The reaction mixture was stirred at -60°C for 4 hours. An aqueous ammonium chloride solution was added to the reaction mixture, which was extracted ethyl acetate. The extract was washed with water and brine, dried over anhydrous magnesium sulfate and concentrated. The obtained residue was purified by column chromatography on silica gel (hexane:ethyl acetate=7:3) to give the title compound (1.90 g) having the following physical data.
TLC:Rf 0.36 (hexane:ethyl acetate=3:2);
NMR ($CDCl_3$):d 7.53-7.32 (m, 3H), 7.18 (m, 1H), 7.04 (s, 1H), 6.85 (m, 1H), 6.79 (m, 1H), 5.23 and 5.18 (m and m, 1H), 4.22-4.00 (m, 2H), 3.37 and 3.28 (m and d, J = 6.9 Hz, 1H), 2.99 (m, 1H), 2.65-2.32 (m, 2H), 2.30-1.85 (m, 2H), 1.30-1.00 (m, 3H).

Reference Example 21

4-((2,6-dichlorophenyl)aminocarbonyl)-2-(1-(2,4-difluorophenyl)-1-(t-butyldimethylsilyloxy)methyl)butanoic acid ethyl ester

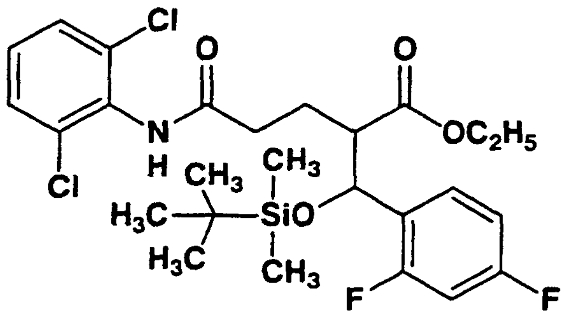

Under an atmosphere of argon, to a solution of the compound prepared in Reference Example 20 (1.85 g) in dimethylformamide (15 ml) were added imidazole (1.69 g) and t-butyldimethylsilyl chloride (1.37g) at room temperature. The reaction mixture was stirred at room temperature for 36 hours. The reaction mixture was diluted with ethyl acetate, washed with 1N hydrochloric acid, water, a saturated aqueous sodium hydrogen carbonate solution, water and brine, dried over anhydrous magnesium sulfate and concentrated. The obtained residue was purified by column chromatography on silica gel (hexane:ethyl acetate=4:1) to give the title compound (2.16 g) having the following physical data.
TLC:Rf 0.73 (hexane:ethyl acetate=3:2);
NMR ($CDCl_3$):d 7.46-7.32 (m, 3H), 7.20-7.00 (m, 2H), 6.86 (m, 1H), 6.76 (m, 1H), 5.23 and 5.18 (d, J = 5.7 Hz and d, J = 7.5 Hz, 1H), 4.27-3.98 (m, 2H), 3.02-1.75 (m, 5H), 1.32 and 1.17 (t, J = 6.9 Hz and t, J = 6.9 Hz, 3H), 0.86 and 0.81 (s and s, 9H), 0.046 and 0.016 (s and s, 3H), -0.21 and -0.27 (s and s, 3H).

Reference Example 22

1-(2,6-dichlorophenyl)-5-(1-(2,4-difluorophenyl)1-(t-butyldimethylsilyloxy)methyl)piperidin-2-one

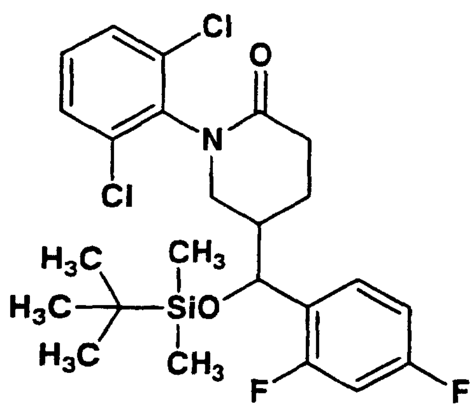

By the same procedure as described in Reference Example 3→Example 2 using the compound prepared in Reference Example 21, the title compound having the following physical data was obtained.
TLC:Rf 0.39 (hexane:ethyl acetate=7:3);
NMR ($CDCl_3$):d 7.49-7.32 (m, 3H), 7.20 (m, 1H), 6.89 (m, 1H), 6.78 (m, 1H), 4.97-4.90 (m, 1H), 3.54 and 3.47 (t, J = 11.4 Hz and t, J = 11.4 Hz, 1H), 3.38 and 3.12 (m and ddd, J = 11.4, 4.8, 1.8 Hz, 1H), 2.68 (m, 1H), 2.49 (m, 1H), 2.36 (m, 1H), 2.12 (m, 1H), 1.86-1.68 (m, 1H), 0.89 and 0.86 (s and s, 9H), 0.046 and 0.020 (s and s, 3H), -0.19 and-0.22

(s and s, 3H).

Example 24

1-(2,6-dichlorophenyl)-5-((2,4-difluorophenyl)hydroxymethyl)piperidin-2-one

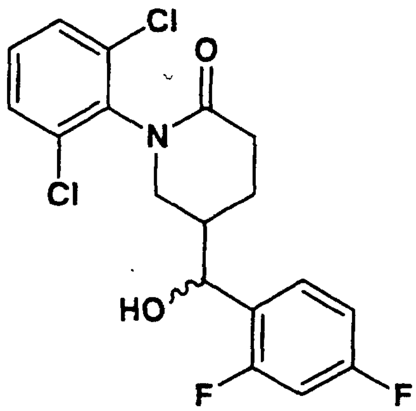

less polar (1)

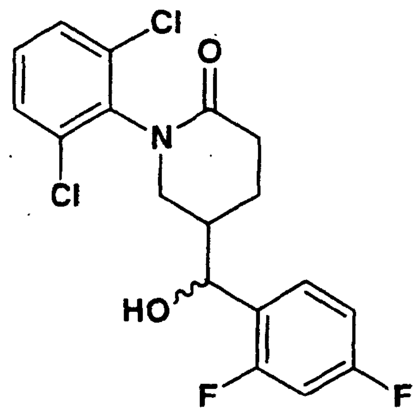

more polar (2)

Under an atmosphere of argon, to a solution the compound prepared in Reference Example 22 (870 mg) in tetrahydrofuran (15 ml) was added t-butylammonium fluoride $3H_2O$ (1.10 g) at room temperature. The reaction mixture was stirred at room temperature for 1 hour. The reaction mixture was diluted with ethyl acetate, washed with 1N hydrochloric acid, water, a saturated aqueous sodium hydrogen carbonate solution, water and brine, dried over anhydrous magnesium sulfate and concentrated. The obtained residue was purified by column chromatography on silica gel (hexane:ethyl acetate=3:2) to give the compound (1) (59 mg) and (2) (353 mg) of the present invention having the following physical data.

Example 24(1)

TLC:Rf 0.50 (ethyl acetate:hexane=3:2);
NMR ($CDCl_3$):d 7.48 (m, 1H), 7.39 (d, J = 7.8 Hz, 2H), 7.22 (m, 1H), 6.94 (m, 1H), 6.82 (m, 1H), 4.96 (dd, J = 8.4, 4.2 Hz, 1H), 3.70-3.57 (m, 2H), 2.63 (ddd, J = 17.7, 5.4, 4.2 Hz, 1H), 2.53-2.38 (m, 2H), 2.09 (d, J = 4.2 Hz, 1H), 1.82-1.62 (m, 2H).

Example 24(2)

TLC:Rf 0.42 (ethyl acetate:hexane=3:2);
NMR ($CDCl_3$):d 7.44 (m, 1H), 7.40-7.32 (m, 2H), 7.19 (m, 1H), 6.80 (m, 1H), 6.92 (m, 1H), 4.95 (dd, J = 6.9, 4.5 Hz, 1H), 3.46 (t, J = 11.4 Hz, 1H), 3.13 (ddd, J = 11.4, 5.4, 2.1 Hz, 1H), 2.73 (ddd, J = 17.7, 5.4, 2.7 Hz, 1H), 2.60-2.40 (m, 2H), 2.22 (m, 1H), 2.09 (d, J = 4.5 Hz, 1H), 1.85 (m, 1H).

Examples 25(1)~25(4)

By the same procedure as described in Reference Example 20→Reference Example 21→Reference Example 22→Example 24 using the corresponding amide derivatives and aldehyde derivatives, the following compounds were obtained.

Example 25(1)

1-(4-t-butoxycarbonylamino-2,6-dichlorophenyl)-5-((2,4-difluorophenyl)hydroxymet hyl)piperidin-2-one

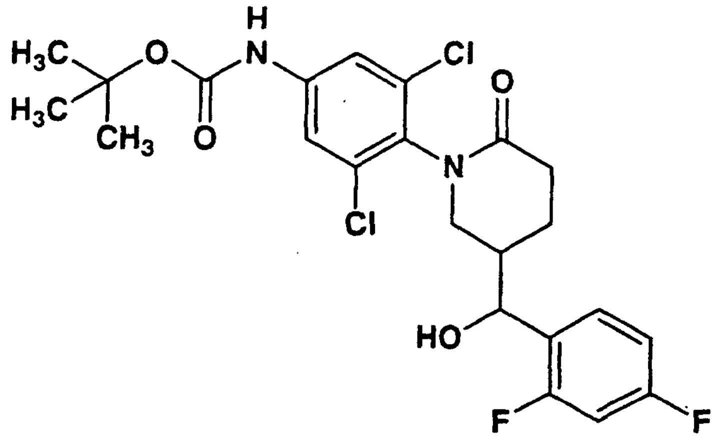

TLC:Rf 0.35 and 0.20 (hexane:ethyl acetate=1:1);
NMR ($CDCl_3$):d 1.50 and 1.51 (s and s, 9H), 1.90-1.60 (m, 1H), 1.99 (m, 1H), 2.56-2.10 (m, 3H), 3.07 (m, 1H), 3.42 (t, J = 10.2 Hz, 1H), 3.60 (m, 1H), 4.95 (m, 1H), 6.98-6.70 (m, 3H), 7.55-7.35 (m, 3H).

Example 25(2) and Example 25(3)

1-(4-bromo-2,6-dichlorophenyl)-5-((2-thienyl)hydroxymethyl)piperidin-2-one

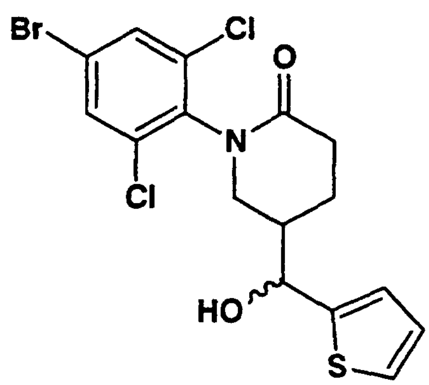

less polar (2)

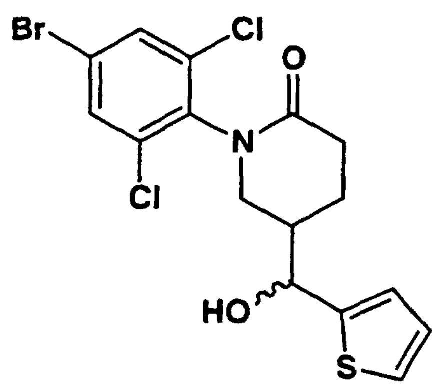

more polar (3)

Example 25(2)

TLC:Rf 0.52 (hexane:ethyl acetate=1:1);
NMR ($CDCl_3$):d 1.76 (m, 2 H) 2.28 (br. s., 1 H) 2.54 (m, 3 H) 3.58 (dd, J=11.90, 9.52 Hz, 1 H) 3.70 (dd, J=11.90, 5.49 Hz, 1 H) 4.90 (d, J=8.42 Hz, 1 H) 7.02 (m, 2 H) 7.32 (d , J=4.94 Hz, 1 H) 7.57 (m, 2 H).

Example 25(3)

TLC:Rf 0.35 (hexane:ethyl acetate=1:1);

NMR ($CDCl_3$):d 1.88 (m, 1 H) 2.35 (m, J=3.48, 1.46 Hz, 2 H) 2.52 (m, J=17.76 Hz, 2 H) 2.69 (m, J=1.83 Hz, 1 H) 3.17 (m, 1 H) 3.32 (dd, J=11.53, 10.44 Hz, 1 H) 4.89 (d, J=7.69 Hz, 1 H) 6.98 (m, 2 H) 7.29 (m, 1 H) 7.51 (m, 2 H).

Example 25(4)

1-(4-bromo-2,6-dichlorophenyl)-5-((2,4-difluorophenyl)hydroxymethyl)piperidin-2-one

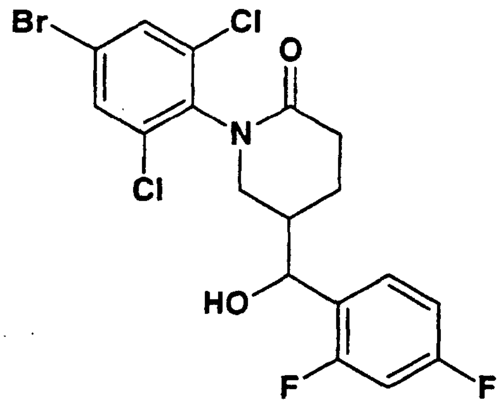

TLC:Rf 0.22 and 0.11 (hexane:ethyl acetate=1:1);
NMR ($CDCl_3$):d 2.78-1.64 (m, 6H), 3.61-3.08 (m, 2H), 4.97 (m, 1H), 6.98-6.77 (m, 2H), 7.60-7.40 (m, 3H).

Reference Example 23

4-((2,6-dichlorophenyl)aminocarbvnyl)-2-(2-phenyl-1-hydroxyethyl)butanoic acid ethyl ester

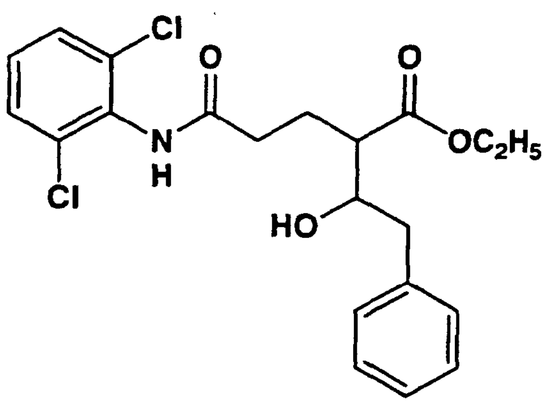

By the same procedure as described in Reference Example 20 using 2-phenylethanal instead of 2,4-difluorobenzaldehyde, the title compound having the following physical data was obtained.
TLC:Rf 0.46 (hexane:ethyl acetate=1:1);
NMR ($CDCl_3$):d 7.37 (d, J = 8.1 Hz, 2H), 7.36-7.02 (m, 7H), 4.31-3.94 (m, 3H), 2.94-2.10 (m, 8H), 1.38-1.20 (m, 3H).

Reference Example 24

4-((2,6-dichlorophenyl)aminocarbonyl)-2-(2-phenyl-1-((imidazol-1-yl)thioxomethoxy)ethyl)butanoic acid ethyl ester

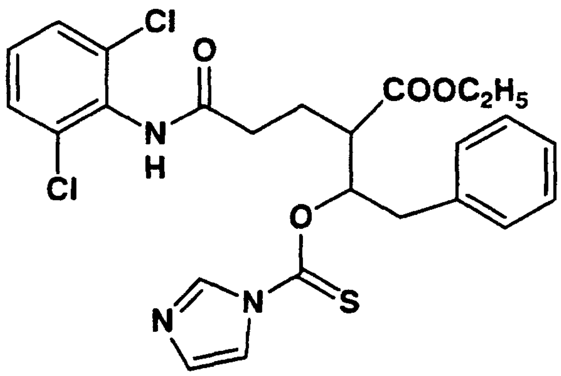

To a solution of the compound prepared in Reference Example 23 (1.32 g) in dichloroethane (20 ml) was added 1,1'-thiocarbonyldiimidazole (1.22 g) at room temperature. The reaction mixture was stirred at room temperature for 2 hours. The reaction mixture was diluted with ethyl acetate, washed with 2N hydrochloric acid and brine, dried over anhydrous magnesium sulfate and concentrated. The obtained residue was purified by column chromatography on silica gel (hexane:ethyl acetate=1:1) to give the compound of the present invention (1.32 g) having the following physical data.
TLC:Rf 0.32 (hexane:ethyl acetate=3:2).

Reference Example 25

4-((2,6-dichlorophenyl)aminocarbonyl)-2-(2-phenylethyl)butanoic acid ethyl ester

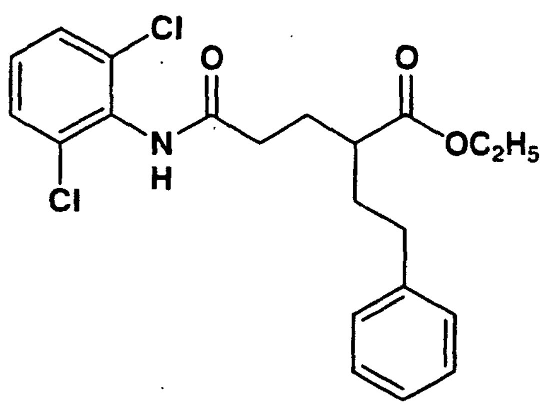

A solution of the compound prepared in Reference Example 24 (1.3 g), 2,2'-azobis-isobutyronitrile (80 mg) and tributyltin hydride (1.29ml) in toluene (20 ml) was stirred at 120°C for 2 hours. The reaction mixture was concentrated, diluted with acetonitrile and washed with hexane, and the acetonitrile layer was concentrated. The obtained residue was purified by column chromatography on silica gel (hexane:ethyl acetate=4:1) to give the compound of the present invention (867 mg) having the following physical data.
TLC:Rf 0.21 (hexane:ethyl acetate=4:1);
NMR ($CDCl_3$):d 7.36 (d, J = 8.1 Hz, 2H), 7.33-7.14 (m, 6H), 7.04 (s, 1H), 4.24-4.15 (m, 2H), 2.70-2.30 (m, 5H), 2.12-1.90 (m, 3H), 1.81 (m, 1H), 1.38-1.20 (m, 3H).

Example 26

1-(2,6-dichlorophenyl)-5-(2-phenylethyl)piperidin-2-one

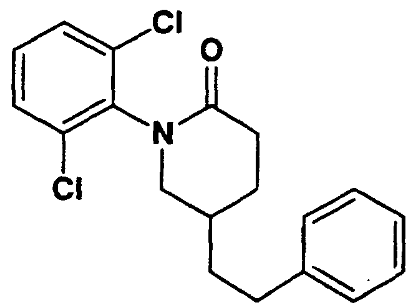

By the same procedure as described in Reference Example 3-Example 2 using the compound prepared in Reference Example 25, the compound of the present invention having the following physical data was obtained.
TLC:Rf 0.35 (toluene:ethyl acetate=4:1);
NMR ($CDCl_3$):d 7.43-7.36 (m, 2H), 7.35-7.16 (m, 6H), 3.48 (ddd, J = 11.4, 5.1, 1.8 Hz, 1H), 3.31 (dd, J = 11.4, 9.9 Hz, 1H), 2.73-2.60 (m, 3H), 2.54 (ddd, J = 16.8, 10.8, 6.3 Hz, 1H),2.18-2.00 (m, 2H), 1.81-1.62 (m, 3H).

Reference Example 26

4-(2,4-difluorophenylmethyl)-5-hydroxypentanoic acid ethyl ester

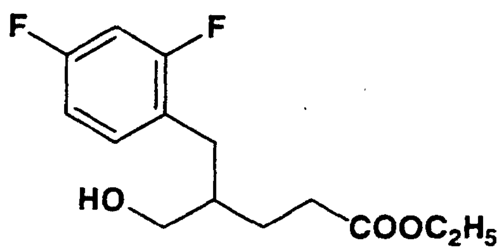

By the same procedure as described in Reference Example 5 using 2-(2,4-difluorophenylmethyl)-4-ethoxycarbonylbutanoic acid, the title compound having the following physical data was obtained.
TLC:Rf 0.41 (hexane:ethyl acetate=1:1);
NMR ($CDCl_3$):d 1.25 (t, J=7.14 Hz, 3 H) 1.71 (m, 4 H) 2.38 (m, 2 H) 2.65 (m, 2 H) 3.50 (m, 2 H) 4.13 (q, J=7.14 Hz, 2 H) 6.80 (m, 2 H) 7.14 (m, 1 H).

Reference Example 27

4-(2,4-difluorophenylmethyl)-5-methylsulfonyloxypentanoic acid ethyl ester

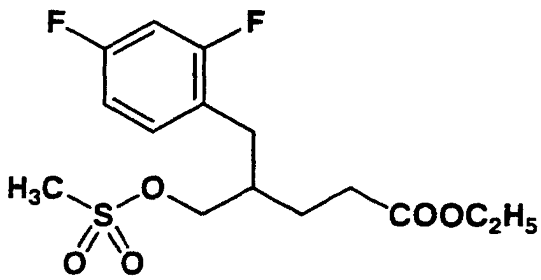

To a solution of the compound prepared in Reference Example 26 (1 g) in pyridine (6 ml) was added methanesulfonyl chloride (0.76 ml) at 0°C. The reaction mixture was stirred at room temperature for 30 minutes. The reaction mixture was poured in 1N hydrochloric acid and extracted with t-butyl methyl ether. The extract was washed with 1N hydrochloric acid, water and brine, dried over anhydrous sodium sulfate and concentrated to give the title compound (1.21 g) having the following physical data.
TLC:Rf 0.50 (hexane:ethyl acetate=1:1);
NMR ($CDCl_3$):d 1.25 (t, J=7.14 Hz, 3 H) 1.77 (m, 2 H) 2.08 (m, 1 H) 2.41 (m, 2 H) 2.71 (m, 2 H) 3.01 (s, 3 H) 4.10 (m, 4 H) 6.82 (m, 2 H) 7.16 (m, 1 H).

Reference Example 28

4-(2,4-difluorophenylmethyl)-5-cyclohexylaminopentanoic acid ethyl ester

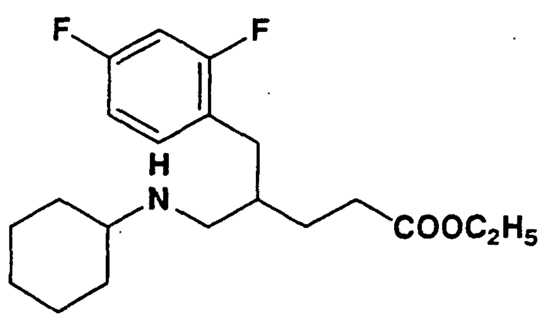

To a solution of cyclohexylamine (0.52 ml) in toluene (2 ml) was added a solution of the compound prepared in Reference Example 27 (150 mg) in toluene (1 ml). The reaction mixture was refluxed for 3.5 hours. The reaction mixture was poured in 1N hydrochloric acid and extracted with ethyl acetate. The extract was washed with a saturated aqueous sodium hydrogen carbonate solution, water and brine, dried over anhydrous sodium sulfate and concentrated to give the title compound (120mg) having the following physical data.
TLC:Rf 0.18 (ethyl acetate:methanol=10:1);
NMR ($CDCl_3$):d 1.03 (m, 2 H) 1.24 (t, J=7.14 Hz, 3 H) 1.41 (m, 2 H) 1.71 (m, 8 H) 2.34 (m, 4 H) 2.61 (m, 4 H) 4.11 (q, J=7.14 Hz, 2 H) 6.80 (m, 2 H) 7.15 (m, 1 H).

Example 27

1-cyclohexyl-5-(2,4-difluorobenzyl)piperidin-2-one

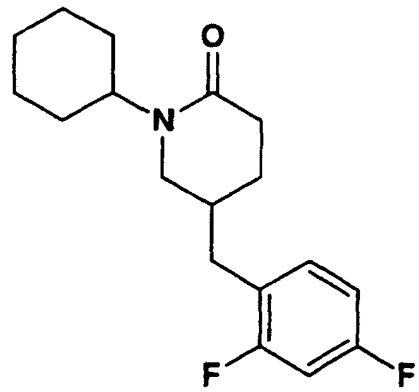

TLC:Rf 0.25 (ethyl acetate:hexane=2: 1);
NMR ($CDCl_3$):d 1.06 (m, 1 H) 1.36 (m, 5 H) 1.62 (m, 3 H) 1.79 (m, 3 H) 2.02 (m, 1 H) 2.32 (m, 1 H) 2.48 (m, 1H) 2.63 (m, 2 H) 2.87 (m, J=11.95, 9.75 Hz, 1 H) 3.21 (m, J=12.09 , 4.67, 1.65 Hz, 1 H) 4.46 (m, 1 H) 6.82 (m, 2 H) 7.11 (m, 1 H).

Examples 28(1) and 28(2)

By the same procedure as described in Reference Example 28→Example 27 using the corresponding amine derivatives instead of cyclohexylamine, the following compounds of the present invention were obtained.

Example 28(1)

1-(2-(2-hydroxyethyl)phenyl)-5-(2,4-difluorobenzyl)piperidin-2-one

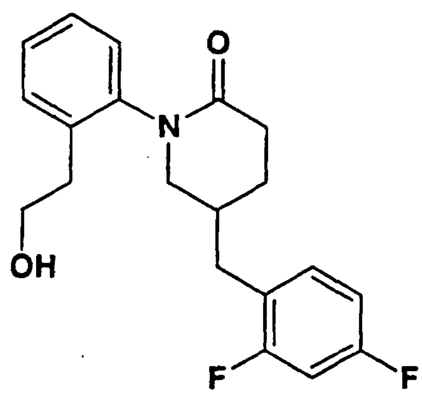

TLC:Rf 0.44 (ethyl acetate:methanol=10:1);
NMR ($CDCl_3$):d 1.73 (m, 1 H) 2.01 (m, 1 H) 2.33 (m, 1 H) 2.63 (m, 7 H) 3.41 (m, 2 H) 3.87 (m, 2 H) 6.82 (m, 2 H) 7.10 (m, 2 H) 7.36 (m, 3 H).

Example 28(2)

1-(3,5-dichlorophenyl)-5-(2,4-difluorobenzyl)piperidin-2-one

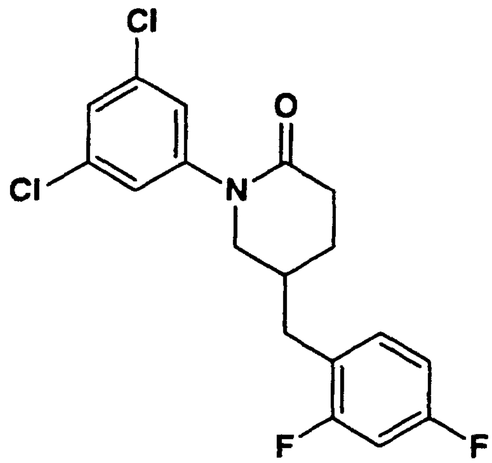

TLC:Rf 0.60 (ethyl acetate:hexane=2:1);
NMR ($CDCl_3$):d 1.67 (m, 1 H) 1.99 (m, 1 H) 2.28 (m, 1 H) 2.51 (m, 1 H) 2.66 (m, 3 H) 3.45 (m, 2 H) 6.83 (m, 2 H) 7.11 (m, 1 H) 7.16 (d, J=1.80 Hz, 2 H) 7.25 (t, J=1.80 Hz, 1 H ).

Reference Example 29

(2S)-4-benzyloxycarbonyl-2-(t-butoxycarbonylamino)butanoic acid 2-(trimethylsilyloxy)ethyl esther

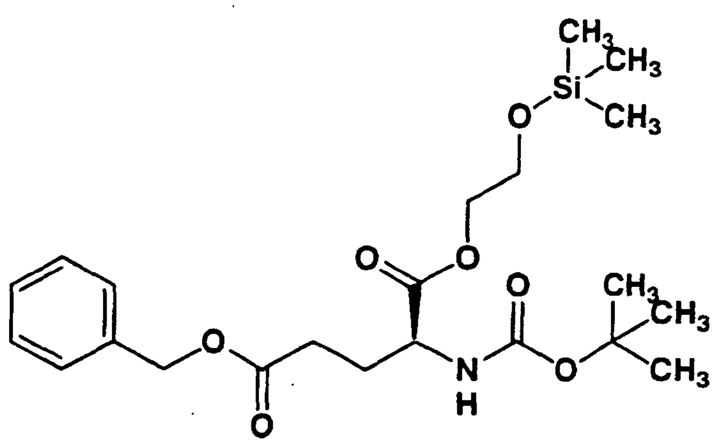

To a solution of (2S)-4-benzyloxycarbonyl-2-(t-butoxycarbonylamino)butanoic acid (5 g) in dimethylformamide (3 ml) were added 2-(trimethylsilyl)ethanol (1.77 g), 1-ethyl-3-[3-(dimethylamino)propyl]carbodiimide (3.08 ml) and dimethylaminopyridine (183 mg) at room temperature. The reaction mixture was stirred at room temperature for 2 hours. The reaction mixture was diluted with ethyl acetate/hexane, washed with 1N hydrochloric acid, water, a saturated aqueous sodium hydrogen carbonate solution, water and brine, dried over anhydrous magnesium sulfate and concentrated to give the title compound (1) (6.5 g) having the following physical data.
TLC:Rf 0.37 (hexane:ethyl acetate=4:1);
NMR ($CDCl_3$):d 0.04 (s, 9 H) 1.00 (m, 2 H) 1.43 (s, 9 H) 1.98 (m, 1 H) 2.21 (m, 1 H) 2.46 (m, 2 H) 4.20 (m, 3 H) 5.12 (s, 3 H) 7.36 (m, 5 H).

Reference Example 30

(2S,4S)-4-benzyloxycarbonyl-5-(2,4-difluorophenyl)-2-(t-butoxycarbonylamino)pentanoic acid 2-(trimethylsilyloxy) ethyl ester

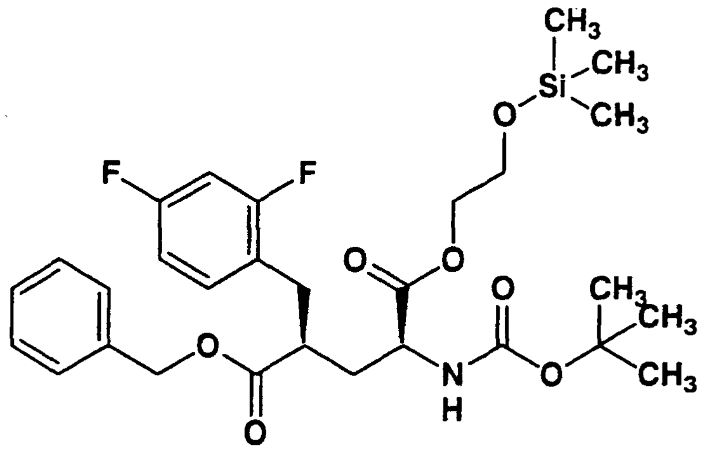

By the same procedure as described in Reference Example 2 using lithium bis(trimethylsilyl)amide and the compound prepared in Reference Example 29 instead of lithium diisopropylamide, the title compound having the following physical data was obtained.
TLC:Rf 0.43 (hexane:ethyl acetate=4:1);
NMR ($CDCl_3$):d 0.04 (s, 9 H) 0.98 (m, 2 H) 1.42 (s, 9 H) 2.02 (m, 2 H) 2.88 (m, 3 H) 4.17 (m, 2 H) 4.38 (m, 1 H) 4.95 (m, 2 H) 5.05 (d, J=12.36 Hz, 1 H) 6.70 (m, 2 H) 7.02 (m, 1 H) 7.18 (m, 2 H) 7.31 (m, 3 H).

Reference Example 31

(2S,4S)-4-benzyloxycarbonyl-5-(2,4-difluorophenyl)-2-aminopentanoic acid 2-(trimethylsilyloxy)ethyl ester

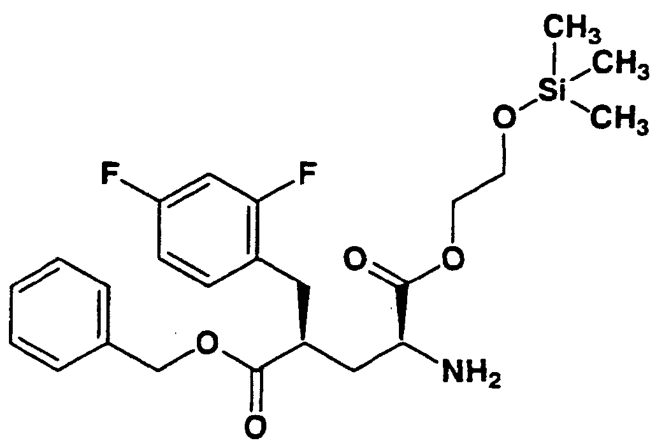

To the compound prepared in Reference Example 30 (1.3 g) was added a solution of 4N hydrochloric acid/ ethyl acetate (5 ml). The reaction mixture was stirred at room temperature for 30 minutes. The reaction mixture was diluted with ethyl acetate, washed with a saturated aqueous sodium hydrogen carbonate solution, water and brine, dried over anhydrous magnesium sulfate and concentrated to give the title compound having the following physical data.

TLC:Rf 0.27(hexane:ethyl acetate=1:1);
NMR ($CDCl_3$):d 0.04 (s, 9 H) 0.97 (m, 2 H) 1.87 (m, 2 H) 2.90 (m, 3 H) 3.43 (m, 1 H) 4.17 (m, 2 H) 5.02 (m, 2 H) 6.73 (m, 2 H) 7.03 (m, 1 H) 7.21 (m, 2 H) 7.32 (m, 3 H).

Reference Example 32

(2S,4S)-4-benzyloxycarbonyl-5-(2,4-difluorophenyl)-2-carbonylaminopentanoic acid 2-(trimethylsilyloxy)ethyl ester

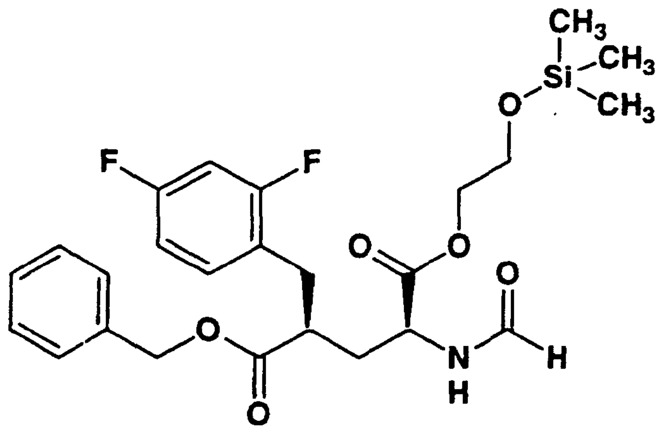

To a solution of the compound prepared in Reference Example 31 in formic acid (6.23 ml) was added acetic anhydride (1.95ml) at 0°C. The reaction mixture was stirred at room temperature for 30minutes. Water was added to the reaction mixture, which was diluted with ethyl acetate. The reaction mixture was washed with a saturated aqueous sodium hydrogen carbonate solution, water and brine, dried over anhydrous magnesium sulfate and concentrated to give the title compound (1.15 g) having the following physical data.
TLC:Rf 0.55 (hexane:ethyl acetate=1:1);
NMR ($CDCl_3$):d 0.04 (s, 9 H) 0.99 (m, 2 H) 2.10 (m, 2 H) 2.87 (m, 2 H) 4.17 (m, 3 H) 4.73 (m, 1 H) 4.99 (s, 2 H) 5.97 (d, J=8.24 Hz, 1 H) 6.73 (m, 2 H) 7.03 (m, 1 H) 7.21 (m, 2 H) 7.35 (m, 3 H) 8.02 (s, 1 H).

Reference Example 33

(2S,4S)-4-benzyloxycarbonyl-5-(2,4-difluorophenyl)-2-isocyanidepentanoic acid 2-(trimethylsilyioxy)ethyl ester

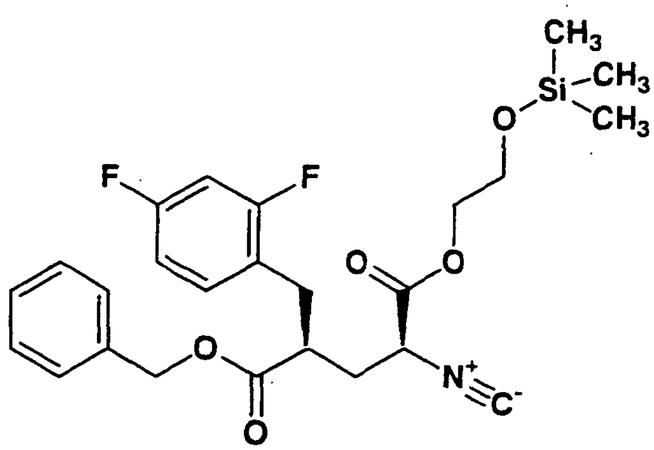

To a solution of the compound prepared in Reference Example 32 (180mg) in dichloromethane was added

triethylamine (135 µl) and phosphoryl chloride (87mg) in dichloromethane (1 ml) at -78°C. The reaction mixture was stirred at room temperature for 2 hours. The reaction mixture was poured in ice water and extracted with ethyl acetate. The extract was washed with a saturated aqueous sodium hydrogen carbonate solution, water and brine, dried over anhydrous magnesium sulfate and concentrated to give the title compound having the following physical data.
TLC:Rf 0.55(hexane:ethyl acetate=4:1);
NMR ($CDCl_3$):d 0.05 (s, 9 H) 1.02 (m, 2 H) 2.02 (m, 1 H) 2.33 (m, 1 H) 2.97 (m, 3 H) 4.22 (m, 3 H) 5.08 (m, 2 H) 6.73 (m, 2 H) 7.00 (m, 1 H) 7.30 (m, 5 H).

Reference Example 34

(4S)-4-benzyloxycarbonyl-5-(2,4-difluorophenyl)pentanoic acid 2-(trimethylsilyloxy)ethyl ester

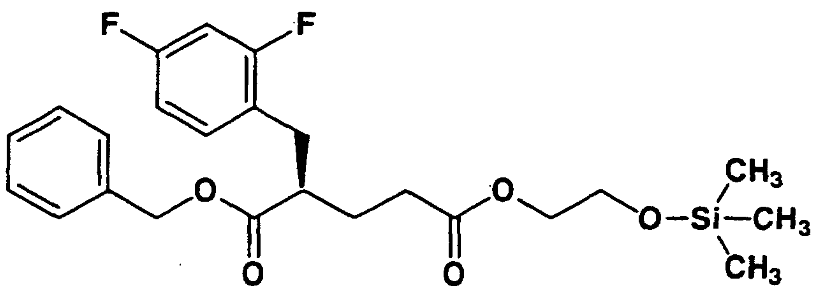

To a solution of tributyltin hydride (148 mg) in toluene (20 ml) were added the compound prepared in Reference Example 33 and 2,2'-azobis-isobutyronitrile (4 mg). The reaction mixture was stirred at 100°C for 30 minutes. The reaction mixture was concentrated. The obtained residue was purified by column chromatography on silica gel (hexane: ethyl acetate= 19:1→9:1) to give the compound of the present invention (132 mg) having the following physical data.
TLC:Rf 0.62 (hexane:ethyl acetate=4:1);
NMR ($CDCl_3$):d 0.03 (s, 9 H) 0.95 (m, 2 H) 1.92 (m, 2 H) 2.29 (m, 2 H) 2.81 (m, 3 H) 4.14 (m, 2 H) 5.04 (m, 2 H) 6.72 (m, 2 H) 7.03 (m, 1 H) 7.21 (m, 2 H) 7.32 (m, 3 H).

Reference Example 35

(4S)-4-benzyloxycarbonyl-5-(2,4-difluorophenyl)pentanoic acid

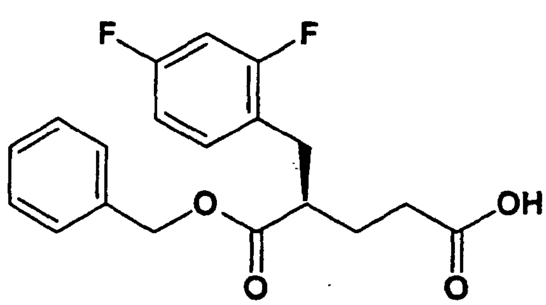

By the same procedure as described in Reference Example 24 using the compound prepared in Reference Example 34, the title compound having the following physical data was obtained.
TLC:Rf 0.41 (hexane:ethyl acetate=1:1);
NMR ($CDCl_3$):d 1.93 (m, 2 H) 2.39 (m, 2 H) 2.85 (m, 3 H) 5.05 (m, 2 H) 6.72 (m, 2 H) 7.03 (m, 1 H) 7.22 (m, 2 H) 7.33 (m, 3 H).

Reference Example 36

(2S)-4-(2,6-dichlorophenylaminocarbonyl)-2-(2,4-difluorophenylmethyl)butanoic acid benzyl ester

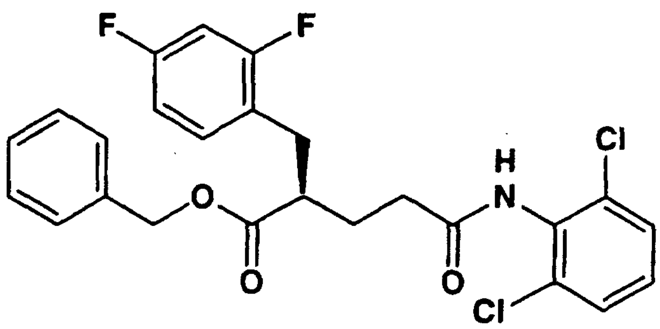

By the same procedure as described in Reference Example 1, using acid chloride which was made from the compound prepared in Reference Example 35 and thionyl chloride, the title compound having the following physical data was obtained.
TLC:Rf 0.37 (hexane:ethyl acetate=7:3);
NMR ($CDCl_3$):d 2.02 (m, 2 H) 2.37 (m, 2 H) 2.89 (m, 3 H) 5.10 (m, 2 H) 6.75 (m, 3 H) 7.05 (m, 1 H) 7.19 (m, 1 H) 7.31 (m, 7 H).

Example 29

(5R)-1-(2,6-dichlorophenyl)-5-(2,4-difluorobenzyl)piperidin-2-one

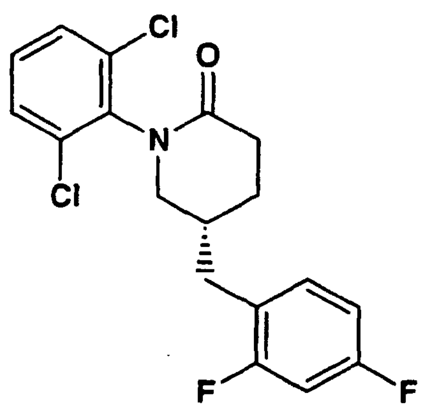

By the same procedure as described in Reference Example 3→Example 2 using the compound prepared in Reference Example 36, the compound of the present invention having the following physical data was obtained.
TLC:Rf 0.34 (ethyl acetate:hexane=2:3);
NMR ($CDCl_3$):d 1.73 (m, 1 H) 1.98 (m, 1 H) 2.38 (m, 1 H) 2.52 (m, 1 H) 2.68 (m, 3 H) 3.36 (d, J=7.97 Hz, 2 H) 6.82 (m, 2 H) 7.17 (m, 2 H) 7.38 (m, 2 H).

Example 30

(5S)-1-(2,6-dichlorophenyl)-5-(2,4-difluorobenzyl)piperidin-2-one

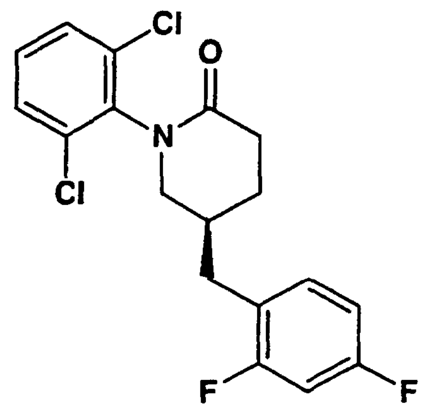

By the same procedure as described in Reference Example 29→Reference Example 30→Reference Example 31→Reference Example 32→Reference Example 33→Reference Example 34→Reference Example 35→Reference Example 36→ Reference Example 3 → Example 2 using (2R)-4-methoxycarbonyl-2-(t-butoxycarbonylamino)butanoic acid instead of (2S)-4-benzyloxycarbonyl-2-(t-butoxycarbonylamino)butanoic acid, the compound of the present invention having the following physical data was obtained.
TLC:Rf 0.34 (ethyl acetate:hexane=2:3);
NMR ($CDCl_3$):d 1.71 (m, 1 H) 1.97 (m, 1 H) 2.37 (m, 1 H) 2.51 (m, 1 H) 2.67 (m, 3 H) 3.35 (d, J=7.97 Hz, 2 H) 6.81 (m, 2 H) 7.17 (m, 2 H) 7.37 (m, 2 H).

Reference Example 37

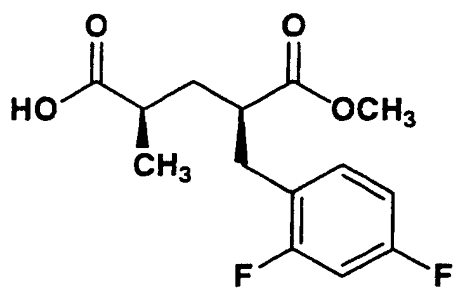

By the same procedure as described in Reference Example 19 using (2R)-4-(methoxycarbonyl)-2-methylbutanoic acid and 2,4-difluorobenzyl bromide, the title compound having the following physical data was obtained.
TLC:Rf 0.40 (ethyl acetate:hexane=1:1);
NMR ($CDCl_3$):d 1.19 (d, J = 7.2 Hz, 3H), 1.50-1.61 (m, 1H), 2.10-2.20 (m, 1H), 2.45-2.55 (m, 1H), 2.70-2.95 (m, 3H), 3.59 (s, 3H), 6.75-6.85 (m, 2H), 7.05-7.15 (m, 1H).

Example 31

(3R,5R)-1-(2,6-dichlorophenyl)-5-(2,4-difluorobenzyl)-3-methylpiperidin-2-one

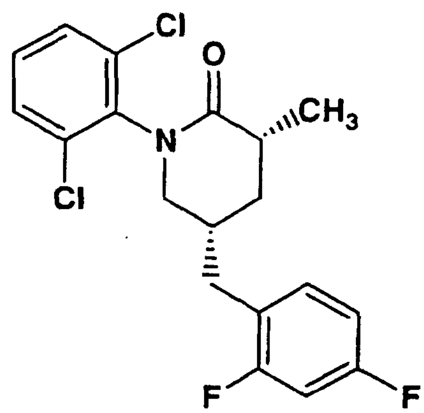

By the same procedure as described in Reference Example 36→Reference Example 3→Example 2 using the compound prepared in Reference Example 37 and 2,6-dichloroaniline, the compound of the present invention having the following physical data was obtained.
TLC:Rf 0.84(hexane:ethyl acetate=1:1);
NMR ($CDCl_3$):d 1.32 (m, 3 H) 1.53 (m, 1 H) 2.02 (m, 1 H) 2.46 (m, 2 H) 2.67 (m, J=7.14 Hz, 2 H) 3.35 (m, J=10.99 Hz, 2 H) 6.82 (m, 2 H) 7.19 (m, J=8.52, 7.42 Hz, 2 H) 7.36 (m, J=8. 52 Hz, 2 H).

Examples 31(1) and 31(2)

By the same procedure as described in Reference Example 36→Reference Example 3→Example 2 using the compound prepared in Reference Example 37 and the corresponding amine derivatives, the following compounds of the present invention were obtained.

Example 31(1)

(3R,5R)-1-(4-nitro-2,6-dimethylphenyl)-5-(2,4-difluorobenzyl)-3-methylpiperidin-2-one

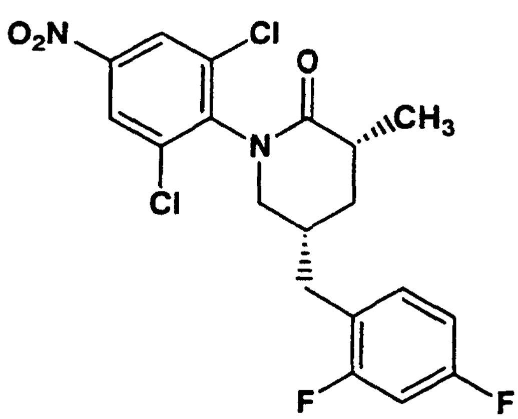

TLC:Rf 0.70(hexane:ethyl acetate=1:1);

NMR ($CDCl_3$):d 1.30 (d, J=7.14 Hz, 3 H) 1.54 (m, 1 H) 2.05 (m, 1 H) 2.55 (m, J=6.59 Hz, 2 H) 2.69 (d, J=7.14 Hz, 2 H) 3.32 (m, 2 H) 6.83 (m, 2 H) 7.12 (m, J=8.10, 6.46 Hz, 1 H) 8.25 (s, 2 H).

Example 31(2)

(3R,5R)-1-(4-bromo-2,6-dimethylphenyl)-5-(2,4-difluorobenzyl)-3-methylpiperidin-2-one

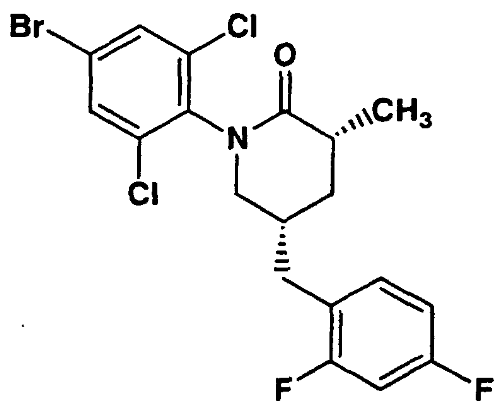

TLC:Rf 0.41 (ethyl acetate:hexane=1:2);
NMR ($CDCl_3$):d 1.29 (d, J=6.87 Hz, 3 H) 1.46 (m, 1 H) 2.06 (m, J=7.14 Hz, 1 H) 2.07 (s, 3 H) 2.15 (s, 3 H) 2.32 (m, 1 H) 2.51 (m, 1 H) 2.65 (d, J=7.14 Hz, 2 H) 3.19 (m, J=11.26 Hz, 2 H) 6.81 (m, 2 H) 7.10 (m, 1 H) 7.22 (s, 2 H).

Example 32

(3S,5R)-1-(2,6-dichlorophenyl)-5-(2,4-difluorobenzyl)-3-(benzyloxycarbonylamino)piperidin-2-one

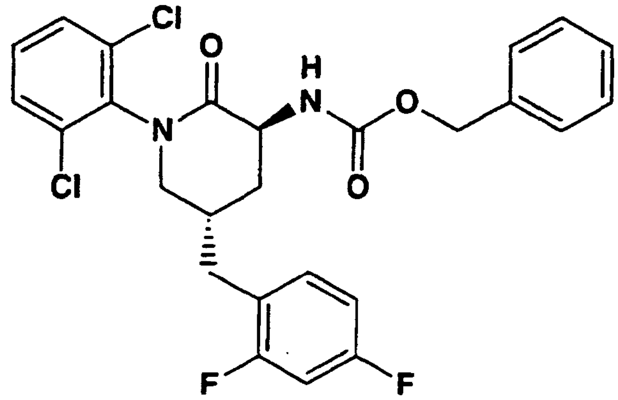

TLC:Rf 0.28 (hexane:ethyl acetate=7:3);
NMR ($CDCl_3$):d 1.95 (m, 1 H) 2.42 (m, 1 H) 2.74 (m, 3 H) 3.30 (dd, J=12.91, 5.77 Hz, 1 H) 3.58 (dd, J=12.91, 8.79 Hz, 1 H) 4.54 (m, 1 H) 5.14 (s, 2 H) 5.81 (m, 1 H) 6.82 (m, 2 H) 7.22 (m, 3 H) 7.36 (m, 6 H).

Example 33

(3S,5R)-1-(2,6-dichlorophenyl)-5-(2,4-difluorobenzyl)-3-aminopiperidin-2-one

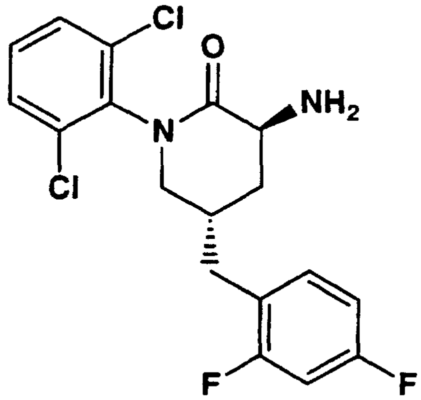

To a solution of the compound prepared in Example 32 (125 mg) in methanol (5 ml) was added 10% palladium-carbon (36 mg). The reaction mixture was stirred for 10 minutes under an atmosphere of hydrogen. The reaction mixture was filtrated through Celite (proprietary name) and concentrated. The obtained residue was purified by preparative TLC (chloroform:methanol=9:1) to give the compound of the present invention (24 mg) having the following physical data.
TLC:Rf 0.48 (chloroform:methanol=9:1);
NMR ($CDCl_3$):d 1.92 (m, 1 H) 2.09 (m, 1 H) 2.64 (m, 1 H) 2.81 (m, 2 H) 3.42 (d, J=6.87 Hz, 2 H) 3.71 (dd, J=7.69, 6.59 Hz, 1 H) 6.82 (m, 2 H) 7.20 (m, 2 H) 7.39 (m, 2H).

Reference Example 37

(4S)-4-(t-butoxycarbonylamino)-N-(2,6-dimethylphenyl)-5-((2-methoxyethoxy)methoxy)pentanamide

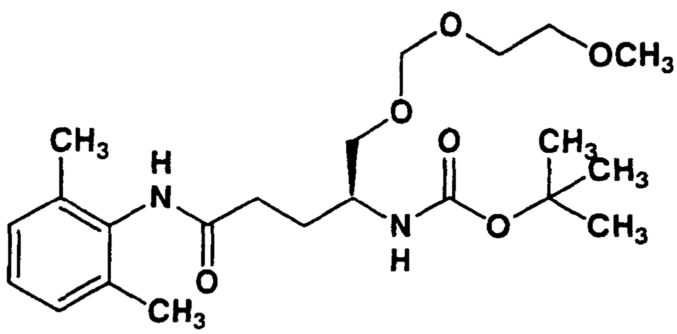

To a solution of (4S)-4-(t-butoxycarbonylamino)-5-((2-methoxyethoxy)methoxy)pentanoic acid (10 g) in N,N-dimethylformamide (30 ml) were added 2,6-dimethylaniline (4.5 g), 1-ethyl-3-(3-dimethylaminopropyl)carbodiimide hydrochloride (27 g), 1-hydroxybenzotriazole (18 g) and triethylamine (18 ml). The reaction mixture was stirred at room temperature overnight. Water was added to the reaction mixture, which was extracted with ethyl acetate. Ethyl acetate layer was washed with brine, dried over anhydrous sodium sulfate and concentrated to give the compound of the present invention (11 g) having the following physical data.
TLC:Rf 0.53 (ethyl acetate:methanol=9:1);
NMR ($CDCl_3$):d 1.45 (s, 9 H) 1.96 (m, 2 H) 2.24 (s, 6 H) 2.48 (m, 2 H) 3.39 (s, 3 H) 3.65 (m, 6 H) 3.94 (m, 1 H) 4.72

(s, 2 H) 5.13 (m, 1 H) 7.10 (m, 3 H) 8.06 (s, 1 H).

Reference Example 38

(4S)-4-amino-N-(2,6-dimethylphenyl)-5-hydroxypentanamide

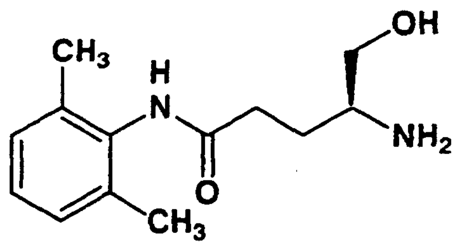

By the same procedure as described in Reference Example 31 using the compound prepared in Reference Example 37, the title compound having the following physical data was obtained.
TLC:Rf 0.27 (ethyl acetate:methanol:28% aqueous ammonia solution=16:3:1);
NMR ($DMSO-d_6$):d 2.15-2.30 (m, 1 H) 2.45-2.60 (m, 1 H) 3.10-3.30 (m, 2 H) 3.40-3.50 (m, 1 H) 3.95-4.20 (m, 2 H) 5.40 (br, s, 1 H) 7.84 (s, 3 H) 10.05 (s, 1 H).

Reference Example 39

(4S)-4-(t-butoxycarboxyamino)-N-(2,6-dimethylphenyl)-5-hydroxypentanamide

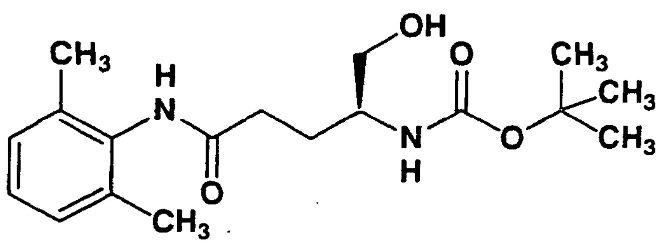

To a suspension of the compound prepared in Reference Example 38 (200mg) in t-butanol (2ml) were added di-t-butyldicarbonate (1 ml), catalytic amount of N,N-dimethylaminopyridine and the reaction mixture was stirred at room temperature for 2.5 hours. Water was added to the reaction mixture, which was extracted with ethyl acetate. Ethyl acetate layer was washed with brine, dried over anhydrous sodium sulfate and concentrated to give the compound of the present invention (200 mg) having the following physical data.
TLC:Rf 0.63 (ethyl acetate:methanol: 28% aqueous ammonia solution=8:1:1);
NMR ($CDCl_3$):d 1.53 (s, 9H) 1.80-2.05 (m, 2H) 2.22 (s, 6H) 2.53 (m, 2H) 2.84 (m, 1H) 3.59-3.80 (m, 3H) 5.14 (m, 1H) 7.02-7.20 (m, 3H) 7.61 (s, 1H).

Reference Example 40

(5S)-1-(2,6-dimethylphenyl)-5-aminopiperidin-2-one

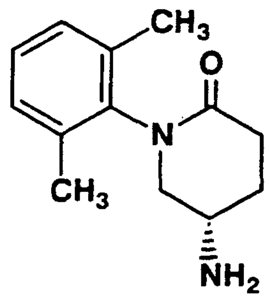

By the same procedure as described in Reference Example 27→Example 1 using the compound prepared in Reference Example 39, the compound of the present invention having the following physical data was obtained.
TLC:Rf 0.41 (ethyl acetate:methanol:28% aqueous ammonia solution=8:1:1);
NMR (DMSO-$d_6$):d 2.08 (s, 3 H) 2.16 (s, 3 H) 2.47 (m, 3 H) 3.07 (m, 1 H) 3.61 (m, 2 H) 4.11 (m, 1 H) 7.16 (m, 3 H) 8.07 (m, 2 H).

Example 34

(5S)-1-(2,6-dimethylphenyl)-5-(2,4-difluorophenylsulfonylamino)piperidin-2-one

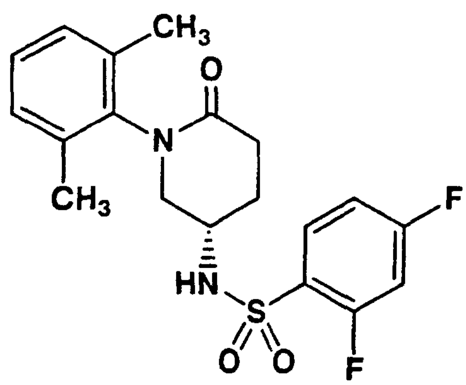

TLC:Rf 0.60 (ethyl acetate);
NMR ($CDCl_3$):d 2.03 (m, 1 H) 2.15 (s, 3 H) 2.18 (s, 3 H) 2.53 (m, 3 H) 3.04 (m, 2 H) 4.10 (m, J=14.28, 7.14 Hz, 1 H) 4.45 (m, J=1.10 Hz, 1 H) 6.95 (m, 2 H) 7.16 (m, 3 H) 7.79 (m, 1 H ).

Example 35

1-(4-(3-ethoxycarbonylpropylcarbonylamino)-2,6-dichlorophenyl)-5-(2,4-difluorobenzyl)piperidin-2-one

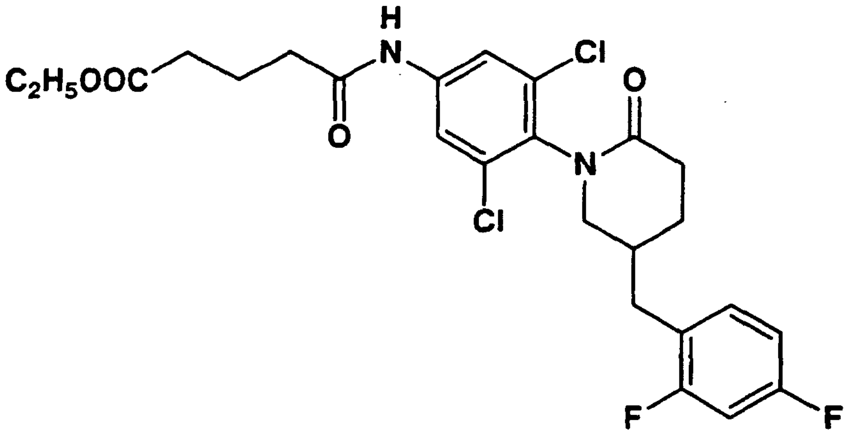

By the same procedure as described in Reference Example 1 using the compound prepared in Example 10 (1), the compound of the present invention having the following physical data was obtained.
TLC:Rf 0.54 (acetone:hexane=1:1);
NMR ($CDCl_3$):d 8.95-8.90 (m, 1H), 7.44-7.32 (m, 2H), 7.18-7.07 (m, 1H), 6.87-6.75 (m, 2H), 4.13 (q, J = 7.2 Hz, 2H), 3.32-3.25 (m, 2H), 2.75-2.65 (m, 3H), 2.62-2.47 (m, 1H), 2.42-2.27 (m, 5H), 2.07-1.89 (m, 3H), 1.80-1.65 (m, 1H), 1.26 (t, J = 7.2 Hz, 3H).

Example 36

1-(4-(3-carboxypropytcarbonylamino)-2,6-dichlorophenyl)-5-(2,4-diftuorobenzyl)piperidin-2-one

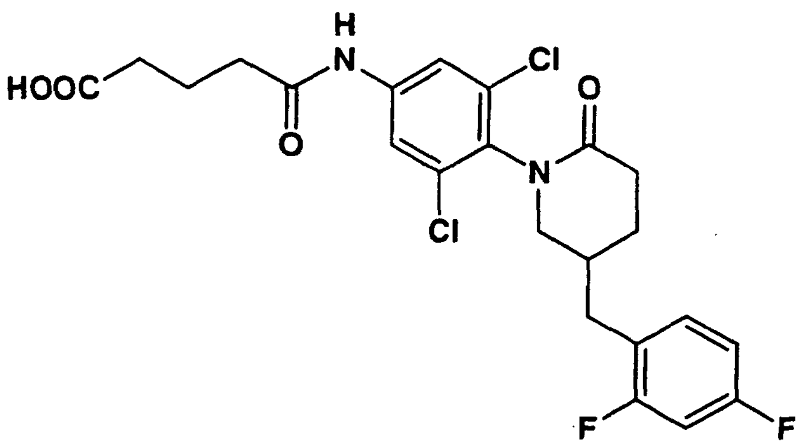

To a solution of the compound prepared in Example 35 (114 mg) in ethanol (2.2 ml) was added a 5N aqueous sodium hydroxide solution (0.26 ml). The reaction mixture was stirred at room temperature for 2.5 hours. Water was added to the reaction mixture, which was extracted with t-butyl methyl ether. The water layer was acidified with 1N hydrochloric acid and the reaction mixture was extracted with ethyl acetate. The ethyl acetate layer was washed with brine, dried over anhydrous sodium sulfate and concentrated to give the compound of the present invention (88 mg) having the following physical data.

TLC:Rf 0.39 (ethyl acetate:methanol=5:1);
NMR (DMSO-$d_6$):d 12.13-11.80 (m, 1H), 10.26 (s, 1H), 7.73 (s, 2H), 7.45-7.33 (m, 1H), 7.23-7.10 (m, 1H), 7.07-6.96 (m, 1H), 3.30-3.14 (m, 2H), 2.70 (d, J = 6.9 Hz, 2H), 2.50-2.17 (m, 7H), 1.92-1.72 (m, 3H), 1.68-1.48 (m, 1H).

Example 37

1-(4-(4-hydroxybutylcarbonylamino)-2,6-dichlorophenyl)-5-(2,4-difluorobenzyl)piperidin-2-one

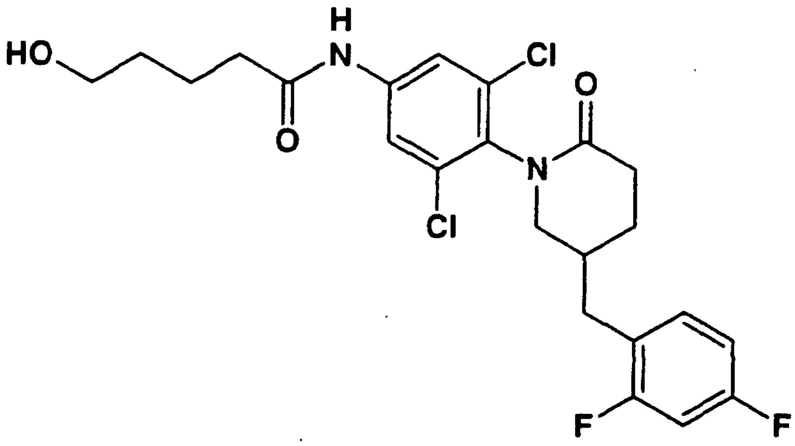

By the same procedure as described in Reference Example 5 using the compound prepared in Example 36, the compound of the present invention having the following physical data was obtained.
TLC:Rf 0.51 (ethyl acetate:methanol=5:1);
NMR ($CDCl_3$):d 9.07 (brs, 1H), 7.37 and 7.31 (each d, J = 2.1 Hz, each 1H), 7.18-7.08 (m, 1H), 6.88- 6.74 (m, 2H), 3.70-3.60 (m, 2H), 3.35-3.25 (m, 2H), 2.80-2.48 (m, 4H), 2.44-2.23 (m, 3H), 2.07-1.93 (m, 2H), 1.82-1.67 (m, 4H).

Example 38

1-(4-(2-(ethoxycarbonyl)ethylcarbonylamino)-2,6-dichlorophenyl)-5-(2,4-difluorobenzyl)piperidin-2-one

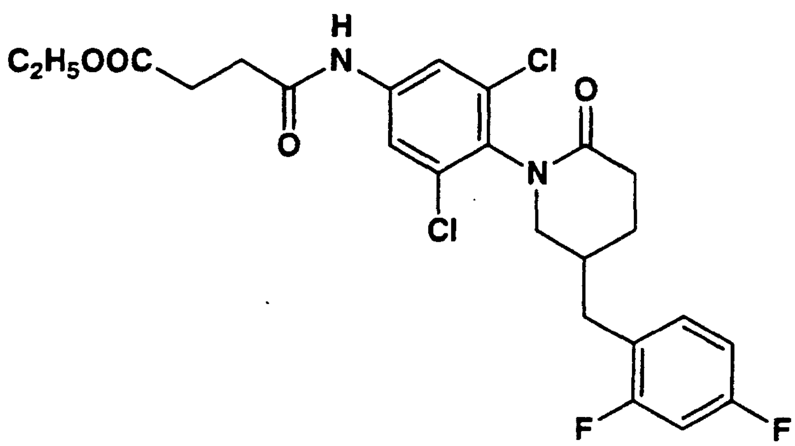

By the same procedure as described in Reference Example 1 using the compound prepared in Example 10 (1) and 3-chlorocarbonylpropanoic acid ethyl ester, the compound of the present invention having the following physical

data was obtained.
TLC:Rf 0.46 (acetone:hexane=1:1);
NMR ($CDCl_3$):d 9.14 (brs, 1H), 7.38 and 7.33 (each d, J = 2.4 Hz, each 1H), 7.33-7.17 (m, 1H), 6.87-6.74 (m, 2H), 4.14 (q, J = 7.2 Hz, 2H), 3.34-3.25 (m, 2H), 2.80-2.47 (m, 8H), 2.45-2.28 (m, 1H), 2.07-1.93 (m, 1H), 1.80-1.67 (m, 1H), 1.26 (t, J = 7.2 Hz, 3H).

Example 39

1-(4-(2-carboxyethylcarbonylamino)-2,6-dichlorophenyl)-5-(2,4-difluorobenzyl)piperidin-2-one

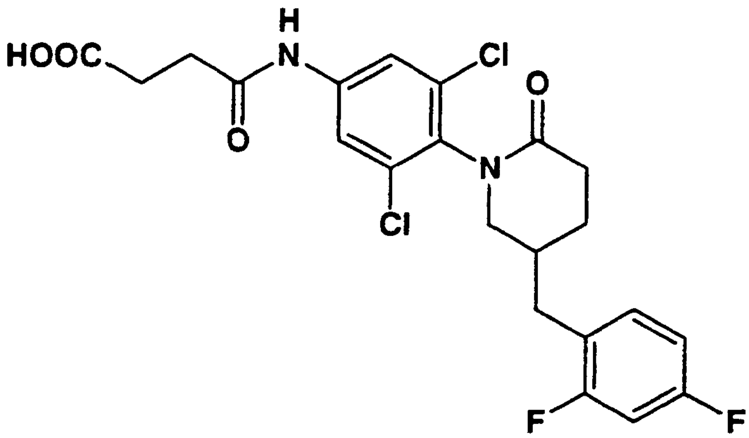

By the same procedure as described in Example 36 using the compound prepared in Example 38, the compound of the present invention having the following physical data was obtained.
TLC:Rf 0.38 (ethyl acetate:methanol:water=7:2:1);
NMR (DMSO-$d_6$):d 12.32-12.00 (m, 1H), 10.36 (s, 1H), 7.73 and 7.72 (each s, each 1H), 7.46-7.34 (m, 1H), 7.26-7.13 (m, 1H), 7.06-6.97 (m, 1H), 3.30-3.13 (m, 2H), 2.70 (d, J = 6.3 Hz, 2H), 2.66-2.37 (m, 6H), 2.34-2.18 (m, 1H), 1.92-1.79 (m, 1H), 1.70-1.50 (m, 1H).

Example 40

1-(4-(3-hydroxypropylcarbonylamino)-2,6-dichlorophenyl)-5-(2,4-difluorobenzyl)piperidin-2-one

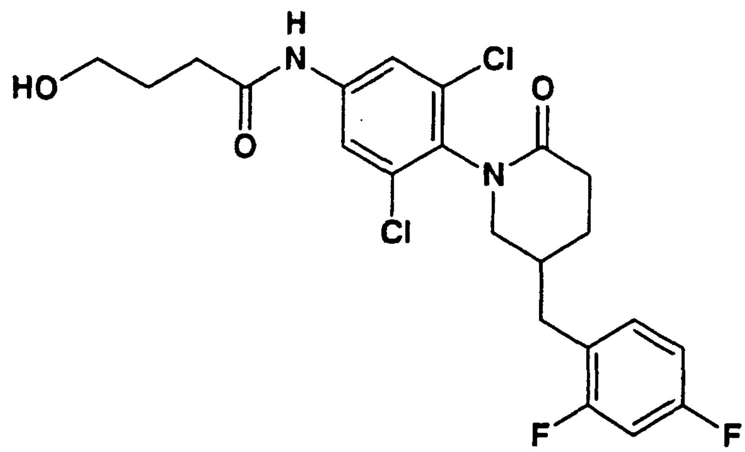

By the same procedure as described in Example 37 using the compound prepared in Example 39, the compound of the present invention having the following physical data was obtained.
TLC:Rf 0.55 (ethyl acetate:methanol=5:1);
NMR ($CDCl_3$):d 9.17 (s, 1H), 7.38-7.30 (m, 2H), 7.18-7.08 (m, 1H), 6.88-6.74 (m, 2H), 3.72-3.61 (m, 2H), 3.37-3.25 (m, 2H), 3.02-2.91 (m, 1H), 2.80-2.49 (m, 4H), 2.46-2.28 (m, 3H), 2.08-1.65 (m, 4H).

Example 41

1-(4-methanesulfonylamino-2,6-dichlorophenyl)-5-(2,4-difluorobenzyl)piperidin-2-one

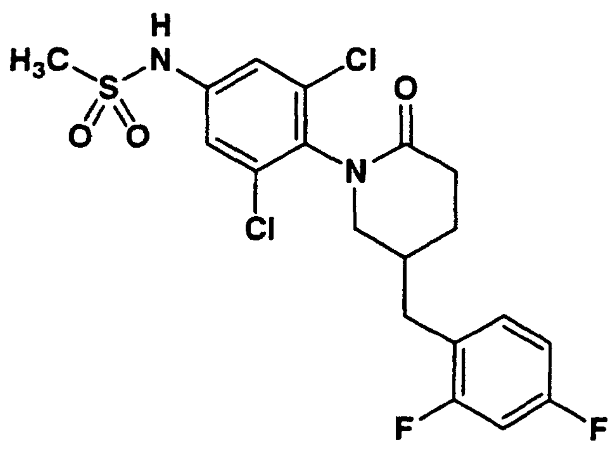

To a solution of the compound prepared in Example 10(1) (124 mg) in dichloromethane (3.2 ml) were added pyridine (0.13 ml) and methanesulfonyl chloride (0.026 ml) at 0°C. The reaction mixture was stirred at room temperature for 1 hour. 1N hydrochloric acid was added to the reaction mixture, which was extracted with t-butyl methyl ether. The extract was washed with a saturated aqueous sodium hydrogen carbonate solution, water and brine, dried over anhydrous sodium sulfate and concentrated. The obtained residue was purified by column chromatography on silica gel

(hexane:ethyl acetate= 2:3→1:3) to give the compound of the present invention (121 mg) having the following physical data.
TLC:Rf 0.32 (ethyl acetate:hexane=2:1);
NMR ($CDCl_3$):d 8.26-8.12 (m, 1H), 7.19-7.02 (m, 3H), 6.78-6.73 (m, 2H), 3.35-3.25 (m, 2H), 3.00 (s, 3H), 2.79-2.49 (m, 4H), 2.46-2.28 (m, 1H), 2.07-1.93 (m, 1H), 1.80-1.64 (m, 1H).

Example 42

1-(2,6-dichloro-4-(pyrrolidin-2,5-dion-1-yl)phenyl)-5-(2,4-difluorobenzyl)piperidin-2-one

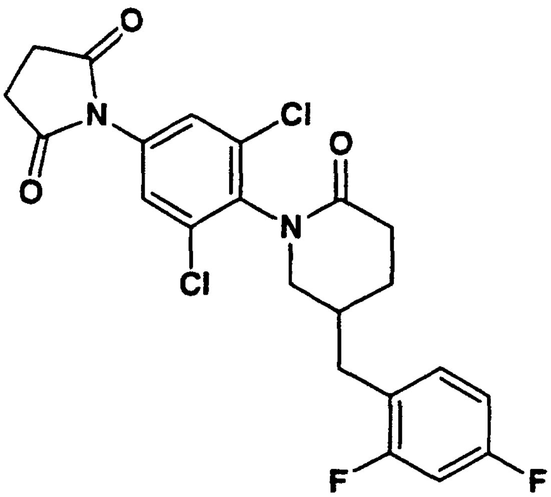

To a solution of the compound prepared in Example 10(1) (76mg) in toluene (4 ml) was added 3,5-dihydrofuran-2,5-dione (21 mg). The reaction mixture was refluxed for 2hours. The reaction mixture was concentrated. To the obtained residue was added acetyl chloride (5 ml). The reaction mixture was refluxed for 45 minutes. The reaction mixture was concentrated. The obtained residue was purified by column chromatography on silica gel (hexane:ethyl acetate=2:3→0:1) to give the compound of the present invention (61 mg) having the following physical data.
TLC:Rf 0.40 (ethyl acetate);
NMR ($CDCl_3$):d 7.47-7.40 (m, 2H), 7.20-7.10 (m, 1H), 6.88-6.73 (m, 2H), 3.35-3.28 (m, 2H), 2.90 (s, 4H), 2.79-2.65 (m, 3H), 2.60-2.47 (m, 1H), 2.45-2.28 (m, 1H), 2.08-1.93 (m, 1H), 1.80-1.65 (m, 1H).

Example 43

1-(2,6-dichloro-4-(piperidin-2,6-dion-1-yl)phenyl)-5-(2,4-difluorobenzyl)piperidin-2-one

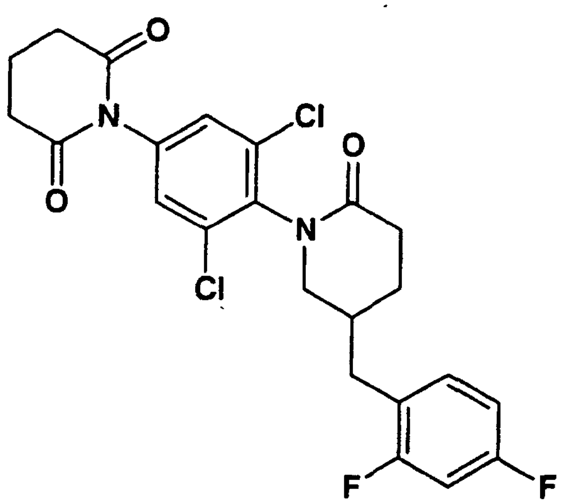

By the same procedure as described in Example 42 using 3,4,5-trihydropyran-2,6-dione instead of 3,5-dihydrofuran-2,5-dione, the compound of the present invention having the following physical data was obtained.
TLC:Rf 0.32 (ethyl acetate);
NMR ($CDCl_3$):d 7.20-7.10 (m, 3H), 6.88-6.76 (m, 2H), 3.34 (d, J = 7.8 Hz, 2H), 2.81 (t, J = 6.6 Hz, 4H), 2.75-2.65 (m, 3H), 2.60-2.27 (m, 2H), 2.13-1.93 (m, 3H), 1.80-1.67 (m, 1H).

Example 44

1-(4-t-butylcarbonylamino-2,6-dichlorophenyl)-5-(2,4-difluorobenzyl)piperidin-2-one

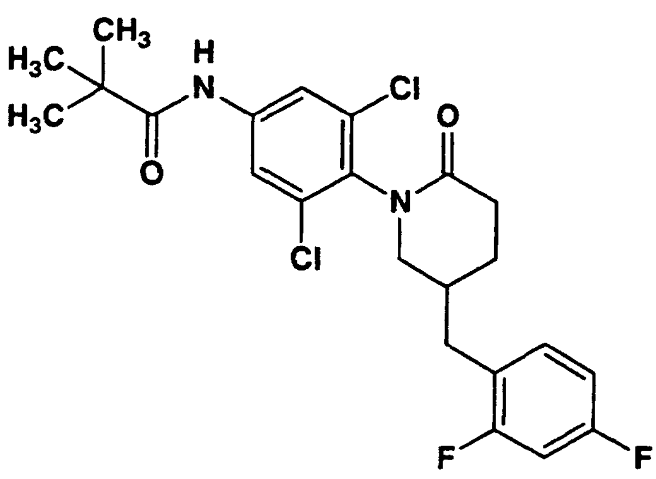

By the same procedure as described in Reference Example 1 using the compound prepared in Example 10 (1) and pivaloyl chloride, the compound of the present invention having the following physical data was obtained.
TLC:Rf 0.46 (ethyl acetate:hexane=2:1);
NMR ($CDCl_3$):d 1.25 (m, 9 H) 1.74 (m, 2 H) 1.97 (m, 1 H) 2.33 (m, 1 H) 2.51 (m, 1 H) 2.68 (m, 2 H) 3.29 (m, 2 H) 6.80

(m, 2H) 7.14 (m, 1 H) 7.46 (d, J=2.20 Hz, 1 H) 7.53 (d, J=2.20 Hz, 1 H) 8.33 (s, 1 H).

Example 45

1-(4-methoxycarbonylamino-2,6-dichlorophenyl)-5-(2,4-difluorobenzyl)piperidin-2-one

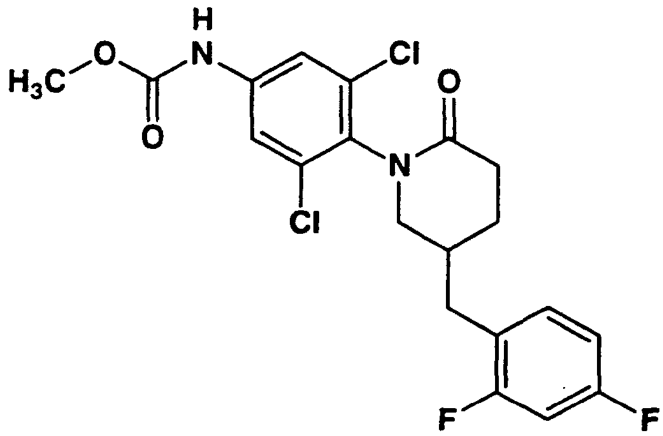

By the same procedure as described in Reference Example 1 using the compound prepared in Example 10 (1) and methyl chloroformate, the compound of the present invention having the following physical data was obtained.
TLC:Rf 0.36 (ethyl acetate:hexane=2:1);
NMR ($CDCl_3$):d 1.75 (m, 1 H) 1.99 (m, 1 H) 2.36 (m, 1 H) 2.55 (m, 1 H) 2.70 (m, 3 H) 3.31 (d, J=7.69 Hz, 2 H) 3.72 (s, 3 H) 6.82 (m, 2 H) 7.13 (m, 1 H) 7.13 (m, J=6.59 Hz, 1 H ) 7.27 (m, 1 H) 7.33 (d, J=2.20 Hz, 1 H) 8.19 (s, 1 H).

Example 46

1-(4-(N,N-diethylamino)methylcarbonylamino-2,6-dichlorophenyl)-5-(2,4-difluorobenzyl)piperidin-2-one

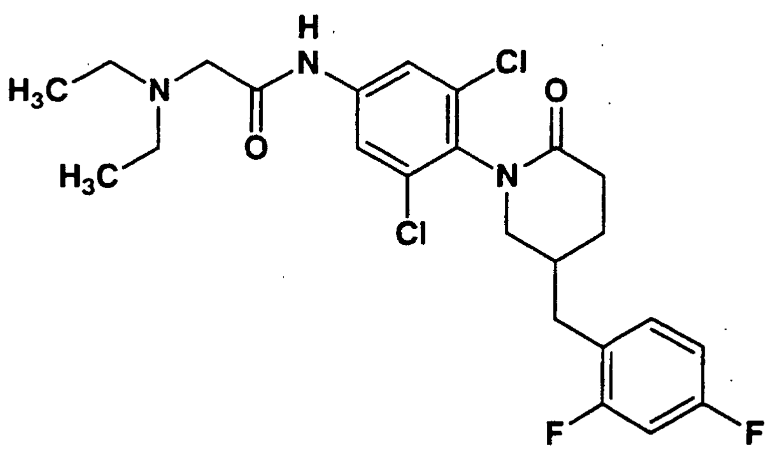

By the same procedure as described in Reference Example 1→Reference Example 17 using the compound prepared in Example 10(1) and chloroacetyl chloride, the compound of the present invention having the following physical data was obtained.
TLC:Rf 0.33 (ethyl acetate:methanol=10:1);
NMR ($CDCl_3$):d 1.08 (t, J=7.14 Hz, 6 H) 1.72 (m, 1 H) 1.95 (m, 1 H) 2.35 (m, 1 H) 2.51 (m, 1 H) 2.67 (m, 7 H) 3.15 (s,

2 H) 3.33 (m, 2 H) 6.82 (m, 2 H) 7.14 (m, 1 H) 7.68 (m, 2 H) 9.55 (m, 1 H).

Example 47

1-((4-(1-methylpiperidin-4-yl)amino)-2,6-dichlorophenyl)-5-(2,6-difluorobenzyl)piperidin-2-one

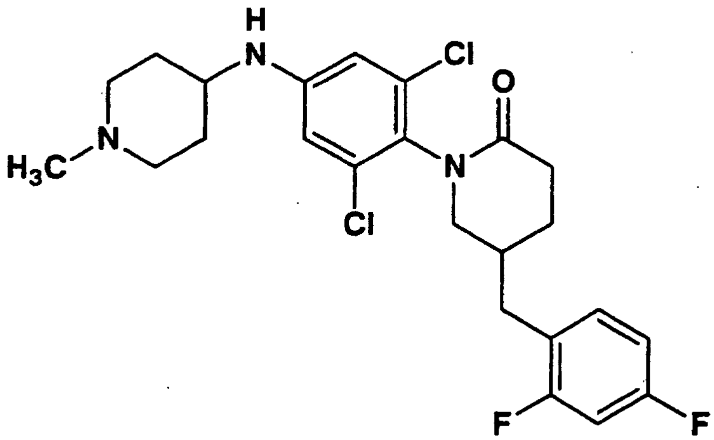

To a solution of the compound prepared in Example 10(1) (100 mg) in dichloroethane (2 ml) were added 1-methylpiperidin-4-one (44 mg), acetic acid (75 µl) and sodium triacetoxyborohydride (165mg) to room temperature for 24 hours. Ethyl acetate was added to the reaction mixture, which was washed with a saturated aqueous sodium hydrogen carbonate solution and brine, dried over anhydrous magnesium sulfate and concentrated. The obtained residue was purified by preparative TLC (chloroform:methanol=9:1) to give the compound of the present invention (19 mg) having the following physical data.
TLC:Rf 0.38 (chloroform:methanol=4:1);
NMR ($CDCl_3$):d 1.50 (m, 2 H) 1.66 (m, 1 H) 1.97 (m, 3 H) 2.14 (m, 2 H) 2.34 (m, 4 H) 2.49 (m, 1 H) 2.65 (m, 3 H) 2.80 (m, 2 H) 3.19 (m, 1 H) 3.31 (d, J=7.97 Hz, 2 H) 3.79 (d, J=7.42 Hz, 1 H) 6.53 (m, 2 H) 6.81 (m, 2 H) 7.15 (m, 1 H).

Examples 47(1)~(4)

By the same procedure as described in Example 47 using the corresponding ketone or aldehyde derivatives instead of 1-methylpiperidin-4-one, the following compounds of the present invention were obtained.

Example 47(1)

1-(4-((1-acetylpipendin-4-yl)amino)-2,6-dichlorophenyl)-5-(2,4-difluorobenzyl)piperidin-2-one

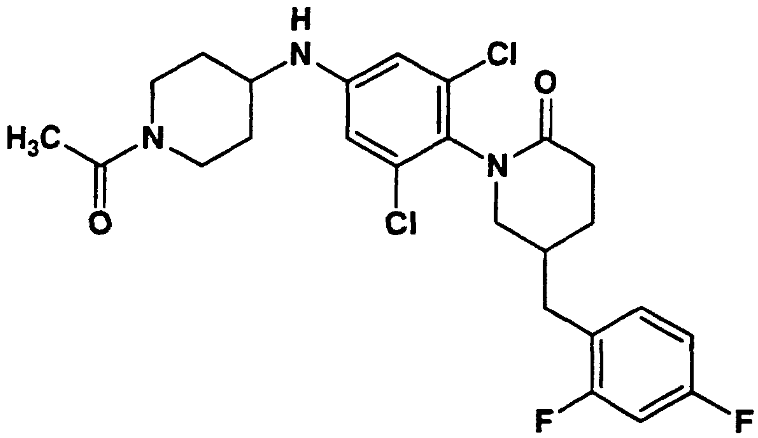

TLC:Rf 0.27 (ethyl acetate:methanol=19:1);
NMR ($CDCl_3$):d 1.33 (m, 2 H) 1.69 (m, 1 H) 2.00 (m, 6 H) 2.33 (m, 1 H) 2.49 (m, 1 H) 2.66 (m, 3 H) 2.86 (m, 1 H) 3.21 (m, 1 H) 3.38 (m, 3 H) 3.79 (m, 1 H) 3.98 (d, J=7.69 Hz, 1 H) 4.45 (m, 1 H) 6.52 (m, 2 H) 6.81 (m, 2 H) 7.14 (m, 1 H).

Example 47(2)

1-(4-(2-hydroxybenzyl)amino-2,6-dichlorophenyl)-5-(2,4-difluorobenzyl)piperidin-2-one

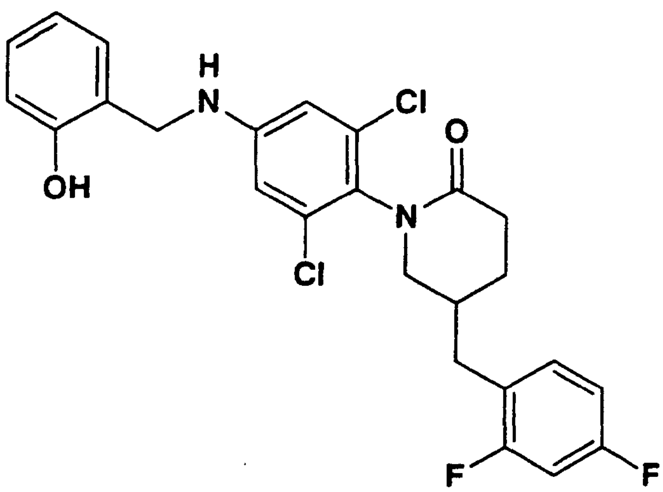

TLC:Rf 0.60 (ethyl acetate:hexane=7:3);
NMR ($CDCl_3$):d 1.67 (m, 1 H) 1.96 (m, 1 H) 2.33 (m, 1 H) 2.50 (m, 1 H) 2.67 (m, 3 H) 3.30 (t, J=5.95 Hz, 2 H) 4.17 (d, J=5.68 Hz, 2 H) 4.63 (t, J=5.68 Hz, 1 H) 6.60 (m, 2 H) 6.82 (m, 4 H) 7.14 (m, 3 H) 7.41 (s, 1 H).

Example 47(3)

1-(4-((2-methylimidazol-4-yl)methylamino)-2,6-dichlorophenyl)-5-(2,4-difluorobenzyl)piperidin-2-one

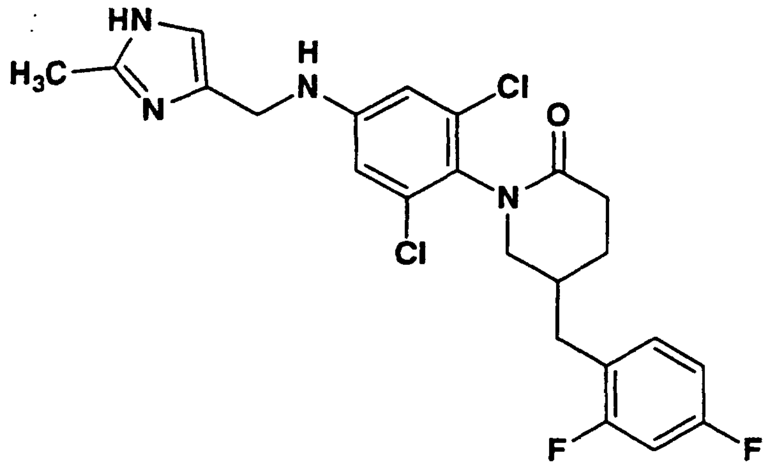

TLC:Rf 0.28 (dichloromethane:methanol=4:1);
NMR ($CDCl_3$):d 1.71 (m, 1 H) 1.97 (m, 1 H) 2.33 (m, 4 H) 2.51 (m, 1 H) 2.67 (m, 3 H) 3.32 (d, J=7.69 Hz, 2 H) 4.03 (d, J=5.13 Hz, 2 H) 4.70 (m, 1 H) 6.41 (m, 3 H) 6.81 (m, 2 H) 7.14 (m, 1H) 10.11 (m, 1 H).

Example 47(4)

1-(4-((1-t-butoxycarbonylpiperidin-4-yl)amino)-2,6-dichlorophenyl)-5-(2,4-difluorobenzyl)piperidin-2-one

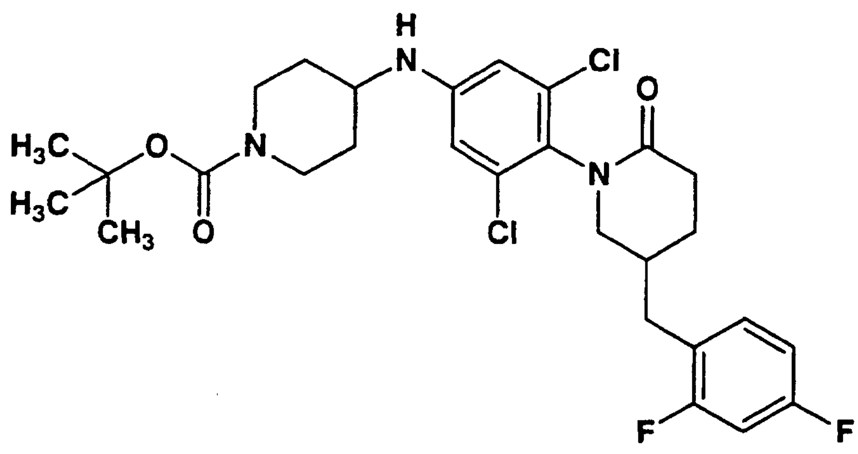

TLC:Rf 0.55 (ethyl acetate:hexane=7:3);
NMR ($CDCl_3$):d 1.32 (m, 2 H) 1.47 (s, 9 H) 1.68 (m, 1 H) 1.96 (m, 3 H) 2.33 (m, 1 H) 2.52 (m, 1 H) 2.66 (m, 3 H) 2.92 (m, 2 H) 3.31 (m, 3 H) 3.77 (d, J=7.87 Hz, 1 H) 4.03 (m, 2 H) 6.54 (m, 2 H) 6.82 (m, 2 H) 7.14 (m, 1 H).

Example 48

1-(4-(piperidin-4-ylamino)-2,6-dichlorophenyl)-5-(2,4-difluorobenzyl)piperidin-2-one

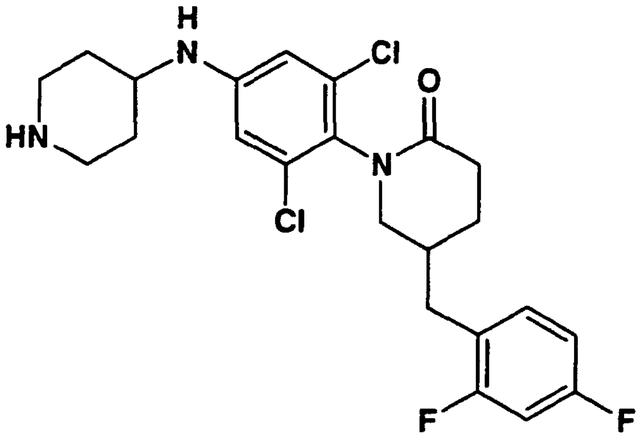

To a solution of the compound prepared in Example 47(4) (193mg) in dichloromethane (1 ml) and water (0.1 ml) was added trifluoroacetic acid (1 ml) at 0°C. The reaction mixture was stirred at room temperature for 1 hour. The reaction mixture was concentrated, dissolved in t-butyl methyl ether/tetrahydrofuran, washed with a 1N sodium hydroxide solution and brine, dried over anhydrous magnesium sulfate and concentrated to give the compound of the present invention (159 mg) having the following physical data.
TLC:Rf 0.13 (chloroform:methanol:aqueous ammonia solution=90:10:1);
NMR ($CDCl_3$):d 1.39 (m, 2 H) 1.68 (m, 1 H) 1.97 (m, 3 H) 2.33 (m, 1 H) 2.52 (m, 2 H) 2.68 (m, 5 H) 3.16 (m, 2 H) 3.29 (m, 3 H) 3.92 (d, J=7.87 Hz, 1 H) 6.53 (m, 2 H) 6.81 (m, 2 H) 7.14 (m, 1 H).

Example 49

1-(4-(1-methanesulfonylpiperidin-4-ylamino)-2,6-dichlorophenyl)-5-(2,4-difluorobenzyl)piperidin-2-one

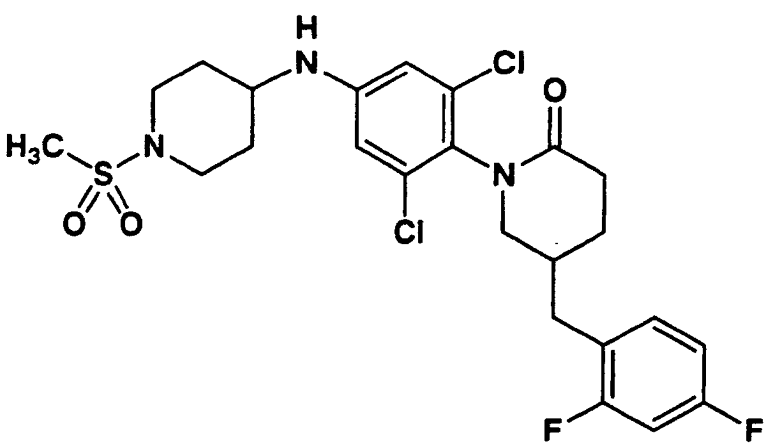

By the same procedure as described in Example 41 using the compound prepared in Example 48, the compound of the present invention having the following physical data was obtained.
TLC:Rf 0.26 (ethyl acetate:hexane=4:1);
NMR ($CDCl_3$):d 1.62 (m, 3 H) 1.95 (m, 1 H) 2.11 (m, 2 H) 2.33 (m, 1 H) 2.49 (m, 1 H) 2.66 (m, 3 H) 2.81 (s, 3 H) 2.90 (m, 2 H) 3.31 (m, 3 H) 3.74 (m, 2 H) 3.92 (d, J=7.69 Hz, 1 H) 6.53 (m, 2 H) 6.82 (m, 2 H) 7.15 (m, 1 H).

Example 50

1-(4-ethylaminocarbonylamino-2,6-dichlorophenyl)-5-(2,4-difluorobenzyl)piperidin-2-one

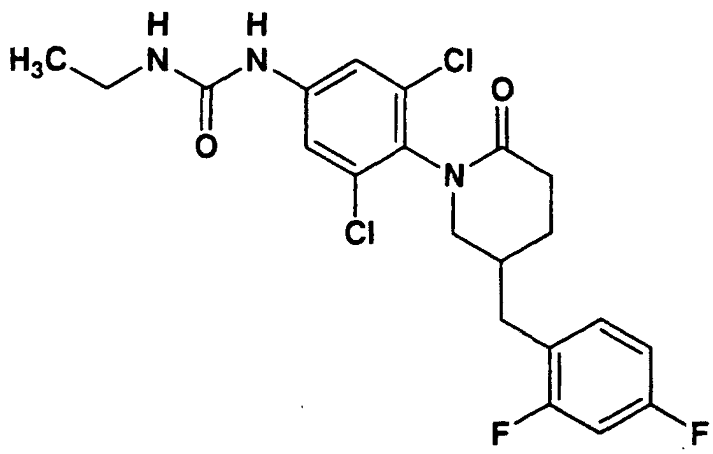

By the same procedure as described in Reference Example 13 using the compound prepared in Example 10 (1) and ethyl isocyanate, the compound of the present invention having the following physical data was obtained.
TLC:Rf 0.25 (ethyl acetate:hexane=3:2);
NMR ($CDCl_3$):d 1.10 (t, J=7.28 Hz, 3 H) 1.73 (m, 1 H) 1.99 (m, 1 H) 2.35 (m, 1 H) 2.53 (m, 1 H) 2.69 (m, 3 H) 3.19 (m, 2 H) 3.35 (d, J=7.69 Hz, 2 H) 5.25 (t, J=5.36 Hz, 1 H) 6.83 (m, 2 H) 7.15 (m, 3 H) 7.78 (s, 1 H).

Example 51

1-(4-(N,N-dimethylamino)-2,6-dichlorophenyl)-5-(2,4-difluorobenzyl)piperidin-2-one

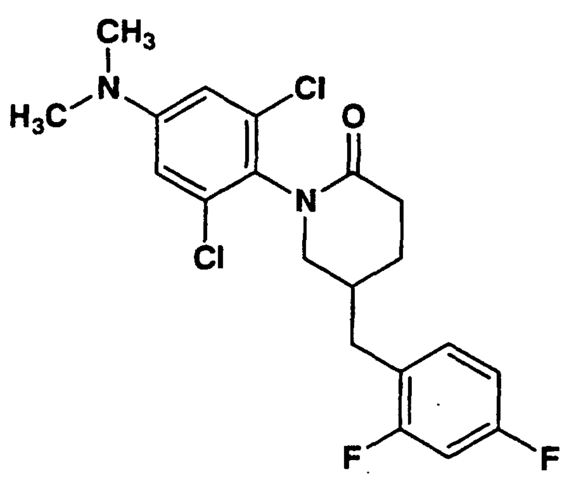

By the same procedure as described in Example 13 using the compound prepared in Example 10(1), the compound of the present invention having the following physical data was obtained.
TLC:Rf 0.57 (ethyl acetate);
NMR ($CDCl_3$):d 1.69 (m, 1 H) 1.95 (m, 1 H) 2.34 (m, 1 H) 2.50 (m, 1 H) 2.67 (m, 3 H) 2.94 (s, 6 H) 3.33 (d, J=8.00 Hz, 2 H) 6.64 (m, 2 H) 6.81 (m, 2 H) 7.15 (m, 1 H).

Example 52

1-(4-((2S)-2-(t-butoxycarbonylamino)-3-benzyloxypropanoylamino)-2,6-dichlorophenyl)-5-(2,4-difluorobenzyl) piperidin-2-one

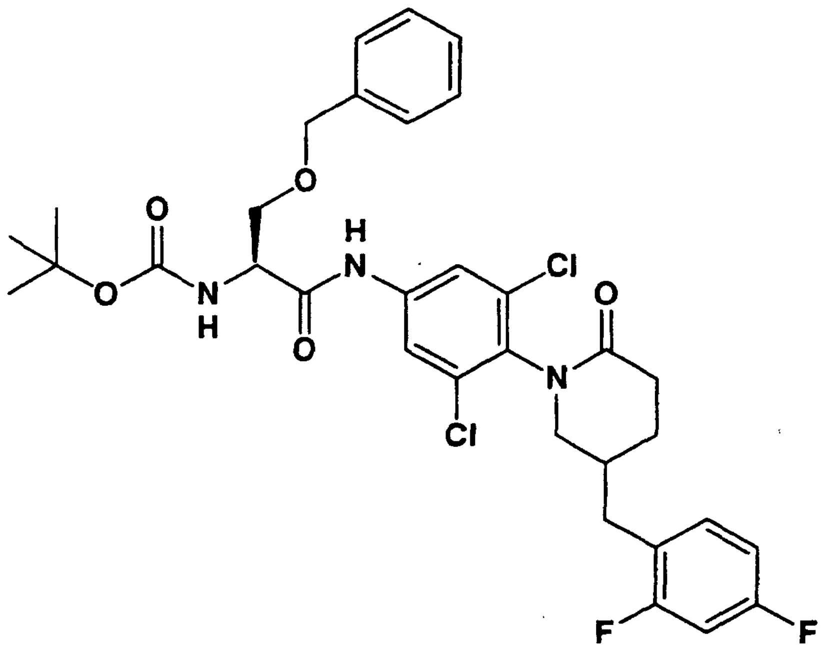

By the same procedure as described in Reference Example 37 using the compound prepared in Example 10 (1) and N-(t-butoxycarbonyl)-O-benzyl-L-serine, the compound of the present invention having the following physical data was obtained.

TLC:Rf 0.34 (ethyl acetate:hexane=2:1);

NMR ($CDCl_3$):d 9.27-9.17 (m, 1 H), 7.73-7.22 (m, 6H), 7.20-7.04 (m, 2H), 6.88-6.74 (m, 2H), 5.64-5.50 (m, 1 H), 4.60-4.38 (m, 3H), 3.91-3.60 (m, 2H), 3.34-3.25 (m, 2H), 2.78-2.63 (m, 3H), 2.62-2.47 (m, 1H), 2.43-2.26 (m, 1H), 2.05-1.82 (m, 1H), 1.80-1.60 (m, 1H), 1.51-1.40 and 0.98-0.91 (each m, totally 9H).

Example 53

1-(4-((2S)-2-(t-butoxycarbonylamino)-3-hydroxypropanoylamino)-2,6-dichlorophenyl)-5-(2,4-difluorobenzyl)piperidin-2-one

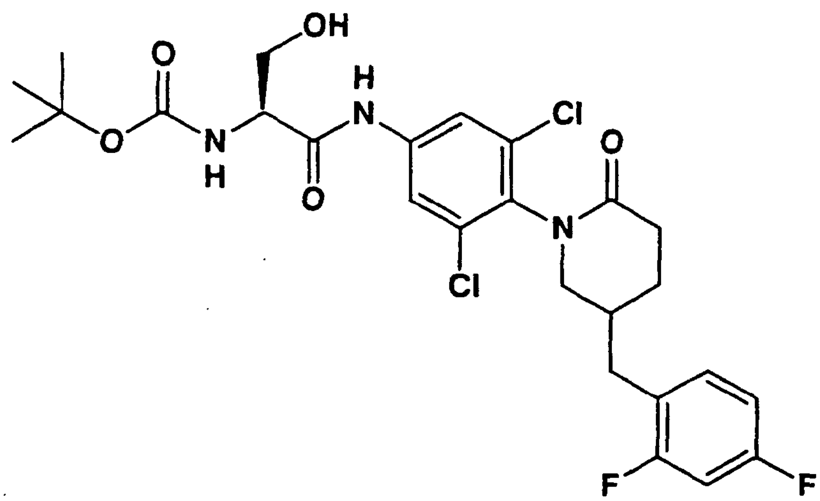

By the same procedure as described in Reference Example 15 using the compound prepared in Example 52, the compound of the present invention having the following physical data was obtained.
TLC:Rf 0.29 (ethyl acetate);
NMR ($CDCl_3$):d 9.37-9.24 (m, 1 H), 7.73-7.68 (m, 1 H), 7.46-7.08 (m, 3H), 6.88-6.74 (m, 2H), 5.70-5.58 (m, 1 H), 4.08-3.60 (m, 3 H), 3.40-3.20 (m, 2H), 2.80-2. 25 (m, 5H), 2.07-1.87 (m, 1H), 1.80-1.57 (m, 1H), 1.53-1.40 (m, 9 H).

Example 54

1-(4-(L-serylamino)-2,6-dichlorophenyl)-5-(2,4-difluorobenzyl)piperidin-2-one

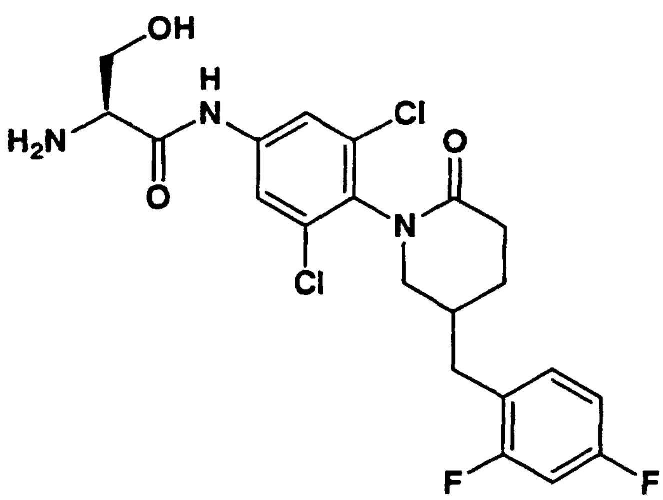

By the same procedure as described in Example 48 using the compound prepared in Example 53, the compound of the present invention having the following physical data was obtained.

TLC:Rf 0.18 (ethyl acetate:methanol:triethylamine=7:2:1);
NMR ($CDCl_3$):d 1.71 (m, 1 H) 2.23 (m, 6 H) 2.64 (m, 1 H) 2.72 (m, 2 H) 3.32 (m, 2 H) 3.60 (m, 1 H) 3.80 (m, 1 H) 3.93 (m, 1 H) 6.81 (m, 2 H) 7.14 (m, 1 H) 7.58 (s, 1 H) 7.71 ( s, 1 H) 9.70 (m, 1 H).

Example 55

1-(4-( L-tyrosylamino)-2,6-dichlorophenyl)-5-(2,4-difluorobenzyl)piperidin-2-one

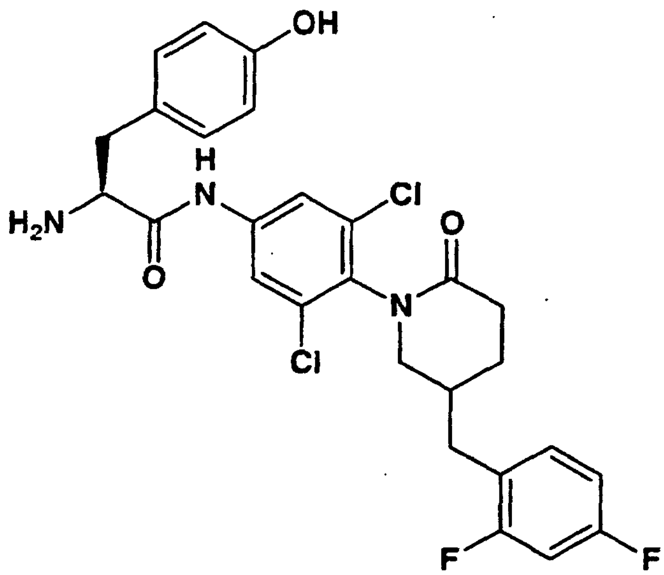

By the same procedure as described in Example 52→Example 53→Example 54 using N-(t-butoxycarbonyl) -O-benzyl-L-tyrosine instead of N-(t-butoxycarbonyl)-O-benzyl-L-serine, the compound of the present invention having the following physical data was obtained.
TLC:Rf 0.51 (ethyl acetate:methanol:triethylamine=7:2:1);
NMR ($CDCl_3$):d 1.82 (m, 5 H) 2.36 (m, 1 H) 2.53 (m, 1 H) 2.72 (m, 4 H) 3.14 (dd, J=13.70, 4.20 Hz, 1 H) 3.35 (m, 2 H) 3.66 (dd, J=8.60, 4.20 Hz, 1 H) 6.70 (d, J=8.40 Hz, 2 H) 6.82 (m, 2 H) 7.01 (d, J=8.40 Hz, 2 H) 7.14 (m, 1 H) 7.52 (m, 1 H) 7.74 (m, 1 H) 9.56 (m, 1 H).

Example 56

1-(4-(L-asparaginylamino)-2,6-dichlorophenyl)-5-(2,4-difluorobenzyl)piperidin-2-one

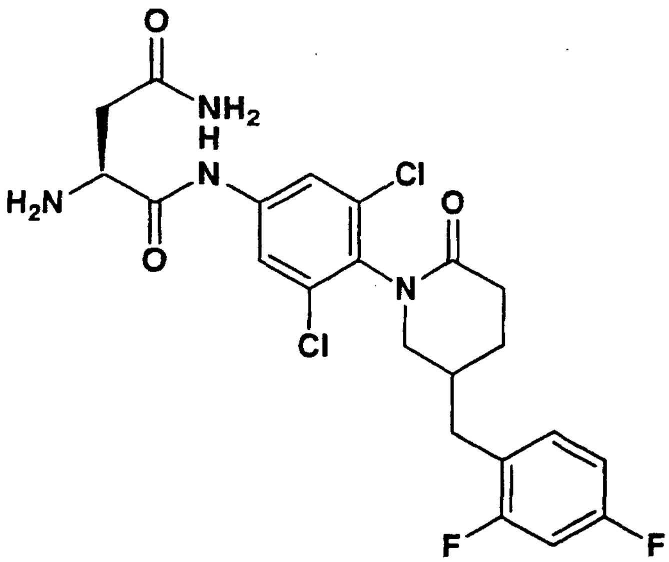

By the same procedure as described in Example 52→Example 53→Example 54 using N-(t-butoxycarbonyl)-L-asparagine instead of N-(t-butoxycarbonyl)-O-benzyl-L-serine, the compound of the present invention having the following physical data was obtained.

TLC:Rf 0.22 (ethyl acetate:methanol:triethylamine=7:2:1);

NMR ($CDCl_3$):d 1.82 (m, 4 H) 2.34 (m, 1H) 2.51 (m, 1 H) 2.69 (m, 5 H) 3.32 (m, 2 H) 3.76 (t, J=6.00 Hz, 1 H) 5.42 (m, 1 H) 5.99 (m, 1 H) 6.82 (m, 2 H) 7.14 (m, 1 H) 7.62 (s, 1 H) 7.69 (s, 1 H) 9.77 (m, 1 H).

Example 57

1-(4-(N-(pyridin-2-ylmethyl)-N-(t-butoxycarbonyl)amino)-2,6-dichlorophenyl)-5-(2,6-difluorobenzyl)piperidin-2-one

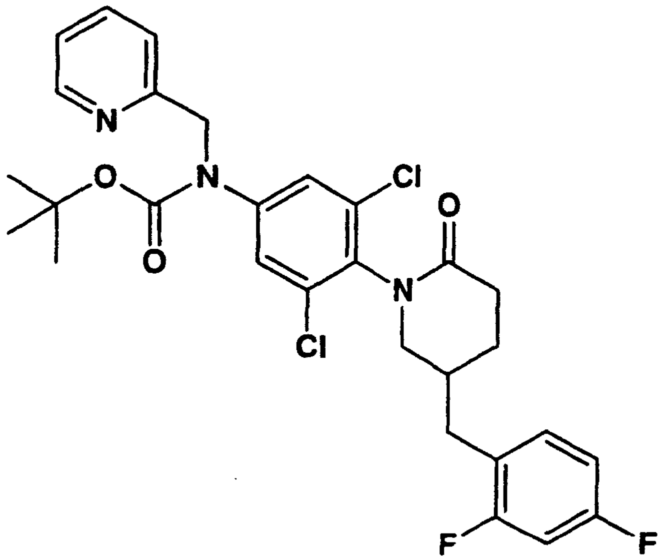

By the same procedure as described in Example 13 using the compound prepared in Example 16(10) and 2-bromomethylpyridine, the compound of the present invention having the following physical data was obtained.
TLC:Rf 0.26 (hexane:ethyl acetate=1:1);
NMR ($CDCl_3$):d 1.37 (s, 9 H) 1.70 (m, 1 H) 1.97 (m, 1 H) 2.35 (m, 1 H) 2.50 (m, 1 H) 2.69 (m, 3 H) 3.30 (d, J=8.24 Hz, 2 H) 4.90 (s, 2 H) 6.82 (m, 2 H) 7.19 (m, 3 H) 7.46 (m, 2 H) 7.69 (m, 1 H) 8.58 (m, 1 H).

Example 58

1-(4-(pyridin-2-ylmethylamino)-2,6-dichlorophenyl)-5-(2,6-difluorobenzyl)piperidin-2-one

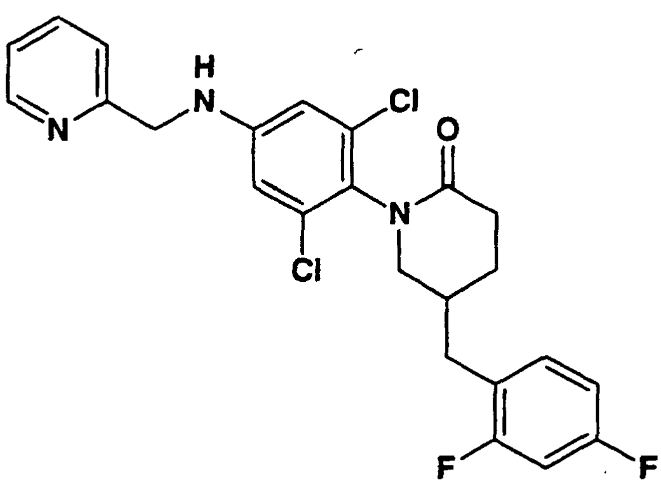

TLC:Rf 0.34 (ethyl acetate:hexane=4;1);
NMR ($CDCl_3$):d 1.69 (m, 1 H) 1.92 (m, 1 H) 2.32 (m, 1 H) 2.49 (m, 1 H) 2.66 (m, 3 H) 3.32 (d, J=7.97 Hz, 2 H) 4.37 (d,

J=4.94 Hz, 2 H) 5.29 (t, J=5.22 Hz, 1 H) 6.63 (m, 2 H) 6.81 (m, 2 H) 7.20 (m, 3 H) 7.67 (td, J=7.69, 1.92 Hz, 1 H) 8.58 (m, 1 H).

Example 59

1-(4-(N-ethoxycarbonylmethyl-N-t-butoxycarbonylamino)-2,6-dichlorophenyl)-5-(2,4-difluorobenzyl)piperidin-2-one

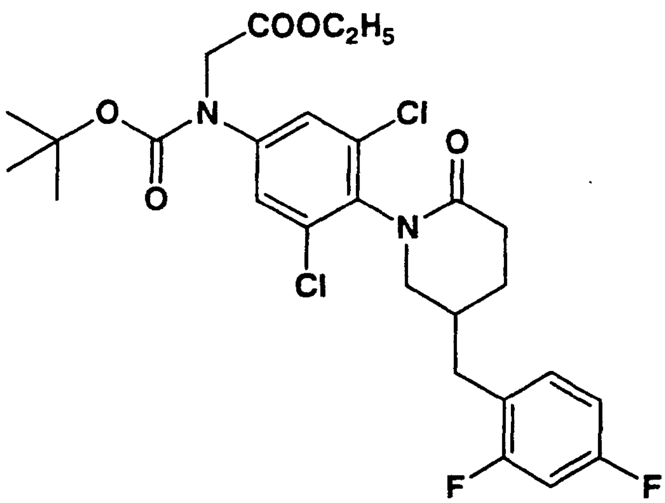

By the same procedure as described in Example 13 using the compound prepared in Example 16(10) and ethyl 2-bromoacetate, the compound of the present invention having the following physical data was obtained.
TLC:Rf 0.38 (ethyl acetate);
NMR ($CDCl_3$):d 7.36 (s, 2H), 7.20-7.08 (m, 1H), 6.89-6.75 (m, 2H), 4.33-4.19 (m, 4H), 3.38-3.26 (m, 2H), 2.80-2.30 (m, 5H), 2.07-1.93 (m, 1H), 1.80-1.63 (m, 1H), 1.47 (s, 9H), 1.38-1.22 (m, 3 H).

Example 60

1-(4-ethoxycarbonylmethylamino-2,6-dichlorophenyl)-5-(2,4-difluorobenzyl)piperidin-2-one

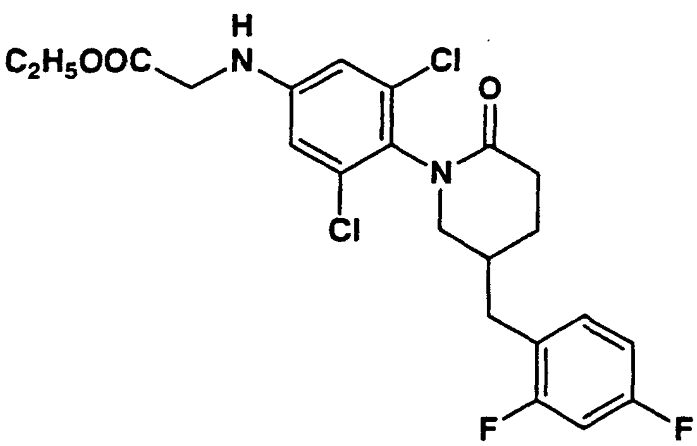

By the same procedure as described in Reference Example 31 using the compound prepared in Example 59, the compound of the present invention having the following physical data was obtained.
TLC:Rf 0.35 (ethyl acetate:hexane=2:1);

NMR ($CDCl_3$):d 1.31 (t, J=7.14 Hz, 3 H) 1.69 (m, 1 H) 1.95 (m, 1 H) 2.33 (m, 1 H) 2.49 (m, 1 H) 2.66 (m, 3 H) 3.31 (d, J=7.69 Hz, 2 H) 3.83 (d, J=5.22 Hz, 2 H) 4.26 (q, J=7.1 4 Hz, 2 H) 4.55 (t, J=4.94 Hz, 1 H) 6.56 (s, 2 H) 6.81 (m, 2 H) 7.14 (m, 1 H).

Example 61

1-(4-carboxymethylamino-2,6-dichlorophenyl)-5-(2,4-difluorobenzyl)piperidin-2-one

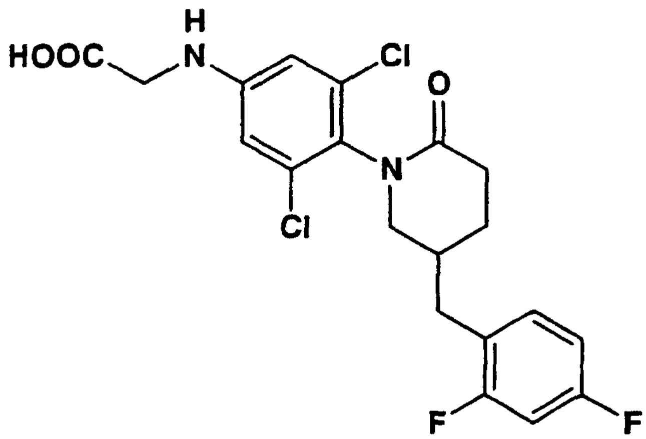

By the same procedure as described in Example 36 using the compound prepared in Example 60, the compound of the present invention having the following physical data was obtained.
TLC:Rf 0.16 (ethyl acetate:methanol=3:1);
NMR (DMSO-$d_6$):d 1.57 (m, 1 H) 1.84 (m, 1 H) 2.24 (m, 1 H) 2.39 (m, 2 H) 2.69 (m, 2 H) 3.18 (m, 2 H) 3.85 (m, 2 H) 6.55 (m, 1 H) 6.66 (s, 2 H) 7.02 (m, 1 H) 7.18 (m, 1 II) 7.38 (m, 1 H) 12.68 (m, 1 H).

Example 62

1-(4-(N-carboxymethyl-N-t-butoxycarbonylamino)-2,6-dichlorophenyl)-5-(2,4-difluorobenzyl)piperidin-2-one

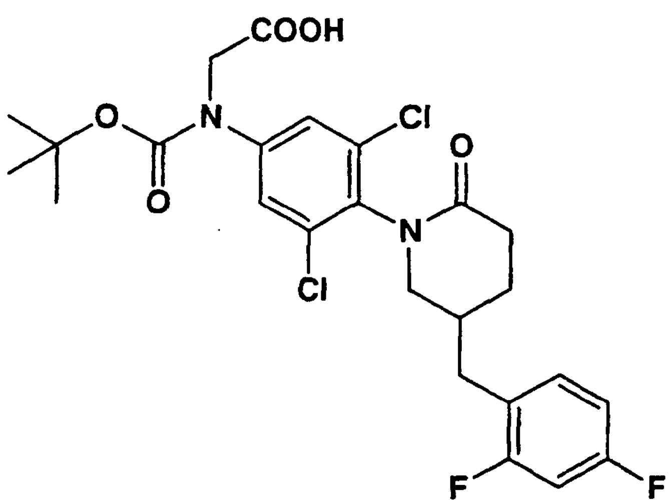

By the same procedure as described in Example 36 using the compound prepared in Example 59, the compound of the present invention having the following physical data was obtained.

TLC:Rf 0.18 (ethyl acetate);
NMR ($CDCl_3$):d 1.45 (s, 9 H) 1.72 (m, 1 H) 2.00 (m, 1 H) 2.36 (m, 1 H) 2.55 (m, 1 H) 2.71 (m, 3 H) 3.33 (d, J=7.97 Hz, 2 H) 4.19 (s, 2 H) 6.82 (m, 2 H) 7.14 (m, 1 H) 7.37 (m, 2 H).

Example 63

1-(4-(N-(2-hydroxyethyl)-N-t-butoxycarbonylamino)-2,6-dichlorophenyl)-5-(2,4-difluorobenzyl)piperidin-2-one

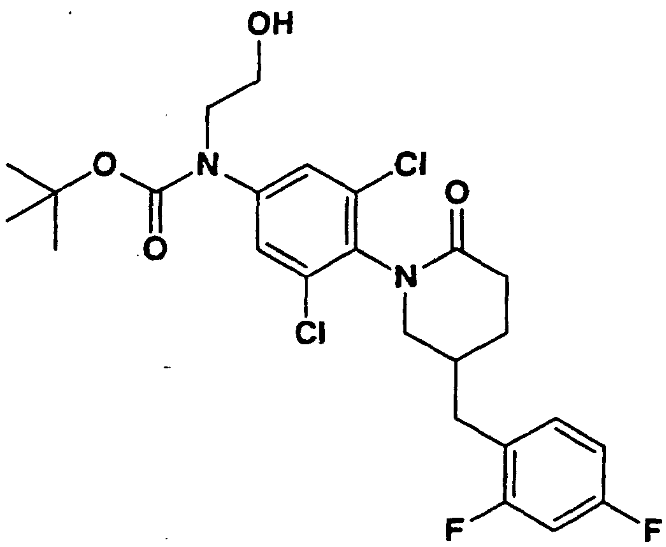

To a solution of the compound prepared in Example 59 (3.28 g) in dimethylformamide (20 ml) were added N-hydroxysuccinimide (1.53 g) and 1-ethyl-3-(3-dimethylaminopropyl)carbodiimide (2.08 g) at room temperature for 1 hour. The reaction mixture was poured in water and extracted with ethyl acetate and hexane (1:1). The extract was washed with brine, dried over anhydrous magnesium sulfate and concentrated. To a solution of the obtained residue in tetrahydrofuran (20 ml) were added sodium borohydride (447 mg) and water (870 μl) at 0°C. The reaction mixture was stirred at room temperature for 30 minutes. 1N hydrochloric acid was added to the reaction mixture, which was extracted with ethyl acetate. The extracted was washed with a saturated aqueous sodium hydrogen carbonate solution and brine, dried over anhydrous magnesium sulfate and concentrated. The obtained residue was washed with isopropyl ether/hexane to give the compound of the present invention (2.08 g) having the following physical data.
TLC:Rf 0.37 (hexane:ethyl acetate=1:4);
NMR ($CDCl_3$):d 1.47 (s, 9 H) 1.71 (m, 1 H) 1.99 (m, 1 H) 2.17 (m, 1 H) 2.36 (m, 1 H) 2.51 (m, 1 H) 2.68 (m, 3 H) 3.33 (d, J=7.69 Hz, 2 H) 3.79 (m, 4 H) 6.83 (m, 2 H) 7.15 (m, 1 H) 7.35 (m, 2 H).

Example 64

1-(4-(2-hydroxyethyl)amino-2,6-dichlorophenyl)-5-(2,4-difluorobenzyl)piperidin-2-one

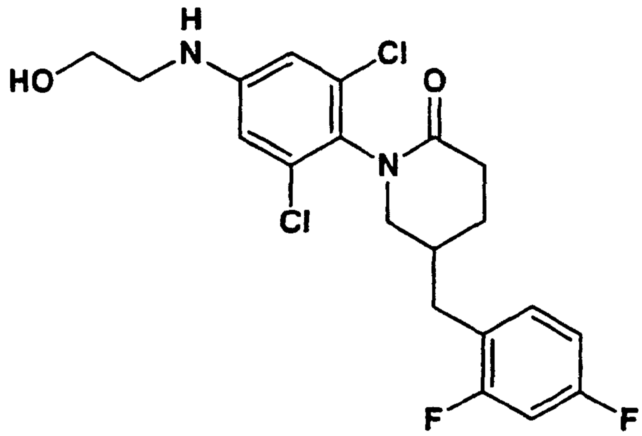

By the same procedure as described in Reference Example 31 using the compound prepared in Example 63, the compound of the present invention having the following physical data was obtained.
TLC:Rf 0.28 (ethyl acetate);
NMR ($CDCl_3$):d 1.69 (m, 1 H) 1.95 (m, 1 H) 2.10 (t, J=5.49 Hz, 1 H) 2.33 (m, 1 H) 2.49 (m, 1 H) 2.66 (m, 3 H) 3.17 (q, J=5.49 Hz, 2 H) 3.33 (d, J=7.97 Hz, 2 H) 3.78 (m, 2 H) 4.44 (t, J=5.49 Hz, 1 H) 6.53 (s, 2 H) 6.81 (m, 2 H) 7.15 (m, 1 H).

Example 65

1-(4-(N-aminocarbonylmethyl-N-t-butoxycarbonylamino)-2,6-dichlorophenyl)-5-(2,4-difluorobenzyl)piperidin-2-one

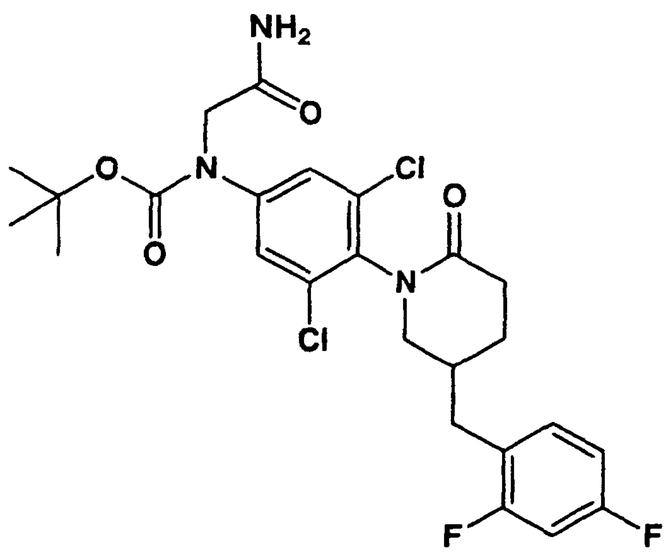

To a solution of the compound prepared in Example 59 (395mg) in dimethylformamide (5 ml) were added N-hydroxysuccinimide (168mg) and 1-ethyl-3-(3-dimethylaminopropyl)carbodiimide (228mg). The reaction mixture was stirred at room temperature for 1 hour. The reaction mixture was poured in water and extracted with ethyl acetate and hexane (1:1). The extract was washed with brine, dried over anhydrous magnesium sulfate and concentrated. To a

solution of the obtained residue in tetrahydrofuran (10 ml) was added aqueous ammonia solution (5.0ml). The reaction mixture was stirred at room temperature for 15 minutes. The reaction mixture was diluted with ethyl acetate, washed with water and brine, dried over anhydrous magnesium sulfate and concentrated. The obtained residue was purified by column chromatography on silica gel (hexane:ethyl acetate= 1:9→0: 1) to give the compound of the present invention (348mg) having the following physical data.
TLC:Rf 0.22 (hexane:ethyl acetate=1:9);
NMR ($CDCl_3$):d 1.48 (s, 9 H) 1.70 (m, 1 H) 1.98 (m, 1 H) 2.35 (m, 1 H) 2.51 (m, 1 H) 2.69 (m, 3 H) 3.32 (d, J=7.97 Hz, 2 H) 4.17 (s, 2 H) 5.46 (br. s., 1 H) 5.91 (br. s., 1 H) 6.83 (m, 2 H) 7.14 (m, 1 H) 7.40 (m, 2 H).

Example 66

1-(4-aminocarbonylmethylamino-2,6-dichlorophenyl)-5-(2,4-difluorobenzyl)piperidin-2-one

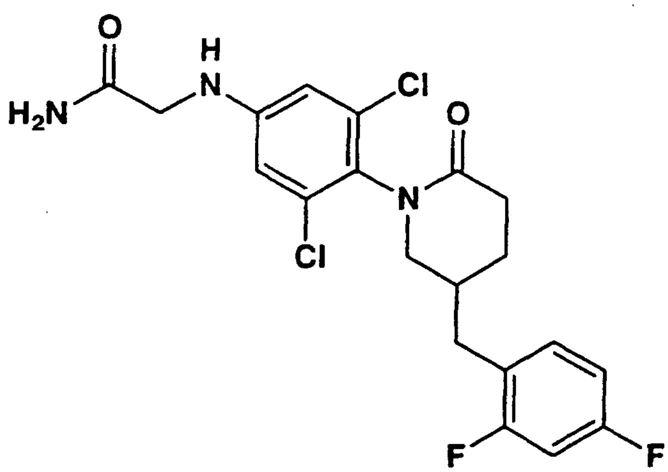

By the same procedure as described in Example 48 using the compound prepared in Example 65, the compound of the present invention having the following physical data was obtained.
TLC:Rf 0.39 (ethyl acetate:methanol=19:1);
NMR ($CDCl_3$):d 1.70 (m, 1 H) 1.97 (m, 1 H) 2.35 (m, 1 H) 2.50 (m, 1 H) 2.66 (m, 3 H) 3.32 (d, J=7.69 Hz, 2 H) 3.47 (d, J=5.22 Hz, 2 H) 5.26 (t, J=5.22 Hz, 1 H) 5.40 (m, 1 H) 6.44 (s, 2 H) 6.66 (m, 1 H) 6.82 (m, 2 H) 7.14 (m, 1 H).

Example 67

1-(4-(N,N-dimethylamino)carbonylmethylamino-2,6-dichlorophenyl)-5-(2,4-difluorobenzyl)piperidin-2-one

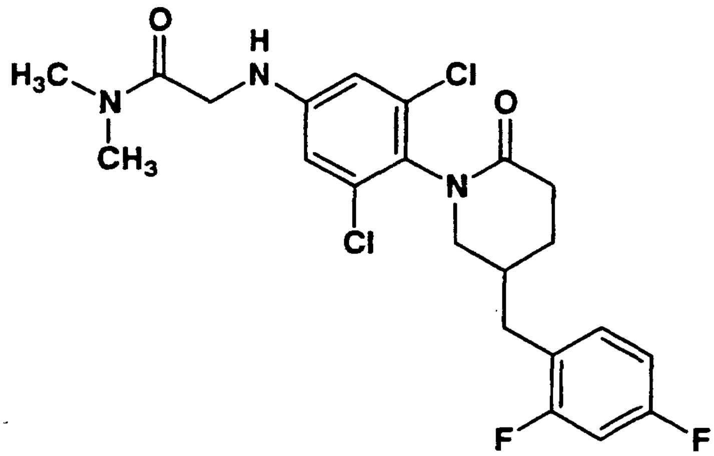

By the same procedure as described in Reference Example 37→Example 48 using the compound prepared in Example 59 and dimethylamine, the compound of the present invention having the following physical data was obtained.
TLC:Rf 0.29 (ethyl acetate);
NMR ($CDCl_3$):d 1.68 (m, 1 H) 1.95 (m, 1 H) 2.34 (m, 1 H) 2.49 (m, 1 H) 2.66 (m, 3 H) 3.03 (s, 3 H) 3.04 (s, 3 H) 3.33 (d, J=7.69 Hz, 2 H) 3.78 (d, J=4.12 Hz, 2 H) 5.19 (m, 1 H ) 6.58 (m, 2 H) 6.82 (m, 2 H) 7.15 (m, 1 H).

Examples 67(1)~67(8)

By the same procedure as described in Example 67 using the corresponding amine derivatives instead of dimethylamine, the following compounds of the present invention were obtained.

Example 67(1)

1-(4-methylaminocarbonylmethylamino-2,6-dichlorophenyl)-5-(2,4-difluorobenzyl)piperidin-2-one

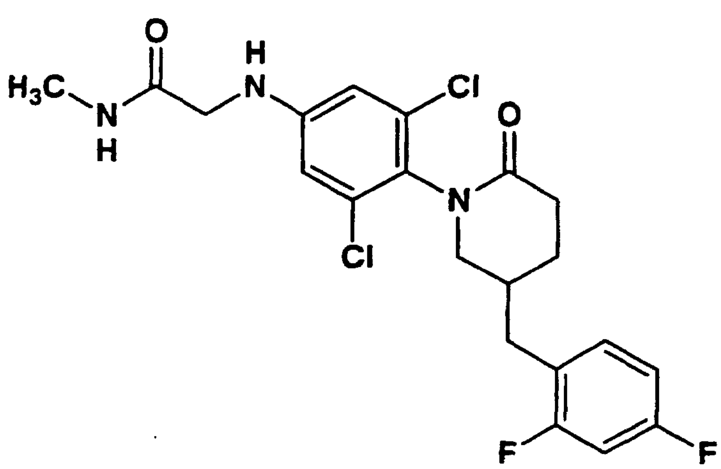

TLC:Rf 0.38 (ethyl acetate:methanol=19:1);
NMR ($CDCl_3$):d 1.69 (m, 1 H) 1.97 (m, 1 H) 2.34 (m, 1 H) 2.50 (m, 1 H) 2.66 (m, 3 H) 2.81 (d, J=4.94 Hz, 3 H) 3.32 (d, J=7.69 Hz, 2 H) 3.56 (d, J=5.49 Hz, 2 H) 5.01 (t, J=5.49 Hz, 1 H) 6.47 (s, 2 H) 6.54 (m, 1 H) 6.82 (m, 2 H) 7.15 (m, 1 H).

Example 67(2)

1-(4-(4-methylpiperazin-1-ylcarbonylmethylamino)-2,6-dichlorophenyl)-5-(2,4-difluorobenzyl)piperidin-2-one

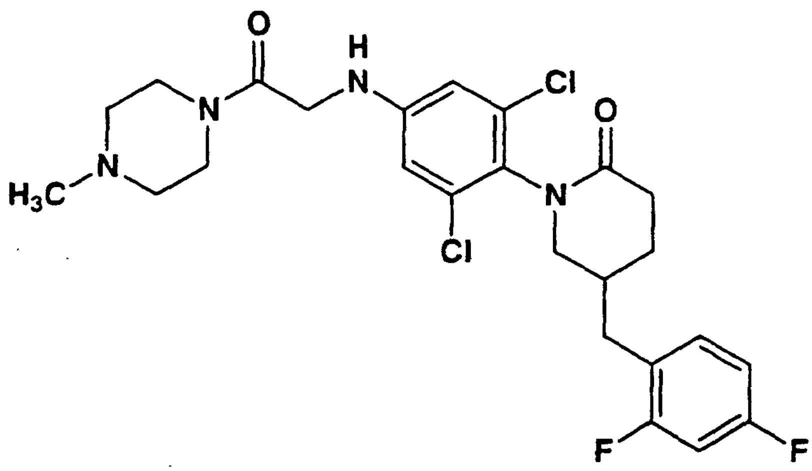

TLC:Rf 0.34 (chloroform:methanol=9:1);
NMR ($CDCl_3$):d 1.67 (m, 1 H) 1.95 (m, 1 H) 2.31 (m, 4 H) 2.44 (m, 5 H) 2.66 (m, 3 H) 3.32 (d, J=7.97 Hz, 2 H) 3.45 (m, 2 H) 3.68 (m, 2 H) 3.80 (d, J=3.85 Hz, 2 H) 5.20 (t, J=3 .85 Hz, 1 H) 6.58 (m, 2 H) 6.82 (m, 2 H) 7.15 (m, 1 H).

Example 67(3)

1-(4-(N,N-dimethylamino)aminocarbonylmethylamino-2,6-dichlorophenyl)-5-(2,4-difluorobenzyl)piperidin-2-one

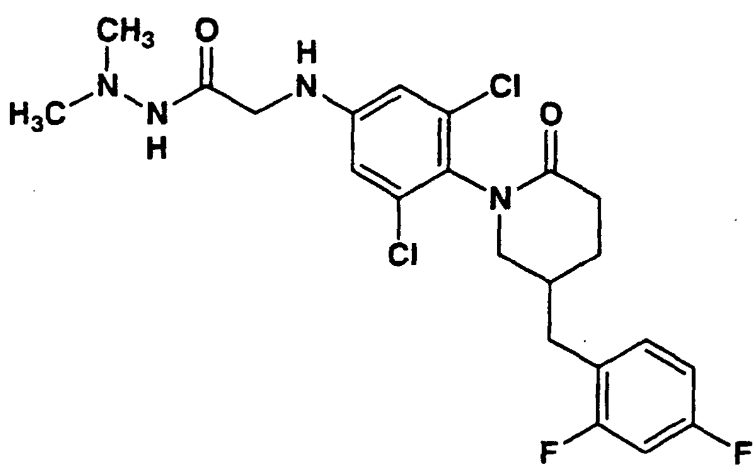

TLC:Rf 0.45 (chloroform:methanol=9:1);
NMR ($CDCl_3$):d 1.70 (m, 1 H) 1.95 (m, 1 H) 2.33 (m, 1 H) 2.49 (m, 7 H) 2.69 (m, 3 H) 3.32 (m, 2 H) 3.42 and 3.95 (d, J=5.22 Hz, and d, J=4.40 Hz, 1 H) 4.94 and 5.07 (t, J=4.40 Hz, and m, 1 H) 6.22 and 7.49(s and s, 1 H) 6.45 and 6.60

(s and m, 2 H) 6.82 (m, 2 H) 7.15 (m, 1 H).

Example 67(4)

1-(4-aminoaminocarbonylmethylamino-2,6-dichlorophenyl)-5-(2,4-difluorobenzyl)piperidin-2-one

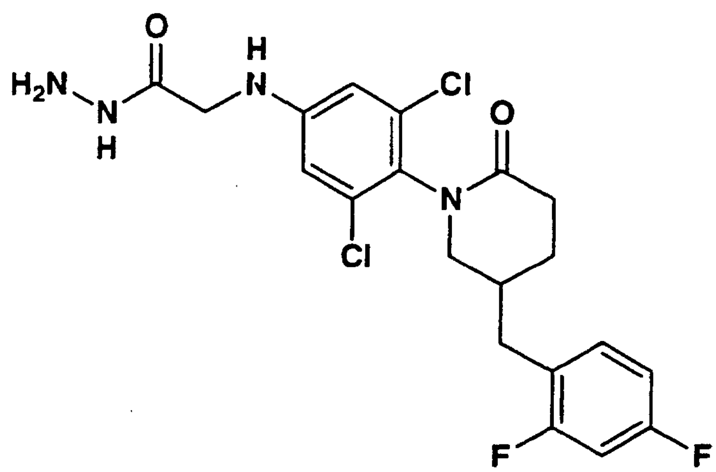

TLC:Rf 0.34 (chloroform:methanol=9:1);
NMR ($CDCl_3$):d 1.68 (m, 1 H) 1.97 (m, 1 H) 2.34 (m, 1 H) 2.50 (m, 1 H) 2.65 (m, 3 H) 3.32 (d, J=7.69 Hz, 2 H) 3.53 (d, J=5.49 Hz, 2 H) 3.82 (m, 2 H) 5.18 (m, 1 H) 6.46 (m, 2 H ) 6.83 (m, 2 H) 7.14 (m, 1 H) 7.89 (br. s., 1 H).

Example 67(5)

1-(4-(2-hydroxyethylamino)carbonylmethylamino-2,6-dichlorophenyl)-5-(2,4-difluorobenzyl)piperidin-2-one

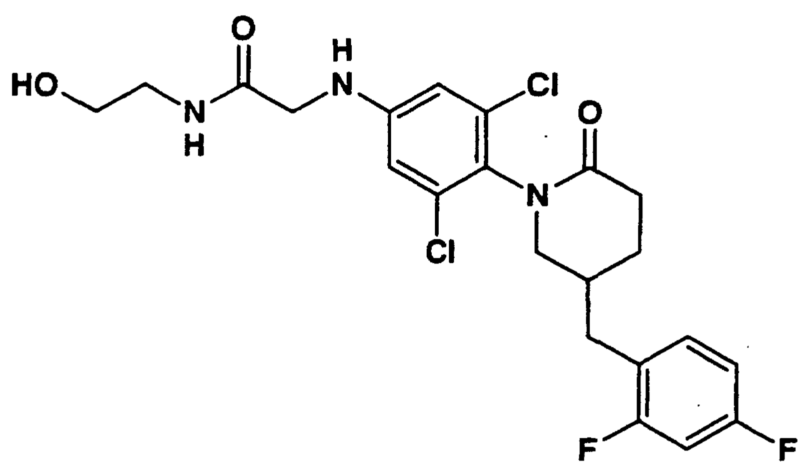

TLC:Rf 0.25 (ethyl acetate:methanol=19:1);
NMR ($CDCl_3$):d 1.65 (m, 1 H) 1.97 (m, 1 H) 2.33 (m, 1 H) 2.51 (m, 1 H) 2.66 (m, 3 H) 3.18 (m, 1 H) 3.33 (d, J=7.51 Hz, 2 H) 3.39 (m, 2 H) 3.50 (d, J=5.49 Hz, 2 H) 3.64 (m, 2 H ) 5.10 (m, 1 H) 6.48 (s, 2 H) 6.82 (m, 2 H) 7.06 (m, 1 H) 7.14 (m, 1 H).

Example 67(6)

1-(4-t-butylaminocarbonylmethylamino-2,6-dichlorophenyl)-5-(2,4-difluorobenzyl)piperidin-2-one

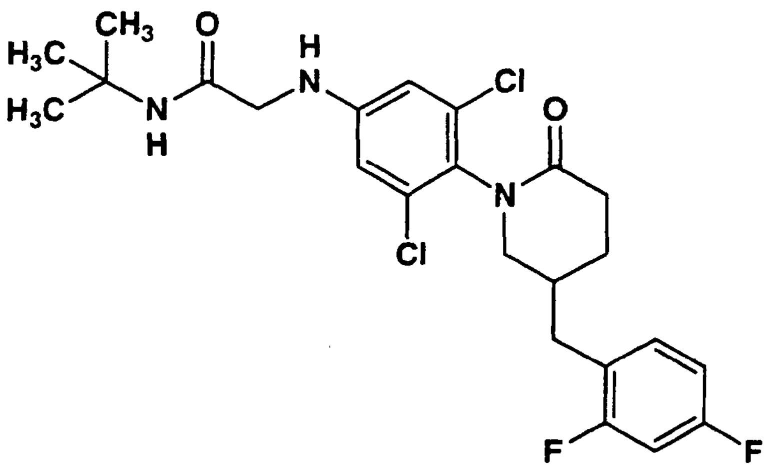

TLC:Rf 0.36 (ethyl acetate);
NMR ($CDCl_3$):d 1.31 (s, 9 H) 1.71 (m, 1 H) 1.96 (m, 1 H) 2.35 (m, 1 H) 2.50 (m, 1 H) 2.67 (m, 3 H) 3.34 (m, 4 H) 5.11 (m, 1 H) 6.42 (m, 3 H) 6.82 (m, 2 H) 7.14 (m, 1 H).

Example 67(7)

1-(4-(2-(N,N-dimethylamino)ethyl)aminocarbonylmethylamino-2,6-dichlorophenyl)-5-(2,4-difluorobenzyl)piperidin-2-one

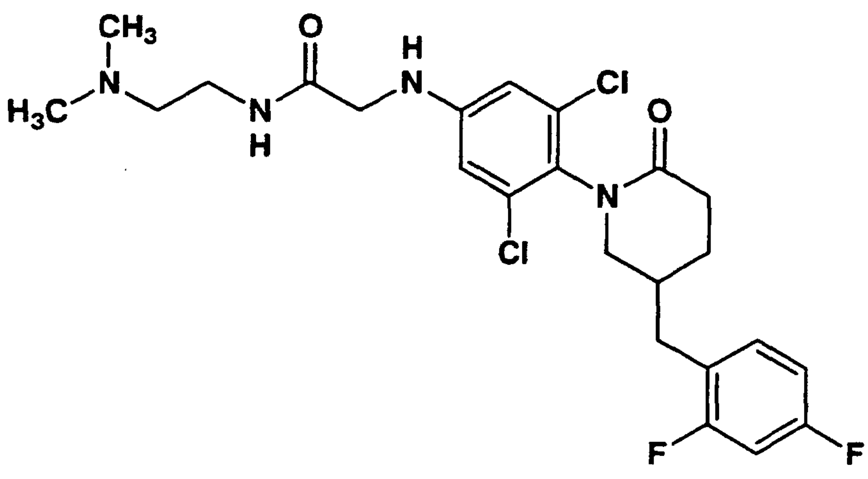

TLC:Rf 0.29 (dichloromethane:methanol=4:1);
NMR ($CDCl_3$):d 1.70 (m, 1 H) 1.93 (m, 1 H) 2.17 (s, 6 H) 2.35 (m, 3 H) 2.49 (m, 1 H) 2.66 (m, 3 H) 3.33 (m, 4 H) 3.62 (d, J=5.13 Hz, 2 H) 5.02 (t, J=5.22 Hz, 1 H) 6.49 (m, 2 H ) 6.81 (m, 3 H) 7.14 (m, 1 H).

Example 67(8)

1-(4-benzyloxyaminocarbonylmethylamino-2,6-dichlorophenyl)-5-(2,4-difluorobenzyl)piperidin-2-one

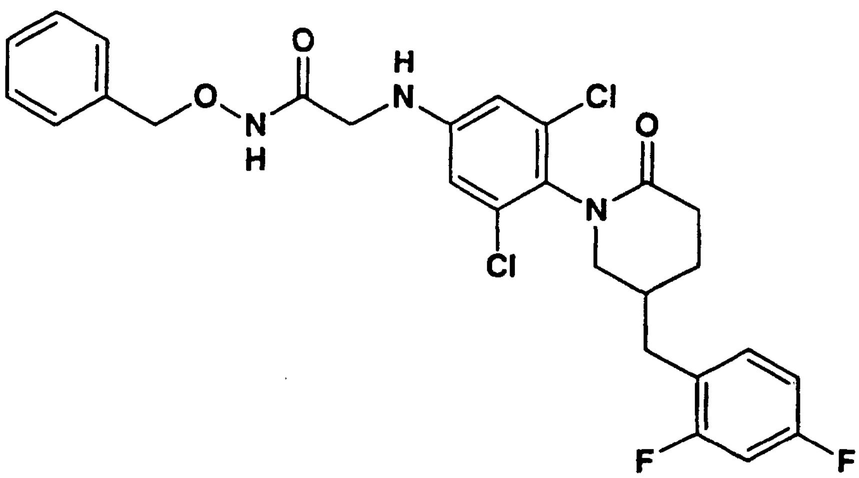

TLC:Rf 0.26 (ethyl acetate:hexane=4: 1);
NMR ($CDCl_3$):d 1.70 (m, 1 H) 1.95 (m, 1 H) 2.47 (m, 3 H) 2.71 (d, J=7.14 Hz, 2 H) 3.31 (d, J=7.69 Hz, 2 H) 3.44 (m, 2 H) 4.91 (m, 3 H) 6.41 (m, 2 H) 6.82 (m, 2 H) 7.14 (m, 1 H ) 7.37 (m, 5 H) 9.43 (br. s., 1 H).

Example 68

1-(4-hydroxyaminocarbonylmethylamino-2,6-dichlorophenyl)-5-(2,4-difluorobenzyl)piperidin-2-one

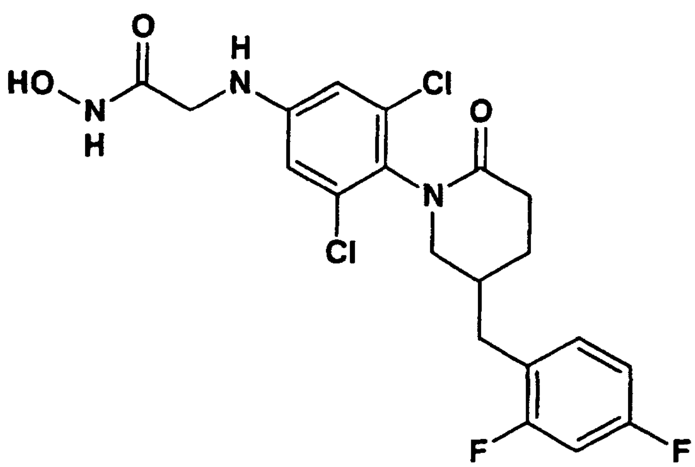

By the same procedure as described in Reference Example 15 using the compound prepared in Example 67 (8), the compound of the present invention having the following physical data was obtained.
TLC:Rf 0.29 (chloroform:methanol=9:1);
NMR (DMSO-$d_6$):d 1.56 (m, 1 H) 1.85 (m, 1 H) 2.27 (m, 3 H) 2.69 (d, J=7.14 Hz, 2 H) 3.28 (m, 2 H) 3.60 (d, J=6.04 Hz, 2 H) 6.66 (m, 3 H) 7.02 (m, 1 H) 7.18 (m, 1 H) 7.37 (m, 1 H) 8.9 1 (br. s., 1 H) 10.59 (br. s., 1 H).

Example 69

1-(4-(3-(N,N-dimethylamino)propyl)amino-2,6-dichlorophenyl)-5-(2,4-difluorobenzyl)piperidin-2-one

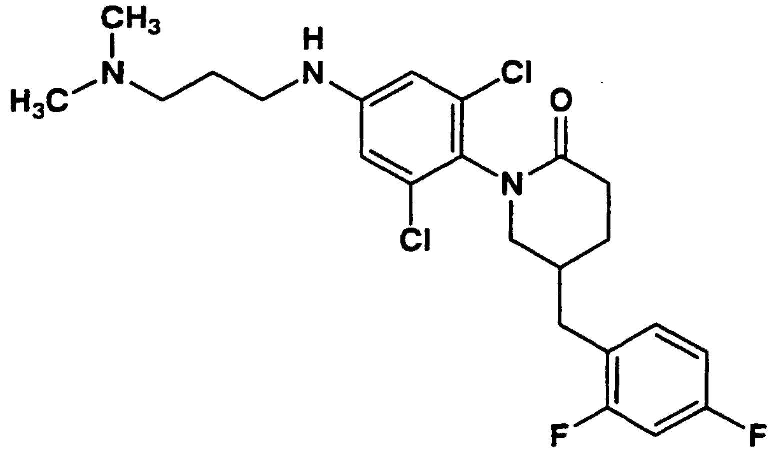

By the same procedure as described in Reference Example 13→Reference Example 15→Reference Example 27→Reference Example 28→Example 48 using the compound prepared in Example 16(10) and 3-bromo-1-benzyloxypropane, the compound of the present invention having the following physical data was obtained.
TLC:Rf 0.13 (chloroform:methanol=4:1);
NMR ($CDCl_3$):d 1.71 (m, 3 H) 1.94 (m, 1 H) 2.24 (s, 6 H) 2.35 (m, 3 H) 2.49 (m, 1 H) 2.66 (m, 3 H) 3.12 (m, 2 H) 3.32 (d, J=7.97 Hz, 2 H) 5.16 (m, 1 H) 6.52 (m, 2 H) 6.81 (m, 2 H) 7.15 (m, 1 H).

Example 70

1-((4-(2-oxo-1,3-oxazolidin-3-yl))-2,6-dichlorophenyl)-5-(2,4-difluorobenzyl)piperidin-2-one (compound 1) and 1-(4-(N-(2-(N',N'-dimethylamino)ethyl))-N-(t-butoxycarbonyl)amino-2,6-dichlorophenyl)-5-(2,4-difluorobenzyl) piperidin-2-one (compound 2)

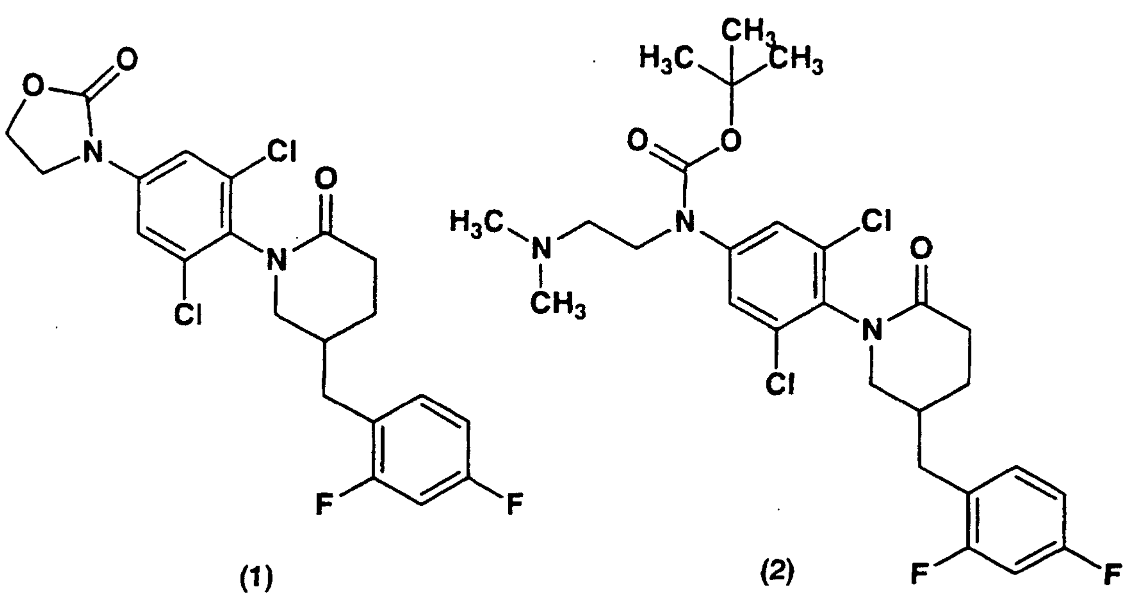

(1) (2)

By the same procedure as described in Reference Example 27→Reference Example 28 using the compound prepared in Example 63 and dimethylamine, the compounds (1) and (2) of the present invention having the following physical data respectively was obtained.

Example 70(1)

TLC:Rf 0.22 (ethyl acetate:hexane=4:1);
NMR ($CDCl_3$):d 1.71 (m, 1 H) 1.98 (m, 1 H) 2.37 (m, 1 H) 2.51 (m, 1 H) 2.68 (m, 3 H) 3.34 (d, J=7.69 Hz, 2 H) 3.99 (m, 2 H) 4.49 (m, 2 H) 6.82 (m, 2 H) 7.15 (m, 1 H) 7.61 (m, 2 H).

Example 70(2)

TLC:Rf 0.51 (chloroform:methanol=9:1);
NMR ($CDCl_3$):d 1.48 (s, 9 H) 1.70 (m, 1 H) 1.99 (m, 1 H) 2.26 (s, 6 H) 2.36 (m, 1 H) 2.51 (m, 3 H) 2.69 (m, 3 H) 3.32 (d, J=7.97 Hz, 2 H) 3.68 (t, J=7.42 Hz, 2 H) 6.82 (m, 2 H) 7.14 (m, 1 H) 7.36 (m, 2 H).

Example 71

1-(4-(2-(N,N-dimethylamino)ethyl)amino-2,6-dichlorophenyl)-5-(2,4-difluorobenzyl)piperidin-2-one

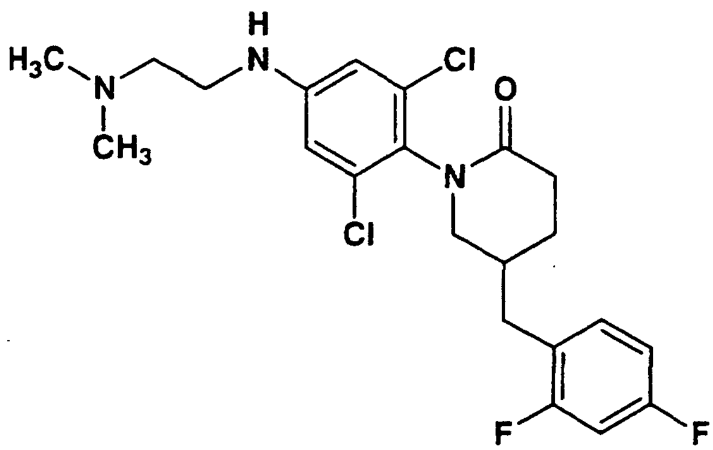

By the same procedure as described in Reference Example 31 using the compound prepared in Example 70 (2), the compound of the present invention having the following physical data was obtained.
TLC:Rf 0.36 (chloroform:methanol=9:1);
NMR ($CDCl_3$):d 1.69 (m, 1 H) 1.95 (m, 1 H) 2.23 (s, 6 H) 2.34 (m, 1 H) 2.49 (m, 3 H) 2.66 (m, 3 H) 3.05 (q, J=5.04 Hz, 2 H) 3.33 (d, J=7.69 Hz, 2 H) 4.61 (t, J=4.53 Hz, 1 H) 6 .57 (s, 2 H) 6.81 (m, 2 H) 7.15 (m, 1 H).

Example 72

1-(4-(2-(morpholin-4-yl)ethylamino)-2,6-dichlorophenyl)-5-(2,4-difluorobenzyl)piperidin-2-one

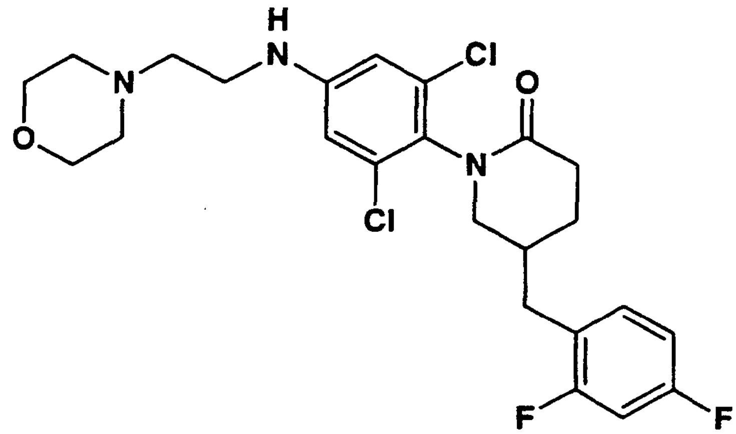

By the same procedure as described in Example 70 using morpholine instead of dimethylamine, the compound of the present invention having the following physical data was obtained.
TLC:Rf 0.48 (chloroform:methanol=9:1);
NMR ($CDCl_3$):d 1.67 (m, 1 H) 1.95 (m, 1 H) 2.33 (m, 1 H) 2.49 (m, 5 H) 2.65 (m, 5 H) 3.09 (q, J=4.85 Hz, 2 H) 3.33 (d, J=7.69 Hz, 2 H) 3.71 (m, 4 H) 4.60 (m, 1 H) 6.59 (m, 2 H ) 6.81 (m, 2 H) 7.15 (m, 1H).

Examples 72(1)~72(5)

By the same procedure as described in Example 72 using the corresponding amine derivatives instead of morpholine, the following compounds of the present invention were obtained.

Example 72(1)

1-(4-(2-(N,N-diethylamino)ethyl)amino-2,6-dichlorophenyl)-5-(2,4-difluorobenzyl)piperidin-2-one

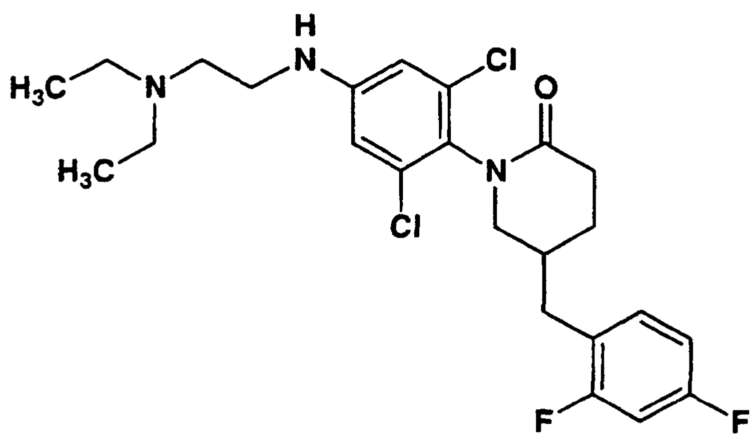

TLC:Rf 0.46 (chloroform:methanol=9:1);
NMR ($CDCl_3$):d 1.01 (t, J=7.14 Hz, 6 H) 1.69 (m, 1 H) 1.92 (m, 1 H) 2.33 (m, 1 H) 2.51 (m, 5 H) 2.65 (m, 5 H) 3.02 (m,

2 H) 3.32 (d, J=8.24 Hz, 2 H) 4.67 (m, 1 H) 6.57 (m, 2 H) 6.81 (m, 2 H) 7.14 (m, 1 H).

Example 72(2)

1-(4-(2-(piperidin-1-yl)ethyl)amino-2,6-dichlorophenyl)-5-(2,4-difluorobenzyl)piperidin-2-one

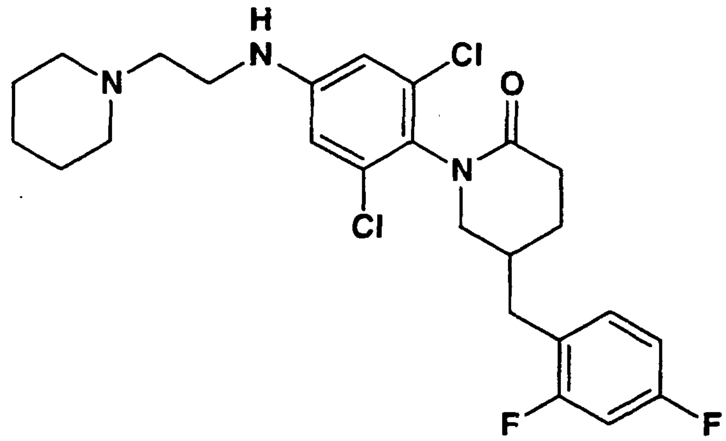

TLC:Rf 0.51 (chloroform:methanol=9:1);
NMR ($CDCl_3$):d 1.46 (m, 2 H) 1.65 (m, 5 H) 1.94 (m, 1 H) 2.33 (m, 5 H) 2.50 (m, 3 H) 2.66 (m, 3 H) 3.06 (m, 2 H) 3.33 (d, J=7.97 Hz, 2 H) 4.72 (m, 1 H) 6.58 (m, 2 H) 6.81 (m, 2 H) 7.15 (m, 1 H).

Example 72(3)

1-(4-(2-(imidazol-1-yl)ethylamino)-2,4-dichlorophenyl)-5-(2,6-difluorobenzyl)piperidin-2-one

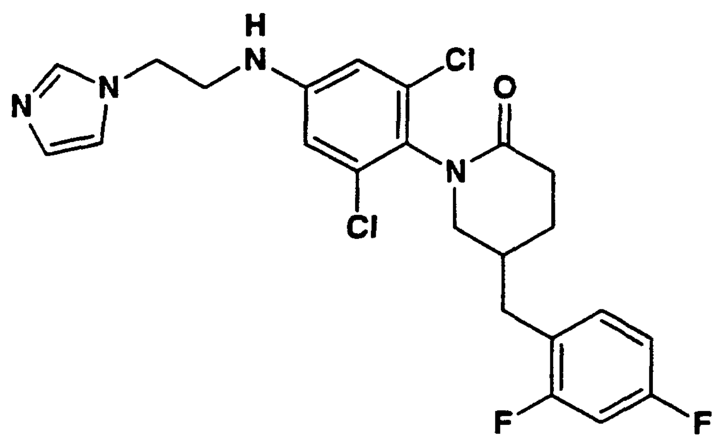

TLC:Rf 0.45 (chloroform:methanol=9:1);
NMR ($CDCl_3$):d 1.69 (m, 1 H) 1.95 (m, 1 H) 2.33 (m, 1 H) 2.50 (m, 1 H) 2.66 (m, 3 H) 3.33 (m, 4 H) 4.11 (m, 2 H) 4.67 (m, 1 H) 6.42 (s, 2 H) 6.82 (m, 2 H) 6.95 (s, 1 H) 7.07 ( s, 1 H) 7.14 (m, 1 H) 7.51 (s, 1 H).

Example 72(4)

1-(4-(2-pyrrolidin-1-yl)ethylamino-2,6-dichtorophenyl-5-(2,4-difluorobenzyl)piperidin-2-one

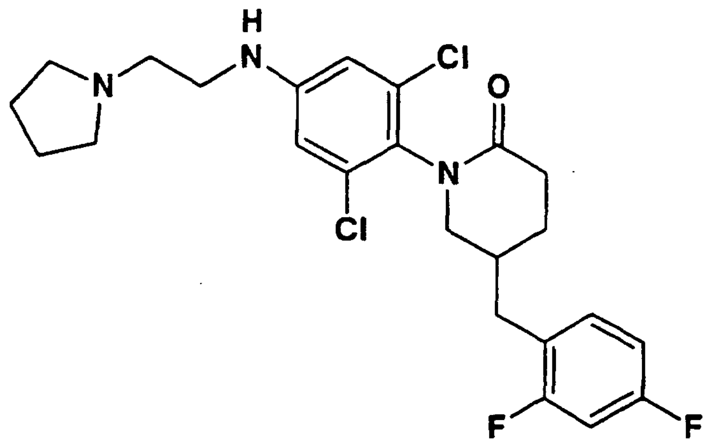

TLC:Rf 0.19 (chloroform:methanol=9:1);
NMR ($CDCl_3$):d 1.74 (m, 5 H) 1.94 (m, 1 H) 2.33 (m, 1 H) 2.49 (m, 5 H) 2.67 (m, 5 H) 3.10 (m, 2 H) 3.32 (d, J=7.69 Hz, 2 H) 4.64 (t, J=4.67 Hz, 1 H) 6.57 (m, 2 H) 6.81 (m, 2 H ) 7.15 (m, 1 H).

Example 72(5)

1-(4-(2-(4-methylpiperazin-1-yl)ethylamino)-2,6-dichlorophenyl)-5-(2,4-difluorobenzyl)piperidin-2-one

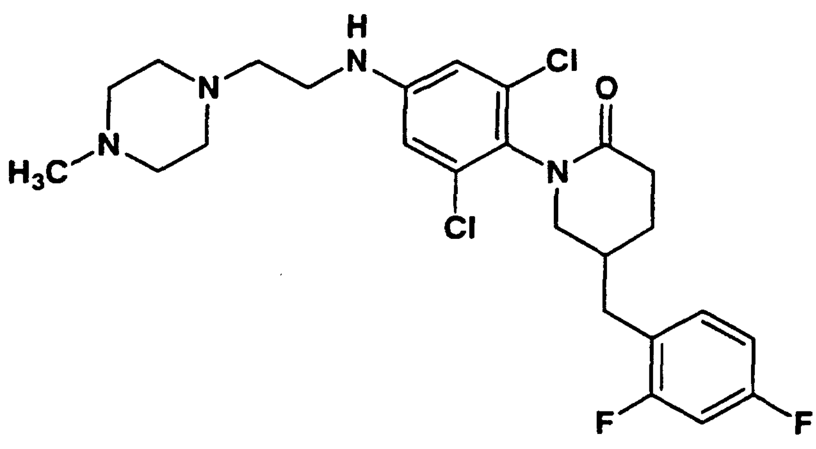

TLC:Rf 0.47 (chloroform:methanol=9:1);
NMR ($CDCl_3$):d 1.68 (m, 1 H) 1.96 (m, 1 H) 2.38 (m, 12 H) 2.64 (m, 6 H) 3.07 (m, 2 H) 3.33 (d, J=7.69 Hz, 2 H) 4.63 (m, 1 H) 6.57 (m, 2 H) 6.81 (m, 2 H) 7.15 (m, 1 H).

Example 73

1-(4-(N-(2-aminoethyl)-N-(t-butoxycarbonyl)amino)-2,6-dichlorophenyl)-5-(2,4-difluorobenzyl)piperidin-2-one

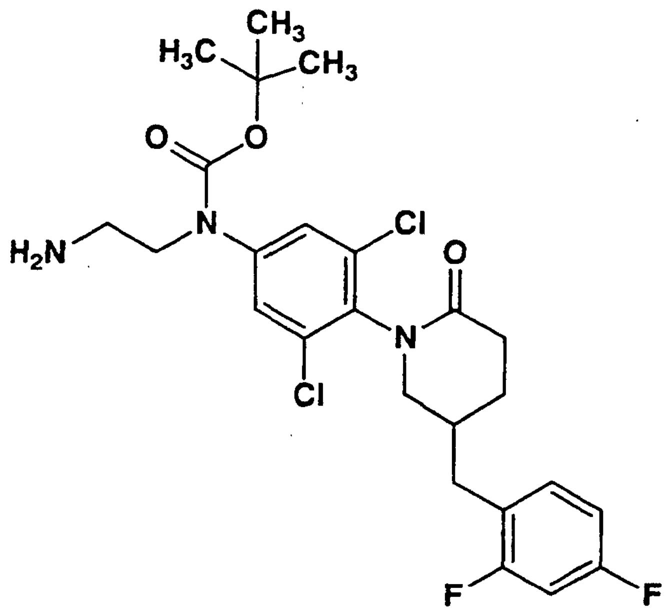

To a solution of the compound prepared in Example 63 (677 mg) in tetrahydrofuran (15 ml) were added triphenylphosphine (658 mg), phthalimide (206 mg) and diethylazodicarboxylate (40% solution in toluene, 1.02 ml). The reaction mixture was stirred at room temperature for 1 hour. The reaction mixture was concentrated and the residue was purified by column chromatography on silica gel (hexane:ethyl acetate=1:1) to give the phthalamide derivative (776 mg). To a solution of the obtained phthalamide derivative (241mg) in methanol (5 ml) was added hydrazine $1H_2O$ (90 μl). The reaction mixture was stirred at room temperature for 16 hours. The reaction mixture was filtrated and the filtrate was concentrated. The residue was purified by preparative TLC (dichloromethane:methanol=9:1) to give the compound of the present invention (112 mg) having the following physical data.
TLC:Rf 0.22(chloroform:methanol=9:1);
NMR ($CDCl_3$):d 1.47 (s, 9 H) 1.71 (m, 1 H) 1.98 (m, 1 H) 2.35 (m, 1 H) 2.51 (m, 1 H) 2.69 (m, 3 H) 2.90 (m, 2 H) 3.32 (d, J=7.69 Hz, 2 H) 3.66 (t, J=6.87 Hz, 2 H) 6.82 (m, 2 H) 7.16 (m, 1 H) 7.33 (m, 2 H).

Example 74

1-(4-(2-methanesulfonylaminoethyl)amino-2,6-dichlorophenyl)-5-(2,4-difluorobenzyl)piperidin-2-one

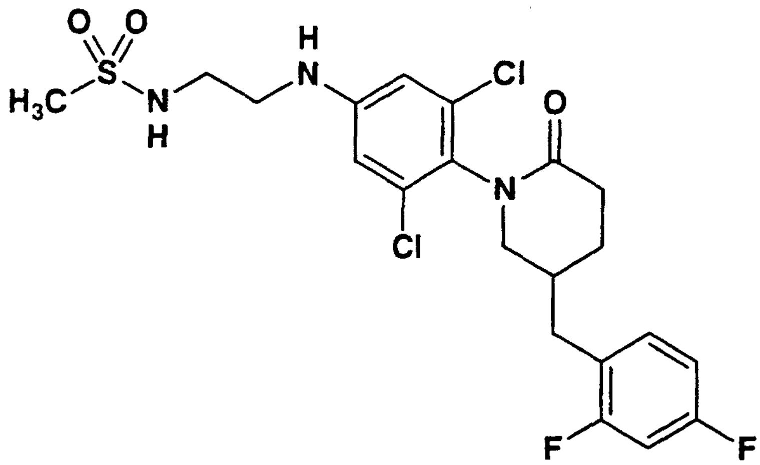

By the same procedure as described in Example 41→Example 48 using the compound prepared in Example 73, the compound of the present invention having the following physical data was obtained.
TLC:Rf 0.40 (chloroform:methanol=9:1);
NMR ($CDCl_3$):d 1.68 (m, 1 H) 1.95 (m, 1 H) 2.34 (m, 1 H) 2.51 (m, 1 H) 2.68 (m, 3 H) 2.93 (s, 3 H) 3.12 (m, 2 H) 3.26 (m, 2 H) 3.33 (d, J=7.69 Hz, 2 H) 4.85 (m, 1 H) 5.41 (m, 1 H) 6.41 (s, 2 H) 6.82 (m, 2 H) 7.15 (m, 1 H).

Example 75

1-(4-(N-(2-methylcarbonylaminoethyl)-N-(t-butoxycarbonyl)amino)-2,6-dichlorophenyl)-5-(2,4-difluorobenzyl) piperidin-2-one

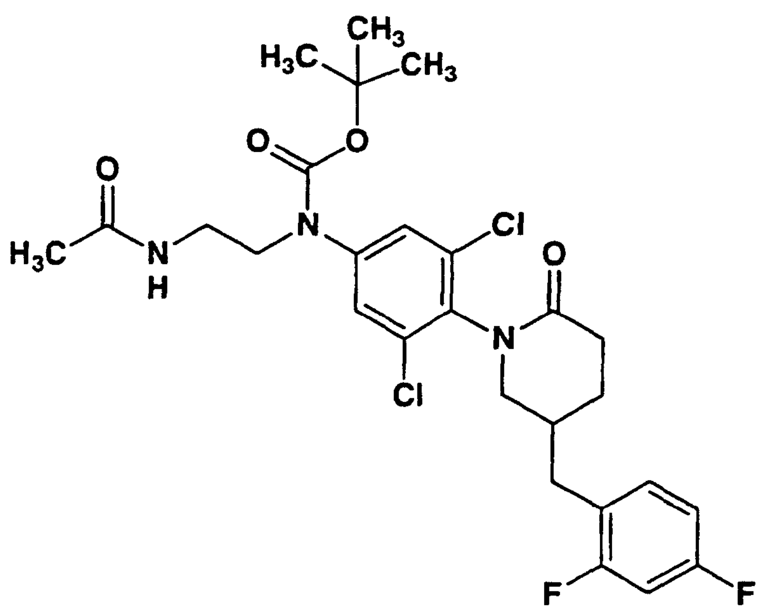

To a solution of the compound prepared in Example 73 (279 mg) in tetrahydrofuran (5 ml) were added triethylamine (220 μl) and acetic anhydride (162 mg). The reaction mixture was stirred at room temperature for 30 minutes. Ethyl acetate was added to the reaction mixture, which was washed with 1N hydrochloric acid, a saturated aqueous sodium hydrogen carbonate solution and brine, dried over anhydrous magnesium sulfate and concentrated. The obtained residue was purified by column chromatography on silica gel (ethyl acetate:methanol=1:0→19:1) to give the compound of the present invention (148 mg) having the following physical data.
TLC:Rf 0.49 (ethyl acetate:methanol=19:1);
NMR ($CDCl_3$):d 1.47 (s, 9 H) 1.71 (m, 1 H) 1.98 (m, 4 H) 2.36 (m, 1 H) 2.51 (m, 1 H) 2.68 (m, 3 H) 3.33 (d, J=7.69 Hz, 2 H) 3.45 (m, 2 H) 3.76 (t, J=5.77 Hz, 2 H) 5.92 (m, 1 H) 6.83 (m, 2 H) 7.15 (m, 1 H) 7.30 (m, 2 H).

Example 76

1-(4-(2-methylcarbonylaminoethyl)amino-2,6-dichlorophenyl)-5-(2,4-difluorobenzyl)piperidin-2-one

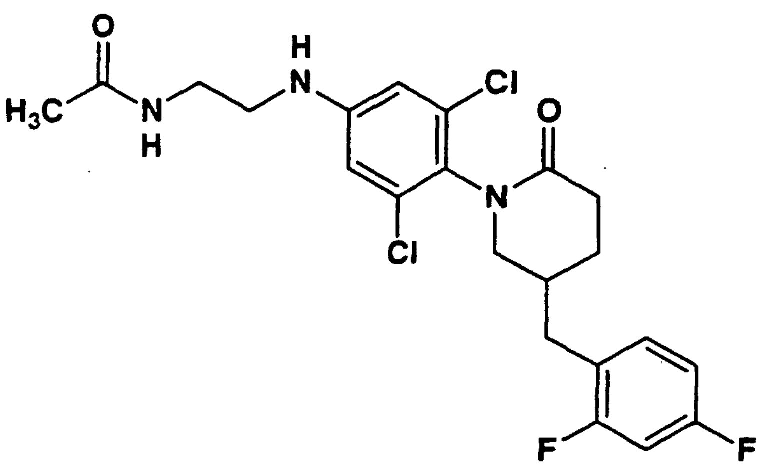

By the same procedure as described in Example 48 using the compound prepared in Example 75, the compound of the present invention having the following physical data was obtained.
TLC:Rf 0.29 (ethyl acetate:methanol=19:1);
NMR ($CDCl_3$):d 1.70 (m, 1 H) 1.95 (m, 4 H) 2.33 (m, 1 H) 2.49 (m, 1 H) 2.66 (m, 3 H) 3.04 (m, 2 H) 3.36 (m, 4 H) 4.88 (t, J=4.94 Hz, 1 H) 6.42 (s, 2 H) 6.57 (m, 1 H) 6.82 (m, 2 H) 7.14 (m, 1 H).

Example 77

1-(4-(N-(2-aminocarbonylaminoethyl)-N-(t-butoxycarbonyl)amino)-2,6-dichlorophenyl)-5-(2,4-difluorobenzyl) piperidin-2-one

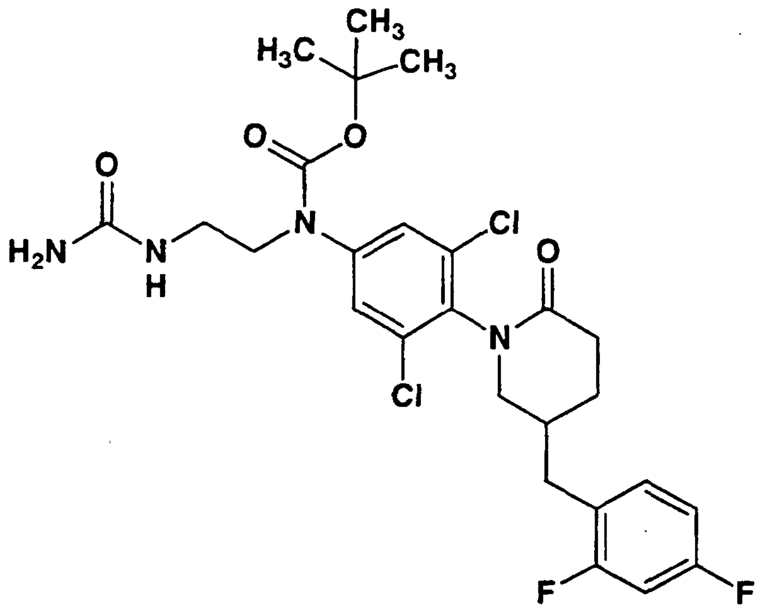

To a solution of the compound prepared in Example 73 (203 mg) in acetic acid (1 ml) and water (1 ml) was added sodium cyanate (200 mg). The reaction mixture was stirred at 80°C for 1 hour. Ethyl acetate was added to the reaction mixture, which was washed with a saturated aqueous sodium hydrogen carbonate solution and brine, dried over anhydrous magnesium sulfate and concentrated. The obtained residue was purified by preparative TLC (ethyl acetate:methanol=19:1) to give the compound of the present invention (92 mg) having the following physical data.
TLC:Rf 0.42 (ethyl acetate:methanol=19:1);
NMR ($CDCl_3$):d 1.48 (s, 9 H) 1.71 (m, 1 H) 2.00 (m, 1 H) 2.36 (m, 1 H) 2.50 (m, 1 H) 2.67 (m, 3 H) 3.37 (m, 4 H) 3.75 (m, 2 H) 4.53 (br. s., 2 H) 4.90 (m, 1 H) 6.84 (m, 2 H) 7.14 (m, 1 H) 7.33 (m, 2 H).

Example 78

1-(4-(2-aminocarbonylaminoethyl)amino-2,6-dichlorophenyl)-5-(2,4-difluorobenzyl)piperidin-2-one

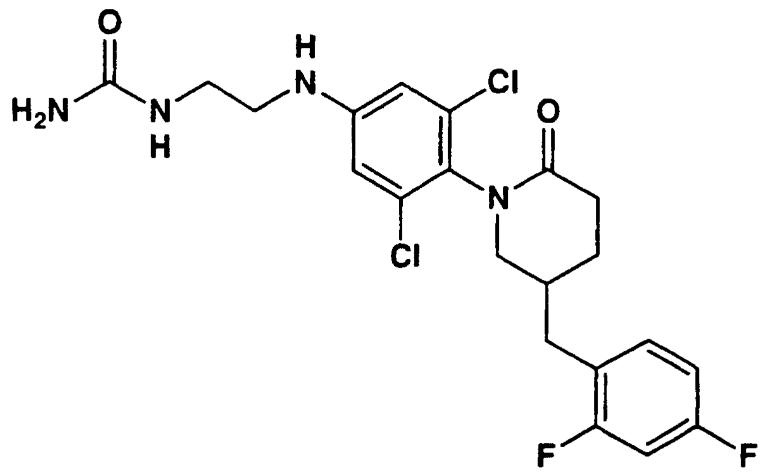

By the same procedure as described in Example 48 using the compound prepared in Example 77, the compound of the present invention having the following physical data was obtained.
TLC:Rf 0.36 (ethyl acetate:methanol=19:1);
NMR ($CDCl_3$):d 1.69 (m, 1 H) 1.96 (m, 1 H) 2.33 (m, 1 H) 2.49 (m, 1 H) 2.65 (m, 3 H) 3.04 (m, 2 H) 3.31 (m, 4 H) 4.67 (s, 2 H) 5.03 (m, 1 H) 5.67 (m, 1 H) 6.45 (m, 2 H) 6.82 ( m, 2 H) 7.15 (m, 1 H).

Example 79

1-(4-(2-(4,4-dimethyl-2,5-dioxoimidazolidin-1-yl)ethylamino)-2,6-dichlorophenyl)-5-(2,4-difluorobenzyl)piperidin-2-one

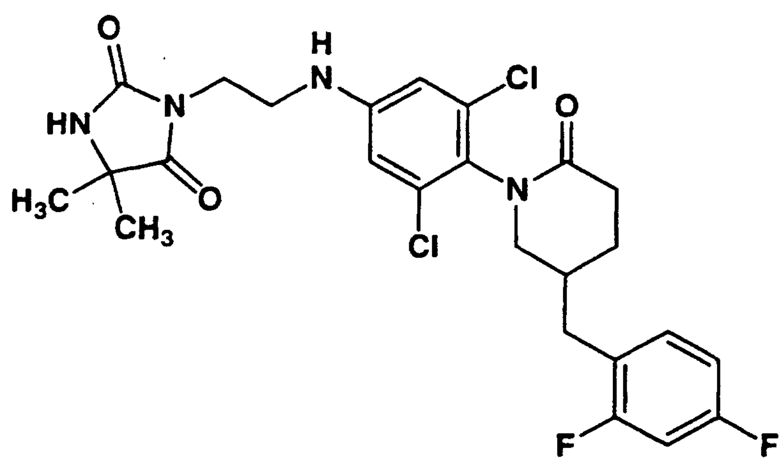

To a solution of the compound prepared in Example 63 (200 mg) in tetrahydrofuran (10 ml) were added triphenylphosphine (195 mg), 4,4-dimethyl-2,5-dioxoimidazolidine (58 mg) and diethylazodicarboxylate (40% solution in toluene, 302 μl) and the mixture was stirred at room temperature for 1 hour. The reaction mixture was concentrated. The obtained residue was purified by column chromatography on silica gel (hexane:ethyl acetate=3:7) to give the imidazolidine compound. To a solution of the imidazolidine compound in dichloromethane (2 ml) and water (0.2 ml)

was added trifluoroacetic acid (2 ml) at 0°C. The reaction mixture was stirred at room temperature for 1 hour. The reaction mixture was concentrated and dissolved in ethyl acetate. The ethyl acetate solution was washed with a saturated aqueous sodium hydrogen carbonate solution and brine, dried over anhydrous magnesium sulfate and concentrated. The residue was purified by preparative TLC (hexane:ethyl acetate=1:4) to give the compound of the present invention (145 mg) having the following physical data.
TLC:Rf 0.33 (ethyl acetate:methanol=19:1);
NMR ($CDCl_3$):d 1.39 (s, 6 H) 1.68 (m, 1 H) 1.94 (m, 1 H) 2.33 (m, 1 H) 2.48 (m, 1 H) 2.65 (m, 3 H) 3.31 (m, 4 H) 3.77 (m, 2 H) 4.49 (t, J=5.31 Hz, 1 H) 5.46 (s, 1 H) 6.56 (m, 2 H) 6.81 (m, 2 H) 7.14 (m, 1 H).

Example 80

1-(3-amino-6-chlorophenyl)-5-(2,4-difluorobenzyl)piperidin-2-one

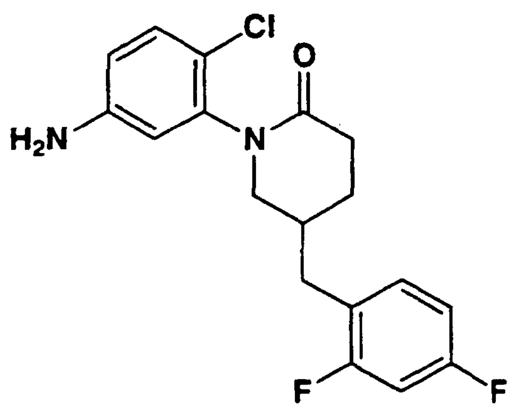

By the same procedure as described in Reference Example 31 using the compound prepared in Example 16 (11), the compound of the present invention having the following physical data was obtained.
TLC:Rf 0.55 (ethyl acetate:methanol=5:1);
NMR ($CDCl_3$):d 7.25-7.06 (m, 2H), 6.87-6.76 (m, 2H), 6.61-6.46 (m, 2H), 3.79-3.60 (m, 2H), 3.47-3.14 (m, 2H), 2.74-2.20 (m, 5H), 2.05-1.90 (m, 1H), 1.79-1.60 (m, 1H).

Example 81

1-(3-methylcarbonylamino-6-chlorophenyl)-5-(2,4-difluorobenzyl)piperidin-2-one

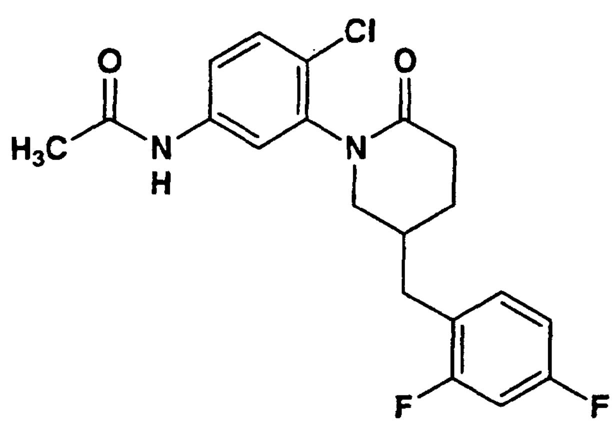

By the same procedure as described in Example 75 using the compound prepared in Example 80, the compound of the present invention having the following physical data was obtained.
TLC:Rf 0.55 (ethyl acetate:methanol=5:1);
NMR ($CDCl_3$):d 8.73-8.63 (m, 1H), 7.53-7.41 (m, 1H), 7.27-7.19 (m, 1H), 7.17-7.08 (m, 1H), 7.02-6.90 (m, 1H), 6.88-6.64 (m, 2H), 3.50-3.25 (m, 2H), 2.75-2.25 (m, 5H), 2.08-1.91 (m, 4H), 1.82-1.67 (m, 1H).

Example 82

1-(3-methanesulfonylamino-6-chlorophenyl)-5-(2,4-difluorobenzyl)piperidin-2-one

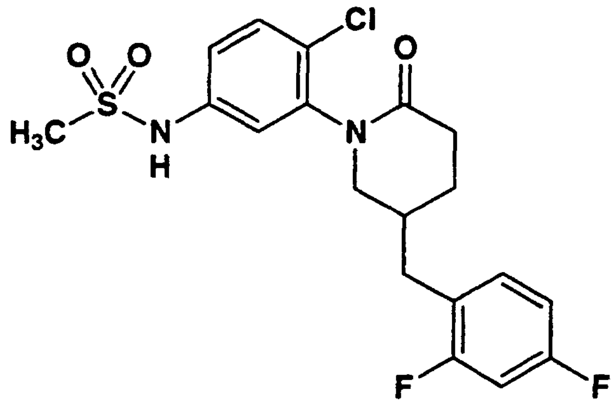

By the same procedure as described in Example 41 using the compound prepared in Example 80, the compound of the present invention having the following physical data was obtained.
TLC:Rf 0.41 (ethyl acetate);
NMR ($CDCl_3$):d 7.44-7.28 (m, 2H), 7.19-6.93 (m, 3H), 6.88-6.72 (m, 2H), 3.52-3.18 (m, 2H), 3.07-2.90 (m, 3H), 2.78-2.48 (m, 4H), 2.41-2.26 (m, 1H), 2.10-1.92 (m, 1H), 1.83-1.67 (m, 1H).

Example 83

1-(3-ethoxycarbonylmethylamino-6-chlorophenyl)-5-(2,4-difluorobenzyl)piperidin-2-one

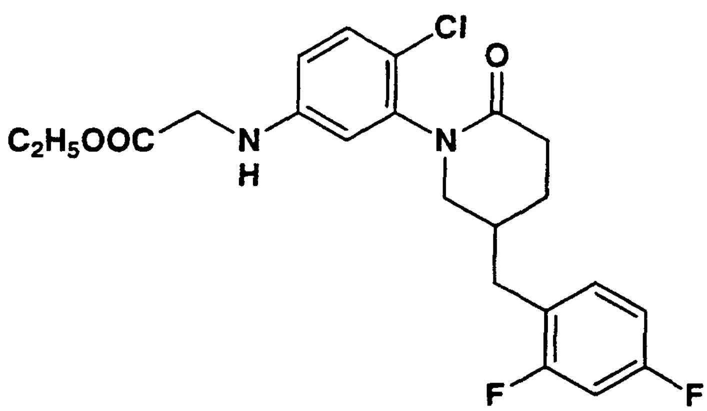

By the same procedure as described in Example 51 using ethyl bromoacetate and the compound prepared in Example 80 instead of methyl iodide, the compound of the present invention having the following physical data was obtained.
TLC:Rf 0.24 (ethyl acetate:hexane=2:1);

NMR ($CDCl_3$):d 7.26-7.08 (m, 2H), 6.88-6.74 (m, 2H), 6.53-6.39 (m, 2H), 4.47-4.34 (m, 1H), 4.30-4.18 (m, 2H), 3.90-3.78 (m, 2H), 3.50-3.18 (m, 2H), 2.74-2.20 (m, 5H), 2.08-1.90 (m, 1H), 1.80-1.62 (m, 1H), 1.37-1.22 (m, 3H).

Example 84

1-(3-carboxylmethylamino-6-chlorophenyl)-5-(2,4-difluorobenzyl)piperidin-2-one

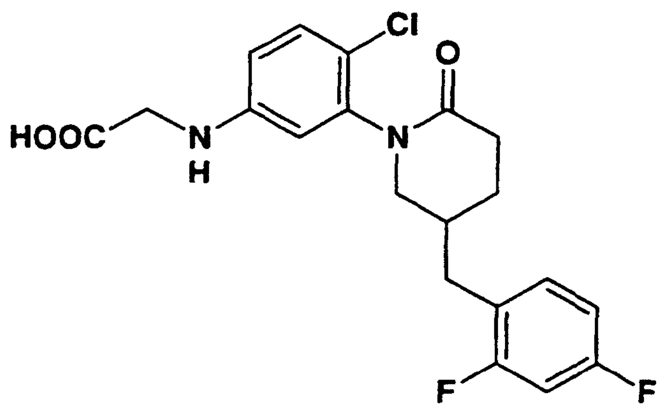

By the same procedure as described in Example 36 using the compound prepared in Example 83, the compound of the present invention having the following physical data was obtained.
TLC:Rf 0.51 (ethyl acetate:methanol:water=6:3:1);
NMR ($CDCl_3$):d 7.45-7.32 (m, 1H), 7.25-7.12 (m, 2H), 7.08-6.98 (m, 1H), 6.53-6.45 (m, 2H), 3.82-3.72 (m, 2H), 3.25-3.15 (m, 2H), 2.74-2.66 (m, 2H), 2.41-2.13 (m, 3H), 1.90-1.78 (m, 1H), 1.70-1.47 (m, 1H).

Example 85

1-(3-(2-hydroxyethylamino)-6-chlorophenyl)-5-(2,4-difluorobenzyl)piperidin-2-one

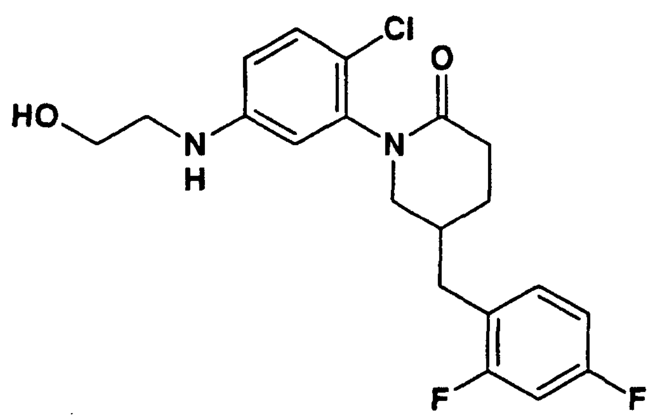

By the same procedure as described in Example 37 using the compound prepared in Example 84, the compound of the present invention having the following physical data was obtained.
TLC:Rf 0.46 (ethyl acetate:methanol=5:1);
NMR ($CDCl_3$):d 7.14-7.05 (m, 2H), 6.84-6.67 (m, 2H), 6.28-6.10 (m, 2H), 3.92-3.75 (m, 2H), 3.27-3.123 (m, 3H), 2.61-2.47 (m, 4H), 2.39-2.25 (m, 2H), 1.85-1.62 (m, 2H).

Example 86

1-(4-(2-(N,N-dimethylamino)ethyl)amino-2,6-dichlorophenyl)-5-(2-fluorobenzyl)piperidin-2-one

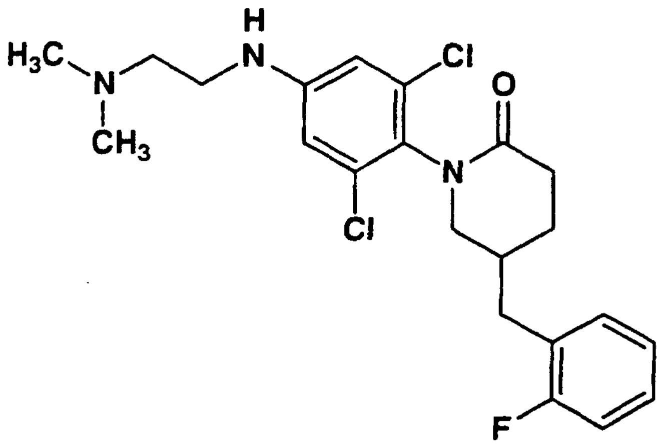

By the same procedure as described in Example 13→Example 48 using the compound prepared in Example 16(12) and 2-(N,N-dimethylamino)-1-chloroethane, the compound of the present invention having the following physical data was obtained.
TLC:Rf 0.39 (chloroform:methanol=4:1);
NMR ($CDCl_3$):d 1.70 (m, 1 H) 1.96 (m, 1 H) 2.23 (m, 6 H) 2.39 (m, 2 H) 2.52 (t, J=5.70 Hz, 2 H) 2.66 (m, 1 H) 2.74 (d, J=7.10 Hz, 2 H) 3.05 (q, J=4.80 Hz, 2 H) 3.35 (d, J=8.00 Hz, 2 H) 4.62 (m, 1 H) 6.57 (m, 2 H) 7.05 (m, 2 H) 7.19 (m, 2 H).

Example 87

1-(2,6-dichlorophenyl)-5-(4-aminobenzyl)piperidin-2-one

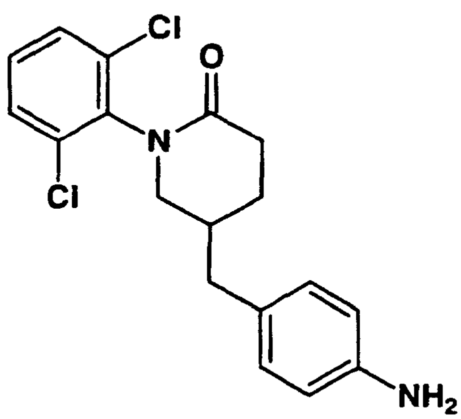

By the same procedure as described in Example 10 using the compound prepared in Example 16(4), the compound of the present invention having the following physical data was obtained.
TLC:Rf 0.31 (ethyl acetate:hexane=2:1);
NMR ($CDCl_3$):d 7.41-7.33 (m, 2H), 7.19 (t, J = 7.8 Hz, 1H), 6.97 and 6.63 (each d, J = 8.4 Hz, each 2H), 3.66-3.52 (m, 2H), 3.37-3.25 (m, 2H), 2.73-2.42 (m, 4H), 2.39-2.20 (m, 1H), 2.07-1.93 (m, 1H), 1.75-1.60 (m, 1H).

Example 88

1-(2,6-dichlorophenyl)-5-(4-cyclohexylcarbonylaminobenzyl)piperidin-2-one

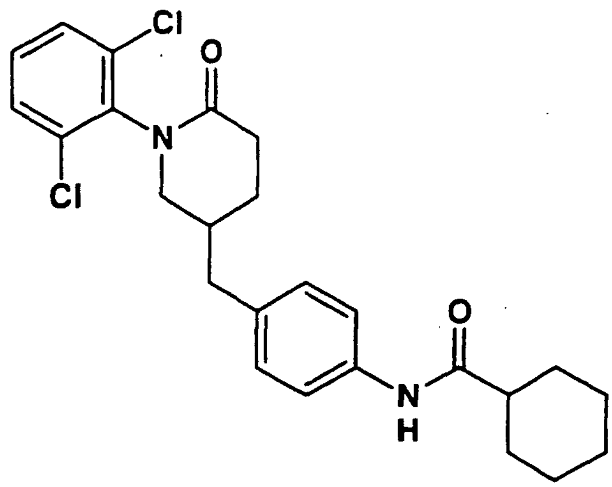

By the same procedure as described in Example 12 using the compound prepared in Example 87 and cyclohexanecarbonyl chloride, the compound of the present invention having the following physical data was obtained.
TLC:Rf 0.54 (chloroform:methanol=9:1);
NMR (DMSO-$d_6$):d 9.72 (s, 1H), 7.58-7.50 (m, 2H), 7.50 (d, J = 8.1 Hz, 2H), 7.37 (t, J = 7.8 Hz, 1H), 7.13 (d, J = 8.1 Hz, 2H), 3.26-3.16 (m, 2H), 2.66-2.59 (m, 2H), 2.50-2.39 (m, 2H), 2.38-2.20 (m, 2H), 1.98-1.51 (m, 7H), 1.48-1.11 (m, 5H).

Example 89

1-(2,6-dichlorophenyl)-5-(4-methylcarbonylaminobenzyl)piperidin-2-one

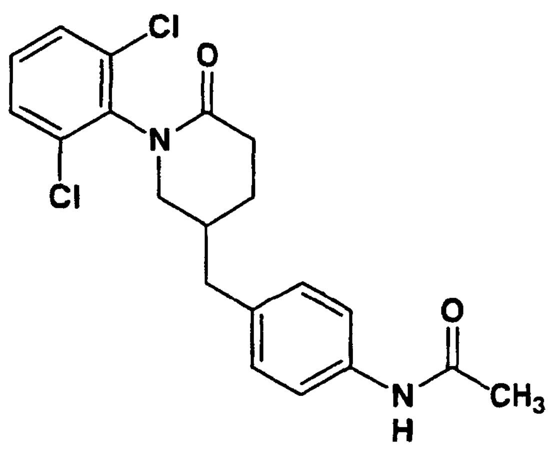

By the same procedure as described in Example 75 using the compound prepared in Example 87, the compound of the present invention having the following physical data was obtained.
TLC:Rf 0.42 (chloroform:methanol=9:1);
NMR (DMSO-$d_6$):d 9.85 (s, 1H), 7.58-7.52 (m, 2H), 7.47 (d, J = 8.4 Hz, 2H), 7.37 (t, J = 8.1 Hz, 1H), 7.13 (d, J = 8.4 Hz, 2H), 3.25-3.16 (m, 2H), 2.66-2.60 (m, 2H), 2.50-2.39 (m, 2H), 2.33-2.19 (m, 1H), 2.00 (s, 3 H), 1.94-1.82 (m, 1H), 1.67-1.51 (m, 1H).

Example 90

1-(4-amino-2-chlorophenyl)-5-(2,4-difluorobenzyl)piperidin-2-one

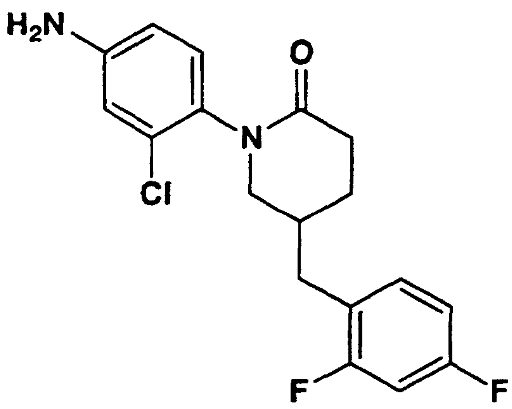

By the same procedure as described in Reference Example 31 using the compound prepared in Example 16 (9), the compound of the present invention having the following physical data was obtained.
TLC:Rf 0.22 (ethyl acetate);
NMR ($CDCl_3$):d 1.70 (m, 1 H) 1.94 (m, 1 H) 2.26 (m, 1 H) 2.56 (m, 4 H) 3.30 (m, 2 H) 3.76 (m, 2 H) 6.54 (m, 1 H) 6.77 (m, 3 H) 6.95 (dd, J=13.32, 8.38 Hz, 1 H) 7.12 (m, 1 H).

Example 91

1-(2,6-dichlorophenyl)-5-(2-hydroxymethylbenzyl)piperidin-2-one

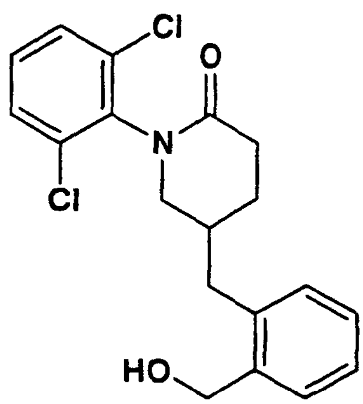

To a solution of the compound prepared in Example 16(8) (3.86 g) in methanol (20 ml) was added concentrated hydrochloric acid (0.5 ml). The reaction mixture was stirred at 65°C for 3.5 hours. The reaction mixture was neutralized with a saturated aqueous sodium hydrogen carbonate solution and then the reaction mixture was concentrated and extracted with ethyl acetate. The extract was washed with water and brine, dried over anhydrous magnesium sulfate and concentrated. The obtained residue was washed with isopropyl ether to give the compound of the present invention (2.74g) having the followed physical data.
TLC:Rf 0.26 (hexane:ethyl acetate=2:3);
NMR ($CDCl_3$):d 1.77 (m, 1H), 2.01 (m, 1H), 2.57-2.35 (m, 2H), 2.66 (m, 1H), 2.87-2.67 (m, 2H), 3.45-3.32 (m, 2H), 4.75 (d, J =10.2 Hz, 2H),7.33-7.17 (m, 4H),7.43-7.35 (m, 3H).

Example 92

1-(2,6-dichlorophenyl)-5-(2-aminomethylbenzyl)piperidin-2-one hydrochloride

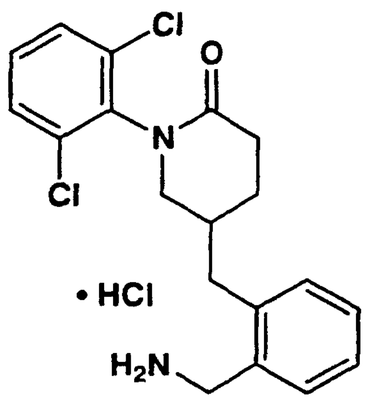

By the same procedure as described in Example 73→Reference Example 31 using the compound prepared in Example 91, the compound of the present invention having the following physical data was obtained.
TLC:Rf 0.18 (chloroform:methanol=9:1);
NMR ($CD_3OD$):d 1.80 (m, 1 H) 2.01 (m, 1 H) 2.37 (m, 1 H) 2.52 (m, 1 H) 2.63 (m, 1 H) 2.86 (m, 2 H) 3.38 (m, 1 H) 3.49 (m, 1 H) 4.22 (s, 2 H) 7.35 (m, 4 H) 7.42 (m, 1 H) 7.49 (m , 2 H).

Example 93

1-(2,6-dichlorophenyl)-5-(2-(1-methyl-3-t-butylpyrazol-5-yl)aminocarbonylaminomethylbenzyl)piperidin-2-one

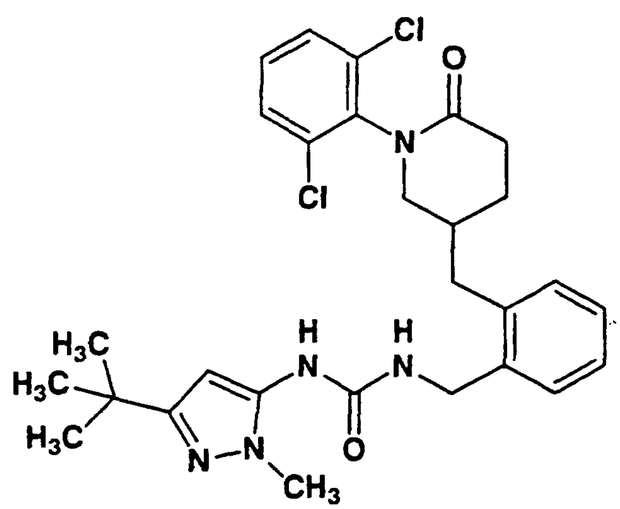

To a solution of the compound prepared in Example 92 (130 mg) in tetrahydrofuran (5 ml) was added N-(3-t-butyl-1-methylpyrazol-5-yl)-1-(4-dimethylidenehydropyridyl)carboamide (108 mg) at room temperature. The reaction mixture was stirred at room temperature for 15 minutes. 1N hydrochloric acid was added to the reaction mixture, which was extracted with ethyl acetate. The extract was washed 1N hydrochloric acid, a saturated aqueous sodium hydrogen carbonate solution and brine, dried over anhydrous sodium sulfate and concentrated. The obtained residue was purified by preparative TLC (chloroform:methanol=9:1, twice) to give the compound of the present invention having the following

physical data.
TLC:Rf 0.50 (chloroform:methanol 9:1);
NMR ($CDCl_3$):d 1.25 (s, 9 H) 1.73 (m, 1 H) 1.95 (m, 1 H) 2.56 (m, 5 H) 3.38 (d, J=7.69 Hz, 2 H) 3.62 (s, 3 H) 4.45 (d, J=5.49 Hz, 2 H) 5.11 (m, 1 H) 5.97 (s, 1 H) 6.37 (s, 1 H) 7.26 (m, 7 H).

Example 94

1-(2,6-dichlorophenyl)-5-(2-benzylaminocarbonylaminomethylbenzyl)piperidin-2-one

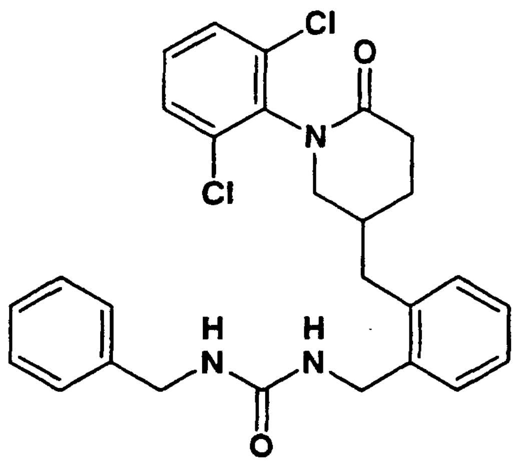

By the same procedure as described in Reference Example 13 using the compound prepared in Example 92 and benzylisocyanate, the compound of the present invention having the following physical data was obtained.
TLC:Rf 0.34 (chloroform:methanol 19:1);
NMR (DMSO-$d_6$):d 1.65 (m, 1 H), 1.89 (m, 1 H), 2.38 (m, 3 H), 2.71 (m, 2 H), 3.29 (m, 2 H), 4.21 (d, J=5.49 Hz, 2 H), 4.28 (d, J=4.94 Hz, 2 H), 6.38 (m, 2 H), 7.24 (m, 10 H), 7.57 (m, 2 H).

Examples 94(1)-94(6)

By the same procedure as described in Example 94 using the corresponding isocyanate instead of benzylisocyanate, the following compounds of the present invention were obtained.

Example 94(1)

1-(2,6-dichlorophenyl)-5-(2-phenylaminocarbonylaminomethylbenzyl)piperidin-2-one

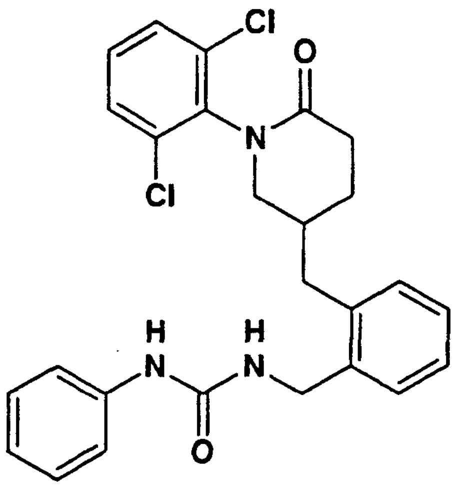

TLC:Rf 0.33 (chloroform:methanol 19:1);
NMR (DMSO-$d_6$):d 1.64 (m, 1 H), 1.92 (m, 1 H), 2.39 (m, 3 H), 2.76 (d, J=6.87 Hz, 2 H), 3.28 (m, 2 H), 4.33 (d, J=5.49 Hz, 2 H), 6.49 (t, J=5.49 Hz, 1 H), 6.88 (t, J=7.28 Hz, 1 H), 7.27 (m, 9 H), 7.54 (m, 2 H), 8.4 6 (s, 1 H).

Example 94(2)

1-(2,6-dichlorophenyl)-5-(2-cyclohexylaminocarbonylaminomethylbenzyl)piperidin-2-one

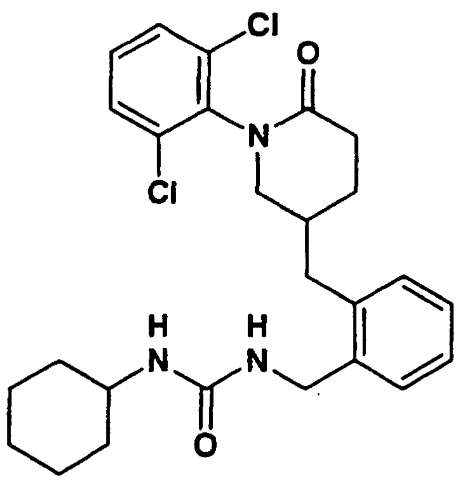

TLC:Rf 0.32 (chloroform:methanol 19:1);
NMR (DMSO-$d_6$):d 1.15 (m, 6 H), 1.67 (m, 7 H), 2.37 (m, 3 H), 2.73 (m, 2 H), 3.30 (m, 2 H), 4.22 (m, 2 H), 5.76 (d, J=7.69 Hz, 1 H), 6.05 (t, J=5.49 Hz, 1 H), 7.15 (m, 4 H), 7.37 (t, J=7.97 Hz, 1 H), 7.55 (m, 2 H).

Example 94(3)

1-(2,6-dichlorophenyl)-5-(2-(3-trifluoromethylphenyl)aminocarbonylaminomethylbenzyl)piperidin-2-one

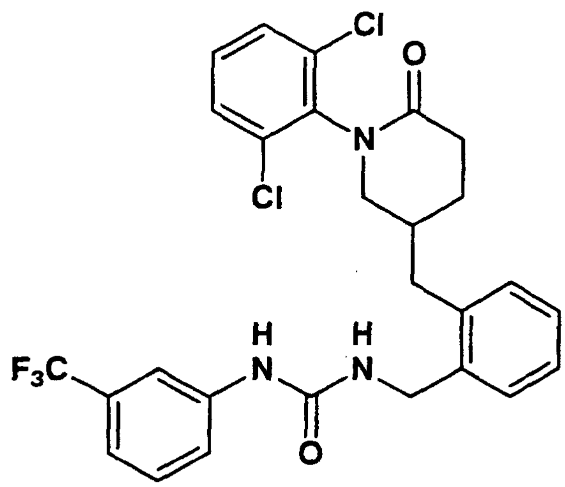

TLC:Rf 0.32 (chloroform:methanol 19:1);
NMR (DMSO-$d_6$):d 1.67 (m, 1 H), 1.89 (m, 1 H), 2.38 (m, 3 H), 2.76 (m, 2 H), 3.27 (m, 2 H), 4.36 (d, J=5.22 Hz, 2 H), 6.67 (t, J=5.22 Hz, 1 H), 7.37 (m, 10 H), 7.96 (s, 1 H), 8.88 (s, 1 H).

Example 94(4)

1-(2,6-djchlorophenyl)-5-(2-ethylaminocarbonylaminomethybenzyl)piperidin-2-one

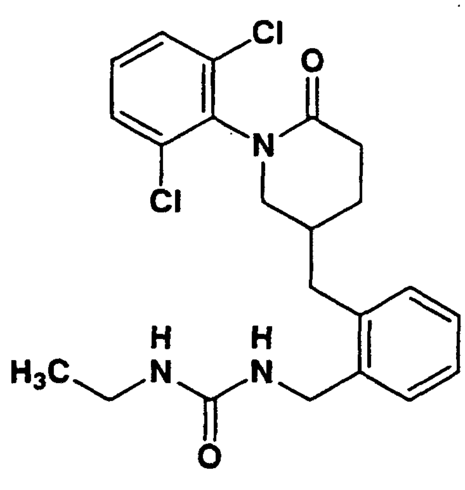

TLC:Rf 0.29 (chloroform:methanol 19:1);
NMR (DMSO-$d_6$):d 0.97 (t, J=7.00 Hz, 3 H), 1.65 (m, 1 H), 1.88 (m, 1 H), 2.35 (m, 3 H), 2.73 (m, 2 H), 3.00 (m, 2 H), 3.28 (m, 2 H), 4.23 (d, J=5.49 Hz, 2 H), 5.81 (t, J=4.67 Hz, 1 H), 6.16 (t, J=5.49 Hz, 1 H), 7.1 8 (m, 4 H), 7.38 (t, J=7.97 Hz, 1 H), 7.58 (m, 2 H).

Example 94(5)

1-(2,6-dichlorophenyl)-5-(2-(3-methylphenyl)aminocarbonylaminomethylbenzyl)piperidin-2-one

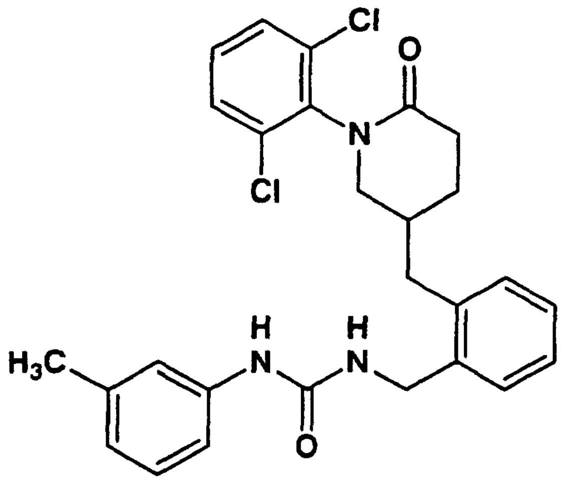

TLC:Rf 0.49 (chloroform:methanol 19:1);
NMR (DMSO-$d_6$):d 1.66 (m, 1 H), 1.89 (m, 1 H), 2.22 (s, 3 H), 2.37 (m, 3 H), 2.76 (d, J=6.87 Hz, 2 H), 3.25 (m, 2 H), 4.33 (d, J=5.08 Hz, 2 H), 6.47 (t, J=5.08 Hz, 1 H), 6.70 (d, J=7.14 Hz, 1 H), 7.20 (m, 8 H), 7.5 3 (m, 2 H), 8.37 (s, 1 H).

Example 94(6)

1-(2,6-dichlorophenyl)-5-(2-(2-trifluoromethylphenyl)aminocarbonylaminomethylbenzyl)piperidin-2-one

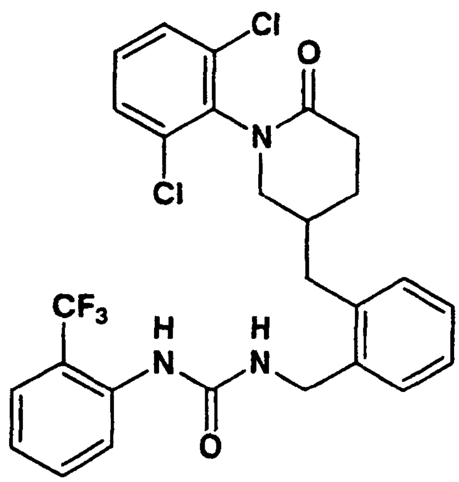

TLC:Rf 0.51 (chloroform:methanol 19:1);
NMR (DMSO-$d_6$):d 1.67 (m, 1 H), 1.88 (m, 1 H), 2.35 (m, 3 H), 2.77 (m, 2 H), 3.29 (m, 2 H), 4.35 (d, J=5.22 Hz, 2 H), 7.25 (m, 7 H), 7.57 (m, 4 H), 7.80 (s, 1 H), 7.97 (d, J=8.24 Hz, 1 H).

Example 95

1-(2,6-dichlorophenyl)-5-(2-methylcarbonylaminomethylbenzyl)piperidin-2-one

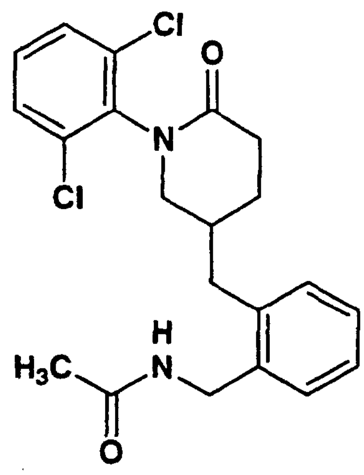

By the same procedure as described in Example 75 using the compound prepared in Example 92, the compound of the present invention having the following physical data was obtained.
TLC:Rf 0.61 (ethyl acetate:methanol=9:1);
NMR ($CDCl_3$):d 1.75 (m, 1 H) 1.99 (s, 3 H) 2.00 (m, 1 H) 2.33 (m, 1 H) 2.48 (m, 1 H) 2.69 (m, 3 H) 3.38 (m, 2 H) 4.47 (d, J=5.49 Hz, 2 H) 5.67 (s, 1 H) 7.23 (m, 5 H) 7.37 (m, 2 H).

Example 96

1-(2,6-dichlorophenyl)-5-(2-(3,8,8-trimethyl-5,6,7,8-tetrahydronaphthalene-2-yl)aminocarbonylaminomethylbenzyl) piperidin-2-one

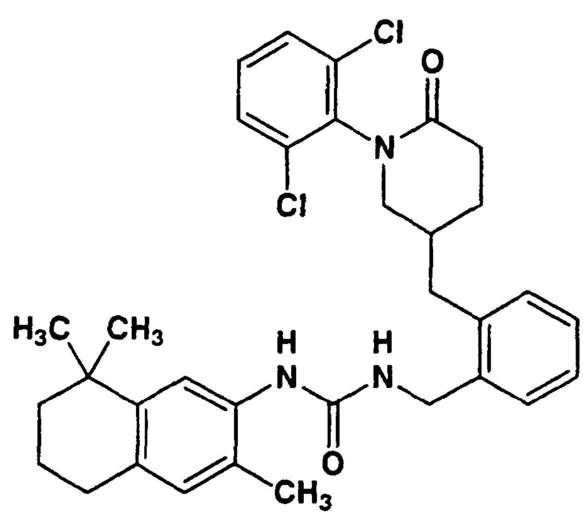

To a solution of 1,1'-carbonylimidazole (45 mg) in tetrahydrofuran(3ml) was added a solution of the compound prepared in Example 92 (100 mg) in tetrahydrofuran (1ml) at 0°C. The mixture was stirred at room temperature for 30 minutes. To the reaction mixture was added a solution of 3,8,8-trimethyl-2-5,6,7,8-tetrahydronaphthylamine (55 mg) in tetrahydrofuran (1 ml). The reaction mixture was stirred at room temperature overnight. To the reaction mixture were

added ethyl acetate and 1N hydrochloric acid sequentially and the reaction mixture was extracted. The water layer was alkalinized with a saturated aqueous sodium hydrogen carbonate. The water layer was extracted with ethyl acetate. The extract was washed with brine, dried over anhydrous sodium sulfate and concentrated. The obtained residue was purified by preparative TLC (chloroform:methanol=9:1) to give the compound of the present invention (12 mg )having the following physical data.
TLC:Rf 0.34 (chloroform:methanol 40:1);
NMR ($CDCl_3$):d 1.18 (s, 6 H), 1.67 (m, 5 H), 1.95 (m, 1 H), 2.17 (s, 3 H), 2.37 (m, 2 H), 2.72 (m, 5 H), 3.38 (d, J=7.69 Hz, 2 H), 4.48 (d, J=5.22 Hz, 2 H), 4.71 (t, J=5.22 Hz, 1 H), 5.91 (s, 1 H), 6.89 (s, 1 H), 7.20 (m, 6 H), 7.37 (m, 2 H).

Example 97

1-(2,6-dichlorophenyl)-5-(2-(1-methyl-3-t-butylpyrazol-5-yl)aminocarbonyloxymethylbenzyl)piperidin-2-one

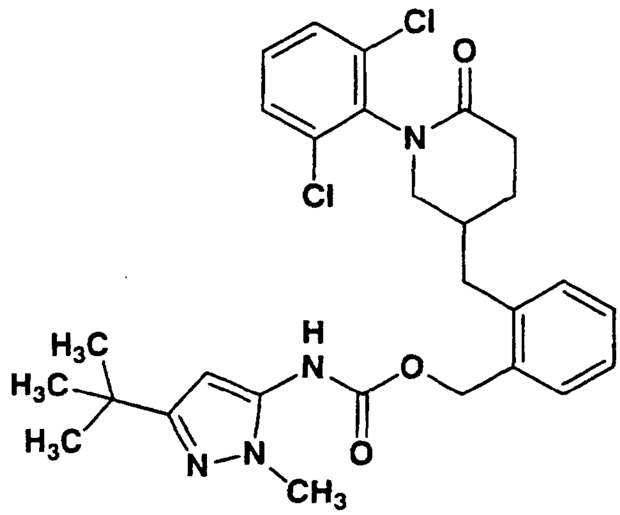

By the same procedure as described in Example 93 using the compound prepared in Example 91, the compound of the present invention having the following physical data was obtained.
TLC:Rf 0.34 (chloroform:methanol 19:1);
NMR (DMSO-$d_6$):d 1.17 (s, 9 H), 1.66 (m, 1 H), 1.85 (m, 1 H), 2.36 (m, 3 H), 2.78 (m, 2 H), 3.29 (m, 2 H), 3.53 (s, 3 H), 5.20 (s, 2 H), 5.93 (s, 1 H), 7.26 (m, 3 H), 7.39 (m, 2 H), 7.54 (m, 2 H), 9.57 (s, 1 H).

Example 98

1-(2,6-dichlorophenyl)-5-(2-formylbenzyl)piperidin-2-one

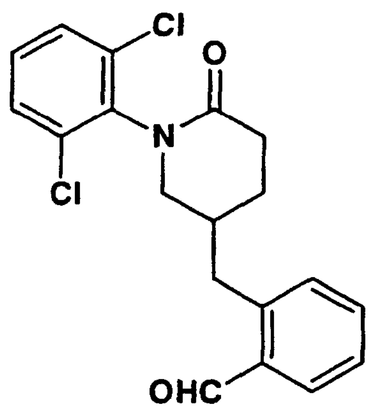

To a solution of the compound prepared in Example 91 (170 mg) in ethyl acetate (3 ml) and dimethylsulfoxyde (3 ml) were added triethylamine (387 µl) and sulfur trioxide pyridine complex (223 mg) at 0°C. The reaction mixture was stirred at room temperature for 30 minutes. To the reaction mixture was added ethyl acetate. The reaction mixture was washed with 1N hydrochloric acid, a saturated aqueous sodium hydrogen carbonate solution and brine, dried over anhydrous magnesium sulfate and concentrated to give the compound of the present invention (169 mg) having the following physical data.
TLC:Rf 0.49 (hexane:ethyl acetate=3:2);
NMR ($CDCl_3$):d 1.79 (m, 1 H) 1.99 (m, 1 H) 2.35 (m, 1 H) 2.48 (m, 1 H) 2.67 (m, 1 H) 3.07 (dd, J=12.91, 7.42 Hz, 1 H) 3.21 (dd, J=12.91, 6.04 Hz, 1 H) 3.42 (d, J=7.97 Hz, 2 H) 7.20 (t, J=7.97 Hz, 1 H) 7.29 (m, 1 H) 7.38 (m, 2 H) 7.50 (m, 2 H) 7.84 (m, 1 H) 10.19 (s, 1 H).

Example 99

1-(2,6-dichlorophenyl)-5-(2-(2-methoxycarbonylvinyl)benzyl)piperidin-2-one

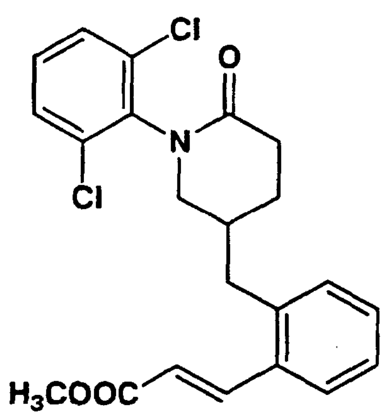

To a solution of the compound prepared in Example 98 (165 mg) in acetonitrile (10 ml) were added diisopropylethylamine (120 µl), lithium chloride (26 mg) and dimethylmethoxycarbonylmethyl phosphonate (125 mg). The reaction mixture was stirred at room temperature for 24 hours. To the reaction mixture was added ethyl acetate. The reaction mixture was washed with 1N hydrochloric acid, a saturated aqueous sodium hydrogen carbonate solution and brine, dried over anhydrous magnesium sulfate and concentrated to give the compound of the present invention (190

mg) having the following physical data.
TLC:Rf 0.49 (hexane:ethyl acetate=3:2);
NMR ($CDCl_3$):d 1.72 (m, 1 H) 1.99 (m, 1 H) 2.30 (m, 1 H) 2.49 (m, 1 H) 2.68 (m, 1 H) 2.85 (m, 2 H) 3.38 (m, 2 H) 3.83 (s, 3 H) 6.39 (d, J=15.66 Hz, 1 H) 7.30 (m, 6 H) 7.59 (m, 1 H) 8.00 (d, J=15.66 Hz, 1 H).

Example 100

1-(2,6-dichlorophenyl)-5-(2-(2-methoxycarbonylvinyl)benzyl)piperidin-2-one

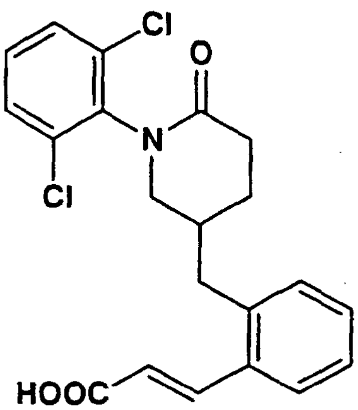

By the same procedure as described in Example 36 using the compound prepared in Example 99, the compound of the present invention having the following physical data was obtained.
TLC:Rf 0.43 (ethyl acetate);
NMR ($CDCl_3$):d 1.76 (m, 1 H) 1.99 (m, 1 H) 2.32 (m, 1 H) 2.51 (m, 1 H) 2.69 (m, 1 H) 2.87 (m, 2 H) 3.36 (m, 2 H) 6.41 (d, J=15.66 Hz, 1 H) 7.29 (m, 6 H) 7.64 (dd, J=7.42, 1.37 Hz, 1 H) 8.09 (d, J=15.66 Hz, 1 H).

Example 101

1-(2,6-dichlorophenyl)-5-(2-(1-methyl-3-t-butylpyrazol-5-yl)aminocarbonyl)ethylpiperidin-2-one

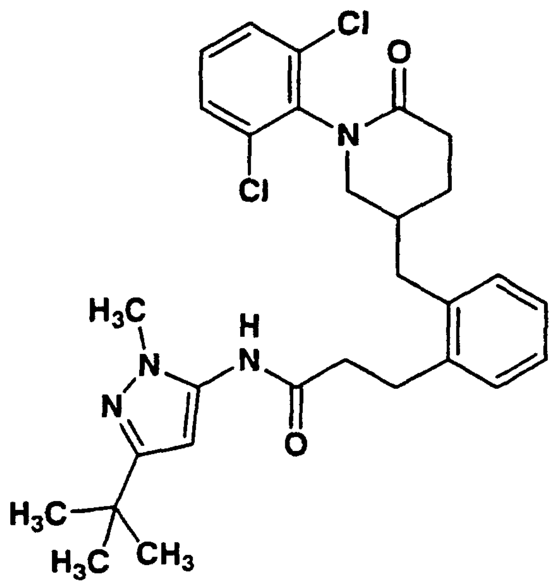

By the same procedure as described in Reference Example 37→Reference Example 15 using the compound prepared in Example 100 and 5-amino-1-methyl-3-t- butylpyrazole, the compound of the present invention having the following physical data was obtained.
TLC:Rf 0.52 (ethyl acetate);
NMR ($CDCl_3$):d 1.26 (s, 9 H) 1.74 (m, 1 H) 2.00 (m, 1 H) 2.39 (m, 2 H) 2.68 (m, 5 H) 3.07 (t, J=7.55 Hz, 2 H) 3.38 (m, 2 H) 3.51 (s, 3 H) 6.04 (s, 1 H) 7.17 (m, 6 H) 7.35 (m, 2 H).

Example 102

1-(2,6-dimethylphenyl)-5-(2-hydroxybenzyl)piperidin-2-one

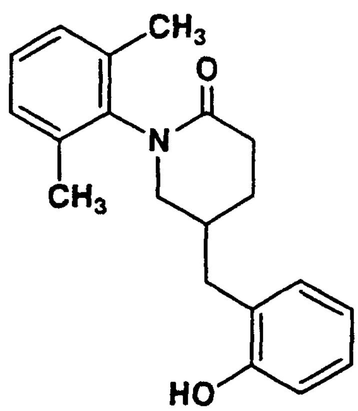

By the same procedure as described in Example 48 using the compound prepared in Example 16(3), the compound of the present invention having the following physical data was obtained.

TLC:Rf 0.37(hexane:ethyl acetate=1:4);
NMR ($CDCl_3$):d 1.62 (m, 1H).1.98 (m, 1H), 2.09 (s, 3H), 2.21 (s, 3H), 2.55-2.35 (m, 2H), 2.73-2.62 (m, 3H), 3.30-3.19 (m, 2H), 6.12 (s, 1H), 6.65 (m, 1H), 6.80 (m, 1H), 7.14-6.99 (m, 5H).

Example 103

1-(2,6-dimethylphenyl)-5-(2-isobutyloxybenzyl)piperidin-2-one

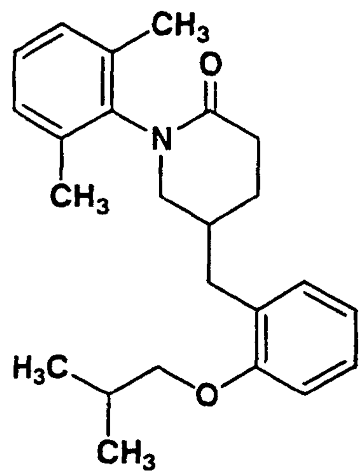

By the same procedure as described in Example 51 using the compound prepared in Example 102 and 1-iodo-2-methylpropane, the compound of the present invention having the following physical data was obtained.
TLC:Rf 0.56 (ethyl acetate:hexane=4:1);
NMR ($CDCl_3$):d 7.18 (m, 1H), 7.16-7.04 (m, 4H), 6.88-6.81 (m, 2H), 3.79-3.68 (m, 2H), 3.30-3.20 (m, 2H), 2.76 (dd, J = 12.9, 6.3 Hz, 1H), 2.71-2.58 (m, 2H), 2.48 (ddd, J = 18.3, 12.0, 6.6 Hz, 1H), 2.41 (m, 1H), 2.22 (s, 3H), 2.15-1.94 (m, 5H), 1.63 (m, 1H), 1.04 (d, J = 6.9 Hz, 6H).

Example 104

1-(2,6-dimethylphenyl)-5-(2-ethoxycarbonylmethoxybenzyl)piperidin-2-one

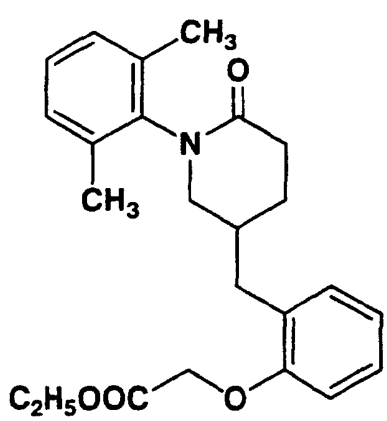

By the same procedure as described in Example 103 using ethyl bromoacetate instead of 1-iodo-2-methylpropane, the compound of the present invention having the following physical data was obtained.

TLC:Rf 0.35 (hexane:ethyl acetate=2:3);
NMR ($CDCl_3$):d 1.27 (t, J=7.14 Hz, 3 H) 1.71 (m, 1 H) 2.01 (m, 1 H) 2.12 (s, 3 H) 2.22 (s, 3 H) 2.49 (m, 2 H) 2.66 (m, 1 H) 2.77 (d, J=7.14 Hz, 2 H) 3.26 (m, 2 H) 4.24 (q, J=7.14 Hz, 2 H) 4.63 (s, 2 H) 6.73 (m, 1 H) 6.93 (m, 1 H) 7.12 (m, 5 H).

Example 105

1-(2,6-dimethylphenyl)-5-(2-carboxymethoxybenzyl)piperidin-2-one

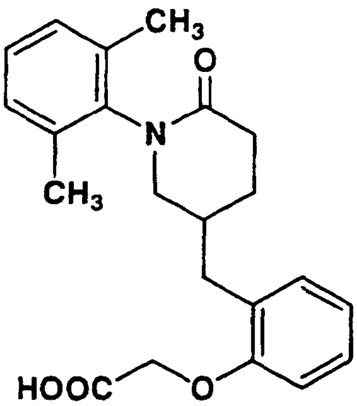

By the same procedure as described in Example 36 using the compound prepared in Example 104, the compound of the present invention having the following physical data was obtained.
TLC:Rf 0.24 (chloroform:methanol=9: 1);
NMR ($CDCl_3$):d 1.67 (m, 1 H) 2.01 (m, 1 H) 2.11 (s, 3 H) 2.22 (s, 3 H) 2.56 (m, 2 H) 2.74 (m, 3 H) 3.26 (m, 2 H) 4.65 (m, 2 H) 6.74 (m, 1 H) 6.91 (m, 1 H) 7.12 (m, 5 H).

Example 106

1-(2,6-dimethylphenyl)-5-(2-(1-methyl-3-t-butylpyrazol-5-yl)aminocarbonylmethyloxyphenylmethyl)piperidin-2-one

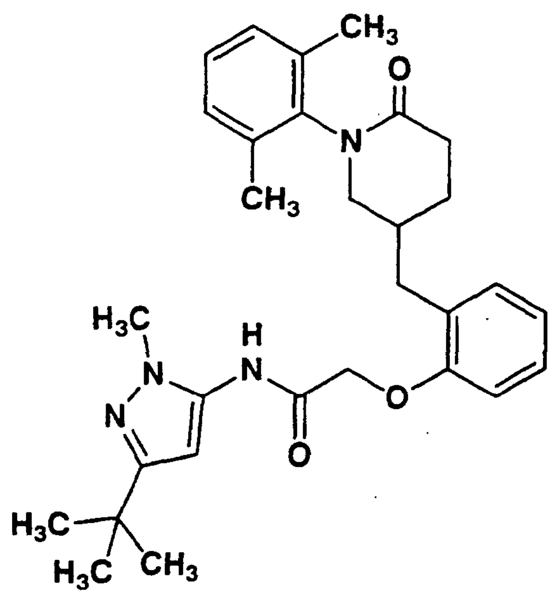

By the same procedure as described in Example 37 using the compound prepared in Example 105 and 5-amino-1-methyl-3-t-butylpyrazole, the compound of the present invention having the following physical data was obtained.
TLC:Rf 0.28 (ethyl acetate);
NMR ($CDCl_3$):d 1.29 (s, 9 H) 1.70 (m, 1 H) 2.04 (m, 4 H) 2.20 (s, 3 H) 2.45 (m, 2 H) 2.68 (m, 1 H) 2.78 (d, J=7.42 Hz, 2 H) 3.27 (m, 2 H) 3.61 (s, 3 H) 4.69 (s, 2 H) 6.14 (s, 1 H) 6.88 (d, J=8.24 Hz, 1 H) 7.07 (m, 4 H) 7.23 (m, 2 H) 7.92 (s, 1 H).

Example 107

1-(4-methoxycarbonyl-2,6-dimethylphenyl)-5-(2,4-difluorobenzyl)piperidin-2-one

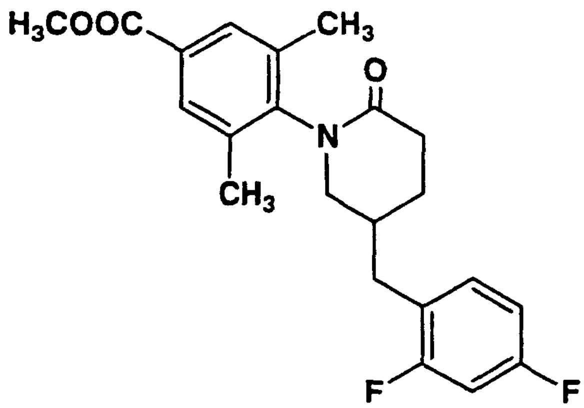

To a solution of the compound prepared in Example 16(1) (0.93 g) in dimethylformamide (4.6 ml) were added methanol (4.6 ml), triethylamine (0.96 ml), 1,1'-bis(diphenylphosphono)ferracene (126 mg) and palladium acetate (51 mg). Under an atmosphere of carbon monoxide, the reaction mixture was stirred 70°C for 2.5 hours. The reaction mixture was diluted with ethyl acetate and filtrated through Celite (proprietary name). Water was added to the filtrate, which was extracted with t-butyl methyl ether. The extract was washed with brine, dried over anhydrous magnesium sulfate and concentrated. The obtained residue was purified by column chromatography on silica gel (hexane:ethyl acetate=1:1→0:1) to give the compound of the present invention (670 mg) having the following physical data.
TLC:Rf 0.23 (ethyl acetate:hexane=2:1);
NMR ($CDCl_3$):d 7.80-7.73 (m, 2H), 7.17-7.07 (m, 1H), 6.87-6.73 (m, 2H), 3.89 (s, 3H), 3.27-3.12 (m, 2H), 2.74-2.62 (m, 3H), 2.60-2.45 (m, 1H), 2.40-2.22 (m, 4H), 2.16 (s, 3H), 2.07-1.95 (m, 1H), 1.78-1.61 (m, 1H).

Example 108

1-(4-carboxyl-2,6-dimethylphenyl)-5-(2,4-difluorobenzyl)piperidin-2-one

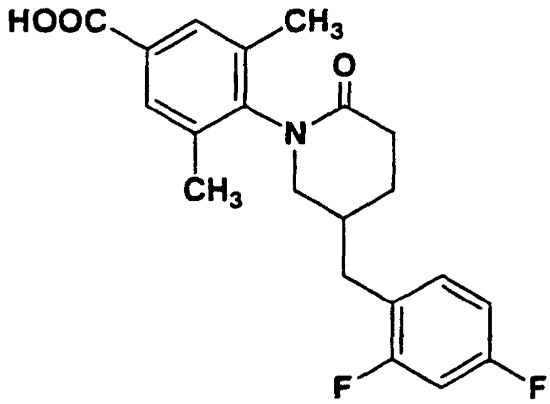

By the same procedure as described in Example 36 using the compound prepared in Example 107, the compound of the present invention having the following physical data was obtained.
TLC:Rf 0.40 (ethyl acetate: methanol =5: 1);
NMR (DMSO-$d_6$):d 13.00-12.70 (m, 1H), 7.66 (s, 2H), 7.43-7.33 (m, 1H), 7.23-7.13 (m, 1H), 7.07-6.95 (m, 1H), 3.30-3.05 (m, 2H), 2.76-2.65 (m, 2H), 2.47-2.35 (m, 2H), 2.30-2.20 (m, 1H), 2.16 and 2.07 (each s, each 3H), 1.90-1.79 (m, 1H), 1.74-1.55 (m, 1H).

Example 109

1-(4-(2-(N,N-dimethylamino)ethyl)aminocarbonyl-2,6-dimethylphenyl)-5-(2,4-difluorobenzyl)piperidin-2-one

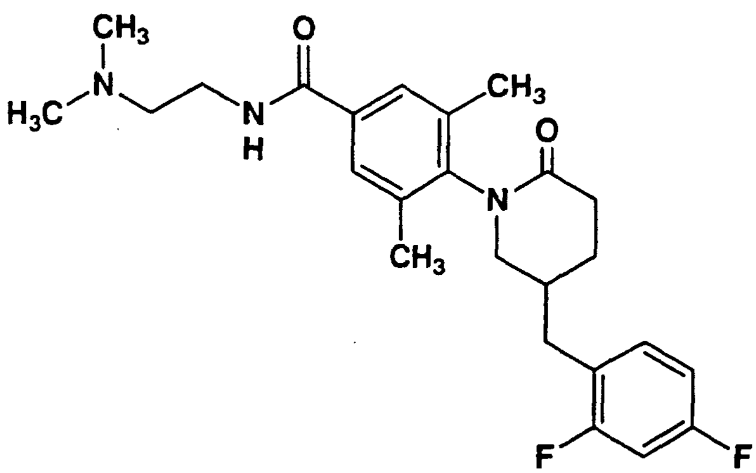

To a solution of the compound prepared in Example 108 (60 mg) in toluene (1 ml) were added oxalyl chloride (15 µl) and dimethylformamide (2 drops). The reaction mixture was stirred at room temperature for 1.5 hours. The obtained solution was added to a solution of 2-(N,N-dimethylamine)ethylamine (21 µl), dimethylformamide (1 ml) and triethylamine (68 µl) at room temperature. The reaction mixture was stirred at room temperature overnight. The reaction mixture was poured in water and extracted with ethyl acetate. The extract was washed with brine, dried over anhydrous

magnesium sulfate and concentrated. The obtained residue was washed t-butyl methyl ether/hexane to give the compound of the present invention (21 mg) having the following physical data.
TLC:Rf 0.49 (ethyl acetate:methanol:triethylamine=10:9:1);
NMR ($CDCl_3$):d 7.48 (s, 2H), 7.18-7.07 (m, 1H), 6.88-6.67 (m, 3H), 3.49 (brq, J = 5.7 Hz, 2H), 3.29-3.12 (m, 2H), 2.77-2.62 (m, 3H), 2.60-2.45 (m, 4H), 2.26 (s, 9H), 2.16 (s, 3H), 2.08-1.96 (m, 1H), 1.75-1.67 (m, 1H).

Examples 109(1)~109(7)

By the same procedure as described in Example 109 using the corresponding amine derivatives instead of 2-(N,N-dimethylamine)ethylamine, the following compound of the present invention were obtained.

Example 109(1)

1-(4-(3-(N,N-dimethylamino)propylaminocarbonyl)-2,6-dimethylphenyl)-5-(2,4-difluorobenzyl)piperidin-2-one

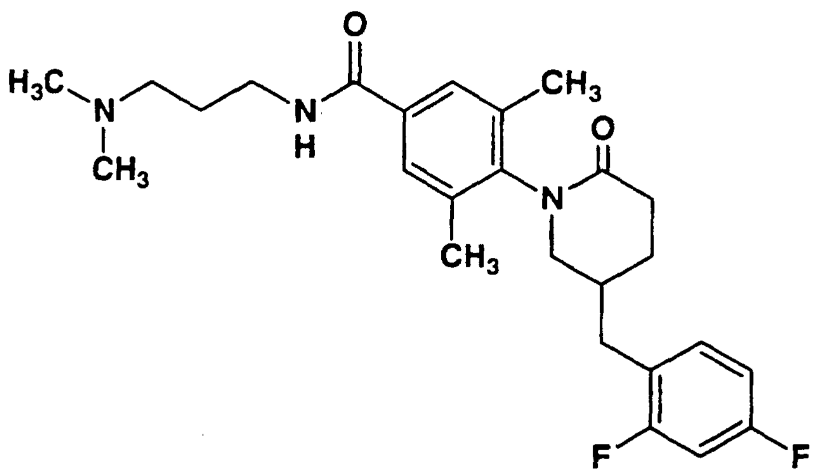

TLC:Rf 0.32 (ethyl acetate:methanol:triethylamine=10:9:1);
NMR ($CDCl_3$):d 1.67 (m, 1 H) 1.81 (m, 2 H) 2.00 (m, 1 H) 2.15 (s, 3 H) 2.25 (s, 3 H) 2.35 (m, 7 H) 2.53 (m, 3 H) 2.69 (m, 3 H) 3.20 (m, 2 H) 3.53 (m, 2 H) 6.80 (m, 2 H) 7.11 ( m, 1 H) 7.51 (m, 2 H) 8.29 (m, 1 H).

Example 109(2)

1-(4-methylaminocarbonyl-2,6-dimethylphenyl)-5-(2,4-difluorobenzyl)piperidin-2-one

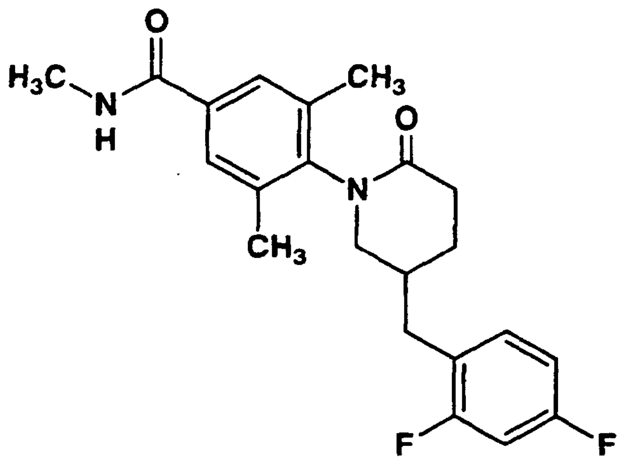

TLC:Rf 0.61 (ethyl acetate: methanol:triethylamine=7:2: 1);
NMR ($CDCl_3$):d 1.70 (m, 1 H) 2.01 (m, 1 H) 2.14 (s, 3 H) 2.23 (s, 3 H) 2.31 (m, 1 H) 2.52 (m, 1 H) 2.68 (m, 3 H) 2.97 (m, 3 H) 3.20 (m, 2 H) 6.13 (m, 1 H) 6.82 (m, 2 H) 7.12 ( m, 1 H) 7.44 (s, 2 H).

Example 109(3)

1-(4-(N,N-dimethylamino)carbonyl-2,6-dimethylphenyl)-5-(2,4-difluorobenzyl)piperidin-2-one

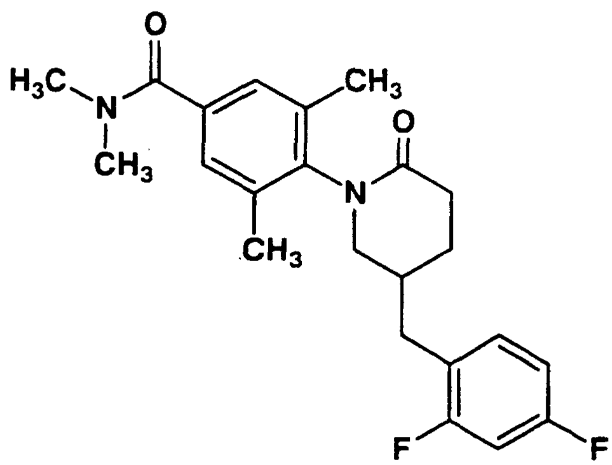

TLC:Rf 0.25 (ethyl acetate: triethylamine=9:1);
NMR ($CDCl_3$):d 1.69 (m, 1 H) 2.01 (m, 1 H) 2.11 (s, 3 H) 2.21 (s, 3 H) 2.29 (m, 1 H) 2.52 (m, 1 H) 2.69 (m, 3 H) 3.07 (m, 8 H) 6.82 (m, 2 H) 7.11 (m, 3 H).

Example 109(4)

1-(4-(rnorpholin-4-yl)carbonyl-2,6-dimethylphenyl)-5-(2,4-difluorobenzyl)piperidin-2-one

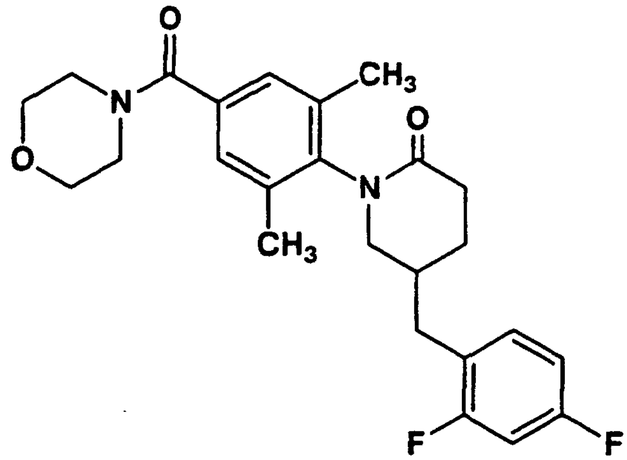

TLC:Rf 0.28 (ethyl acetate: triethylamine=9:1);
NMR ($CDCl_3$):d 1.69 (m, 1 H) 2.01 (m, 1 H) 2.12 (s, 3 H) 2.22 (s, 3 H) 2.31 (m, 1 H) 2.52 (m, 1 H) 2.69 (m, 3 H) 3.16 (m, 2 H) 3.63 (m, 8 H) 6.81 (m, 2 H) 7.11 (m, 3 H).

Example 109(5)

1-(4-(4-methylpiperazin-1-yl)carbonyl-2,6-dimethylphenyl)-5-(2,4-difluorobenzyl)piperidin-2-one hydrochloride

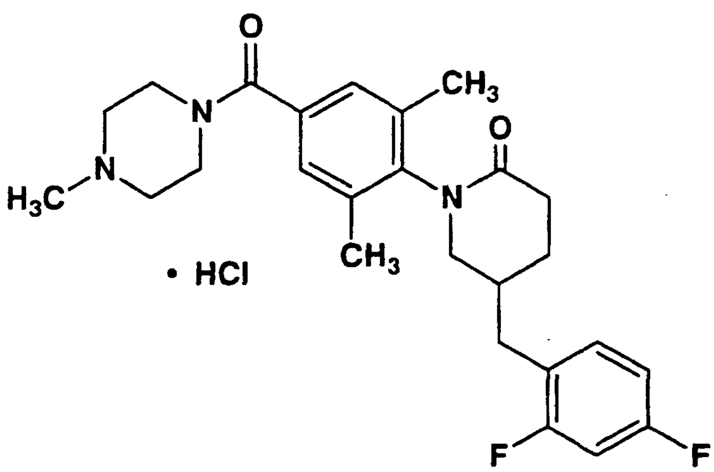

TLC:Rf 0.51 (ethyl acetate:methanol:triethylamine=7:2:1);
NMR (DMSO-$d_6$):d 1.64 (m, 1 H) 1.84 (m, 1 H) 2.06 (s, 3 H) 2.15 (s, 3 H) 2.28 (m, 1 H) 2.41 (m, 4 H) 2.73 (m, 7 H) 3.06 (m, 4 H) 3.25 (m, 2 H) 7.03 (m, 1 H) 7.19 (m, 3 H) 7.40 (m, 1 H) 10.97 (m, 1 H).

Example 109(6)

1-(4-(3-ethoxycarbonylpropyl)aminocarbonyl-2,6-dimethylphenyl)-5-(2,4-difluorobenzyl)piperidin-2-one

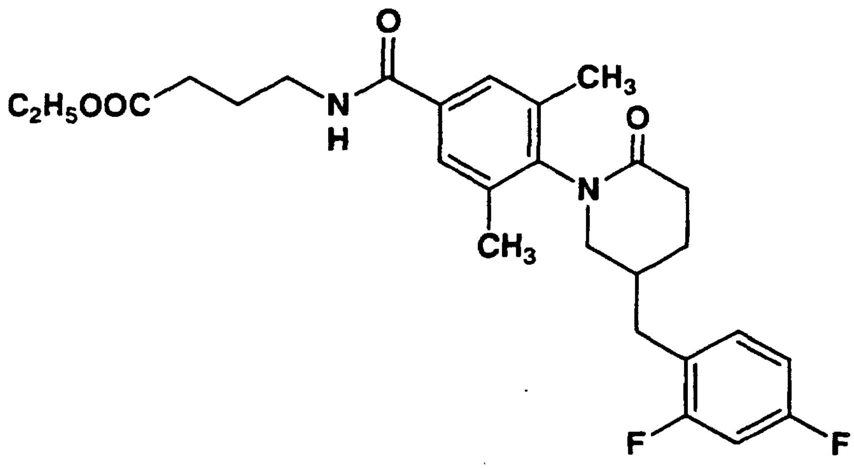

TLC:Rf 0.47 (ethyl acetate:methanol=5:1);
NMR ($CDCl_3$):d 7.48 (brs, 2H), 7.19-7.06 (m, 1H), 6.88-6.73 (m, 2H), 6.50-6.40 (m, 1H), 4.14 (q, J = 7.2 Hz, 2H), 3.48 (brq, J = 6.6Hz, 2H), 3.28-3.11 (m, 2H), 2.74-2.62 (m, 3H), 2.59-2.46 (m, 1H), 2.42 (t, J = 7.2 Hz, 2H), 2.36-2.21 (m, 4H), 2.15 (s, 3H), 2.06-1.87 (m, 3H), 1.75-1.65 (m, 1H), 1.25 (t, J = 7.2 Hz, 3H).

Example 109(7)

1-(4-(2-ethoxycarbonylethyl)aminocarbonyl-2,6-dimethylphenyl)-5-(2,4-difluorobenzyl)piperidin-2-one

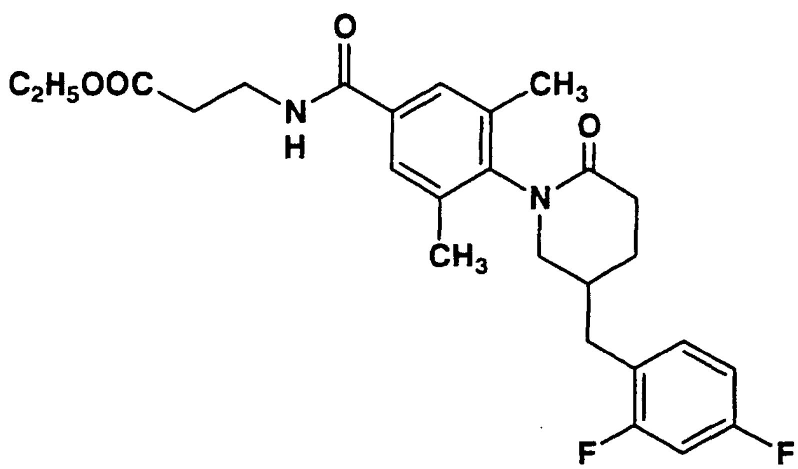

TLC:Rf 0.52 (ethyl acetate:methanol=5:1);
NMR ($CDCl_3$):d 7.47 (brs, 2H), 7.18-7.07 (m, 1H), 6.90-6.68 (m, 3H), 4.17 (q, J = 7.2 Hz, 2H), 3.70 (brq, J = 6.0 Hz, 2H), 3.29-3.10 (m, 2H), 2.75-2.45 (m, 6H), 2.39-2.22 (m, 4H), 2.15 (s, 3H), 2.07-1.94 (m, 1H), 1.76-1.65 (m, 1H), 1.28 (t, J = 7.2 Hz, 3H).

Examples 110(1) and 110(2)

By the same procedure as described in Example 36 using the compounds prepared in Example 109(6) or Example 109(7), the following compound of the present invention were obtained.

Example 110(1)

1-(4-(3-carboxypropyl)aminocarbonyl-2,6-dimethylphenyl)-5-(2,4-difluorobenzyl)piperidin-2-one

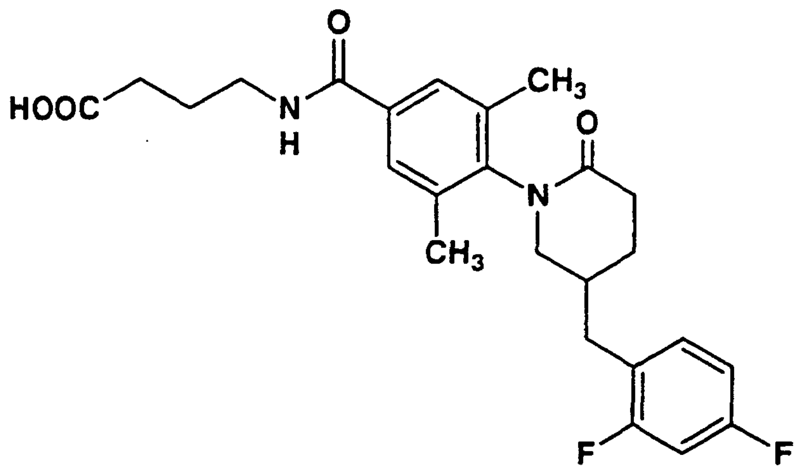

TLC:Rf 0.37 (ethyl acetate:methanol:water=7:2:1);
NMR ($CDCl_3$):d 7.49 (s, 2H), 7.19-7.08 (m, 1H), 7.07-6.93 (m, 1H), 6.89-6.65 (m, 2H), 3.47-3.32 (m, 2H), 3.28-3.12 (m, 2H), 2.75-2.63 (m, 3H), 2.60-2.45 (m, 1H), 2.40-2.26 (m, 3H), 2.21 and 2.17 (each s, each 3H), 2.08-1.94 (m, 1H), 1.92-1.79 (m, 2H), 1.75-1.59 (m, 1H).

Example 110(2)

1-(4-(2-carboxyethyl)aminocarbonyl-2,b-dimethylphenyl)-5-(2,4-difluorobenzyl)piperidin-2-one

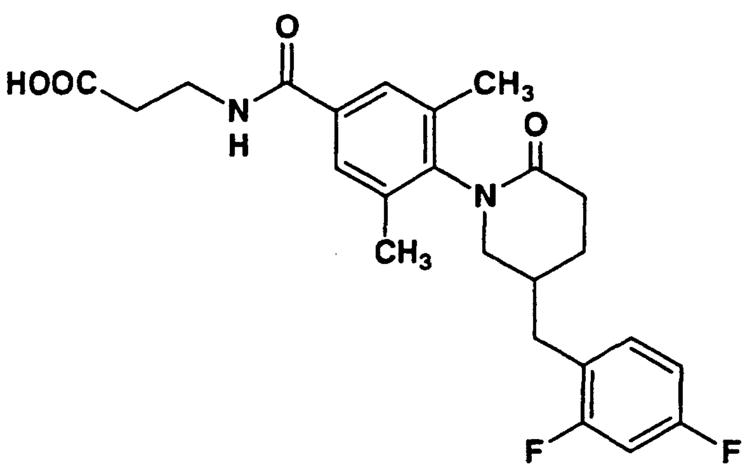

TLC:Rf 0.29 (ethyl acetate:methanol:water=7:2:1);

NMR ($CDCl_3$):d 7.45 (s, 2H), 7.18-7.07 (m, 1H), 6.90-6.73 (m, 3H), 3.68-3.55 (m, 2H), 3.27-3.12 (m, 2H), 2.76-2.65 (m, 3H), 2.62-2.46 (m, 3H), 2.40-2.26 (m, 1H), 2.23 and 2.14 (each s, each 3H), 2.07-1.93 (m, 1H), 1.75-1.63 (m, 1H).

Example 111

1-(4-(t-butoxycarbonyl)amino-2,6-dimethylphenyl)-5-(2,4-difluorobenzyl)piperidin-2-one

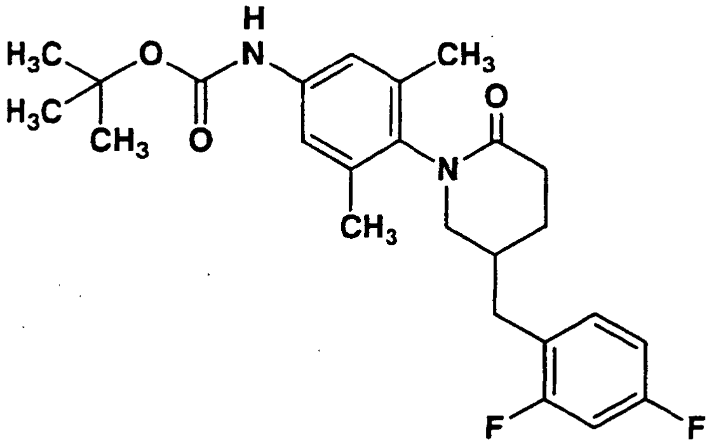

To a solution the compound prepared in Example 108 (500 mg) in toluene (5 ml) were added butanol (5ml), triethylamine (0.23 ml) and diphenylphosphorylazide (0.35 ml). The reaction mixture was stirred at 90°C for 3 hours. To the reaction mixture was added ethyl bromoacetate (0.14 ml). The reaction mixture was stirred at room temperature. The reaction mixture was concentrated. The obtained residue was purified by column chromatography on silica gel (hexane:ethyl acetate=3:1→1:3) to give the compound of the present invention (486 mg) having the following physical data.
TLC:Rf 0.27 (ethyl acetate:hexane=3:1);
NMR ($CDCl_3$):d 7.16-7.05 (m, 3H), 6.87-6.73 (m, 2H), 6.55 (s, 1H), 3.26 -3.10 (m, 2H), 2.74-2.60 (m, 3H), 2.53-2.45 (m, 1H), 2.33-2.21 (m, 1H), 2.16 (s, 3H), 2.06 (s, 3H), 2.05-1.92 (m, 1H), 1.73-1.60 (m, 1H), 1.50 (s, 9H).

Example 112

1-(4-amino-2,6-dimethylphenyl)-5-(2,4-difluorobenzyl)piperidin-2-one

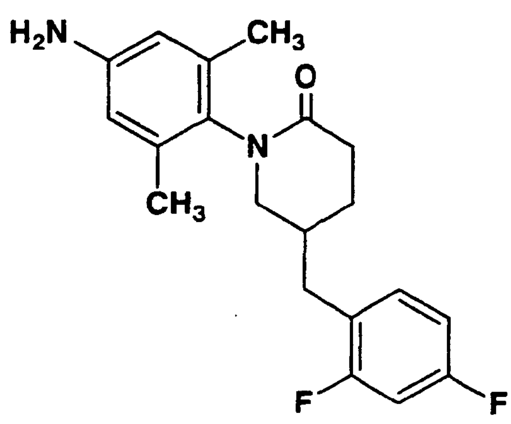

By the same procedure as described in Reference Example 31 using the compounds prepared in Example

111, the compound of the present invention having the following physical data was obtained.
TLC:Rf 0.42 (ethyl acetate:methanol=5:1);
NMR ($CDCl_3$):d 1.64 (m, 1 H) 1.94 (m, 1 H) 2.01 (s, 3 H) 2.11 (s, 3 H) 2.28 (m, 1 H) 2.49 (m, 1 H) 2.67 (m, 3 H) 3.19 (m, 2 H) 3.55 (m, 2 H) 6.40 (s, 2 H) 6.83 (m, 2 H) 7.12 (m, 1H).

Example 112(1)

1-(4-aminomethyl-2,6-dimethylphenyl)-5-(2,4-difluorobenzyl)piperidin-2-one

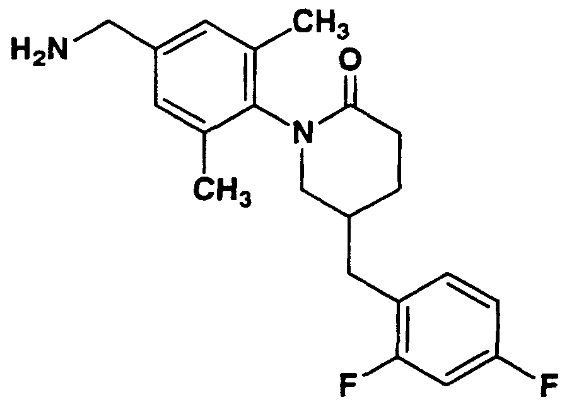

By the same procedure as described in Reference Example 3→Example 73 using the compounds prepared in Example 107, the compound of the present invention having the following physical data was obtained.
TLC:Rf 0.34 (ethyl acetate:methanol:triethylamine=7:2:1);
NMR ($CDCl_3$):d 1.67 (m, 1 H) 1.87 (m, 2 H) 2.00 (m, 1 H) 2.11 (s, 3 H) 2.20 (s, 3 H) 2.32 (m, 1H) 2.51 (m, 1H) 2.68 (m, 3 H) 3.21 (m, 2 H) 3.79 (s, 2 H) 6.81 (m, 2 H) 7.05 ( m, 2 H) 7.13 (m, 1 H).

Example 113

1-(4-(4-(tetrahydropyran-2-yloxy)but-1-ynyl)-2,6-dimethylphenyl)-5-(2,4-difluorobenzyl)piperidin-2-one

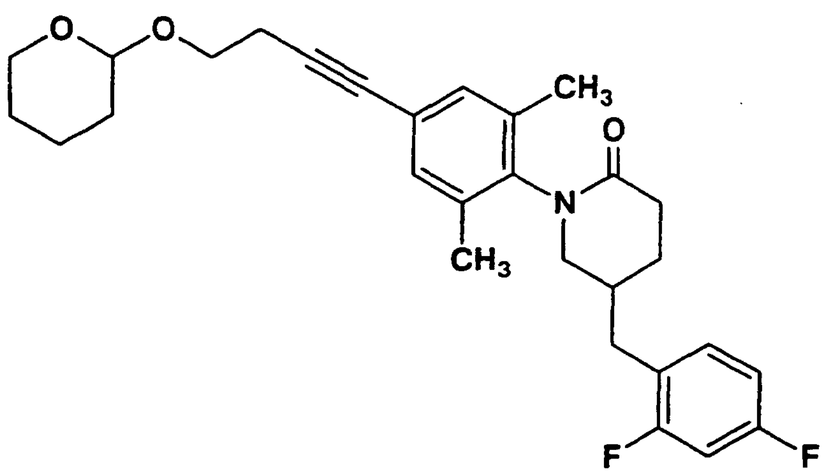

To a solution of the compound prepared in Example 16(1) (5 g) in dimethylformamide (36 ml) were added 3-butynyloxytetrahydro-2-pyran (2.83 g) and triethylamine (12 ml) under an atmosphere of argon. To the mixture was

added bis(triphenylphosphine)palladium(II) chloride (0.86 g). The reaction mixture was stirred at 90°C for 1 hour. The reaction mixture was poured in ice water and extracted with t-butyl methyl ether. The extract was washed with water and brine, dried over anhydrous sodium sulfate and concentrated. The obtained residue was purified by column chromatography on silica gel (ethyl acetate:hexane=1:2→3:1) to give the compound of the present invention (5.09 g) having the following physical data.
TLC:Rf 0.24 (ethyl acetate);
NMR ($CDCl_3$):d 7.16-7.07 (m, 3H), 6.87-6.76 (m, 2H), 4.70-4.57 (m, 1H), 3.96-3.80 (m, 2H), 3.67-3.40 (m, 2H), 3.30-3.13 (m, 2H), 2.75-2.60 (m, 5H), 2.58-2.40 (m, 1H), 2.36-2.23 (m, 1H), 2.18 and 2.16 (each s, totally 3H), 2.08 and 2.07 (each s, totally 3H), 2.02-1.94 (m, 1H), 1.89-1.77 (m, 2H), 1.74-1.46 (m, 5H).

Example 114

1-(4-(4-hydroxybut-1-ynyl)-2,6-dimethylphenyl)-5-(2,4-difluorobenzyl)piperidin-2-one

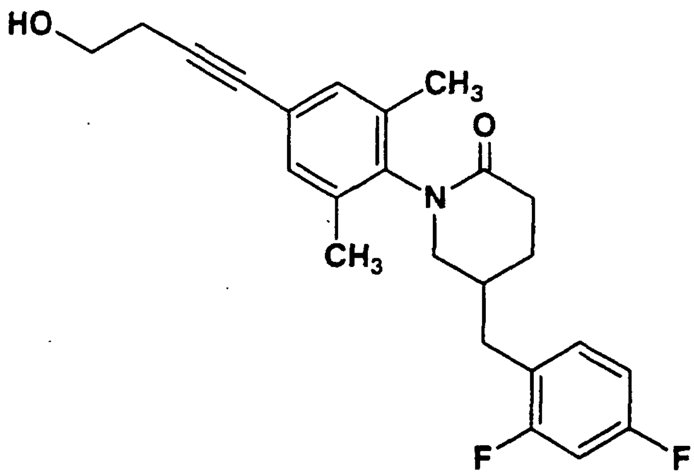

To a solution of the compound prepared in Example 113 (5.09 g) in methanol (50 ml) was added 2N hydrochloric acid (11 ml). The reaction mixture was stirred at room temperature for 10 minutes. The reaction mixture was poured in water and extracted with t-butyl methyl ether. The extract was washed with water and brine, dried over anhydrous sodium sulfate and concentrated. The obtained residue was purified by column chromatography on silica gel (hexane:ethyl acetate=2:3→methanol:ethyl acetate=1:3) to give the compound of the present invention (0.94 g) having the following physical data.
TLC:Rf 0.29 (ethyl acetate);
NMR ($CDCl_3$):d 1.69 (m, 1 H) 1.87 (m, 1 H) 1.98 (m, 1 H) 2.08 (s, 3 H) 2.17 (s, 3 H) 2.29 (m, 1 H) 2.50 (m, 1 H) 2.66 (m, 5 H) 3.20 (m, 2 H) 3.77 (m, 2 H) 6.81 (m, 2 H) 7.12 ( m, 3H).

Example 115

1-(4-(4-(N,N-dimethylamino)but-1-ynyl))-2,6-dimethylphenyl)-5-(2,4-difluorobenzyl)piperidin-2-one

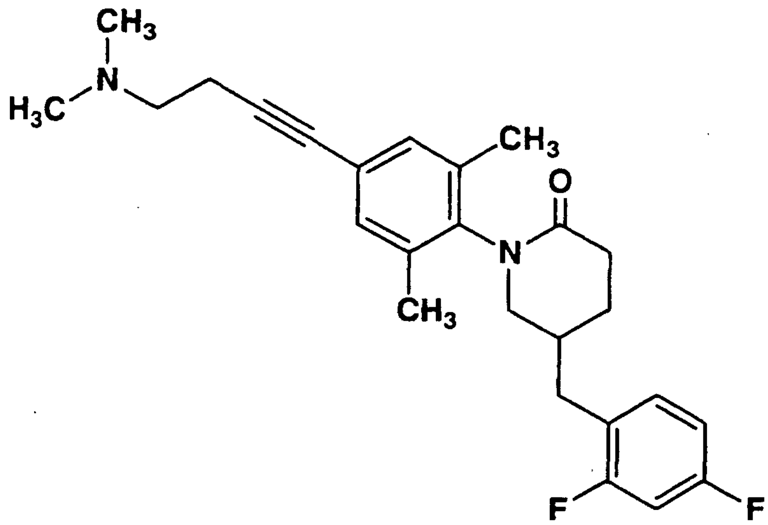

By the same procedure as described in Example 70 using the compounds prepared in Example 114 and dimethylamine, the compound of the present invention having the following physical data was obtained.
TLC:Rf 0.53 (ethyl acetate:methanol:triethylamine=7:2:1);
NMR ($CDCl_3$):d 1.65 (m, 1 H) 1.97 (m, 1 H) 2.07 (s, 3 H) 2.16 (s, 3 H) 2.29 (m, 7 H) 2.51 (m, 5 H) 2.66 (m, 3 H) 3.18 (m, 2 H) 6.81 (m, 2 H) 7.11 (m, 3 H).

Examples 115(1) and 115(2)

By the same procedure as described in Example 115 using the corresponding amine derivatives instead of dimethylamine, the following compounds of the present invention were obtained.

Example 115(1)

1-(4-(4-morpholinobut-1-ynyl)-2,6-dimethylphenyl)-5-(2,4-difluorobenzyl)piperidin-2-one

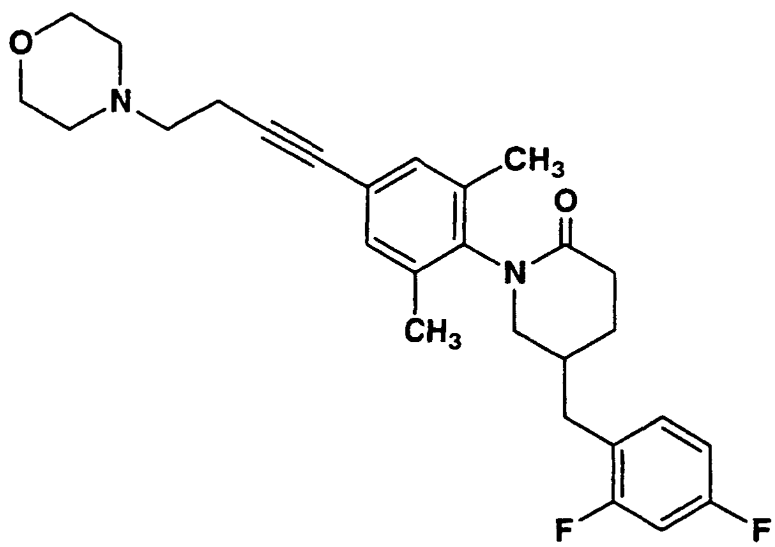

TLC:Rf 0.62 (ethyl acetate:methanol:triethylamine=7:2:1);
NMR ($CDCl_3$):d 1.67 (m, 1 H) 1.98 (m, 1 H) 2.07 (s, 3 H) 2.16 (s, 3 H) 2.29 (m, 1 H) 2.57 (m, 12 H) 3.19 (m, 2 H) 3.72 (m, 4 H) 6.82 (m, 2 H) 7.11 (m, 3 H).

Example 115(2)

1-(4-(4-(4-methylpiperazin-1-yl)but-1-ynyl)-2,6-dimethylphenyl)-5-(2,4-difluorobenzyl)piperidin-2-one

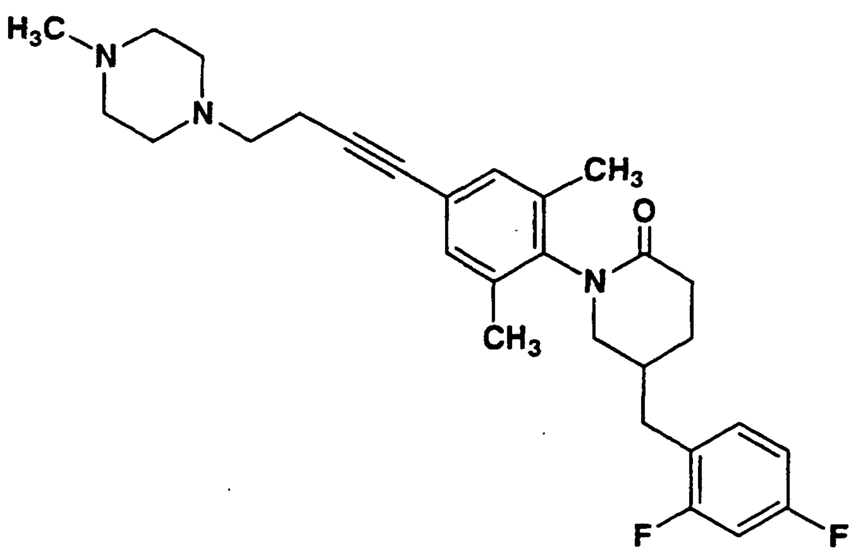

TLC:Rf 0.40 (ethyl acetate:methanol:triethylamine=7:2: 1);
NMR ($CDCl_3$):d 1.66 (m, 1 H) 1.98 (m, 1 H) 2.06 (s, 3 H) 2.16 (s, 3 H) 2.30 (m, 4 H) 2.55 (m, 16 H) 3.19 (m, 2 H) 6.81

(m, 2 H) 7.13 (m, 3 H).

Example 116

1-(4-(4-(N,N-dimethylamino)butyl)-2,6-dimethylphenyl)-5-(2,4-difluorobenzyl)piperidin-2-one

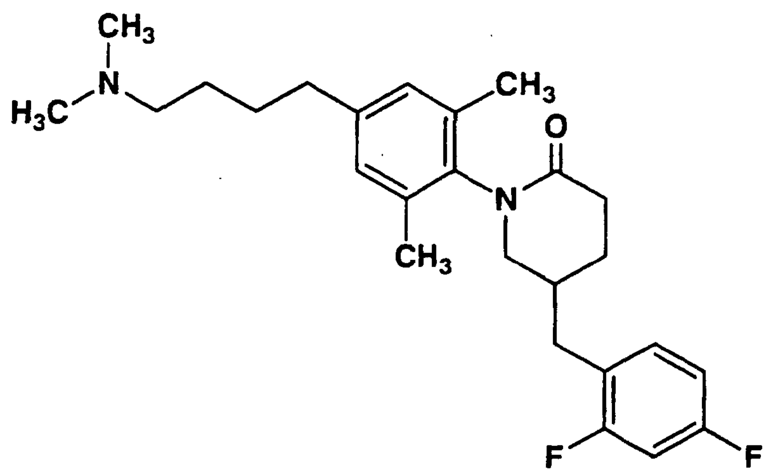

By the same procedure as described in Reference Example 15 using the compounds prepared in Example 115, the compound of the present invention having the following physical data was obtained.
TLC:Rf 0.44 (ethyl acetate:methanol:triethylamine=7:2:1);
NMR ($CDCl_3$):d 1.58 (m, 5 H) 1.98 (m, 1 H) 2.07 (s, 3 H) 2.17 (s, 3 H) 2.21 (s, 6 H) 2.30 (m, 3 H) 2.50 (m, 3 H) 2.69 (m, 3 H) 3.20 (m, 2 H) 6.80 (m, 2 H) 6.89 (s, 2 H) 7.13 (m, 1 H).

Example 117

(E)-1-(4-(2-(2-(N,N-dimethylamino)ethyl)vinyl)-2,6-dimethylphenyl)-5-(2,4-difluorobenzyl)piperidin-2-one

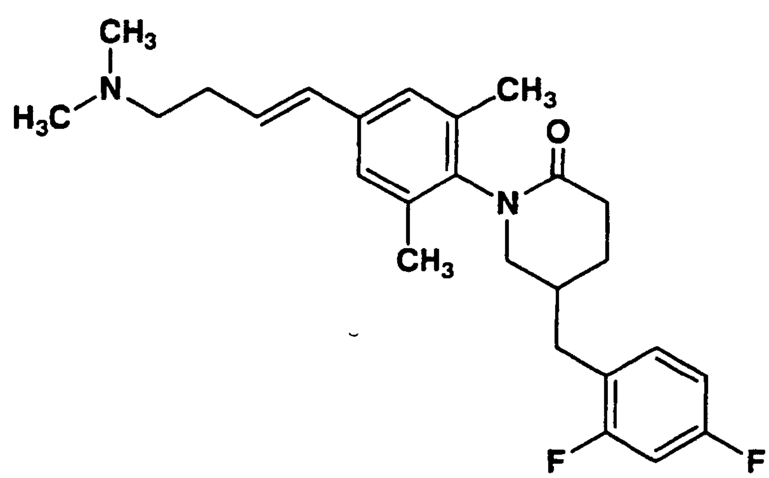

To a solution of the compound prepared in Example 115 (74 mg) in tetrahydrofuran (3.4 ml) were added quinoline (11 mg) and palladium-barium sulfate (22 mg) under an atmosphere of argon. Under an atmosphere of hydrogen, the reaction mixture was stirred at room temperature for 4 hours. The reaction mixture was filtrated through Celite (proprietary name) and concentrated. The obtained residue was purified by column chromatography on silica

gel (ethyl acetate:methanol:triethylamine=1:0:0→20:1:1) to give the compound of the present invention (57 mg) having the following physical data.
TLC:Rf 0.51 (ethyl acetate:methanol:triethylamine=7:2:1);
NMR ($CDCl_3$):d 1.69 (m, 1 H) 2.00 (m, 1 H) 2.10 (s, 3 H) 2.20 (s, 3 H) 2.24 (s, 6 H) 2.29 (m, 1 H) 2.39 (m, 2 H) 2.52 (m, 3 H) 2.67 (m, 3 H) 3.21 (m, 2 H) 5.63 (m, 1 H) 6.36 ( m, 1 H) 6.82 (m, 2 H) 7.01 (s, 2 H) 7.13 (m, 1 H).

Example 118

1-(4-(5-hydroxypent-1-ynyl)-2,6-dimethylphenyl)-5-(2,4-difluorobenzyl)piperidin-2-one

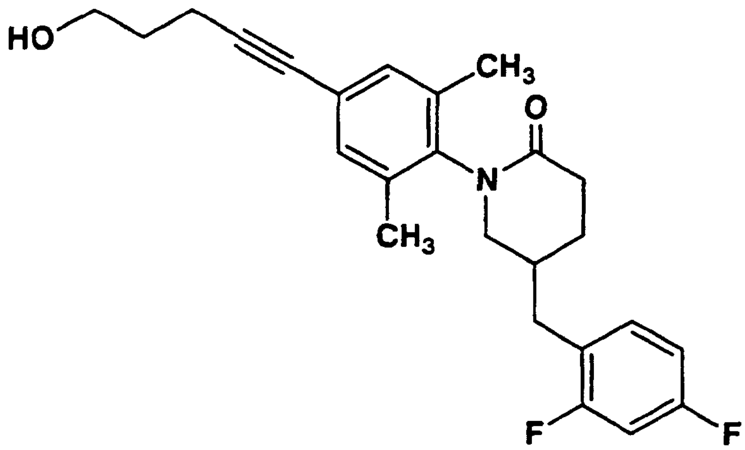

To a solution of the compound prepared in Example 16(1) (408 mg) in dimethylformamide (2.5 ml) were added triethylamine (2.5 ml) and 4-pentyn-1-ol (168 mg). Under an atmosphere of argon, to the mixture were added tetrakis (triphenylphosphine)palladium(0) (46 mg) and copper iodide (23 mg). The reaction mixture was stirred at 80°C for 2 hours. The reaction mixture was diluted with ethyl acetate, washed 1N hydrochloric acid, a saturated anhydrous sodium hydrogen carbonate solution and brine, dried over anhydrous sodium sulfate and concentrated. The obtained residue was purified by column chromatography on silica gel (hexane:ethyl acetate=3:7→1:9) to give the compound of the present invention (276 mg) having the following physical data.
TLC:Rf 0.28(hexane:ethyl acetate=1:4);
NMR ($CDCl_3$):d 1.51 (m, 1 H) 1.68 (m, 1 H) 1.83 (m, 2 H) 2.00 (m, 1 H) 2.07 (s, 3 H) 2.16 (s, 3 H) 2.29 (m, 1 H) 2.50 (m, 3 H) 2.66 (m, 3 H) 3.18 (m, 2 H) 3.80 (m, 2 H) 6.81 (m, 2 H) 7.14 (m, 3 H).

Example 119

1-(4-(5-hydroxypentyl)-2,6-dimethylphenyl)-5-(2,4-difluorobenzyl)piperidin-2-one

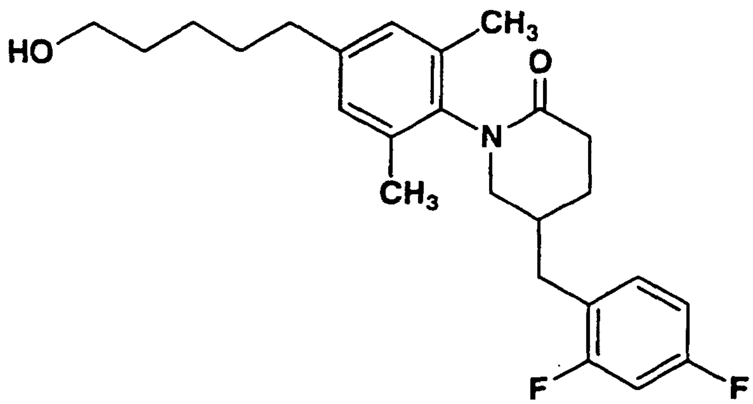

By the same procedure as described in Reference Example 15 using the compounds prepared in Example 118, the compound of the present invention having the following physical data was obtained.
TLC:Rf 0.28 (ethyl acetate);
NMR ($CDCl_3$):d 1.41 (m, 2 H) 1.65 (m, 6 H) 1.99 (m, 1 H) 2.08 (s, 3 H) 2.17 (s, 3 H) 2.29 (m, 1 H) 2.50 (m, 3 H) 2.67 (m, 3 H) 3.21 (m, 2 H) 3.64 (q, J=6.23 Hz, 2 H) 6.81 (m, 2 H) 6.89 (s, 2 H) 7.12 (m, 1 H).

Example 120

1-(4-(4-formylbutyl)-2,6-dimethylphenyl)-5-(2,4-difluorobenzyl)piperidin-2-one

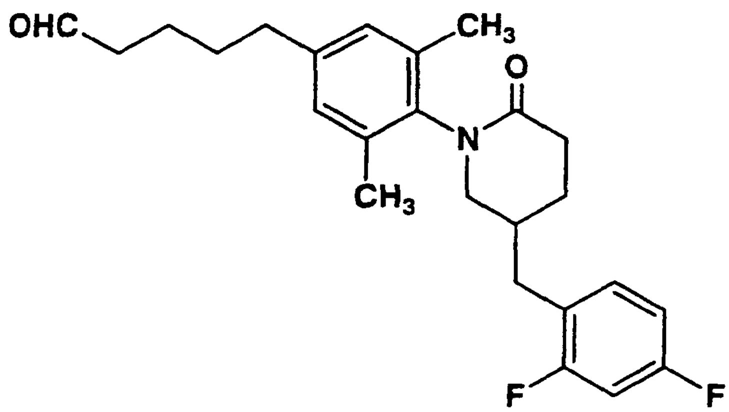

By the same procedure as described in Example 98 using the compounds prepared in Example 119, the compound of the present invention having the following physical data was obtained.
TLC:Rf 0.52 (ethyl acetate);
NMR ($CDCl_3$):d 1.68 (m, 5 H) 2.00 (m, 1 H) 2.08 (s, 3 H) 2.17 (s, 3 H) 2.30 (m, 1 H) 2.45 (m, 2 H) 2.53 (m, 3 H) 2.67 (m, 3 H) 3.23 (m, 2 H) 6.81 (m, 2 H) 6.89 (s, 2 H) 7.13 (m, 1 H) 9.77 (t, J=1.92 Hz, 1 H).

Example 121

1-(4-(4-carboxybutyl)-2,6-dimethylphenyl)-5-(2,4-difluorobenzyl)piperidin-2-one

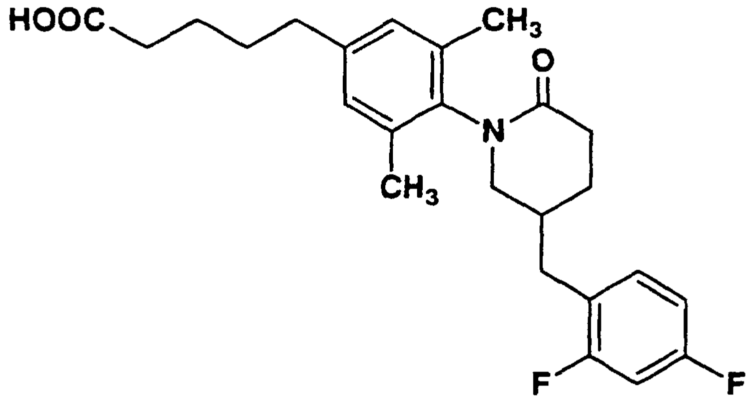

To a solution of the compound prepared in Example 120 (133 mg) in t-butanol (3.0 ml) and water (0.8 ml) were added 2-methyl-2-butene (99 mg), sodium dihydrogen phosphate (38 mg)and 85% sodium chlorite (99 mg). The reaction mixture was stirred at room temperature for 1 hour. The reaction mixture was diluted with ethyl acetate, washed with 1N hydrochloric acid, water and brine, dried over anhydrous sodium sulfate and concentrated. The obtained residue was purified by preparative TLC (ethyl acetate) to give the compound of the present invention (108 mg ) having the following physical data.
TLC:Rf 0.28 (ethyl acetate);
NMR ($CDCl_3$):d 1.66 (m, 5 H) 1.99 (m, 1 H) 2.08 (s, 3 H) 2.17 (s, 3 H) 2.31 (m, 3 H) 2.53 (m, 3 H) 2.66 (m, 3 H) 3.22 (m, 2 H) 6.80 (m, 2 H) 6.88 (s, 2 H) 7.11 (m, 1 H).

Example 122

(E)-1-(4-(2-(methoxycarbonyl)vinyl)-2,6-dimethylphenyl)-5-(2,4-difluorobenzyl)piperidin-2-one

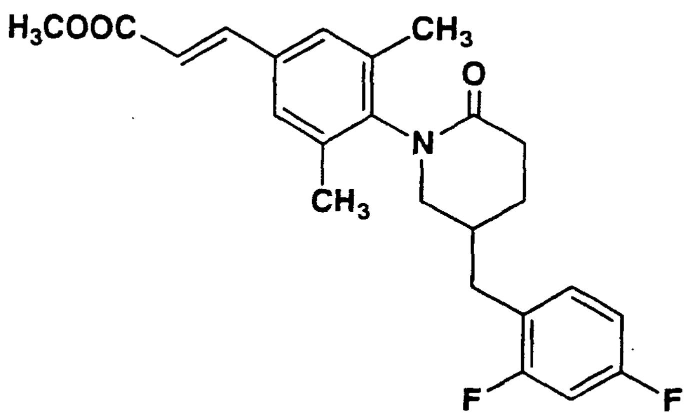

By the same procedure as described in Example 113 using 2-propenoic acid methyl ester instead of 2-(3-butynyloxy)tetrahydro-2H-pyran, the compound of the present invention having the following physical data was obtained.
TLC:Rf 0.27 (ethyl acetate:hexane=2:1);
NMR ($CDCl_3$):d 1.68 (m, 1 H) 2.01 (m, 1 H) 2.13 (s, 3 H) 2.23 (s, 3 H) 2.32 (m, 1 H) 2.51 (m, 1 H) 2.68 (m, 3 H) 3.21 (m, 2 H) 3.80 (s, 3 H) 6.37 (d, J=15.90 Hz, 1 H) 6.81 (m, 2 H) 7.12 (m, 1H) 7.25 (m, 2 H) 7.60 (d, J=15.90 Hz, 1 H).

Example 123

(E)-1-(4-(2-(carboxyl)vinyl)-2,6-dimethylphenyl)-S-(2,4-difluorobenzyl)piperidin-2-one

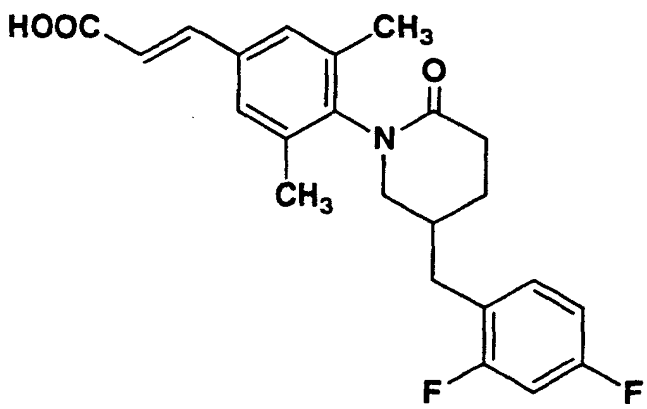

By the same procedure as described in Example 36 using the compound prepared in Example 122, the compound of the present invention having the following physical data was obtained.
TLC:Rf 0.43 (ethyl acetate:methanol=5:1);
NMR (DMSO-$d_6$):d 1.63 (m, 1 H) 1.82 (m, 1 H) 2.03 (s, 3 H) 2.13 (s, 3 H) 2.25 (m, 1 H) 2.42 (m, 2 H) 2.69 (m, 2 H) 3.09 (m, 1 H) 3.23 (m, 1 H) 6.46 (d, J=16.10 Hz, 1 H) 7.03 (m, 1 H) 7.18 (m, 1 H) 7.38 (m, 3 H) 7.48 (d, J=16.10 Hz, 1 H) 12.36 (m, 1 H).

Example 124

1-(4-methoxycarbonyl-2,6-dichlorophenyl)-5-(2,4-difluorobenzyl)piperidin-2-one

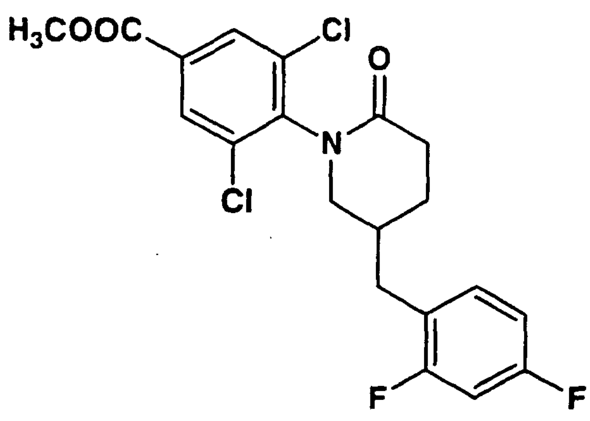

By the same procedure as described in Example 107 using the compound prepared in Example 16(13), the compound of the present invention having the following physical data was obtained.
TLC:Rf 0.34 (ethyl acetate:hexane=1:1);
NMR ($CDCl_3$):d 1.75 (m, 1 H) 2.01 (m, 1 H) 2.38 (m, 1 H) 2.53 (m, 1 H) 2.70 (m, 3 H) 3.35 (m, 2 H) 3.93 (s, 3 H) 6.83 (m, 2 H) 7.15 (m, 1 H) 8.04 (m, 2 H).

Example 125

1-(4-carboxyl-2,6-dichlorophenyl)-5-(2,4-difluorobenzyl)piperidin-2-one

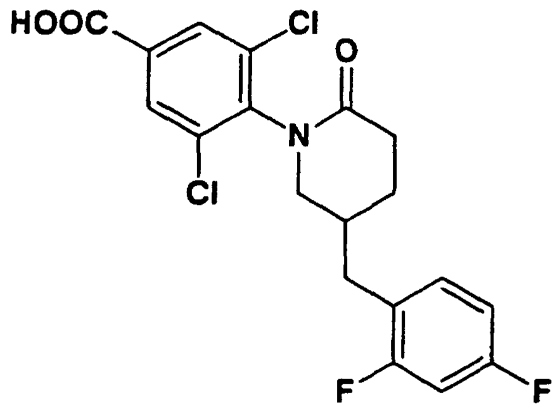

By the same procedure as described in Example 36 using the compound prepared in Example 124, the compound of the present invention having the following physical data was obtained.
TLC:Rf 0.33 (ethyl acetate:methanol=4:1);
NMR (DMSO-$d_6$):d 1.63 (m, 1 H) 1.87 (m, 1 H) 2.30 (m, 1 H) 2.45 (m, 2 H) 2.72 (m, 2 H) 3.27 (m, 2 H) 7.02 (m, 1 H) 7.19 (m, 1 H) 7.39 (m, 1 H) 7.97 (m, 2 H) 13.64 (m, 1H).

Example 126

1-(4-(2-(N,N-dimethylamino)ethyl)aminocarbonyl-2,6-dichlorophenyl)-5-(2,4-difluorobenzyl)piperidin-2-one

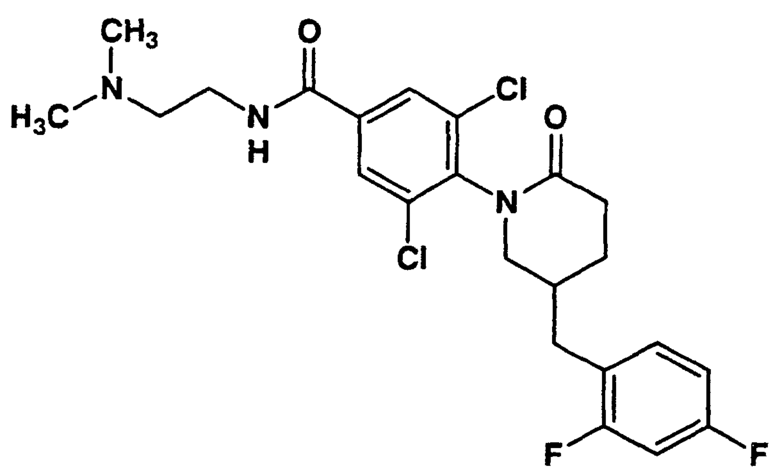

By the same procedure as described in Example 109 using the compound prepared in Example 125, the compound of the present invention having the following physical data was obtained.
TLC:Rf 0.47 (ethyl acetate:methanol:triethylamine=7:2:1);
NMR ($CDCl_3$):d 1.73 (m, 1 H) 2.00 (m, 1 H) 2.27 (s, 6 H) 2.38 (m, 1 H) 2.53 (m, 3 H) 2.70 (m, 3 H) 3.35 (m, 2 H) 3.49 (q, J=4.80 Hz, 2 H) 6.84 (m, 3 H) 7.15 (m, 1 H) 7.78 (m, 2 H).

Example 127

1-(4-(N,N-dimethylamino)methyl-2,6-dichlorophenyl)-5-(2,4-difluorobenzyl)piperidin-2-one

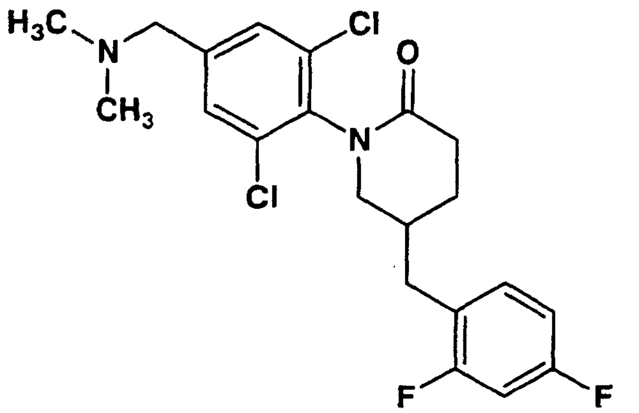

By the same procedure as described in Example 70 using the compound prepared in Example 7 and dimethylamine, the compound of the present invention having the following physical data was obtained.
TLC:Rf 0.42 (ethyl acetate:methanol=19:1);
NMR ($CDCl_3$):d 1.72 (m, 1 H) 1.98 (m, 1 H) 2.25 (s, 6 H) 2.36 (m, 1 H) 2.51 (m, 1 H) 2.69 (m, 3 H) 3.35 (m, J=5.77 Hz, 4 H) 6.82 (m, 2 H) 7.15 (m, 1 H) 7.35 (m, 2 H).

Example 127(1)

1-(4-(4-methylpiperazin-1-ymethyl)-2,6-dichlorophenyl)-5-(2,4-difluorobenzyl)piperidin-2-one

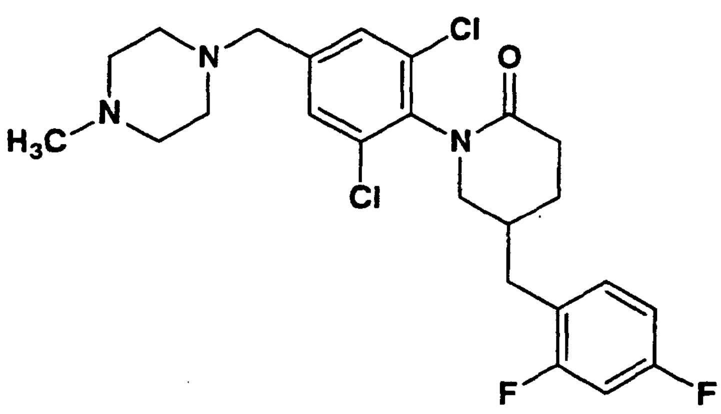

By the same procedure as described in Example 127 using 1-methylpiperazine instead of dimethylamine, the compound of the present invention having the following physical data was obtained.
TLC:Rf 0.23 (ethyl acetate:triethylamine=9:1);
NMR ($CDCl_3$):d 1.73 (m, 1 H) 1.98 (m, 1 H) 2.30 (s, 3 H) 2.45 (m, 10 H) 2.65 (m, 1 H) 2.72 (m, 2 H) 3.34 (d, J=7.90 Hz, 2 H) 3.43 (s, 2 H) 6.82 (m, 2 H) 7.15 (m, 1 H) 7.36 (m, 2 H).

Example 128

1-(4-(piperazin-1-yl)-2,6-dichlorophenyl)-5-(2,6-difluorobenzyl)piperidin-2-one

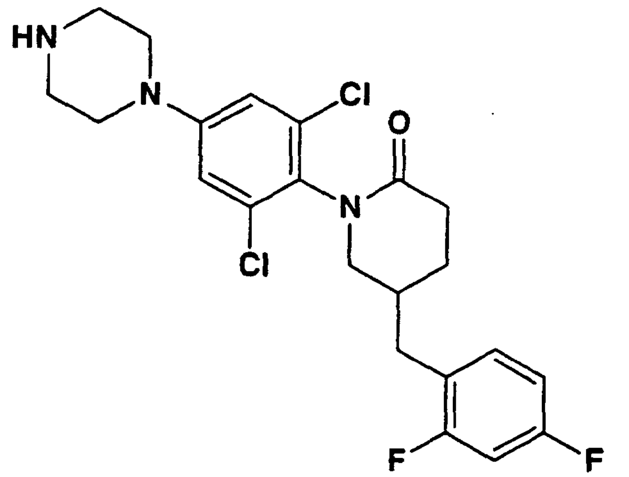

To a solution of the compound prepared in Example 16(13) (500 mg) in toluene (5 ml) were added piperazine (766 mg) and sodium t-butoxide (160 mg) under an atmosphere of argon. To the mixture was added dichlorobis(tri-o-tolylphosphine)palladium(II) (44 mg). The reaction mixture was stirred at 100°C for 4 hours. The reaction mixture was diluted with ethyl acetate, filtrated through Celite (proprietary name) and filtrated. The filtrate was concentrated. The obtained residue was purified by column chromatography on silica gel (ethyl acetate:methanol:triethylamine=1:0:0→10:2:1) to give the compound of the present invention (96 mg) having the following physical data.
TLC:Rf 0.20 (chloroform:methanol=4:1);
NMR ($CDCl_3$):d 1.70 (m, 1 H) 1.95 (m, 1 H) 2.33 (m, 1 H) 2.49 (m, 1 H) 2.67 (m, 3 H) 2.99 (m, 4 H) 3.15 (m, 4 H) 3.33 (d, J=7.40 Hz, 2 H) 6.83 (m, 4 H) 7.14 (m, 1 H).

Example 128(1)

1-(4-(4-methylpiperazin-1-yl)-2,6-dichlorophenyl)-5-(2,4-difluorobenzyl)piperidin-2-one

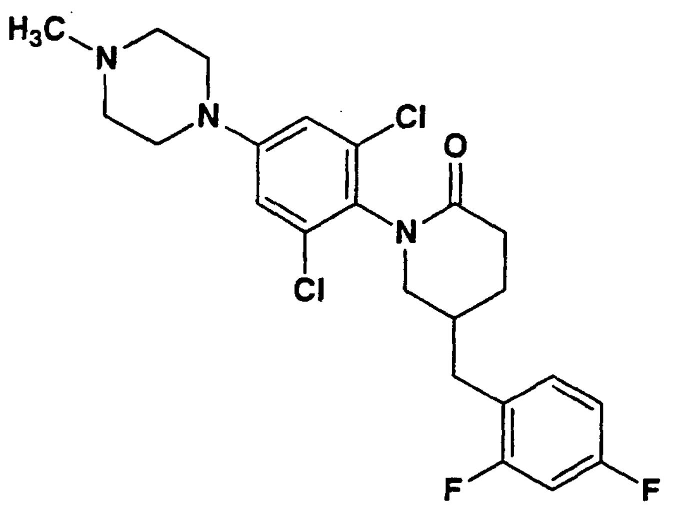

By the same procedure as described in Example 128 using 1-methylpiperazine instead of piperazine, the compound of the present invention having the following physical data was obtained.
TLC:Rf 0.58 (chloroform:methanol=4:1);
NMR ($CDCl_3$):d 1.71 (m, 1 H) 1.95 (m, 1 H) 2.34 (m, 4 H) 2.52 (m, 5 H) 2.67 (m, 3 H) 3.21 (m, 4 H) 3.32 (d, J=7.70 Hz, 2 H) 6.81 (m, 4 H) 7.14 (m, 1 H).

Example 129

1-(4-(3-hydroxy-1-propynyl)-2,6-dichlorophenyl)-5-(2,4-difluorobenzyl)piperidin-2-one

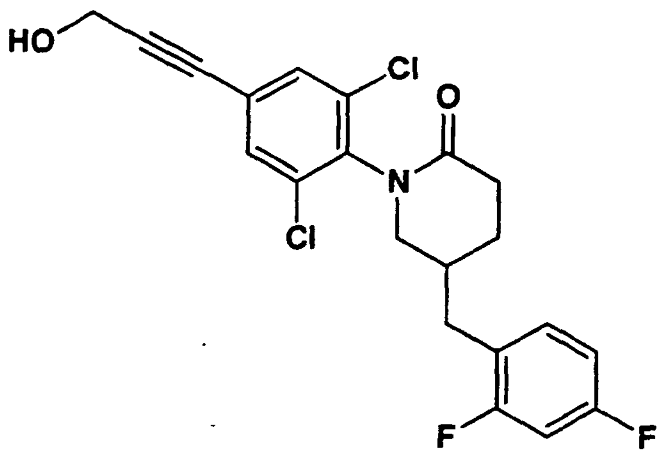

By the same procedure as described in Example 118 using the compound prepared in Example 16(3) and 2-propyn-1-ol, the compound of the present invention having the following physical data was obtained.
TLC:Rf 0.45 (ethyl acetate:hexane=2:1);
NMR ($CDCl_3$):d 1.70 (m, 1 H) 1.78 (t, J=6.30 Hz, 1 H) 1.98 (m, 1 H) 2.37 (m, 1 H) 2.51 (m, 1 H) 2.69 (m, 3 H) 3.33 (d, J=8.00 Hz, 2 H) 4.47 (d, J=6.30 Hz, 2 H) 6.82 (m, 2 H) 7.14 (m, 1 H) 7.43 (m, 2 H).

Example 130

1-(4-(3-(N,N-dimethylamino)propyl)-2,6-dichlorophenyl)-5-(2,4-difluorobenzyl)piperidin-2-one

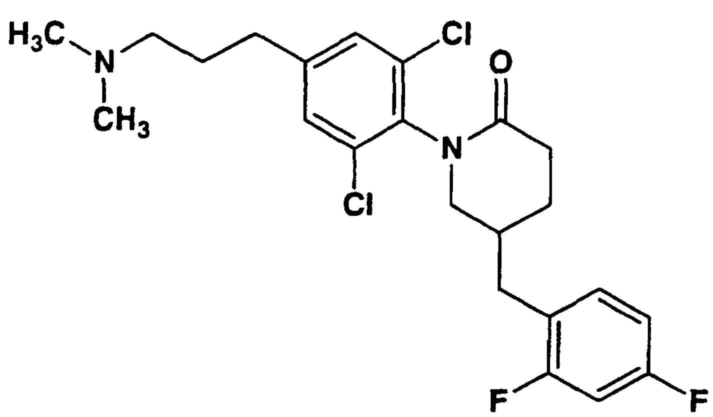

By the same procedure as described in Example 70→Reference Example 15 using the compound prepared in Example 129, the compound of the present invention having the following physical data was obtained.

TLC:Rf 0.46 (ethyl acetate:methanol:triethylamine=8:1:1);
NMR ($CDCl_3$):d 1.74 (m, 3 H) 1.97 (m, 1 H) 2.23 (s, 6 H) 2.29 (t, J=7.20 Hz, 2 H) 2.38 (m, 1 H) 2.56 (m, 4 H) 2.72 (m, 2 H) 3.34 (d, J=7.70 Hz, 2 H) 6.82 (m, 2 H) 7.14 (m, 1 H ) 7.20 (m, 2 H).

Example 131

1-(4-(3-aminopropyl)-2,6-dichlorophenyl)-5-(2,4-difluorobenzyl)piperidin-2-one hydrochloride

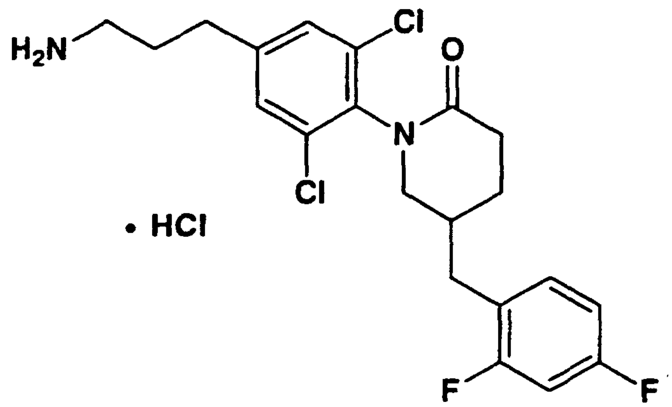

By the same procedure as described in Reference Example 15→Example 73 using the compound prepared in Example 129, the compound of the present invention having the following physical data was obtained.
TLC:RF 0.27 (chloroform:methanol:aqueous ammonia solution=90:10:1);
NMR ($CDCl_3$):d 1.72 (m, 1 H) 1.98 (m, 3 H) 2.46 (m, 7 H) 2.92 (m, 2 H) 3.33 (d, J=7.42 Hz, 2 H) 6.81 (m, 2 H) 7.14 (m, 1 H) 7.28 (m, 2 H) 8.16 (m, 3 H).

Example 132

1-(4-(4-hydroxybut-1-ynyl)-2,6-dichlorophenyl)-5-(2,4-difluorobenzyl)piperidin-2-one

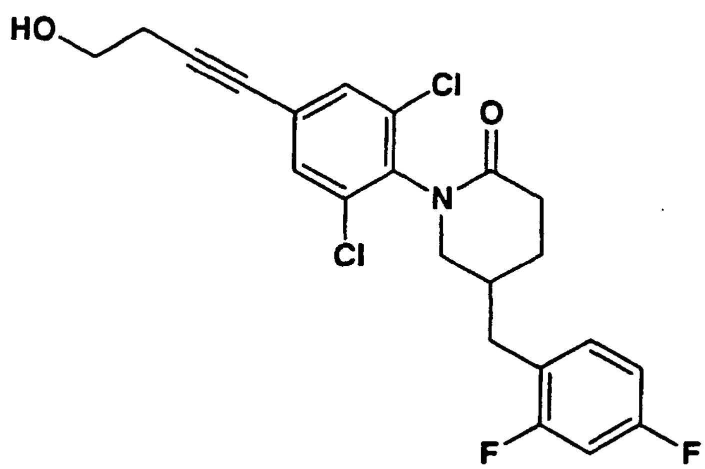

By the same procedure as described in Reference Example 118 using the compound prepared in Example 16(3) and 3-butyn-1-ol, the compound of the present invention having the following physical data was obtained.
TLC:Rf 0.38 (chloroform:methanol=9:1);

NMR ($CDCl_3$):d 1.70 (m, 1 H) 1.82 (t, J=6.20 Hz, 1 H) 1.98 (m, 1 H) 2.35 (m, 1 H) 2.50 (m, 1 H) 2.68 (m, 5 H) 3.33 (d, J=8.00 Hz, 2 H) 3.79 (q, J=6.20 Hz, 2 H) 6.82 (m, 2 H) 7.14 (m, 1 H) 7.41 (m, 2 H).

Example 133

1-(4-(4-(N,N-dimethylamino)butyl)-2,6-dichlorophenyl)-5-(2,4-difluorobenzyl)piperidin-2-one

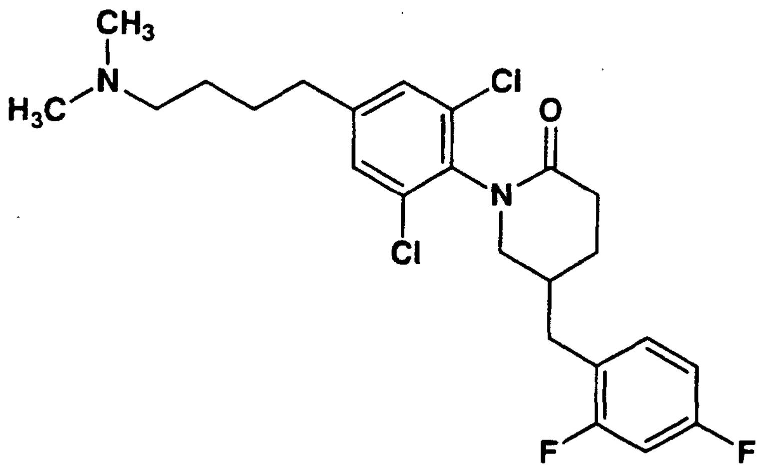

By the same procedure as described in Example 130 using the compound prepared in Example 132, the compound of the present invention having the following physical data was obtained.
TLC:Rf 0.21 (chloroform:methanol=9:1);
NMR ($CDCl_3$):d 1.50 (m, 2 H) 1.68 (m, 3 H) 1.98 (m, 1 H) 2.21 (s, 6 H) 2.27 (m, 2 H) 2.36 (m, 1 H) 2.55 (m, 4 H) 2.72 (m, 2 H) 3.33 (d, J=7.69 Hz, 2 H) 6.81 (m, 2 H) 7.16 (m, 3 H).

Example 134

(E)-1-(4-(2-(methyloxycarbonyl)vinyl)-2,6-dimethylphenyl)-5-(2,4-difluorobenzyl)piperidin-2-one

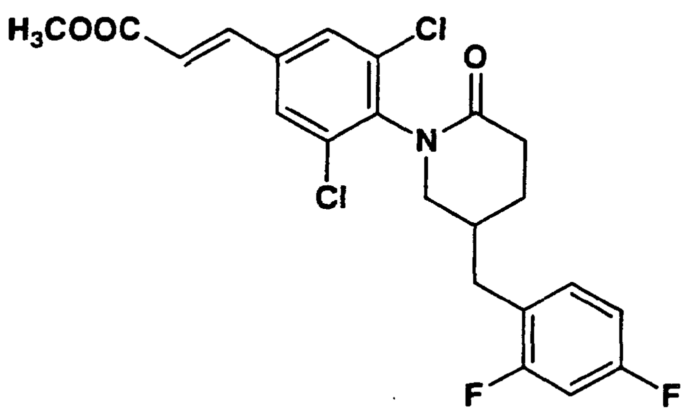

By the same procedure as described in Example 122 using the compound prepared in Example 16(3), the compound of the present invention having the following physical data was obtained.
TLC:Rf 0.50 (ethyl acetate:hexane=2:1);
NMR ($CDCl_3$):d 1.73 (m, 1 H) 2.00 (m, 1 H) 2.38 (m, 1 H) 2.53 (m, 1 H) 2.66 (m, 1 H) 2.73 (m, 2 H) 3.35 (m, 2 H) 3.82 (s, 3 H) 6.41 (d, J=15.90 Hz, 1 H) 6.82 (m, 2 H) 7.15 (m, 1H) 7.53 (m, 3H).

Example 135

(E)-1-(4-(2-aminocarbonylvinyl)-2,6-dimethylphenyl)-5-(2,4-difluorobenzyl)piperidin-2-one

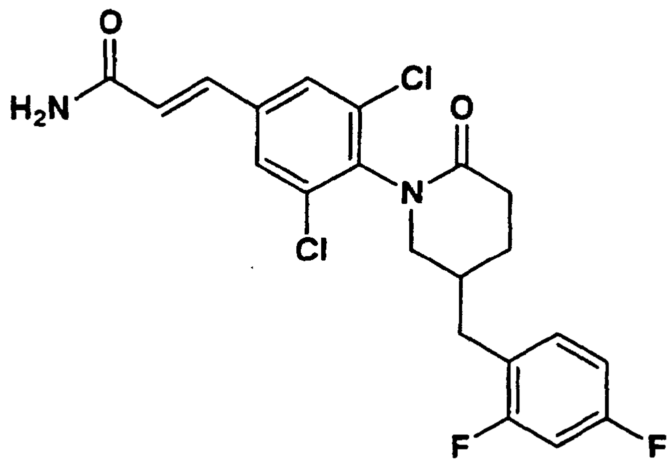

By the same procedure as described in Example 108→Example 109 using aqueous ammonia solution instead of the compound prepared in Example 134 and 2-(N,N-dimethylamine)ethylamine, the compound of the present invention having the following physical data was obtained.
TLC:Rf 0.54 (ethyl acetate:methanol=9:1);
NMR ($CDCl_3$):d 1.74 (m, 1 H) 2.00 (m, 1 H) 2.38 (m, 1 H) 2.54 (m, 1 H) 2.71 (m, 3 H) 3.36 (m, 2 H) 5.46 (m, 1 H) 6.04 (m, 1 H) 6.24 (d, J=15.60 Hz, 1 H) 6.82 (m, 2 H) 7.15 (m, 1 H) 7.42 (m, 3 H).

Example 136

(E)-1-(4-(2-cyanovinyl)-2,6-dimethylphenyl)-5-(2,4-difluorobenzyl)piperidin-2-one

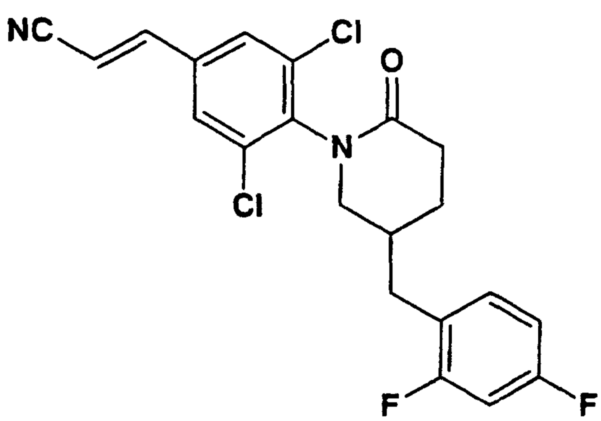

To a solution of the compound prepared in Example 135 (312 mg) in dichloromethane (7.1 ml) were added pyridine (0.34 ml) and anhydrous trifluoromethanesulfonic acid (0.24 ml) at 0°C under an atmosphere of argon. The reaction mixture was stirred at room temperature for 20 minutes. The reaction mixture was poured in 1N hydrochloric acid and extracted with ethyl acetate. The extract was washed with water and brine, dried over anhydrous sodium sulfate and concentrated to give the compound of the present invention (296 mg) having the following physical data.
TLC:Rf 0.52 (ethyl acetate:hexane=2:1);

NMR ($CDCl_3$):d 1.74 (m, 1 H) 1.99 (m, 1 H) 2.37 (m, 1 H) 2.53 (m, 1 H) 2.70 (m, 3 H) 3.33 (m, 2 H) 5.89 (d, J=16.50 Hz, 1 H) 6.82 (m, 2 H) 7.15 (m, 1 H) 7.27 (d, J=16.50 Hz, 1 H) 7.46 (m, 2 H).

Example 137

1-(4-(2-cyanoethyl)-2,6-dichlorophenyl)-5-(2,4-difluorobenzyl)piperidin-2-one

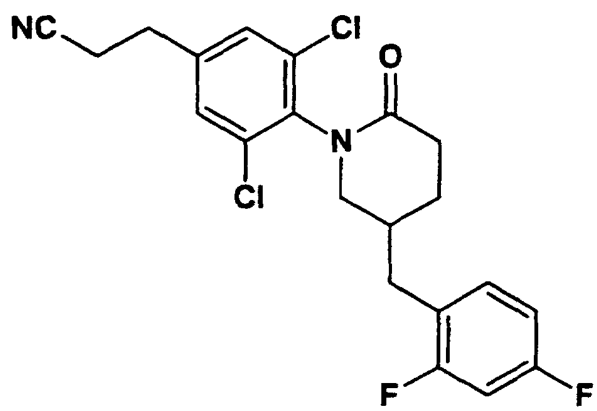

By the same procedure as described in Reference Example 15 using the compound prepared in Example 136, the compound of the present invention having the following physical data was obtained.
TLC:Rf 0.38 (ethyl acetate:hexane);
NMR ($CDCl_3$):d 1.73 (m, 1 H) 1.98 (m, 1 H) 2.36 (m, 1 H) 2.51 (m, 1 H) 2.68 (m, 5 H) 2.92 (t, J=7.40 Hz, 2 H) 3.34 (m, 2 H) 6.82 (m, 2 H) 7.15 (m, 1 H) 7.26 (m, 2 H).

Example 138

1-(4-(2-methoxycarbonylethyl)-2,6-dichlorophenyl)-5-(2,4-difluorobenzyl)piperidin-2-one

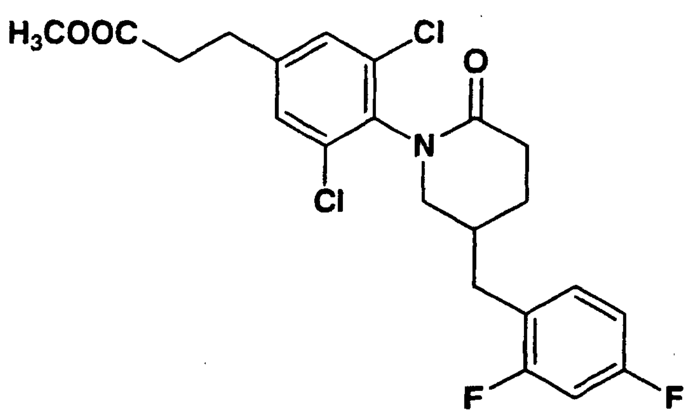

By the same procedure as described in Reference Example 15 using the compound prepared in Example 134, the compound of the present invention having the following physical data was obtained.
TLC:Rf 0.46 (ethyl acetate:hexane=2:1);
NMR ($CDCl_3$):d 1.71 (m, 1 H) 1.97 (m, 1 H) 2.35 (m, 1 H) 2.51 (m, 1 H) 2.63 (m, 3 H) 2.72 (m, 2 H) 2.90 (t, J=7.80 Hz, 2 H) 3.33 (d, J=8.00 Hz, 2 H) 3.69 (s, 3 H) 6.82 (m, 2 H ) 7.15 (m, 1 H) 7.23 (m, 2 H).

Example 139

1-(4-(2-methylaminocarbonyl)ethyl-2,6-dichlorophenyl)-5-(2,4-difluorobenzyl)piperidin-2-one

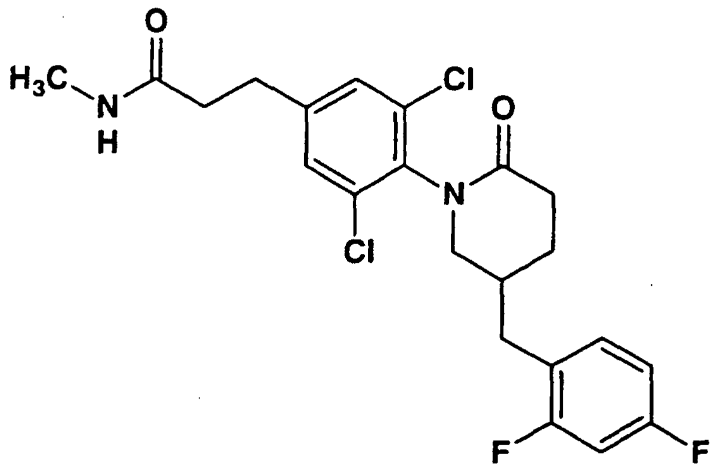

By the same procedure as described in Example 108→Example 109 using the compound prepared in Example 138 and methylamine instead of 2-(N,N-dimethylamine)ethylamine, the compound of the present invention having the following physical data was obtained.
TLC:Rf0.41 (ethyl acetate:methanol=9:1);
NMR ($CDCl_3$):d 1.74 (m, 1 H) 1.98 (m, 1 H) 2.37 (m, 3 H) 2.52 (m, 1 H) 2.65 (m, 1 H) 2.72 (m, 2 H) 2.78 (d, J=4.90 Hz, 3 H) 2.92 (m, 2 H) 3.33 (d, J=7.90 Hz, 2 H) 5.61 (m, 1 H ) 6.82 (m, 2 H) 7.14 (m, 1 H) 7.21 (s, 2 H).

Example 140

(3R,5R)-1-(4-methoxycarbonyl-2,6-dimethylphenyl)-5-(2,4-difluorobenzyl)-3-methylpiperidin-2-one

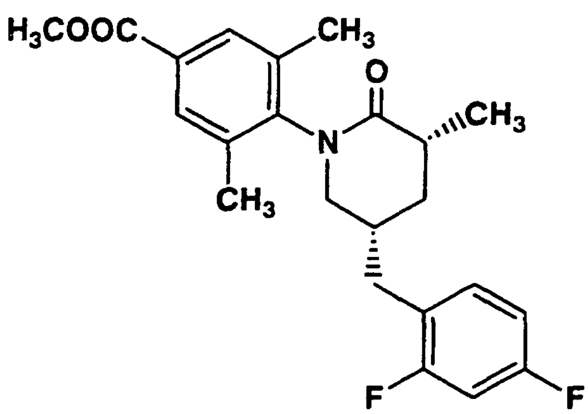

By the same procedure as described in Example 107 using the compound prepared in Example 31(2), the compound of the present invention having the following physical data was obtained.
TLC:Rf 0.33 (hexane:ethyl acetate=1:1);
NMR ($CDCl_3$):d 1.30 (d, J=7.14 Hz, 3 H) 1.46 (m, J=12.64 Hz, 1 H) 2.06 (m, 1 H) 2.15 (s, 3 H) 2.22 (s, 3 H) 2.36 (m, 1 H) 2.52 (m, 1 H) 2.66 (d, J=7.14 Hz, 2 H) 3.22 (d, J=11.26 Hz, 2 H) 3.89 (s, 3 H) 6.82 (m, 2 H) 7.12 (m, J=6.59 Hz, 1 H) 7.76 (s, 2 H).

Example 141

(3R,5R)-1-(4-carboxy-2,6-dimethylphenyl)-5-(2,4-difluorobenzyl)-3-methylpiperidin-2-one

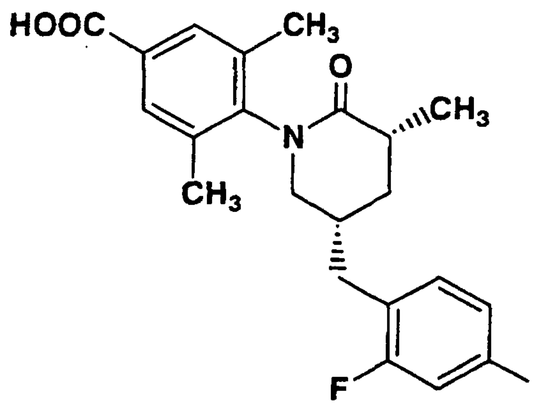

By the same procedure as described in Example 36 using the compound prepared in Example 140, the compound of the present invention having the following physical data was obtained.
TLC:Rf 0.15 (ethyl acetate:hexane=1:1);
NMR ($CDCl_3$):d 1.32 (d, J=7.14 Hz, 3 H) 1.48 (m, 1 H) 2.07 (m, 1 H) 2.15 (s, 3 H) 2.22 (s, 3 H) 2.37 (m, 1 H) 2.56 (m, 1 H) 2.66 (d, J=7.14 Hz, 2 H) 3.24 (m, J=11.54 Hz, 2 H) 6.82 (m , 2 H) 7.11 (m, 1 H) 7.71 (s, 2 H).

Example 142

(3R,5R)-1-(4-(2-(N,N-dimethylamino)ethylaminocarbonyl)-2,6-dimethyiphenyl)-5-(2,4-difluorobenzyl)-3-methylpiperidin-2-one

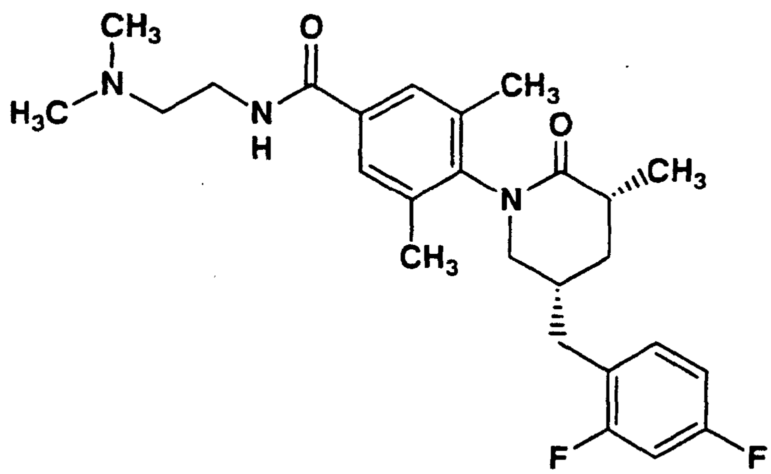

By the same procedure as described in Example 109 using the compound prepared in Example 141, the compound of the present invention having the following physical data was obtained.
TLC:Rf 0.15 (chloroform:methanol=9:1);
NMR ($CDCl_3$):d 1.30 (d, J=7.14 Hz, 3 H) 1.46 (m, 1 H) 2.14 (s, 3 H) 2.22 (s, 3 H) 2.25 (m, J=15.93 Hz, 1 H) 2.27 (s, 6 H) 2.51 (m, 4 H) 2.65 (d, J=7.14 Hz, 2 H) 3.18 (m, 2 H) 3.50 (m, J=5.77 Hz, 2 H) 6.79 (m, 3 H) 7.11 (m, 1 H) 7.47 (s, 2 H).

Example 143

(3R,5R)-1-(4-(3-(N,N-dimethylamino)-1-propynyl)-2,6-dimethylphenyl)-5-(2,4-difluorobenzyl)-3-methylpiperidin-2-one

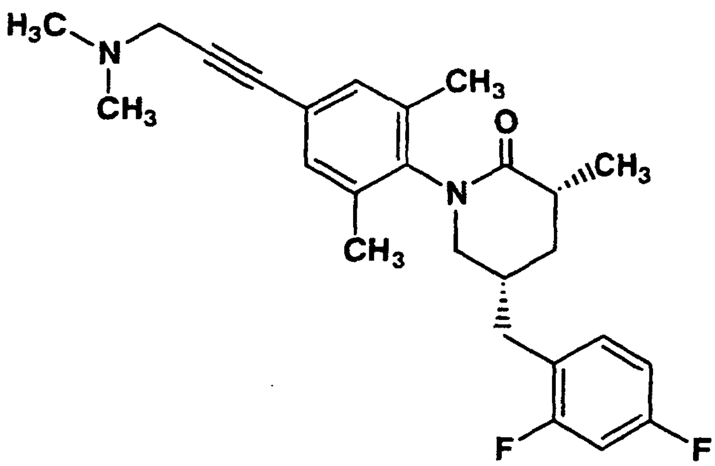

By the same procedure as described in Example 118 using N,N-dimethyl-2-propynylamine and the compound prepared in Example 32(2) instead of 4-pentyn-1-ol, the compound of the present invention having the following physical data was obtained.
TLC:Rf 0.15 (ethyl acetate:methanol=9:1);
NMR ($CDCl_3$):d 1.30 (d, J=7.14 Hz, 3 H) 1.45 (m, J=12.64 Hz, 1 H) 2.06 (m, 1 H) 2.06 (s, 3 H) 2.14 (s, 3 H) 2.33 (m, J=10.16 Hz, 1 H) 2.34 (s, 6 H) 2.52 (m, 1 H) 2.65 (d, J=7.14 Hz, 2 H) 3.20 (m, J=11.54 Hz, 2 H) 3.45 (s, 2 H) 6.81 (m, 2 H) 7.11 (m, J=6.32 Hz, 1 H) 7.16 (s, 2 H).

Example 144

(3R,5R)-1-(4-(3-(N,N-dimethylamino)propynyl)-2,6-dimethylphenyl)-5-(2,4-difluorobenzyl)-3-methylpiperidin-2-one

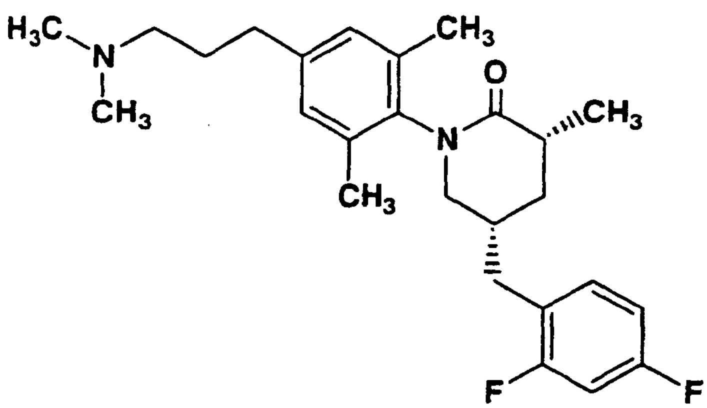

By the same procedure as described in Reference Example 15 using the compound prepared in Example 143, the compound of the present invention having the following physical data was obtained.
TLC:Rf 0.36 (chloroform:methanol=9:1);
NMR ($CDCl_3$):d 1.30 (d, J=6.87 Hz, 3 H) 1.45 (m, 1 H) 1.75 (m, 2 H) 2.07 (m, 1 H) 2.07 (s, 3 H) 2.14 (s, 3 H) 2.23 (s, 6 H) 2.30 (m, 3 H) 2.53 (m, 3 H) 2.64 (d, J=7.14 Hz, 2 H) 3.22 (m, J=11.26 Hz, 2 H) 6.81 (m, 2 H) 6.89 (s, 2 H) 7.11 (m, 1 H).

Example 145

1-(4-carboxyl-2,6-dimethylphenyl)-5-(2,4-difluorobenzyl)tetrahydropyrimidin-2(1H)-one

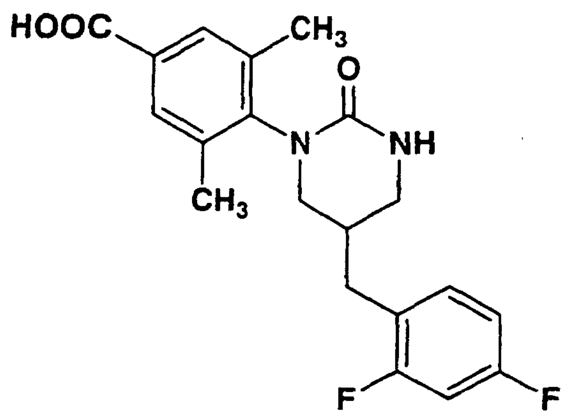

By the same procedure as described in Example 107→Example 108 using the compound prepared in Example 20, the compound of the present invention having the following physical data was obtained.
TLC:Rf 0.45 (ethyl acetate:methanol=19:1);
NMR (DMSO-$d_6$):d 2.10 (s, 3 H) 2.20 (s, 3 H) 2.35 (m, 1 H) 2.71 (d, J=7.14 Hz, 2 H) 3.12 (m, 4 H) 6.58 (m, J=3.30 Hz, 1 H) 7.03 (m, 1 H) 7.19 (m, 1 H) 7.39 (m, 1 H) 7.63 (s, 2 H).

Example 146

1-(4-t-butyloxycarbonylamino-2,6-dimethylphenyl)-5-(2,4-difluorobenzyl)tetrahydropyrimidin-2(1H)-one

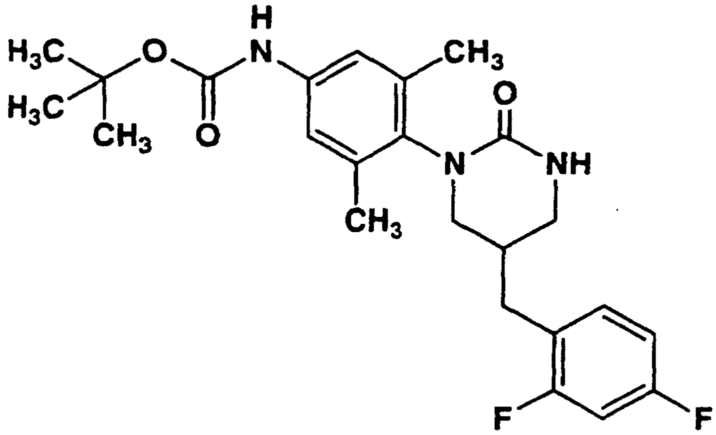

By the same procedure as described in Example 111 using the compound prepared in Example 145, the compound of the present invention having the following physical data was obtained.
TLC:Rf 0.43 (ethyl acetate);
NMR ($CDCl_3$):d 1.50 (s, 9 H) 2.14 (s, 3 H) 2.23 (s, 3 H) 2.50 (m, 1 H) 2.73 (m, 2 H) 3.26 (m, 4 H) 4.77 (d, J=1.10 Hz, 1 H) 6.40 (s, 1 H) 6.82 (m, 2 H) 7.12 (m, 3 H).

Example 147

1-(4-amino-2,6-dimethylphenyl)-5-(2,4-difluorobenzyl)tetrahydropyrimidin-2(1H)-one

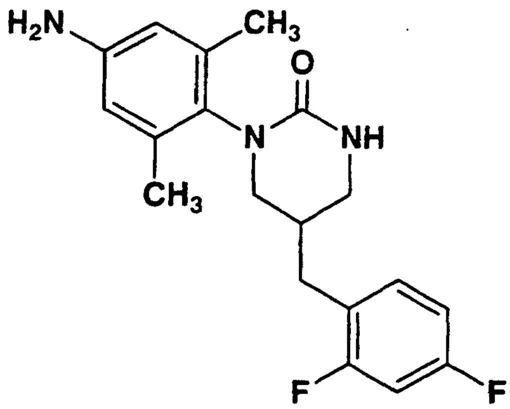

By the same procedure as described in Reference Example 31 using the compound prepared in Example 146, the compound of the present invention having the following physical data was obtained.
TLC:Rf 0.43 (ethyl acetate);
NMR ($CDCl_3$):d 2.08 (s, 3 H) 2.17 (s, 3 H) 2.49 (m, J=2.75 Hz, 1 H) 2.72 (m, 2 H) 3.26 (m, 4 H) 3.55 (s, 2 H) 4.92 (s, 1 H) 6.38 (s, 2 H) 6.81 (m, 2 H) 7.13 (m, 1 H).

Example 148

1-(4-(2-(N,N-dimethylamino)ethylaminocarbonyl)-2,6-dimethylphenyl)-5-(2,4-difluorobenzyl)tetrahydropyrimidin-2 (1H)-one

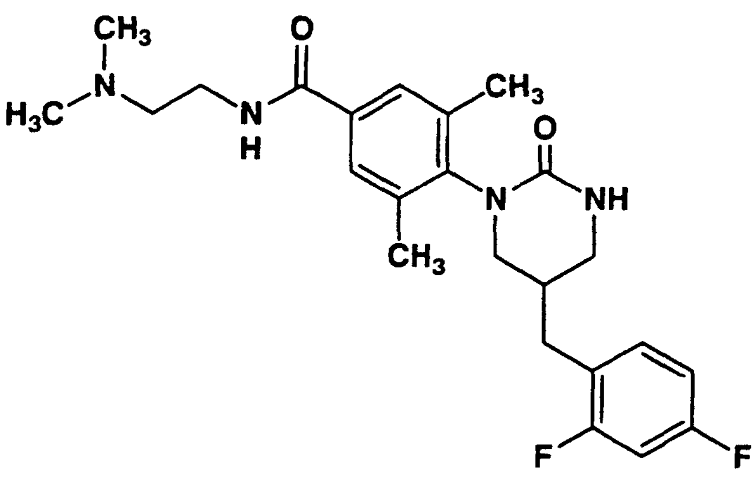

TLC:Rf 0.45 (ethyl acetate:methanol:triethylamine=8:1:1);
NMR ($CDCl_3$):d 2.22 (s, 3 H) 2.27 (s, 6 H) 2.31 (s, 3 H) 2.51 (m, 3 H) 2.74 (m, J=6.87 Hz, 2 H) 3.28 (m, 4 H) 3.50 (m, 2 H) 4.84 (m, J=3.85 Hz, 1 H) 6.73 (m, 1H) 6.83 (m, 2 H) 7.14 (m, J=6.59 Hz, 1 H) 7.46 (s, 2 H).

Example 149

1-(2,6-dimethylphenyl)-5-(2-methoxycarbonylbenzyl)piperidin-2-one

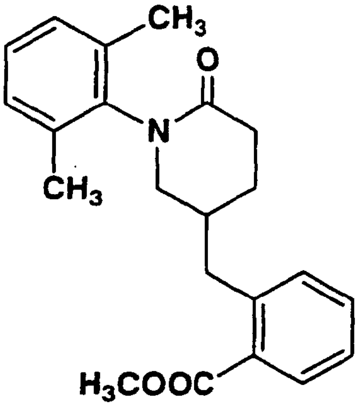

By the same procedure as described in Example 107 using the compound prepared in Example 16(5), the compound of the present invention having the following physical data was obtained.
TLC:Rf 0.32 (ethyl acetate:toluene=3:1);
NMR ($CDCl_3$):d 1.66 (m, 1 H) 2.04 (m, 1 H) 2.11 (s, 3 H) 2.23 (s, 3 H) 2.33 (m, 1 H) 2.46 (m, 1 H) 2.66 (m, 1 H) 2.94 (m, 1 H) 3.12 (m, 1 H) 3.28 (m, 2 H) 3.88 (m, 3 H) 7.09 ( m, 3 H) 7.27 (m, 2 H) 7.43 (m, 1 H) 7.96 (dd, J=7.97, 1.37 Hz, 1 H).

Example 150

1-(2,6-dimethylphenyl)-5-(2-carboxybenzyl)piperidin-2-one

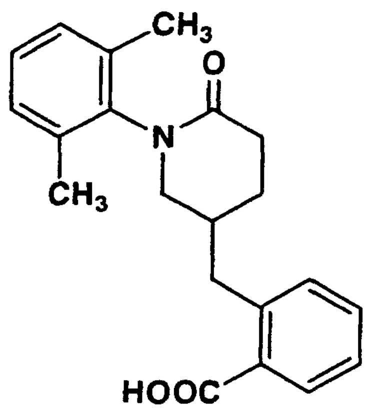

By the same procedure as described in Example 36 using the compound prepared in Example 149, the compound of the present invention having the following physical data was obtained.
TLC:Rf 0.36 (ethyl acetate:methanol=5:1);
NMR (DMSO-$d_6$):d 1.64 (m, 1 H) 1.83 (m, 1 H) 1.99 (m, 3 H) 2.13 (m, 3 H) 2.32 (m, 2 H) 2.99 (m, 3 H) 3.29 (m, 2 H) 7.07 (s, 3 H) 7.30 (m, 2 H) 7.46 (m, 1 H) 7.82 (d, J=8.06 Hz, 1 H) 12.93 (m, 1 H).

Example 151

1-(2,6-dimethylphenyl)-5-(2-(2-methoxycarbonylvinyl)benzyl)piperidin-2-one

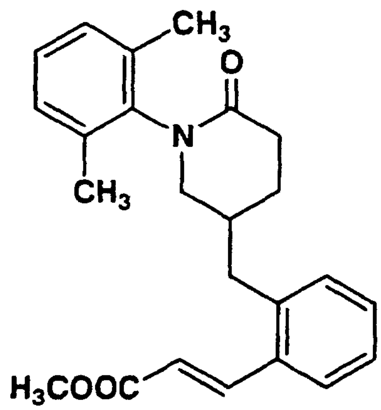

By the same procedure as described in Example 122 using the compound prepared in Example 16(5), the compound of the present invention having the following physical data was obtained.
TLC:Rf 0.35 (ethyl acetate);
NMR ($CDCl_3$):d 1.65 (m, 1 H) 2.00 (m, 4 H) 2.21 (m, 4 H) 2.46 (m, 1 H) 2.66 (m, 1 H) 2.84 (m, 2 H) 3.25 (m, 2 H) 3.82 (s, 3 H) 6.39 (d, J=15.66 Hz, 1 H) 7.04 (m, 2 H) 7.17 (m, 1 H) 7.30 (m, 2 H) 7.59 (m, 2 H) 7.99 (d, J=15.66 Hz, 1 H).

Example 152

(3R,5R)-1-(4-amino-2,6-dichlorophenyl)-5-(2,4-dinuorobenzyl)-3-methylpiperidin-2-one

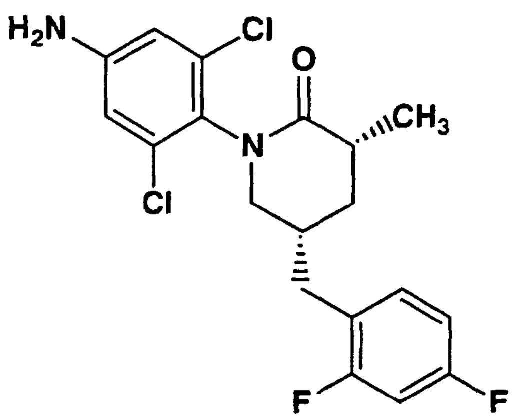

By the same procedure as described in Example 10 using the compound prepared in Example 31(1), the compound of the present invention having the following physical data was obtained.
TLC:Rf 0.73(hexane:ethyl acetate=1:1);
NMR ($CDCl_3$):d 1.28 (d, J=7.14 Hz, 3 H) 1.46 (m, J=12.36 Hz, 1 H) 1.99 (m, 1 H) 2.43 (m, 2 H) 2.65 (d, J=7.42 Hz, 2 H) 3.31 (m, J=10.71 Hz, 2 H) 3.85 (s, 2 H) 6.61 (s, 2 H) 6.81 (m, 2 H) 7.12 (m, 1 H).

Example 153

(3R,5R)-1-(4-(t-butoxycarbonylamino)-2,6-dichlorophenyl)-5-(2,4-difluorobenzyl)-3-methylpiperidin-2-one

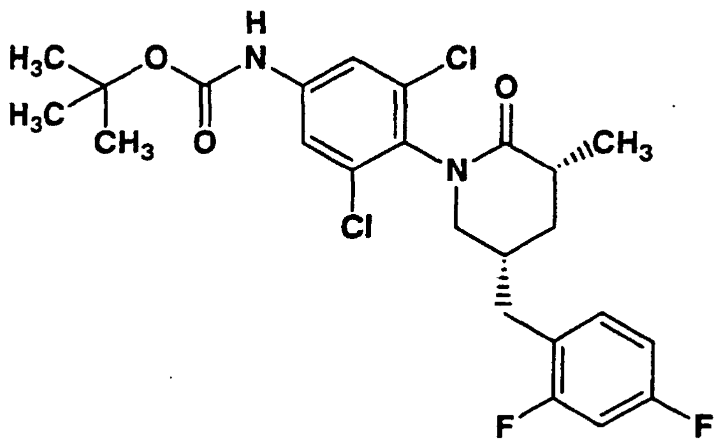

By the same procedure as described in Reference Example 39 using the compound prepared in Example 152, the compound of the present invention having the following physical data was obtained.
TLC:Rf 0.52 (hexane:ethyl acetate=2:1);
NMR ($CDCl_3$):d 1.32 (d, J=6.87 Hz, 3 H) 1.50 (m, 1 H) 1.48 (s, 9 H) 2.05 (m, 1 H) 2.37 (m, 1 H) 2.53 (m, 1 H) 2.65 (d, J=7.42 Hz, 2 H) 3.27 (m, 2 H) 6.82 (m, 2 H) 7.10 (m, 2 H) 7.51 (m, J=2.20 Hz, 1 H) 7.75 (s, 1 H).

Example 154

(3R,5R)-1-(4-(2-(N,N-dimethylamino)ethylamino)-2,6-dichlorophenyl)-5-(2,4-difluorobenzyl)-3-methylpiperidin-2-one

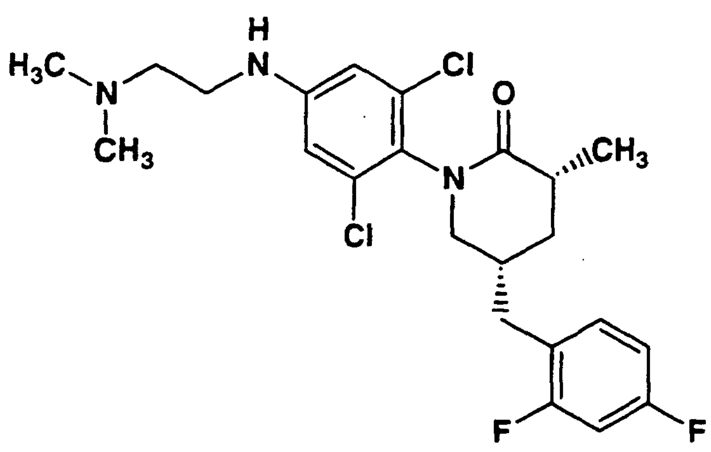

By the same procedure as described in Example 57→Example 58 using the compound prepared in Example 153, the compound of the present invention having the following physical data was obtained.
TLC:Rf 0.02 (ethyl acetate:methanol=9:1);
NMR ($CDCl_3$):d 1.28 (d, J=7.14 Hz, 3 H) 1.46 (m, 1 H) 1.98 (m, 1 H) 2.23 (s, 6 H) 2.53 (m, 4 H) 2.64 (d, J=7.69 Hz, 2 H) 3.06 (m, J=6.32 Hz, 2 H) 3.29 (m, 2 H) 4.60 (m, 1 H) 6.56 (s, 2 H) 6.81 (m, 2 H) 7.12 (m, 1 H).

Example 155

1-(2,6-dimethylphenyl)-5-(3-phenylpropyl)piperidin-2-one

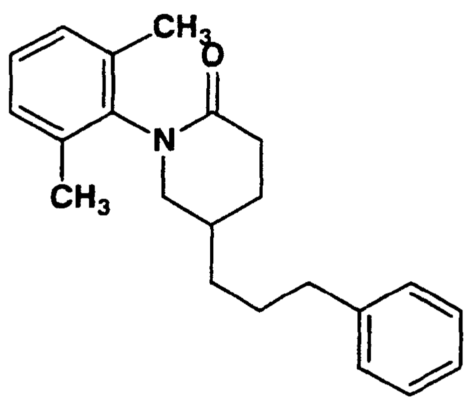

By the same procedure as described in Reference Example 15 using the compound prepared in Example 18, the compound of the present invention having the following physical data was obtained.
TLC:Rf 0.45 (ethyl acetate:hexane=3:2);
NMR ($CDCl_3$):d 7.32-7.22 (m, 3H), 7.21-7.06 (m, 5H), 3.28 (ddd, J =, 12.0, 5.1, 1.8 Hz, 1H), 3.12 (dd, J = 12.0, 10.2 Hz, 1H), 2.70-2.59 (m, 3H), 2.51 (ddd, J = 17.4, 11.1, 5.7 Hz, 1H), 2.18 (s, 3H), 2.17 (s, 3H), 2.07-1.92 (m, 2H), 1.78-1.55 (m, 3H), 1.49-1.36 (m, 2H).

Examples 156

(3RS,SRS)-1-(2,6-dichlorophenyl)-5-(2,4-difluorobenzyl)-3-hydropiperidin-2-one (compound 1) and (3RS,5SR)-1-(2,6-dichlorophenyl)-5-(2,4-difluorobenzyl)-3-hydropiperidin-2-one(compound 2)

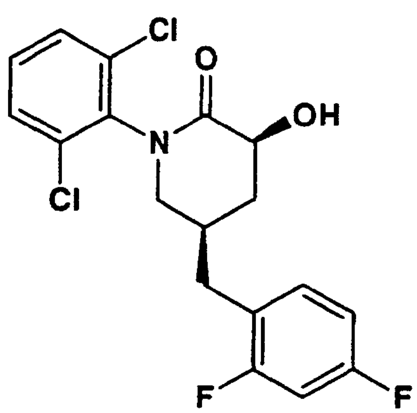

(1)

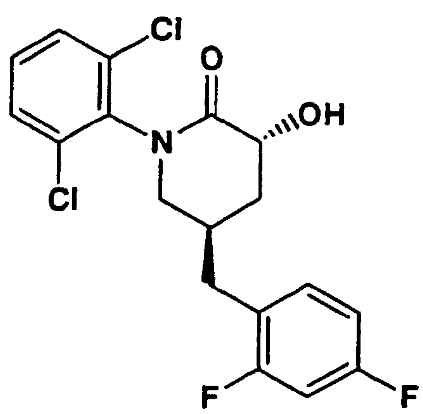

(2)

To a solution of the compound prepared in Example 1(1) (315 mg) in tetrahydrofuran (5 ml) was added lithium bis(trimethylsilyl)amide (1.0M solution in tetrahydrofuran, 900 µl) at -78°C under an atmosphere of argon. The mixture was stirred at same temperature for 30 minutes. To the mixture was added a solution of 3-phenyl-2-(phenylsulfonyl)-1,2-oxaziridine (245 mg) in tetrahydrofuran (3 ml) at -70°C. The reaction mixture was stirred at -70°C for 6 hours. A

saturated aqueous ammonium chloride solution was added to the reaction mixture, which was extracted with ethyl acetate. The extract was washed water and brine, dried over anhydrous magnesium sulfate and concentrated. The obtained residue was purified by column chromatography on silica gel (hexane:ethyl acetate=3:2) to give the compound (1) (29 mg) and the compound (2) (37 mg) of the present invention having the following physical data respectively.

Example 156(1)

TLC:Rf 0.56 (ethyl acetate:hexane=1:1);
NMR ($CDCl_3$):d 1.78 (m, 1 H) 2.36 (m, 1 H) 2.52 (m, 1 H) 2.75 (m, 2 H) 3.39 (m, 2 H) 3.50 (s, 1 H) 4.21 (dd, J=11.81, 5.77 Hz, 1 H) 6.83 (m, 2 H) 7.14 (m, 1 H) 7.26 (m, 1 H) 7.40 (m, 2 H).

Example 156(2)

TLC:Rf 0.51 (ethyl acetate:hexane=1:1);
NMR ($CDCl_3$):d 2.11 (m, 2 H) 2.76 (m, 3 H) 3.43 (m, 3 H) 4.44 (m, 1 H) 6.83 (m, 2 H) 7.21 (m, 2 H) 7.39 (d, J=7.97 Hz, 2 H).

Examples 157

(3RS,5RS)-1-(2,6-dimethyiphenyl)-5-(2,4-difluorobenzyl)-3-methylpiperidin-2-one (compound 1) and (3RS,5SR)-1-(2,6-dimethylphenyl)-5-(2,4-difluorobenzyl)-3-methylpiperidin-2-one (compound 2)

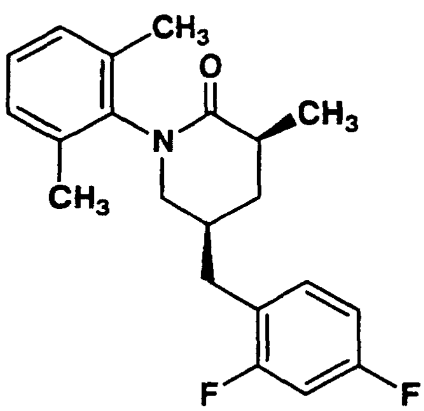

(1)

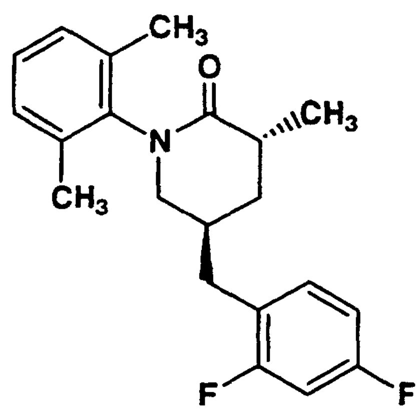

(2)

By the same procedure as described in Example 14 using the compound prepared in Example 5, the compound (1) and the compound (2)of the present invention having the following physical data were obtained respectively.

Example 157(1)

TLC:Rf 0.53 (hexane:ethyl acetate=1:1);
NMR ($CDCl_3$):d 7.18-7.02 (m, 4H), 6.88-6.75 (m , 2H), 3.23 (dd, J = 12.0, 11.1 Hz, 1H), 3.16 (ddd, J = 12.0, 5.1, 1.8 Hz, 1H), 2.65 (d, J = 7.2 Hz, 2H), 2.60-2.45 (m, 1H), 2.45-2.25 (m ,1H), 2.18 (s, 3H), 2.15-2.00 (m ,4H), 1.46 (ddd, J = 12.6, 12.3, 12.3 Hz, 1H), 1.31 (d, J = 6.9 Hz, 3H).

Example 157(2)

TLC:Rf 0.40 (hexane:ethyl acetate=1:1);
NMR ($CDCl_3$):d 7.18-7.02 (m, 4H), 6.88-6.75 (m ,2H), 3.30-3.15 (m ,2H), 2.82-2.60 (m, 3H), 2.60-2.40 (m, 1H), 2.20 (s, 3H), 2.10 (s, 3H), 1.88 (ddd, J = 13.5, 10.2, 6.6 Hz, 1H), 1.77 (dddd, J = 13.5,3.9,3.9,1.5 Hz, 1H), 1.34 (d, J = 7.2 Hz, 3H).

Example 158

(5S)-1-(2,6-dimethylphenyl)-5-(methyl(2,4-difluorophenylsulfonyl)amino)piperidin-2-one

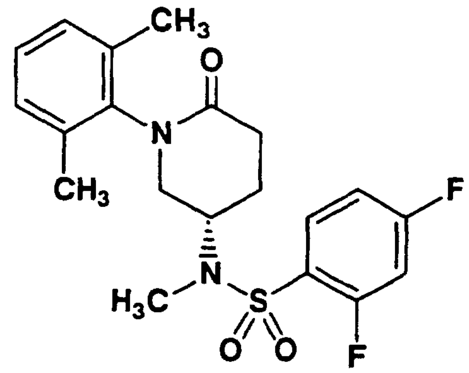

By the same procedure as described in Example 51 using the compound prepared in Example 34, the compound of the present invention having the following physical data was obtained.
TLC:Rf 0.40 (hexane:ethyl acetate=1:4);
NMR ($CDCl_3$):d 2.17 (s, 3 H) 2.23 (m, 1 H) 2.24 (s, 3 H) 2.63 (m, 4 H) 2.66 (s, 3 H) 3.51 (m, J=12.64, 10.16 Hz, 1 H) 4.14 (m, 1 H) 6.94 (m, 2 H) 7.13 (m, 3 H) 7.78 (m, 1 H).

Example 158(1)

(5S)-1-(2,6-dimethylphenyl)-5-((2-hydroxyethyl)(2,4-difluorophenylsulfonyl)amino)piperidin-2-one

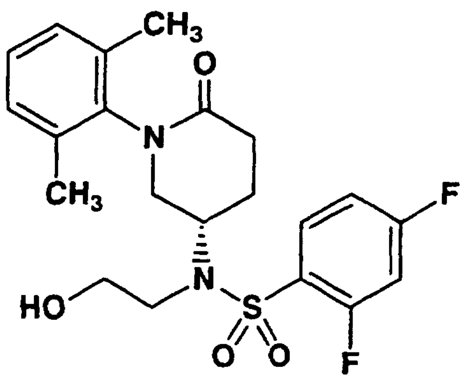

By the same procedure as described in Example 51 using 2-bromoethanol instead of methyl iodide, and the compound prepared in Example 34, the compound of the present invention having the following physical data was obtained.
TLC:Rf 0.22 (hexane:ethyl acetate=1:4);
NMR ($CDCl_3$):d 1.84 (m, 1 H) 2.17 (s, 3 H) 2.22 (s, 3 H) 2.54 (m, 4 H) 2.92 (dd, J=14.01, 3.02 Hz, 1 H) 3.06 (m, 1 H) 3.23 (m, 1 H) 3.66 (m, 3 H) 4.25 (m, 1 H) 6.95 (m, 2 H) 7.12 (m, 3 H) 7.77 (m, 1 H).

Example 159

1-(4-(N-methylcarbonyl-N-methylamino)-2,6-dichlorophenyl)-5-(2,4-difluorobenzyl)piperidin-2-one

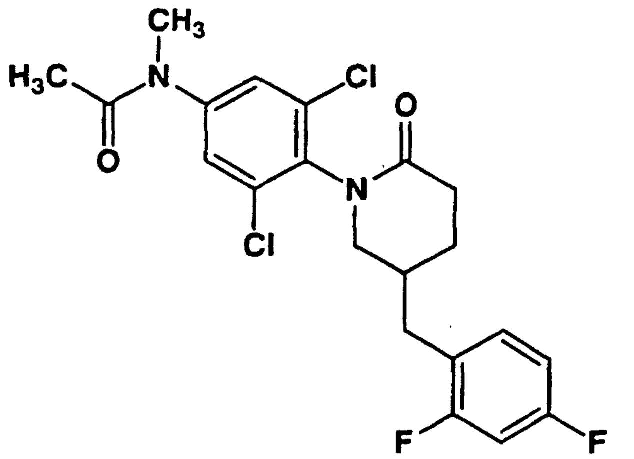

By the same procedure as described in Example 13 using the compound prepared in Example 12(1), the compound of the present invention having the following physical data was obtained.
TLC:Rf 0.33 (ethyl acetate);
NMR ($CDCl_3$):d 1.74 (m, 1 H) 2.04 (m, 4 H) 2.39 (m, 1 H) 2.53 (m, 1 H) 2.66 (dd, J=5.77, 3.57 Hz, 1 H) 2.73 (d, J=7.14 Hz, 2 H) 3.26 (s, 3 H) 3.36 (d, J=7.14 Hz, 2 H) 6.83 (m, 2 H) 7.16 (m, 1 H) 7.26 (m, 2 H).

Example 160

1-(2,6-dichlorophenyl)-5-(2,4-difluorobenzyl)-3-methyltetrahydropyrimidin-2(1H)-one

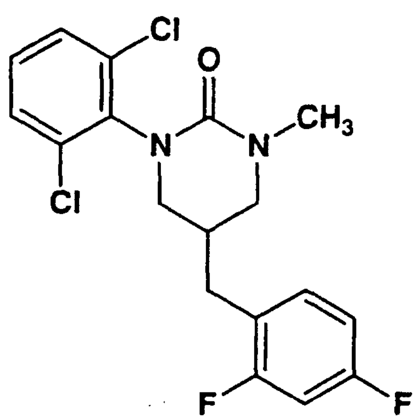

By the same procedure as described in Example 13 using the compound prepared in Example 19, the compound of the present invention having the following physical data was obtained.
TLC:Rf 0.53 (ethyl acetate:hexane=4:1);
NMR ($CDCl_3$):d 7.40-7.34 (m, 2H), 7.21-7.12 (m, 2H), 6.88-6.77 (m, 2H), 3.47-3.37 (m, 2H), 3.32 (m, 1H), 3.20 (dd, J = 12.0, 8.7 Hz, 1H), 2.99 (s, 3H), 2.89-2.71 (m, 2H), 2.63 (m, 1H).

Example 161

1-(2,6-dichlorophenyl)-5-(2,4-difluorobenzyl)piperazin-2-one

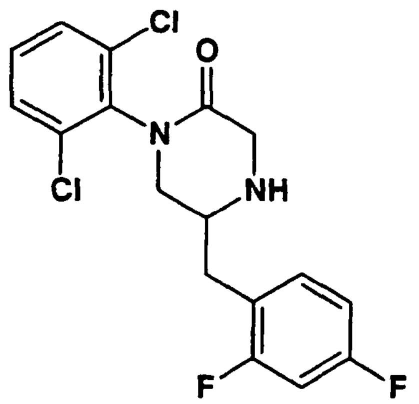

By the same procedure as described in Example 114 using the compound prepared in Example 19, the compound of the present invention having the following physical data was obtained.
TLC:Rf 0.19 (ethyl acetate:hexane=4:1);
NMR ($CDCl_3$):d 2.85 (m, 2 H) 3.36 (m, 1 H) 3.50 (m, 2 H) 3.68 (d, J=17.58 Hz, 1 H) 3.81 (d, J=17.58 Hz, 1 H) 6.85 (m, 2 H) 7.23 (m, 2 H) 7.40 (m, 2 H).

Example 162

1-(2,6-dichlorophenyl)-5-(2,4-difluorobenzyl)-4-methylpiperazin-2-one hydrochloride

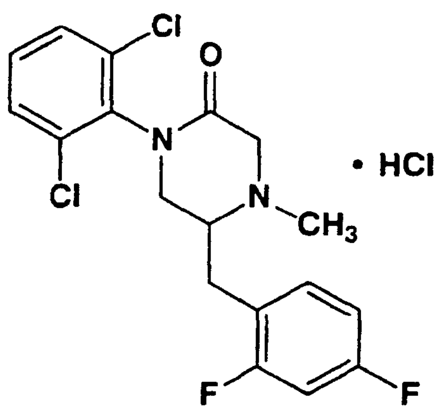

By the same procedure as described in Example 47→Reference Example 31 using the compound prepared in Example 161 and formaldehyde, the compound of the present invention having the following physical data was obtained.
TLC:Rf 0.31 (ethyl acetate:hexane=4:1);
NMR ($CD_3OD$):d 3.18 (m, 4 H) 3.50 (dd, J=14.28, 4.40 Hz, 1 H) 3.60 (dd, J=14.01, 4.12 Hz, 1 H) 3.95 (dd, J=14.01, 9.61 Hz, 1 H) 4.34 (m, 3 H) 7.03 (m, 2 H) 7.49 (m, 4 H).

Example 163

1-(2,6-dichlorophenyl)-5-(2,4-difluorophenylcarbonyl)piperidin-2-one

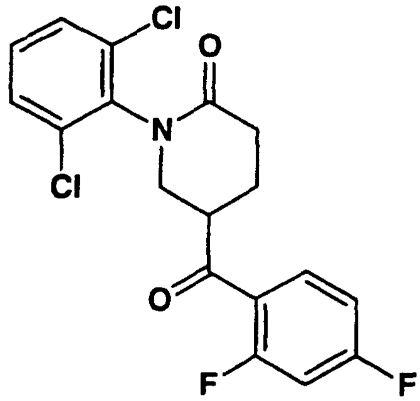

By the same procedure as described in Example 98 using the compound prepared in Example 24, the compound of the present invention having the following physical data was obtained.
TLC:Rf 0.33 (ethyl acetate:hexane=2:3);
NMR ($CDCl_3$):d 7.94 (m, 1H), 7.42-7.38 (m, 2H), 7.23 (m, 1H), 7.01 (m, 1H), 6.94 (m, 1H), 3.99-3.79 (m, 2H), 3.62 (m, 1H), 2.80 (ddd, J = 17.7, 5.7, 3.9 Hz, 1H), 2.69 (m, ddd, J = 17.7, 11.1, 6.3 Hz, 1H), 2.33 (m, 1H), 2.15 (m, 1H).

Example 164

1-(2,6-dichlorophenyl)-5-(1-(2,4-difluorophenyl)-1-hydroxyethyl)piperidin-2-one

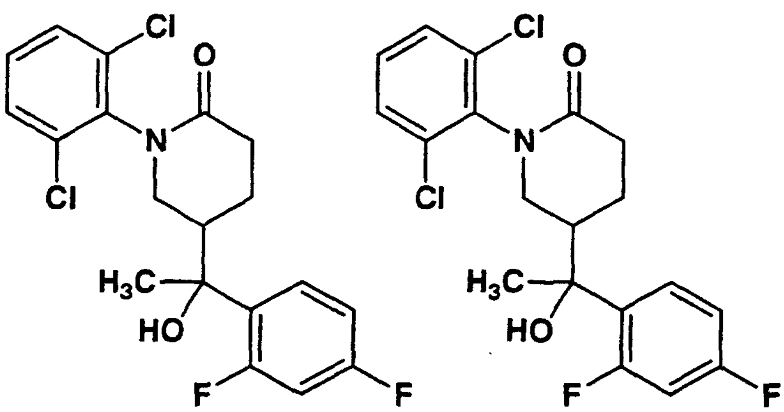

less polar (1) more polar (2)

To a solution of the compound prepared in Example 163 (300 mg) in tetrahydrofuran (5 ml) was added methylmagnesium bromide (0.93M solution in tetrahydrofuran, 1.85 ml) at 0°C under an atmosphere of argon. The reaction mixture was stirred at room temperature for 3 hours. A saturated aqueous ammonium chloride solution was added to the reaction mixture, which was extracted with ethyl acetate. The extract was washed water and brine, dried over anhydrous magnesium sulfate and concentrated. The obtained residue was purified by column chromatography on silica gel (hexane:ethyl acetate=7:3→3:2) to give the compound (1) (125 mg) and the compound (2) (85 mg) of the present invention having the following physical data respectively.

Example 164(1)

TLC:Rf 0.50 (ethyl acetate:hexane=3:2);
NMR ($CDCl_3$):d 7.58 (m, 1H), 7.43-7.37 (m, 2H), 7.22 (t, J = 7.8 Hz, 1H), 6.94-6.78 (m, 2H), 3.71 (t, J = 11.1 Hz, 1H), 3.48 (ddd, J = 11.1, 5.4, 2.1 Hz, 1H), 2.69-2.56 (m, 2H), 2.42 (ddd, J = 18.3, 11.7, 6.0 Hz, 1H), 1.92 (d, J = 1.2 Hz, 1H), 1.81 (m, 1H), 1.66 (d, J = 1.2 Hz, 3H), 1.61 (m, 1H).

Example 164(2)

TLC:Rf 0.32 (ethyl acetate:hexane=3:2);
NMR ($CDCl_3$):d 7.56 (m, 1H), 7.36-7.28 (m, 2H), 7.16 (t, J = 7.8 Hz, 1H), 6.88-6.77 (m, 2H), 3.47 (t, J = 11.4 Hz, 1H), 2.87-2.63 (m, 3H), 2.58 (ddd, J = 18.0, 11.7, 6.3 Hz, 1H), 2.16 (m, 1H), 1.99 (m, 1H), 1.88 (d, J = 1.2 Hz, 1H), 1.71 (d, J = 1.2 Hz, 3H).

Example 165

1-(2,6-dichlorophenyl)-5-(1-(2,4-difluorophenyl)vinyl)piperidin-2-one

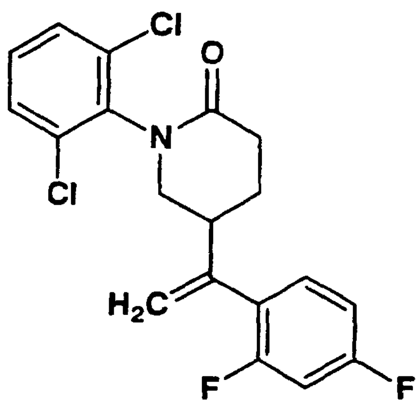

To a solution of the compound prepared in Example 164(1) (75 mg) in toluene (3 ml) was added p-toluenesulfonic acid (7 mg). The reaction mixture was stirred at 130°C for 12 hours. The reaction mixture was diluted with ethyl acetate, washed with a saturated aqueous sodium hydrogen carbonate solution, water and brine, dried over anhydrous magnesium sulfate and concentrated. The obtained residue was purified by column chromatography on silica gel (hexane:ethyl acetate=7:3) to give the compound of the present invention (70 mg) having the following physical data.
TLC:Rf 0.45 (ethyl acetate:hexane=1:1);
NMR ($CDCl_3$):d 7.38 (d, J = 8.1 Hz, 2H), 7.23-7.14 (m, 2H), 6.90- 6.78 (m, 2H), 5.37 (s, 1H), 5.26 (s, 1H), 3.51 (t, J = 11.1 Hz, 1H), 3.40 (ddd, J = 11.1, 5.1, 2.1 Hz, 1H), 3.19 (m, 1H), 2.76 (ddd, J = 18.0, 5.7, 2.7 Hz, 1H), 2.62 (ddd, J = 18.0, 11.4, 6.3 Hz, 1H), 2.18 (m, 1H), 1.93 (m, 1H).

Example 166

1-(2,6-dichlorophenyl)-5-(1-(2,4-difluorophenyl)ethyl)piperidin-2-one

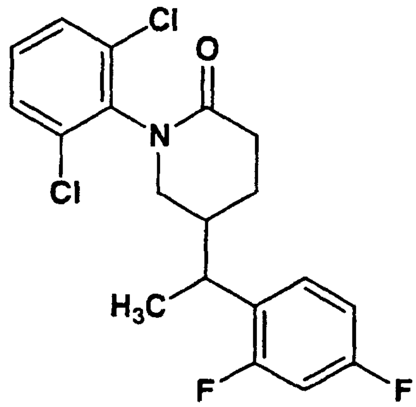

By the same procedure as described in Reference Example 15 using the compound prepared in Example 165, the compound of the present invention having the following physical data was obtained.
TLC:Rf 0.47 (ethyl acetate:hexane=1:1);
NMR ($CDCl_3$):d 7.43-7.38 (m, 2H), 7.25-7.16 (m, 2H), 6.92-6.77 (m, 2H), 3.56 (ddd, J = 11.7, 5.4, 2.1 Hz, 1H), 3.40 (t, J = 11.7 Hz, 1H), 3.03 (m, 1H), 2.59 (ddd, J = 17.7, 5.4, 3.6 Hz, 1H), 2.41 (ddd, J = 17.7, 11.1, 6.3 Hz, 1H), 2.25 (m, 1H), 1.75 (m, 1H), 1.58 (m, 1H), 1.29 (d, J = 6.9 Hz, 3H).

Example 167

1-(2,6-dichlorophenyl)-5-(1-(2,4-difluorophenyl)1-hydroxyiminomethyl)piperidin-2-one

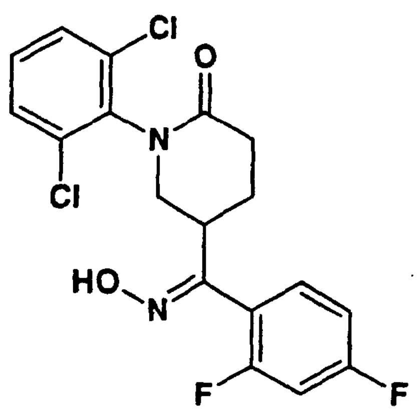

To a solution of the compound prepared in Example 163 (107 mg) in pyridine (3ml) was added hydroxylamine hydrochloride (58 mg). The reaction mixture was stirre at 60°C for 8 hours. The reaction mixture was diluted with ethyl acetate, washed with 2N hydrochloric acid, water, a saturated aqueous sodium hydrogen carbonate solution, water and brine, dried over anhydrous magnesium sulfate and concentrated. The obtained residue was washed with isopropyl ether to give the compound of the present invention (96mg) having the following physical data.
TLC:Rf 0.27 (ethyl acetate:hexane=1:1);
NMR ($CDCl_3$):d 8.12 and 7.51 (s and s, 1H), 7.42-7.33 (m, 2H), 7.29-7.17 (m, 2H), 7.01-6.83 (m, 2H), 4.00-3.16 (m, 3H), 2.81-2.50 (m, 2H), 2.23-1.91 (m, 2H).

Example 168

1-(2,6-dichlorophenyl)-5-(1-(2,4-difluorophenyl)-1-methoxymethyl)piperidin-2-one

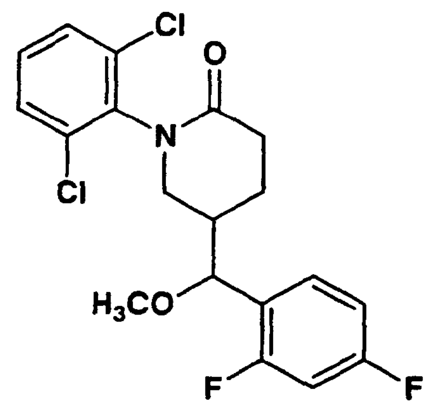

By the same procedure as described in Example 13 using the compound prepared in Example 24, the compound of the present invention having the following physical data was obtained.
TLC:Rf 0.31 (ethyl acetate:hexane=2:3);
NMR ($CDCl_3$):d 7.40-7.32 (m, 3H), 7.19 (t, J = 8.4 Hz, 1H), 7.94 (m, 1H), 6.81 (m, 1H), 4.43 (d, J = 6.9 Hz, 1H), 3.45 (t, J = 11.4 Hz, 1H), 3.23 (s, 3H), 3.10 (ddd, J = 11.4, 5.1, 1.8 Hz, 1H), 2.70 (ddd, J = 17.7, 5.4, 3.0 Hz, 1H), 2.57-2.36 (m, 2H), 2.20 (m, 1H), 1.82 (m, 1H).

Example 169

1-(2,6-dichlorophenyl)-5-(1-(2,4-difluorophenyl)-1-chloromethyl)piperidin-2-one

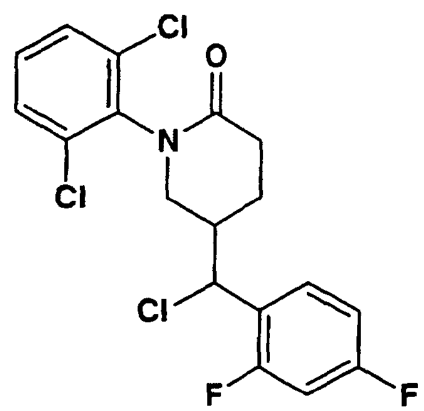

To a solution of the compound prepared in Example 24 (12 mg) in toluene (1 ml) was added thionyl chloride (10 µl). The reaction mixture was stirred at 100°C for 30 minutes. The reaction mixture was concentrated to give the compound of the present invention (12 mg) having the following physical data.
TLC:Rf 0.72 (hexane:ethyl acetate=2:3);
NMR ($CDCl_3$):d 1.71 (m, 1 H) 1.92 (m, 1 H) 2.62 (m, 3 H) 3.16 and 3.62 (m and dd J = 11.81, 9.43 Hz, 1H) 3.34 and 3.38 (t and m, J=11.17 Hz, 1 H) 5.15 (d and d, J=9.34 and J = 8.42 Hz, 1 H) 6.90 (m, 2 H) 7.37 (m, 4 H).

Example 170

(5E)-1-(2,6-dichlorophenyl)-5-(2,4-difluorobenzylidene)piperidin-2-one

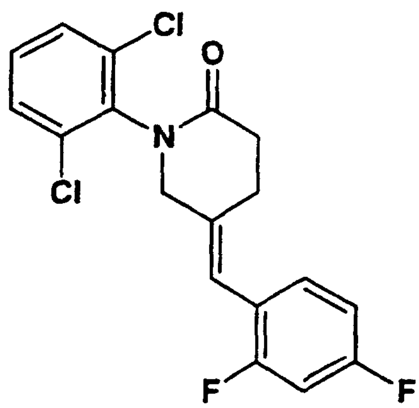

To a solution of the compound prepared in Example 169 (12 mg) in ethanol (2 ml) was added potassium hydroxide (110 mg). The reactioin mixture was stirred at 80°C for 3 hours. To the reaction mixture was 1N hydrochloric acid to neutralize. The reaction mixture was concentrated, diluted with ethyl acetate, washed with water and brine, dried over anhydrous magnesium sulfate and concentrated. The reaction mixture was purified by preparative TLC (toluene:ethyl acetate=4:1, twice) to give the compound of the present invention (1.9 mg) having the following physical data.
TLC:Rf 0.46 (ethyl acetate:toluene=3:7);
NMR ($CDCl_3$):d 2.66 (m, 2 H) 2.78 (m, 2 H) 4.30 (d, J=1.46 Hz, 2 H) 6.44 (s, 1 H) 6.88 (m, 2 H) 7.25 (m, 2 H) 7.42 (m, 2 H).

Example 171

(5E)-1-(4-bromo-2,6-dichlorophenyl)-5-(2,4-difluorobenzylidene)piperidin-2-one

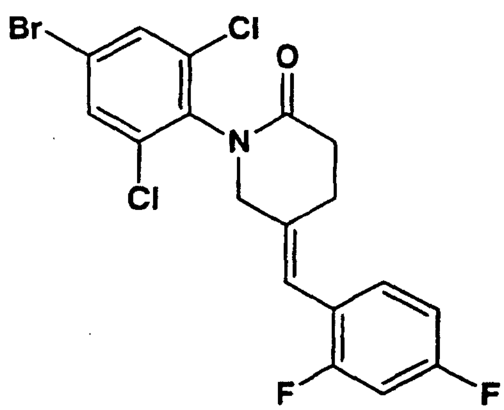

By the same procedure as described in Example 169→Example 170 using the compound prepared in Example 25(4), the compound of the present invention having the following physical data was obtained.
TLC:Rf 0.40 (ethyl acetate:hexane=3:7);
NMR ($CDCl_3$):d 2.64 (m, 2 H) 2.77 (m, 2 H) 4.27 (d, J=1.28 Hz, 2 H) 6.44 (s, 1 H) 6.88 (m, 2 H) 7.24 (m, 1 H) 7.59 (s, 2 H).

Example 172

1-(4-amino-2,6-dichlorophenyl)-5-(1-(2,4-difluorophenyl)1-hydroxyiminomethyl)piperidin-2-one

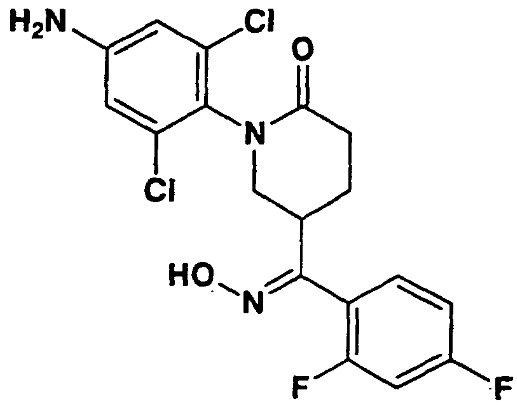

By the same procedure as described in Example 163→Reference Example 31→Example 168 using the compound prepared in Example 25(1), the compound of the present invention having the following physical data was obtained.
TLC:Rf 0.35 (ethyl acetate:hexane=4:1);
NMR ($CDCl_3$):d 2.07 (m, 2 H) 2.65 (m, 2 H) 3.80-3.11 (m, 3 H) 3.91 (s, 2 H) 6.60 (m, 2 H) 6.92 (m, 2 H) 7.23 (m, 1 H) 7.68 and 8.23 (s and s, 1 H).

Reference Example 42

1-(2,6-dichlorophenyl)piperidin-2,5-dione

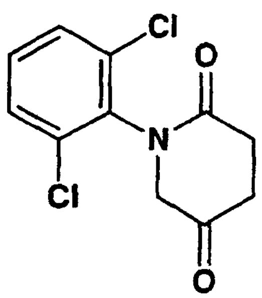

By the same procedure as described in Example 98 using the compound prepared in Reference Example 12, the title compound having the following physical data was obtained.
TLC:Rf 0.40 (hexane:ethyl acetate=1:1);
NMR ($CDCl_3$):d 2.91 (m, 4 H) 4.16 (s, 2 H) 7.28 (m, 1 H) 7.43 (m, 2 H).

Example 173

1-(2,6-dichlorophenyl)-5-(2,4-difluorophenylamino)piperidin-2-one

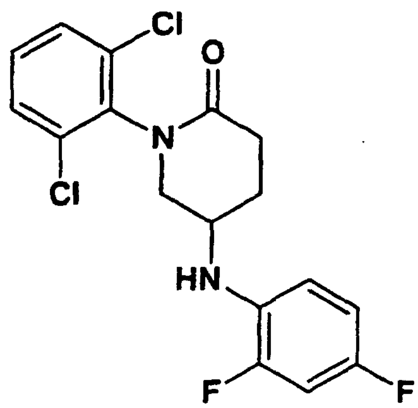

By the same procedure as described in Example 47 using the compound prepared in Reference Example 42 and 2,4-difluoroaniline, the compound of the present invention having the following physical data was obtained.
TLC:Rf 0.33 (ethyl acetate:toluene= 1:4);
NMR ($CDCl_3$):d 2.12 (m, 1 H) 2.26 (m, 1 H) 2.73 (m, 2 H) 3.42 (dd, J=11.95, 6.18 Hz, 1 H) 3.85 (m, 1 H) 4.00 (m, 2 H) 6.76 (m, 3 H) 7.22 (m, 1 H) 7.39 (m, 2 H).

[Formulation example 1]

The following components were admixed in a conventional method, punched out to give 100 tablets each containing 50 mg of active ingredient.
1-(2,6-di chlorophenyl)-5-(2,4-difluorobenzyl)piperidine-2-one (5.0g)
carboxymethylcellulose (disintegrant) (0.2g)
magnesium stearate (lubricant) (0.1g)
microcrystalline cellulose (4.7g)

[Formulation example 2]

The following components were admixed in a conventional method. The solution was sterilized in a conventional method, filled in ampoules 5 ml each and freeze-dried in a conventional method to give 100 ampoules each containing 20 mg of active ingredient.
1-(2,6-di chlorophenyl)-5-(2,4-difluorobenzyl)piperidine-2-one (2.0g)
mannitol (20g)
distilled water (1000ml)

**Claims**

**1.** A piperidin-2-one derivative compound represented by formula (I):

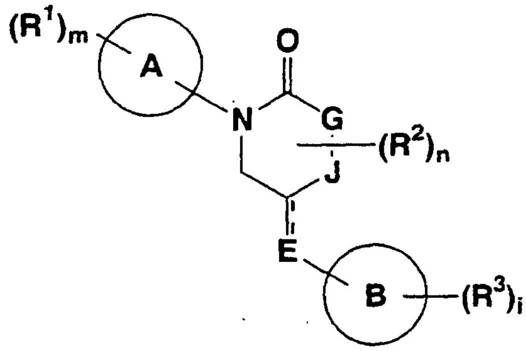

(I)

wherein ring A represents a C5-10 mono- or bi- carbocyclic ring, or a 5-to 10-membered mono- or bi- heterocyclic ring having 1 to 5 nitrogen atom(s), 1 to 2 oxygen atom(s) and/or a sulfur atom;

$R^1$ represents

(1) C1-8 alkyl,
(2) C2-8 alkenyl,
(3) C2-8 alkynyl,
(4) a halogen atom,
(5) $-OR^4$,
(6) $-NR^5R^6$,
(7) $-NR^7COR^8$,
(8) $-CONR^9R^{10}$,
(9) $-COOR^{11}$,
(10) $-SO_2NR^{12}R^{13}$,
(11) $-NR^{14}SO_2R^{15}$,
(12) $-SR^{16}$,
(13) $-S(O)R^{17}$,
(14) $-SO_2R^{18}$,
(15) $-NR^{22}COOR^{23}$,
(16) $-NR^{24}CONR^{25}R^{26}$,
(17) $-COR^{27}$,
(18) $-NO_2$,
(19) -CN,
(20) $-CF_3$,
(21) $-OCF_3$,
(22) Cyc1, or
(23) C1-8 alkyl, C2-8 alkenyl or C2-8 alkynyl substituted with 1 to 5 substituent(s) selected from the group consisting of (a) a halogen atom, (b) $-OR^4$, (c) $-NR^5R^6$, (d) $-NR^7COR^8$, (e) $-CONR^9R^{10}$, (f) $-COOR^{11}$, (g) $-SO_2NR^{12}R^{13}$, (h) $-NR^{14}SO_2R^{15}$, (i) $-SR^{16}$, (j) $-S(O)R^{17}$, (k) $-SO_2R^{18}$, (i) $-NR^{22}COOR^{23}$, (m) $-NR^{24}CONR^{25}R^{26}$, (n) $-COR^{27}$, (o) $-NO_2$, (p) -CN, (q) $-CF_3$, (r) $-OCF_3$, and (s) Cyc1;

$R^4$-$R^{18}$ or $R^{22}$-$R^{27}$ each independently represents

(1) a hydrogen atom,
(2) C1-8 alkyl,
(3) Cyc1, or
(4) C1-8 alkyl substituted with 1 to 5 substituent(s) selected from the group consisting of (a) $-OR^{28}$, (b) $-NR^{29}R^{30}$, (c) $-NR^{31}COR^{32}$, (d) $-CONR^{33}R^{34}$, (e) $-COOR^{35}$, (f) $-SO_2NR^{36}R^{37}$, (g) $-NR^{38}SO_2R^{39}$, (h) $-CONR^{40}NR^{41}R^{42}$, (i) $-CONR^{43}OR^{44}$, and (j) Cycl,

$R^{28}$-$R^{44}$ each independently represents

(1) a hydrogen atom,
(2) C1-8 alkyl,

(3) Cyc1, or
(4) C1-8 alkyl substituted with 1 to 5 substituent(s) selected from the group consisting of $-OR^{45}$, $-NR^{46}R^{47}$, and Cyc1;

$R^{45}$-$R^{47}$ each independently represents a hydrogen atom, C1-8 alkyl, Cyc1, or C1-8 alkyl substituted with Cyc1;

Cyc1 represents a C5-10 mono- or bi- carbocyclic ring or a 5- to 10-membered mono- or bi- heterocyclic ring having 1 to 5 nitrogen atom(s), 1 to 2 oxygen atom(s) and/or a sulfur atom, in which the carbocyclic ring or the heterocyclic ring may be substituted with 1 to 5 of $R^{48}$;

$R^{48}$ represents

(1) C1-8 alkyl,
(2) a halogen atom,
(3) $-NO_2$,
(4) -CN,
(5) $-OR^{49}$,
(6) $-NR^{50}R^{51}$,
(7) $-COOR^{52}$,
(8) $-COR^{53}$,
(9) $-CONR^{54}R^{55}$,
(10) $-NR^{56}COR^{57}$,
(11) $-SO_2NR^{58}R^{59}$,
(12) $-NR^{60}SO_2R^{61}$,
(13) $-SR^{62}$,
(14) $-SO_2R^{63}$,
(15) oxo,
(16) $-CF_3$,
(17) $-OCF_3$, or
(18) C1-8 alkyl substituted with 1 to 5 substituent(s) selected from the group consisting of (a) a halogen atom, (b) $-NO_2$, (c) -CN, (d) $-OR^{49}$, (e) $-NR^{50}R^{51}$, (f) $-COOR^{52}$, (g) $-COR^{53}$, (h) $-CONR^{54}R^{55}$, (i) $-NR^{56}COR^{57}$, (j) $-SO_2NR^{58}R^{59}$, (k) $-NR^{60}SO_2R^{61}$, (l) $-SR^{62}$, (m) $-SO_2R^{63}$, (n) oxo, (o) $-CF_3$, and (p) $-OCF_3$;

$R^{49}$-$R^{63}$ each independently represents, a hydrogen atom, C1-8 alkyl, phenyl, or C1-8 alkyl substituted with phenyl;

$R^2$ represents

(1) C1-8 alkyl,
(2) $OR^{20}$,
(3) $-NR^{64}R^{65}$,
(4) $-COOR^{66}$,
(5) $-CONR^{67}R^{68}$,
(6) $-NR^{69}COR^{70}$,
(7) $-SO_2R^{71}$,
(8) $-SO_2NR^{72}R^{73}$,
(9) $-NR^{74}SO_2R^{75}$,
(10) $-NR^{76}COOR^{77}$,
(11) Cyc2, or
(12) C1-8 alkyl, C2-8 alkenyl or C2-8 alkynyl substituted with 1 to 5 substituent(s) selected from the group consisting of (a) $-OR^{20}$, (b) $-NR^{64}R^{65}$, (c) $-COOR^{66}$, (d) $-CONR^{67}R^{68}$, (e) $-NR^{69}COR^{70}$, (f) $-SO_2R^{71}$, (g) $-SO_2NR^{72}R^{73}$, (h) $-NR^{74}SO_2R^{75}$, (i) $-NR^{76}COOR^{77}$, and (j) Cyc2;

$R^{20}$ and $R^{64}$-$R^{77}$ each independently represents (1) a hydrogen atom, (2) C1-8 alkyl, (3) Cyc2 or (4) C1-8 alkyl substituted with Cyc2;

Cyc2 represents a C5-6 monocarbocyclic ring, or a 5- to 6-membered monoheterocyclic ring having 1 to 4 nitrogen atom(s), 1 to 2 oxygen atom(s) and/or a sulfur atom, in which the carbocyclic ring or the heterocyclic ring may be substituted with 1 to 5 substituent(s) selected from the group consisting of C1-8 alkyl, C1-8 alkoxy, a halogen atom, $-CF_3$ and $-OCF_3$;

G or J each independently represents, a carbon atom, a nitrogen atom, an oxygen atom, or a sulfur atom;

E represents C1-4 alkylene, C2-4 alkenylene, -O-, -S-, $-NR^{21}-$,

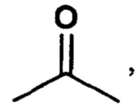

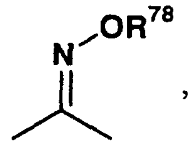

$-NR^{79}SO_2-$ or $-SO_2NR^{80}-$, in which the C1-4 alkylene may be substituted with 1 to 5 of C1-8 alkyl, C1-8 alkoxy, a halogen atom, or hydroxyl;

$R^{21}$ and $R^{78}$-$R^{80}$ each independently represents (1) a hydrogen atom, (2) C1-8 alkyl, (3) Cyc3, (4) C1-8 alkyl substituted with 1 to 5 of Cyc3 or hydroxyl;

Cyc3 represents a C5-6 monocarbocyclic ring, or a 5- to 6-membered monoheterocyclic ring having 1 to 4 nitrogen atom(s), 1 to 2 oxygen atom(s) and/or a sulfur atom, in which the carbocyclic ring or the heterocyclic ring may be substituted with 1 to 5 of C1-8 alkyl, C1-8 alkoxy, a halogen atom, $-CF_3$ or $-OCF_3$;

ring B represents a C5-10 mono- or bi- carbocyclic ring, or a 5- to 10-membered mono- or bi- heterocyclic ring having 1 to 4 nitrogen atom(s), 1 to 2 oxygen atom(s) and/or a sulfur atom;

$R^3$ represents

(1) C1-8 alkyl,
(2) C2-8 alkenyl,
(3) C2-8 alkynyl,
(4) a halogen atom,
(5) $-OR^{81}$,
(6) $-NR^{82}R^{83}$,
(7) $-NR^{84}COR^{85}$,
(8) $-CONR^{86}R^{87}$,
(9) $-COOR^{88}$,
(10) $-SO_2NR^{89}R^{90}$,
(11) $-NR^{91}SO_2R^{92}$,
(12) $-SR^{93}$,
(13) $-S(O)R^{94}$,
(14) $-SO_2R^{95}$,
(15) $-NR^{96}COOR^{97}$,
(16) $-NR^{98}CONR^{99}R^{100}$,
(17) $-OCONR^{101}R^{102}$,
(18) $-NO_2$,
(19) -CN,
(20) $-CF_3$,
(21) $-OCF_3$,
(22) Cyc4, or
(23) C1-8 alkyl, C2-8 alkenyl or C2-8 alkynyl substituted with 1 to 5 substituent(s) selected from the group consisting of (a) a halogen atom, (b) $-OR^{81}$, (c) $-NR^{82}R^{83}$, (d) $-NR^{84}COR^{85}$, (e) $-CONR^{86}R^{87}$, (f) $-COOR^{88}$, (g) $-SO_2NR^{89}R^{90}$, (h) $-NR^{91}SO_2R^{92}$, (i) $-SR^{93}$, (j) $-S(O)R^{94}$, (k) $-SO_2R^{95}$, (l) $-NR^{96}COOR^{97}$, (m) $-NR^{98}CONR^{99}R^{100}$, (n) $-OCONR^{101}R^{102}$, (o) $-NO_2$, (p) -CN, (q) $-CF_3$, (r) $-OCF_3$, and (s) Cyc4;

$R^{81}$-$R^{102}$ each independently represents (1) a hydrogen atom, (2) C1-8 alkyl, (3) Cyc4 or (4) C1-8 alkyl substituted with 1 to 5 substituent(s) selected from the group consisting of Cyc4, $-OR^{103}$, $-CONR^{104}R^{105}$, and $-COOR^{106}$;

$R^{103}$-$R^{116}$ each independently represents, (1) a hydrogen atom, (2) C1-8 alkyl, (3) Cyc4 or (4) C1-8 alkyl substituted with 1 to 5 substituent(s) selected from Cyc4 and $-OR^{107}$;

$R^{107}$ represents (1) a hydrogen atom, (2) C1-8 alkyl, (3) Cyc4 or (4) C1-8 alkyl substituted with Cyc4;

Cyc4 is a C5-10 mono- or bi- carbocyclic ring, or a 5- to 10-membered mono- or bi- heterocyclic ring having 1 to 5 nitrogen atom(s), 1 to 2 oxygen atom(s) and/or a sulfur atom, in which the carbocyclic ring or the heterocyclic ring may be substituted with 1 to 5 of C1-8 alkyl, C1-8 alkoxy, a halogen atom, $-CF_3$ or $-OCF_3$;

m represents 0 or an integer of 1 to 5;

n represents 0 or an integer of 1 to 7;

i represents 0 or an integer of 1 to 12,

wherein

(1) when m is 2 or more, $R^1$ is the same or different,
(2) when n is 2 or more, $R^2$ is the same or different,
(3) when i is 2 or more, $R^3$ is the same or different,
(4) when E is C1-4 alkylene, ---- is a single bond or a double bond,
(5) when E is other than C1-4 alkylene, ---- is a single bond,

or a non-toxic salt thereof.

**2.** The compound according to claim 1, wherein G is a carbon atom, and J is a carbon atom, or a non-toxic salt thereof.

**3.** The compound according to claim 1, wherein G is a nitrogen atom, an oxygen atom or a sulfur atom, and J is a carbon atom, or a non-toxic salt thereof.

**4.** The compound according to claim 1, wherein G is a carbon atom, and J is a nitrogen atom, an oxygen atom or a sulfur atom, or a non-toxic salt thereof.

**5.** The compound according to claim 1, wherein the ring A is a C5-10 mono- or bi- carbocyclic ring, or a non-toxic salt thereof.

**6.** The compound according to claim 1, wherein the ring A is a 5- to 10-membered mono- or bi- heterocyclic ring having 1 to 5 nitrogen atom(s), 1 to 2 oxygen atom(s) and/or a sulfur atom, or a non-toxic salt thereof.

**7.** The compound according to claim 1, wherein the ring B is a C5-10 mono- or bi- carbocyclic ring, or a non-toxic salt thereof.

**8.** The compound according to claim 1, wherein the ring B is a 5- to 10-membered mono- or bi- heterocyclic ring having 1 to 5 nitrogen atom(s), 1 to 2 oxygen atom(s) and/or a sulfur atom, or a non-toxic salt thereof.

**9.** The compound according to claim 1, which is

(1) 1-(2,6-dichlorophenyl)-5-benzylpiperidin-2-one,
(2) 1-(2,6-dichlorophenyl)-5-(2,4-difluorobenzyl)piperidin-2-one,
(3) 1-(2,6-dichlorophenyl)-5-(2-naphtylmethyl)piperidin-2-one,
(4) 1-(2,6-dichlorophenyl)-5-(2,4-dimethylbenzyl)piperidin-2-one,
(5) 1-(2,6-dichlorophenyl)-5-(4-trifluoromethoxybenzyl)piperidin-2-one,
(6) 1-(2,6-dichlorophenyl)-5-(4-trifluoromethylbenzyl)piperidin-2-one,
(7) 1-(2,6-dichlorophenyl)-5-(3,5-difluorobenzyl)piperidin-2-one,
(8) 1-(2,6-dichlorophenyl)-5-(2-chlorobenzyl)piperidin-2-one,
(9) 1-(2,6-dichlorophenyl)-5-(4-fluorobenzyl)piperidin-2-one,
(10) 1-(2,6-dichlorophenyl)-5-(2-fluorobenzyl)piperidin-2-one,
(11) 1-(2,6-dichlorophenyl)-5-(1-naphtylmethyl)piperidin-2-one,
(12) 1-(2,6-dichlorophenyl)-5-(2-methoxybenzyl)piperidin-2-one,
(13) 1-(2,6-dichlorophenyl)-5-(4-ethylbenzyl)piperidin-2-one,
(14) 1-(2,6-dichlorophenyl)-5-benzylpiperidin-2-one,
(15) 1-(2-chlorophenyl)-5-benzylpiperidin-2-one,
(16) 1-(2,6-difluorophenyl)-5-benzylpiperidin-2-one,
(17) 1-(2,6-dimethylphenyl)-5-(2,4-difluorobenzyl)piperidin-2-one,
(18) 1-[2,6-dichloro-4-(1,1,1-triphenylmethoxymethyl)phenyl]-5-(2,4-difluorobenzyl)piperidin-2-one,
(19) 1-(2,6-dichloro-4-hydroxymethylphenyl)-5-(2,4-difluorobenzyl)piperidin-2-one,

(20) 1-(2,6-dichlorophenyl)-5-(2,4-difluorophenoxy)piperidin-2-one,
(21) 1-(2,6-dichlorophenyl)-5-(2,4-difluorothiophenoxy)piperidin-2-one,
(22) 1-(2,6-dichloro-4-nitrophenyl)-5-benzylpiperidin-2-one,
(23) 1-(2,6-dichloro-4-nitrophenyl)-5-(2,4-difluorobenzyl)piperidin-2-one,
(24) 1-(2,6-dichloro-4-aminophenyl)-5-benzylpiperidin-2-one,
(25) 1-(2,6-dichloro-4-aminophenyl)-5-(2,4-difluorobenzyl)piperidin-2-one,
(26) 1-[2,6-dichloro-4-(N-(4-methylimidazol-5-ylmethyl)amino)phenyl]-5-benzylpiperidin-2-one,
(27) 1-[2,6-dichloro-4-(N-(4-methylamino)phenyl)-5-(2,4-difluorobenzyl)piperidin-2-one,
(28) 1-[2,6-dichloro-4-(2-phenylacetylamino)phenyl]-5-(2,4-difluorobenzyl)piperidin-2-one,
(29) 1-(2,6-dichloro-4-acetylaminophenyl)-5-(2,4-difluorobenzyl)piperidin-2-one,
(30) 1-[2,6-dichloro-4-(N-methyl-2-phenylacetylamino)phenyl]-5-(2,4-difluorobenzyl)piperidin-2-one,
(31) (3RS,5RS)-1-(2,6-dichlorophenyl)-5-(2,4-difluorobenzyl)-3-methylpiperidin-2-one,
(32) (3RS,5SR)-1-(2,6-dichtorophenyl)-5-(2,4-difluorobenzyl)-3-methylpiperidin-2-one,
(33) (3RS,5RS)-1-(2,6-dichlorophenyl)-5-(2,4-difluorobenzyl)-3-ethylpiperidin-2-one,
(34) (3RS,5SR)-1-(2,6-dichlorophenyl)-5-(2,4-difluorobenzyl)-3-ethylpiperidin-2-one,
(35) 1-(2,6-dichlorophenyl)-5-(2,4-difluorobenzyl)-3-methoxymethylpiperidin-2-one,
(36) 41-(2,6-dichlorophenyl)-5,5-bis(2,4-difluorobenzyl)piperidin-2-one,
(37) 1-(4-bromo-2,6-dimethylphenyl)-5-(2,4-difluorobenzyl)piperidin-2-one,
(38) 1-(2-methylnaphthalen-1-yl)-5-(2,4-difluorobenzyl)piperidin-2-one,
(39) 1-(2,6-dimethylphenyl)-5-(2-methoxyethoxymethoxybenzyl)piperidin-2-one,
(40) 1-(2,6-dichlorophenyl)-5-(4-nitrobenzyl)piperidin-2-one,
(41) 1-(2,6-dichlorophenyl)-5-(2-cyanobenzyl)piperidin-2-one,
(42) 1-(2,6-dimethylphenyl)-5-(2-bromobenzyl)piperidin-2-one,
(43) 1-(thiazol-2-yl)-5-(2,4-difluorobenzyl)piperidin-2-one,
(44) 1-(2,6-dichlorophenyl)-5-(2-methoxymethoxymethylbenzyl)piperidin-2-one,
(45) 1-(4-t-butoxycarbonylamino-2-chlorophenyl)-5-(2,4-difluorobenzyl)piperidin-2-one,
(46) 1-(4-t-butoxycarbonylamino-2,6-dichlorophenyl)-5-(2,4-difluorobenzyl)piperidin-2-one,
(47) 1-(3-t-butoxycarbonylamino-6-chlorophenyl)-5-(2,4-difluorobenzyl)piperidin-2-one,
(48) 1-(4-t-butoxycarbonylamino-2,6-dichlorophenyl)-5-(2-fluorobenzyl)piperidin-2-one,
(49) 1-(4-bromo-2,6-dichlorophenyl)-5-(2,4-difluorobenzyl)piperidin-2-one,
(50) 1-(2,6-dichlorophenyl)-5-(2,4-difluorobenzyl)-5-hydroxymethylpiperidin-2-one,
(51) 1-(2,6-dimethylphenyl)-5-(3-phenylprop-1-enyl)-5-hydroxymethylpiperidin-2-one,
(52) 1-(2,6-dichlorophenyl)-5-(2,4-difluorobenzyl)tetrahydropyrimidin-2(1H)-one,
(53) 1-(4-bromo-2,6-dichlorophenyl)-5-(2,4-difluorobenzyl)tetrahydropyrimidin-2(1H)-one,
(54) 3-(2,6-dichlorophenyl)-5-(2,4-difluorobenzyl)-1,3-tetrahydroxazin-2-one,
(55) 1-(2,6-dichlorophenyl)-5-(2,4-difluorobenzyl)-4-acetylpiperazin-2-one,
(56) 4-(2,6-dichlorophenyl)-6-(2,4-difluorobenzyl)morpholin-3-one,
(57) 1-(2,6-dichlorophenyl)-5-((2,4-difluorophenyl)hydroxymethyl)piperidin-2-one,
(58) 1-(4-t-butoxycarbonylamino-2,6-dichlorophenyl)-5-((2,4-difluorophenyl)hydroxymethyl)piperidin-2-one,
(59) 1-(4-bromo-2,6-dichlorophenyl)-5-((2-thienyl)hydroxymethyl)piperidin-2-one,
(60) 1-(4-bromo-2,6-dichlorophenyl)-5-(2,4-difluorophenyl)hydroxymethyl)piperidin-2-one,
(61) 1-(2,6-dichlorophenyl)-5-(2-phenylethyl)piperidin-2-one,
(62) 1-cyclohexyl-5-(2,4-difluorobenzyl)pipendin-2-one,
(63) 1-(2-(2-hydroxyethyl)phenyl)-5-(2,4-difluorobenzyl)piperidin-2-one,
(64) 1-(3,5-dichlorophenyl)-5-(2,4-difluorobenzyl)piperidin-2-one,
(65) (5R)-1-(2,6-dichlorophenyl)-5-(2,4-difluorobenzyl)piperidin-2-one,
(66) (5S)-1-(2,6-dichlorophenyl)-5-(2,4-difluorobenzyl)piperidin-2-one,
(67) (3R,5R)-1-(2,6-dichlorophenyl)-5-(2,4-difluorobenzyl)-3-methylpiperidin-2-one,
(68) (3R,5R)-1-(4-nitro-2,6-dimethylphenyl)-5-(2,4-difluorobenzyl)-3-methylpiperidin-2-one,
(69) (3R,5R)-1-(4-bromo-2,6-dimethylphenyl)-5-(2,4-difluorobenzyl)-3-methylpiperidin-2-one,
(70) (3S,5R)-1-(2,6-dichlorophenyl)-5-(2,4-difluorobenzyl)-3-(benzyloxycarbonylamino)piperidin-2-one,
(71) (3S,5R)-1-(2,6-dichlorophenyl)-5-(2,4-dichlorobenzyl-3-aminopiperidin-2-one,
(72) (5S)-1-(2,6-dimethylphenyl)-5-(2,4-difluorophenylsulfonylamino)piperidin-2-one,
(73) 1-(4-(3-ethoxycarbonylpropylcarbonylamino)-2,6-dichlorophenyl)-5-(2,4-difluorobenzyl)piperidin-2-one,
(74) 1-(4-(3-carboxypropylcarbonylamino)-2,6-dichlorophenyl)-5-(2,4-difluorobenzyl)piperidin-2-one,
(75) 1-(4-(4-hydroxybutylcarbonylamino)-2,6-dichlorophenyl)-5-(2,4-difluorobenzyl)piperidin-2-one,
(76) 1-(4-(2-(ethoxycarbonyl)ethylcarbonylamino)-2,6-dichlorophenyl)-5-(2,4-difluorobenzyl)piperidin-2-one,
(77) 1-(4-(2-carboxyethylcarbonylamino)-2,6-dichlorophenyl)-5-(2,4-difluorobenzyl)piperidin-2-one,

(78) 1-( 4-(3-hydroxypropylcarbonylamino)- 2,6-dichlorophenyl)-5-(2,4-difluorobenzyl)piperidin-2-one,
(79) 1-(4-methanesulfonylamino-2,6-dichlorophenyl)-5-(2,4-difluorobenzyl)piperidin-2-one,
(80) 1-(2,6-dichloro-4-(pyrrolidin-2,5-dion-1-yl)phenyl)-5-(2,4-difluorobenzyl)piperidin-2-one,
(81) 1-(2,6-dichloro-4-(piperidin-2,6-dion-1-yl)phenyl)-5-(2,4-difluorobenzyl)piperidin-2-one,
(82) 1-(4-t-butylcarbonylamino-2,6-dichlorophenyl)-5-(2,4-difluorobenzyl)piperidin-2-one,
(83) 1-(4-methoxycarbonylamino-2,6-dichlorophenyl)-5-(2,4-difluorobenzyl)piperidin-2-one,
(84) 1-(4-(N,N-diethylamino)methylcarbonylamino-2,6-dichlorophenyl)-5-(2,4-difluorobenzyl)piperidin-2-one,
(85) 1-((4-(1-methylpiperidin-4-yl)amino)-2,6-dichlorophenyl)-5-(2,6-difluorobenzyl)piperidin-2-one,
(86) 1-(4-((1-acetylpiperidin-4-yl)amino)-2,6-dichlorophenyl)-5-(2,4-difluorobenzyl)piperidin-2-one,
(87) 1-(4-(2-hydroxybenzyl)amino-2,6-dichlorophenyl)-5-(2,4-difluorobenzyl)piperidin-2-one,
(88) 1-(4-((2-methylimidazol-4-yl)methylamino)-2,6-dichlorophenyl)-5-(2,4-difluorobenzyl)piperidin-2-one,
(89) 1-(4-((1-t-butoxycarbonylpiperidin-4-yl)amino)-2,6-dichlorophenyl)-5-(2,4-difluorobenzyl)piperidin-2-one,
(90) 1-(4-(piperidin-4-ylamino)-2,6-dichlorophenyl)-5-(2,4-difluorobenzyl)piperidin-2-one,
(91) 1-(4-(1-methanesulfonylpiperidin-4-ylamino)-2,6-dichlorophenyl)-5-(2,4-difluorobenzyl)piperidin-2-one,
(92) 1-(4-ethylaminocarbonylamino-2,6-dichlorophenyl)-5-(2,4-difluorobenzyl)piperidin-2-one,
(93) 1-(4-(N,N-dimethylamino)-2,6-dichlorophenyl)-5-(2,4-difluorobenzyl)piperidin-2-one,
(94) 1-(4-((2S)-2-(t-butoxycarbonylamino)-3-benzyloxypropanoylamino)-2,6-dichlorophenyl)-5-(2,4-difluorobenzyl)piperidin-2-one,
(95)1-(4-((2S)-2-(t-butoxycarbonylamino)-3-hydroxypropanoylamino)-2,6-dichlorophenyl)-5-(2,4-difluorobenzyl)piperidin-2-one,
(96) 1-(4-(L-serylamino)-2,6-dichlorophenyl)-5-(2,4-difluorobenzyl)piperidin-2-one,
(97) 1-(4-(L-tyrosylamino)-2,6-dichlorophenyl)-5-(2,4-difluorobenzyl)piperidin-2-one,
(98) 1-(4-(L-asparaginylamino)-2,6-dichlorophenyl)-5-(2,4-difluorobenzyl)piperidin-2-one,
(99) 1-(4-(N-(pyridin-2-ylmethyl)-N-(t-butoxycarbonyl)amino)-2,6-dichlorophenyl)-5-(2,6-difluorobenzyl)piperidin-2-one,
(100) 1-(4-(pyridin-2-ylmethylamino)-2,6-dichlorophenyl)-5-(2,6-difluorobenzyl)piperidin-2-one,
(101) 1-(4-(N-ethoxycarbonylmethyl-N-t-butoxycarbonylamino)-2,6-dichlorophenyl)-5-(2,4-difluorobenzyl) piperidin-2-one,
(102) 1-(4-ethoxycarbonylmethylamino-2,6-dichlorophenyl)-5-(2,4-difluorobenzyl)piperidin-2-one,
(103) 1-(4-carboxymethylamino-2,6-dichlorophenyl)-5-(2,4-difluorobenzyl)piperidin-2-one,
(104) 1-(4-(N-carboxymethyl-N-t-butoxycarbonylamino)-2,6-dichlorophenyl)-5-(2,4-difluorobenzyl)piperidin-2-one,
(105) 1-(4-(N-(2-hydroxyethyl)-N-t-butoxycarbonylamino)-2,6-dichlorophenyl)-5-(2,4-difluorobenzyl)piperidin-2-one,
(106) 1-(4-(2-hydroxyethyl)amino-2,6-dichlorophenyl)-5-(2,4-difluorobenzyl)piperidin-2-one,
(107) 1-(4-(N-aminocarbonylmethyl-N-t-butoxycarbonylamino)-2,6-dichlorophenyl)-5-(2,4-difluorobenzyl)piperidin-2-one,
(108) 1-(4-aminocarbonylmethylamino-2,6-dichlorophenyl)-5-(2,4-difluorobenzyl)piperidin-2-one,
(109) 1-(4-(N,N-dimethylamino)carbonylmethylamino)-2,6-dichlorophenyl)-5-(2,4-difluorobenzyl)piperidin-2-one,
(110) 1-(4-methylaminocarbonylmethylamino-2,6-dichlorophenyl)-5-(2,4-difluorobenzyl)piperidin-2-one,
(111) 1-(4-(4-methylpiperadin-1-ylcarbonylmethylamino)-2,6-dichlorophenyl)-5-(2,4-difluorobenzyl)piperidin-2-one,
(112) 1-(4-(N,N-dimethylamino)aminocarbonylmethylamino-2,6-dichlorophenyl)-5-(2,4-difluorobenzyl)piperidin-2-one,
(113) 1-(4-aminoaminocarbonylmethylamino-2,6-dichlorophenyl)-5-(2,4-difluorobenzyl)piperidin-2-one,
(114) 1-(4-(2-hydroxyethylamino)carbonylmethylamino-2,6-dichlorophenyl)-5-(2,4-difluorobenzyl)piperidin-2-one,
(115) 1-(4-t-butylaminocarbonylmethylamino-2,6-dichlorophenyl)-5-(2,4-difluorobenzyl)piperidin-2-one,
(116) 1-(4-(2-(N,N-dimethylamino)ethyl)aminocarbonylmethylamino-2,6-dichlorophenyl)-5-(2,4-difluorobenzyl)piperidin-2-one,
(117) 1-(4-benzyloxyaminocarbonylmethylamino-2,6-dichlorophenyl)-5-(2,4-difluorobenzyl)piperidin-2-one,
(118) 1-(4-hydroxyaminocarbonylmethylamino-2,6-dichlorophenyl)-5-(2,4-difluorobenzyl)piperidin-2-one,
(119) 1-(4-(3-(N,N-dimethylamino)propyl)amino-2,6-dichlorophenyl)-5-(2,4-difluorobenzyl)piperidin-2-one,
(120) 1-(4-(2-oxo-1,3-oxazolidin-3-yl)-2,6-dichlorophenyl)-5-(2,4-difluorobenzyl)piperidin-2-one,
(121) 1-(4-(N-(2-(N',N'-dimethylamino)ethyl)-N-(t-butoxycarbonyl)amino)-2,6-dichlorophenyl)-5-(2,4-difluorobenzyl)piperidin-2-one,

(122) 1-(4-(2-(N,N-dimethylamino)ethyl)amino-2,6-dichlorophenyl)-5-(2,4-difluorobenzyl)piperidin-2-one,
(123) 1-(4-(2-(morpholin-4-yl)ethylamino)-2,6-dichlorophenyl)-5-(2,4-difluorobenzyl)piperidin-2-one,
(124) 1-(4-(2-(N,N-diethylamino)ethyl)amino-2,6-dichlorophenyl)-5-(2,4-difluorobenzyl)piperidin-2-one,
(125) 1-(4-(2-(piperidin-1-yl)ethyl)amino-2,6-dichtorophenyl)-5-(2,4-difluorobenzyl)piperidin-2-one,
(126) 1-(4-(2-(imidazol-1-yl)ethylamino)-2,4-dichlorophenyl)-5-(2,6-difluorobenzyl)piperidin-2-one,
(127) 1-(4-(2-pyrrolidin-1-yl)ethylamino-2,6-dichlorophenyl)-5-(2,4-difluorobenzyl)piperidin-2-one,
(128) 1-(4-(2-(4-methylpiperadin-1-yl)ethylamino)-2,6-dichlorophenyl)-5-(2,4-difluorobenzyl)piperidin-2-one,
(129) 1-(4-(N-(2-aminoethyl)-N-(t-butoxycarbonyl)amino)-2,6-dichlorophenyl)-5-(2,4-difluorobenzyl)piperidin-2-one,
(130) 1-(4-(2-methanesulfonylaminoethyl)amino-2,6-dichlorophenyl)-5-(2,4-difluorobenzyl)piperidin-2-one,
(131) 1-(4-(N-(2-methylcarbonylaminoethyl)-N-(t-butoxycarbonyl)amino)-2,6-dichlorophenyl)-5-(2,4-difluorobenzyl)piperidin-2-one,
(132) 1-(4-(2-methylcarbonylaminoethyl)amino-2,6-dichlorophenyl)-5-(2,4-difluorobenzyl)piperidin-2-one,
(133) 1-(4-(N-(2-aminocarbonylaminoethyl)-N-(t-butoxycarbonyl)amino)-2,6-dichlorophenyl)-5-(2,4-difluorobenzyl)piperidin-2-one,
(134) 1-(4-(2-aminocarbonylaminoethyl)amino-2,6-dichlorophenyl)-5-(2,4-difluorobenzyl)piperidin-2-one,
(135) 1-(4-(2-(4,4-dimethyl-2,5-dioxoimidazolidin-1-yl)ethylamino)-2,6-dichlorophenyl)-5-(2,4-difluorobenzyl)piperidin-2-one,
(136) 1-(3-amino-6-chlorophenyl)-5-(2,4-difluorobenzyl)piperidin-2-one,
(137) 1-(3-methylcarbonylamino-6-chlorophenyl)-5-(2,4-difluorobenzyl)piperidin-2-one,
(138) 1-(3-methanesulfonylamino-6-chlorophenyl)-5-(2,4-difluorobenzyl)piperidin-2-one,
(139) 1-(3-ethoxycarbonylmethylamino-6-chlorophenyl)-5-(2,4-difluorobenzyl)piperidin-2-one,
(140) 1-(3-carboxylmethylamino-6-chlorophenyl)-5-(2,4-difluorobenzyl)piperidin-2-one,
(141) 1-(3-(2-hydroxyethylamino)-6-chlorophenyl)-5-(2,4-difluorobenzyl)piperidin-2-one,
(142) 1-(4-(2-(N,N-dimethylamino)ethyl)amino-2,6-dichlorophenyl)-5-(2-fluorobenzyl)piperidin-2-one,
(143) 1-(2,6-dichlorophenyl)-5-(4-aminobenzyl)piperidin-2-one,
(144) 1-(2,6-dichlorophenyl)-5-(4-cyclohexylcarbonylaminobenzyl)piperidin-2-one,
(145) 1-(2,6-dichlorophenyl)-5-(4-methylcarbonylaminobenzyl)piperidin-2-one,
(146) 1-(4-amino-2-chlorophenyl)-5-(2,4-difluorobenzyl)piperidin-2-one,
(147) 1-(2,6-dichlorophenyl)-5-(2-hydroxymethylbenzyl)piperidin-2-one,
(148) 1-(2,6-dichlorophenyl)-5-(2-aminomethylbenzyl)piperidin-2-one,
(149) 1-(2,6-dichlorophenyl)-5-(2-(1-methyl-3-t-butylpyrazol-5-yl)aminocarbonylaminomethylbenzyl)piperidin-2-one,
(150) 1-(2,6-dichlorophenyl)-5-(2-benzylaminocarbonylaminomethylbenzyl)piperidin-2-one,
(151) 1-(2,6-dichlorophenyl)-5-(2-phenylaminocarbonylaminomethylbenzyl)piperidin-2-one,
(152) 1-(2,6-dichlorophenyl)-5-(2-cyclohexylaminocarbonylaminomethylbenzyl)piperidin-2-one,
(153) 1-(2,6-dichlorophenyl)-5-(2-(3-trifluoromethylphenyl)aminocarbonylaminomethylbenzyl)piperidin-2-one,
(154) 1-(2,6-dichlorophenyl)-5-(2-ethylaminocarbonylaminomethylbenzyl)piperidin-2-one,
(155) 1-(2,6-dichlorophenyl)-5-(2-(3-methylphenyl)aminocarbonylaminomethylbenzyl)piperidin-2-one,
(156) 1-(2,6-dichlorophenyl)-5-(2-(2-trifluoromethylphenyl)aminocarbonylaminomethylbenzyl)piperidin-2-one,
(157) 1-(2,6-dichlorophenyl)-5-(2-methylcarbonylaminomethylbenzyl)piperidin-2-one,
(158) 1-(2,6-dichlorophenyl)-5-(2-(3,8,8-trimethyl-5,6,7,8-tetrahydronaphthalen-2-yl)aminocarbonylaminomethylbenzyl)piperidin-2-one,
(159) 1-(2,6-dichlorophenyl)-5-(2-(1-methyl-3-t-butylpyrazol-5-yl)aminocarbonyloxymethylbenzyl)piperidin-2-one,
(160) 1-(2,6-dichlorophenyl)-5-(2-formylbenzyl)piperidin-2-one,
(161) 1-(2,6-dichlorophenyl)-5-(2-(2-methoxycarbonylvinyl)benzyl)piperidin-2-one,
(162) 1-(2,6-dichlorophenyl)-5-(2-(2-methoxycarbonylvinyl)benzyl)piperidin-2-one,
(163) 1-(2,6-dichlorophenyl)-5-(2-(1-methyl-3-t-butylpyrazol-5-yl)aminocarbonyl)ethylpiperidin-2-one,
(164) 1-(2,6-dimethylphenyl)-5-(2-hydroxybenzyl)piperidin-2-one,
(165) 1-(2,6-dimethylphenyl)-5-(2-isobutyloxybenzyl)piperidin-2-one,
(166) 1-(2,6-dimethylphenyl)-5-(2-ethoxycarbonylmethoxybenzyl)piperidin-2-one,
(167) 1-(2,6-dimethylphenyl)-5-(2-carboxymethoxybenzyl)piperidin-2-one,
(168) 1-(2,6-dimethylphenyl)-5-(2-(1-methyl-3-t-butylpyrazol-5-yl)aminocarbonylmethyloxyphenylmethyl)piperidin-2-one,
(169) 1-(4-methoxycarbonyl-2,6-dimethylphenyl)-5-(2,4-difluorobenzyl)piperidin-2-one,

(170) 1-(4-carboxyl-2,6-dimethylphenyl)-5-(2,4-difluorobenzyl)piperidin-2-one
(171) 1-(4-(2-(N,N-dimethylamino)ethyl)aminocarbonyl-2,6-dimethylphenyl)-5-(2,4-difluorobenzyl)piperidin-2-one,
(172) 1-(4-(3-(N,N-dimethylamino)propylaminocarbonyl-2,6-dimethylphenyl)-5-(2,4-difluorobenzyl)piperidin-2-one,
(173) 1-(4-methylaminocarbonyl-2,6-dimethylphenyl)-5-(2,4-difluorobenzyl)piperidin-2-one,
(174) 1-(4-(N,N-dimethylamino)carbonyl-2,6-dimethylphenyl)-5-(2,4-difluorobenzyl)piperidin-2-one,
(175) 1-(4-(morpholin-4-yl)carbonyl-2,6-dimethylphenyl)-5-(2,4-difluorobenzyl)piperidin-2-one,
(176) 1-(4-(4-methylpiperazin-1-yl)carbonyl-2,6-dimethylphenyl)-5-(2,4-difluorobenzyl)piperidin-2-one,
(177) 1-(4-(3-ethoxycarbonylpropyl)aminocarbonyl-2,6-dimethylphenyl)-5-(2,4-difluorobenzyl)piperidin-2-one,
(178) 1-(4-(2-ethoxycarbonylethyl)aminocarbonyl-2,6-dimethylphenyl)-5-(2,4-difluorobenzyl)piperidin-2-one,
(179) 1-(4-(3-carboxypropyl)aminocarbonyl]-2,6-dimethylphenyl)-5-(2,4-difluorobenzyl)piperidin-2-one,
(180) 1-(4-(2-carboxyethyl)aminocarbonyl-2,6-dimethylphenyl)-5-(2,4-difluorobenzyl)piperidin-2-one,
(181) 1-(4-(t-butoxycarbonyl)amino-2,6-dimethylphenyl)-5-(2,4-difluorobenzyl )piperidin-2-one,
(182) 1-(4-amino-2,6-dimethylphenyl)-5-(2,4-difluorobenzyl)piperidin-2-one,
(183) 1-(4-aminomethyl-2,6-dimethylphenyl)-5-(2,4-difluorobenzyl)piperidin-2-one,
(184) 1-(4-(4-(tetrahydropyran-2-yloxy)but-1-ynyl)-2,6-dimethylphenyl)-5-(2,4-difluorobenzyl)piperidin-2-one,
(185) 1-(4-(4-hydroxybut-1-ynyl)-2,6-dimethylphenyl)-5-(2,4-difluorobenzyl)piperidin-2-one,
(186) 1-(4-(4-(N,N-dimethylamino)but-1-ynyl)-2,6-dimethylphenyl)-5-(2,4-difluorobenzyl)piperidin-2-one,
(187) 1-(4-(4-morpholinobut-1-ynyl)-2,6-dimethylphenyl)-5-(2,4-difluorobenzyl)piperidin-2-one,
(188) 1-(4-(4-(4-methylpiperazin-1-yl)but-1-ynyl)-2,6-dimethylphenyl)-5-(2,4-difluorobenzyl)piperidin-2-one,
(189) 1-(4-(4-(N,N-dimethylamino)butyl)-2,6-dimethylphenyl)-5-(2,4-difluorobenzyl)piperidin-2-one,
(190) (E)-1-(4-(2-(2-(N,N-dimethylamino)ethyl)vinyl)-2,6-dimethylphenyl)-5-(2,4-difluorobenzyl)piperidin-2-one,
(191) 1-(4-(5-hydroxypent-1-ynyl)-2,6-dimethylphenyl)-5-(2,4-difluorobenzyl)piperidin-2-one,
(192) 1-(4-(5-hydroxypentyl)-2,6-dimethylphenyl)-5-(2,4-difluorobenzyl)piperidin-2-one,
(193) 1-(4-(4-formylbutyl)-2,6-dimethylphenyl)-5-(2,4-difluorobenzyl)piperidin-2-one,
(194) 1-(4-(4-carboxylbutyl)-2,6-dimethytphenyl)-5-(2,4-difluorobenzyl)piperidin-2-one,
(195) (E)-1-(4-(2-(methoxycarbonyl)vinyl)-2,6-dimethylphenyl)-5-(2,4-difluorobenzyl)piperidin-2-one,
(196) (E)-1-(4-(2-(carboxyl)vinyl)-2,6-dimethylphenyl)-5-(2,4-difluorobenzyl)piperidin-2-one,
(197) 1-(4-methoxycarbonyl-2,6-dichlorophenyl)-5-(2,4-difluorobenzyl)piperidin-2-one,
(198) 1-(4-carboxyl-2,6-dichlorophenyl)-5-(2,4-difluorobenzyl)piperidin-2-one,
(199) 1-(4-(2-(N,N-dimethylamino)ethyl)aminocarbonyl-2,6-dichlorophenyl)-5-(2,4-difluorobenzyl)piperidin-2-one,
(200) 1-(4-(N,N-dimethylamino)methyl-2,6-dichlorophenyl)-5-(2,4-difluorobenzyl)piperidin-2-one,
(201) 1-(4-(4-methylpiperazin-1-ylmethyl)-2,6-dichlorophenyl)-5-(2,4-difluorobenzyl)piperidin-2-one,
(202) 1-(4-piperazin-1-yl)-2,6-dichlorophenyl)-5-(2,6-difluorobenzyl)piperidin-2-one,
(203) 1-(4-(4-methylpiperazin-1-yl)-2,6-dichlorophenyl)-5-(2,4-difluorobenzyl)piperidin-2-one,
(204) 1-(4-(3-hydroxy-1-propinyl)-2,6-dichlorophenyl)-5-(2,4-difluorobenzyl)piperidin-2-one,
(205) 1-(4-(3-(N,N-dimethylamino)propyl)-2,6-dichlorophenyl)-5-(2,4-difluorobenzyl)piperidin-2-one,
(206) 1-(4-(3-aminopropyl)-2,6-dichlorophenyl)-5-(2,4-difluorobenzyl)piperidin-2-one,
(207) 1-(4-(4-hydroxybut-1-ynyl)-2,6-dichlorophenyl)-5-(2,4-difluorobenzyl)piperidin-2-one,
(208) 1-(4-(4-(N,N-dimethylamino)butyl)-2,6-dichlorophenyl)-5-(2,4-difluorobenzyl)piperidin-2-one,
(209) (E)-1-(4-(2-(methyloxycarbonyl)vinyl)-2,6-dimethylphenyl)-5-(2,4-difluorobenzyl)piperidin-2-one,
(210) (E)-1-(4-(2-aminocarbonylvinyl)-2,6-dimethylphenyl)-5-(2,4-difluorobenzyl)piperidin-2-one,
(211) (E)-1-(4-(2-cyanovinyl)-2,6-dimethylphenyl)-5-(2,4-difluorobenzyl)piperidin-2-one,
(212) 1-(4-(2-cyanoethyl)-2,6-dichlorophenyl)-5-(2,4-difluorobenzyl)piperidin-2-one,
(213) 1-(4-(2-methoxycarbonylethyl)-2,6-dichlorophenyl)-5-(2,4-difluorobenzyl)piperidin-2-one,
(214) 1-(4-(2-methylaminocarbonyl)ethyl)-2,6-dichlorophenyl)-5-(2,4-difluorobenzyl)piperidin-2-one,
(215) (3R,5R)-1-(4-methoxycarbonyl-2,6-dimethylphenyl)-5-(2,4-difluorobenzyl)-3-methylpiperidin-2-one,
(216) (3R,5R)-1-(4-carboxy-2,6-dimethylphenyl)-5-(2,4-difluorobenzyl)-3-methylpiperidin-2-one,
(217) (3R,5R)-1-(4-(2-(N,N-dimethylamino)ethylaminocarbonyl-2,6-dimethylp henyl)-5-(2,4-difluorobenzyl)-3-methylpiperidin-2-one,
(218) (3R,5R)-1-(4-(3-(N,N-dimethylamino)-1-propynyl)-2,6-dimethylphenyl)-5-(2,4-difluorobenzyl)-3-methylpiperidin-2-one,
(219) (3R,5R)-1-(4-(3-(N,N-dimethylamino)propyl)-2,6-dimethylphenyl)-5-(2,4-difluorobenzyl)-3-methylpiperidin-2-one,

(220) 1-(4-carboxyl-2,6-dimethylphenyl)-5-(2,4-difluorobenzyl)tetrahydropyrimidin-2(1H)-one,
(221) 1-(4-t-butyloxycarbonylamino-2,6-dimethylphenyl)-5-(2,4-difluorobenzyl)tetrahydropyrimidin-2(1H)-one,
(222) 1-(4-amino-2,6-dimethylphenyl)-5-(2,4-difluorobenzyl)tetrahydropyrimidin-2(1H)-one,
(223) 1-(4-(2-(N,N-dimethylamino)ethylaminocarbonyl)-2,6-dimethylphenyl)-5-(2,4-difluorobenzyl)tetrahydropyrimidin-2(1H)-one,
(224) 1-(2,6-dimethylphenyl)-5-(2-methoxycarbonylbenzyl)piperidin-2-one,
(225) 1-(2,6-dimethylphenyl)-5-(2-carboxybenzyl)piperidin-2-one,
(226) 1-(2,6-dimethylphenyl)-5-(2-(2-methoxycarbonylvinyl)benzyl)piperidin-2-one,
(227) (3R,5R)-1-(4-amino-2,6-dichlorophenyl)-5-(2,4-difluorobenzyl)-3-methylpiperidin-2-one,
(228) (3R,5R)-1-(4-(t-butoxycarbonylamino)-2,6-dichlorophenyl)-5-(2,4-difluorobenzyl)-3-methylpiperidin-2-one,
(229) (3R,5R)-1-(4-(2-(N,N-dimethylamino)ethylamino)-2,6-dichlorophenyl)-5-(2,4-difluorobenzyl)-3-methylpiperidin-2-one,
(230) 1-(2,6-dimethylphenyl)-5-(3-phenylpropyl)piperidin-2-one,
(231) (3RS,5RS)-1-(2,6-dichlorophenyl)-5-(2,4-difluorobenzyl)-3-hydroxypiperidin-2-one,
(232) (3RS,5SR)-1-(2,6-dichlorophenyl)-5-(2,4-difluorobenzyl)-3-hydroxypiperidin-2-one,
(233) (3RS,5RS)-1-(2,6-dimethylphenyl)-5-(2,4-difluorobenzyl)-3-methylpiperidin-2-one,
(234) (3RS,5SR)-1-(2,6-dimethylphenyl)-5-(2,4-difluorobenzyl)-3-methylpiperidin-2-one,
(235) (5S)-1-(2,6-dimethylphenyl)-5-(methyl(2,4-difluorophenylsulfonyl)amino)piperidin-2-one,
(236) (5S)-1-(2,6-dimethylphenyl)-5-((2-hydroxyethyl)(2,4-difluorophenylsulfonyl)amino)piperidin-2-one,
(237) 1-(4-(N-methylcarbonyl-N-methylamino)-2,6-dichlorophenyl)-5-(2,4-difluorobenzyl)piperidin-2-one,
(238) 1-(2,6-dichlorophenyl)-5-(2,4-difluorobenzyl)-3-methyltetrahydropyrimidin-2(1H)-one,
(239) 1-(2,6-dichlorophenyl)-5-(2,4-difluorobenzyl)piperazin-2-one,
(240) 1-(2,6-dichlorophenyl)-5-(2,4-difluorobenzyl)-4-methylpiperazin-2-one,
(241) 1-(2,6-dichlorophenyl)-5-(2,4-difluorophenylcarbonyl)piperidin-2-one,
(242) 1-(2,6-dichlorophenyl)-5-(1-(2,4-difluorophenyl)-1-hydroxyethyl)piperidin-2-one,
(243) 1-(2,6-dichlorophenyl)-5-(1-(2,4-difluorophenyl)vinyl)piperidin-2-one,
(244) 1-(2,6-dichlorophenyl)-5-(1-(2,4-difluorophenyl)ethyl)piperidin-2-one,
(245) 1-(2,6-dichlorophenyl)-5-(1-(2,4-difluorophenyl)1-hydroxyiminomethyl)piperidin-2-one,
(246) 1-(2,6-dichlorophenyl)-5-(1-(2,4-difluorophenyl)-1-methoxymethyl)piperidin-2-one,
(247) 1-(2,6-dichlorophenyl)-5-(1-(2,4-difluorophenyl)-1-chloromethyl)piperidin-2-one,
(248) (5E)-1-(2,6-dichlorophenyl)-5-(2,4-difluorobenzylidene)piperidin-2-one,
(249) (5E)-1-(4-bromo-2,6-dichlorophenyl)-5-(2,4-difluorobenzylidene)piperidin-2-one,
(250) 1-(4-amino-2,6-dichlorophenyl)-5-(1-(2,4-difluorophenyl)1-hydroxyiminomethyl)piperidin-2-one,
(251) 1-(2,6-dichlorophenyl)-5-(2,4-difluorophenylamino)piperidin-2-one,

or a non-toxic salt thereof.

**10.** A pharmaceutical composition, which comprises the piperidin-2-one derivative compound represented by formula (I) according to claim 1, or a non-toxic salt thereof, as an active ingredient.

**11.** An agent for inhibiting p38MAP kinase, which comprises the piperidin-2-one derivative compound represented by formula (I) according to claim 1, or a non-toxic salt thereof, as an active ingredient.

**12.** An agent for preventing and/or treating various inflammatory diseases, rheumatoid arthritis, osteoarthritis, arthritis, osteoporosis, autoimmune diseases, infectious diseases, sepsis, cachexia, cerebral infarction, Alzheimer's disease, asthma, chronic pulmonary inflammatory diseases, reperfusion injury, thrombosis, glomerulonephritis, diabetes, graft versus host rejection, inflammatory bowel disease, Crohn's disease, ulcerative colitis, multiple sclerosis, tumor growth and metastasis, multiple myeloma, plasma cell leukemia, Castleman's disease, atrial myxoma, psoriasis, dermatitis, gout, adult respiratory distress syndrome (ARDS), arteriosclerosis, post-percutaneous transluminal coronary angioplasty (PTCA) restenosis or pancreatitis, which comprises the piperidin-2-one derivative compound represented by formula (I) according to claim 1, or a non-toxic salt thereof, as an active ingredient.

FIGURE 1

0.1 0.3 1.0 0 ←concentration (μM)

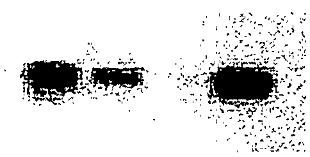

**INTERNATIONAL SEARCH REPORT**

International application No.
PCT/JP02/12174

A. CLASSIFICATION OF SUBJECT MATTER
Int.Cl[7] C07D211/76, 401/12, 211/86, 211/88, 211/96, 417/04, 409/06, 401/10, 413/10, 405/12, 239/10, 241/08, 265/10, 265/32, A61K31/451, 31/454, 31/4545, 31/496, 31/5377,
According to International Patent Classification (IPC) or to both national classification and IPC

B. FIELDS SEARCHED
Minimum documentation searched (classification system followed by classification symbols)
Int.Cl[7] C07D211/76, 401/12, 211/86, 211/88, 211/96, 417/04, 409/06, 401/10, 413/10, 405/12, 239/10, 241/08, 265/10, 265/32, A61K31/451, 31/454, 31/4545, 31/496, 31/5377,

Documentation searched other than minimum documentation to the extent that such documents are included in the fields searched

Electronic data base consulted during the international search (name of data base and, where practicable, search terms used)
REGISTRY(STN), CAPLUS(STN), CAOLD(STN)

C. DOCUMENTS CONSIDERED TO BE RELEVANT

| Category* | Citation of document, with indication, where appropriate, of the relevant passages | Relevant to claim No. |
|---|---|---|
| X | WO 01/54694 A1 (BRISTOL-MYERS SQUIBB CO.), 02 August, 2001 (02.08.01), Claims; example 57 & EP 1253925 A1 & US 2002/91078 A1 | 1,3,5,8, 10-12 |
| X | JP 4-307539 A (Fuji Photo Film Co., Ltd.), 29 October, 1992 (29.10.92), Par. No. [0042] (Family: none) | 1,3,5,8 |
| X | WO 01/76693 A1 (MERCK & CO., INC.), 18 October, 2001 (18.10.01), Example 5 (Family: none) | 1,4,5,8 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>03 February, 2003 (03.02.03) | Date of mailing of the international search report<br>25 February, 2003 (25.02.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/12174

C (Continuation). DOCUMENTS CONSIDERED TO BE RELEVANT

| Category* | Citation of document, with indication, where appropriate, of the relevant passages | Relevant to claim No. |
|---|---|---|
| A | WO 00/12074 A2 (SCIOS INC.), 09 March, 2000 (09.03.00), & JP 2002-523448 A & AU 9957936 A & EP 1107758 A2 & BR 9913654 A & KR 2001082184 A & CN 1323209 A & US 6410540 B1 | 1-12 |
| A | WO 00/71535 A1 (SCIOS INC.), 30 November, 2000 (30.11.00), & JP 2003-500403 A & AU 200054424 A & EP 1178983 A1 & BR 200011274 A & KR 2002019919 A & CN 1351599 A & NO 200105655 A & CZ 200104126 A3 & SK 200101648 A3 & HU 200201261 A2 | 1-12 |
| A | WO 98/28292 A1 (SMITHKLINE BEECHAM CORP.), 02 July, 1998 (02.07.98), & JP 2002-515891 A | 1-12 |
| A | WO 98/06715 A1 (SMITHKLINE BEECHAM CORP.), 19 February, 1998 (19.02.98), & JP 2001-506230 A & EP 922042 A1 | 1-12 |
| E,A | WO 02/102380 A1 (BRISTOL-MYERS SQUIBB PHARMA CO.), 27 December, 2002 (27.12.02), & US 2002/183324 A1 | 1,4,5,7, 10-12 |
| A | OTIS E.FANCHER et al., 4-Alkyl-(or Aralkyl) 1-Aryl-2-piperazinones, J.Med.Chem., 1964, Vol.7, No.2, pages 154 to 158 | 1,4,5,7, 10-12 |

Form PCT/ISA/210 (continuation of second sheet) (July 1998)

## INTERNATIONAL SEARCH REPORT

International application No.
PCT/JP02/12174

Continuation of A. CLASSIFICATION OF SUBJECT MATTER
(International Patent Classification (IPC))

Int.Cl[7] 31/4535, 31/4433, 31/513, 31/495, 31/535, 31/5375, A61P1/04, 1/18, 3/10, 7/00, 7/02, 9/00, 9/10, 11/00, 11/06, 13/12, 17/00, 17/06, 19/00, 19/02, 19/06, 19/10, 25/00, A61P25/28, 29/00, 31/00, 31/04, 35/00, 35/02, 37/02, 37/06, 43/00

(According to International Patent Classification (IPC) or to both national classification and IPC)

Continuation of B. FIELDS SEARCHED
Minimum Documentation Searched(International Patent Classification (IPC))

Int.Cl[7] 31/4535, 31/4433, 31/513, 31/495, 31/535, 31/5375, A61P1/04, 1/18, 3/10, 7/00, 7/02, 9/00, 9/10, 11/00, 11/06, 13/12, 17/00, 17/06, 19/00, 19/02, 19/06, 19/10, 25/00, A61P25/28, 29/00, 31/00, 31/04, 35/00, 35/02, 37/02, 37/06, 43/00

Minimum documentation searched (classification system followed by classification symbols)

<Subject of the search>
Claim 1 involves a large number of compounds in its scope. However, only part of the claimed compounds are supported by the description in the meaning as defined in PCT Article 6 and disclosed therein in the meaning as defined in PCT Article 5.
Thus, the international search was made on the parts supported by the description and disclosed therein, namely, compounds wherein:
(i) G and J are each carbon;
(ii) G is nitrogen, oxygen or sulfur and J is $CH_2$; or
(iii) G is $CH_2$ and J is nitrogen, oxygen or sulfur.

<Inappropriate description in claim>
Compounds (36) and (50) as set forth in claim 9 do not correspond to the compound of claim 1.